# EUROPEAN PATENT APPLICATION

(11) **EP 4 733 299 A1**
(43) Date of publication of application: **29.04.2026**
(21) Application number: 24810425.9
(22) Date of filing: 22.05.2024
(51) Int. Cl.: C07D 277/64, C07D 277/62, A61K 51/00, A61K 31/428, A61P 25/28

(54) **SULFUR-CONTAINING FUSED RING COMPOUND, PHARMACEUTICAL COMPOSITION CONTAINING SAME, AND PREPARATION METHOD THEREFOR AND USE THEREOF**

(30) Priority: 22.05.2023 CN 202310581604
(71) Applicant: Shenzhen Braegen Pharmaceutical Co., Ltd., Shenzhen, Guangdong 518107 (CN)
(72) Inventor: YE, Keqiang, Shenzhen, Guangdong 518000 (CN)
(74) Representative: Michalski Hüttermann & Partner Patentanwälte mbB
(86) International application number: PCT/CN2024/094698
(87) International publication number: WO 2024/240186

(57) **Abstract**

Disclosed are a sulfur-containing fused ring compound, a pharmaceutical composition containing same, and a preparation method therefor and the use thereof. Specifically disclosed is a sulfur-containing fused ring compound as shown in formula A, I-A, II-A, III-A, IV-A, V-A, VI-A or VII, or a pharmaceutically acceptable salt, a tautomer or a radioisotope labeled derivative thereof. The sulfur-containing fused ring compound of the present disclosure can be used as a PET-CT tracer and is used for developing and imaging α-synuclein aggregates in Lewy bodies and Lewy neurites, so as to diagnose diseases of the central nervous system.

## Description

This application claims the benefit of Chinese Patent Application No. CN2023105816049, filed May 22, 2023. The entirety of the aforementioned Chinese patent application is hereby incorporated by reference for all purposes.

### TECHNICAL FIELD

The present disclosure relates to a sulfur-containing fused ring compound; a pharmaceutical composition containing same; a preparation method therefor and the use thereof.

### BACKGROUND

Parkinson's disease (PD) may be characterized by the progressive development of Lewy bodies (LB) and Lewy neurites (LN). These LB and LN consist mostly of aggregations of the protein α-synuclein (Spillantini et al. 1997 Nature; 388: 839-40), which is found in healthy nerve cells as an unfolded membrane-bound proteins. α-Synuclein detaches from the membrane and takes on a β-sheet conformation which permits aggregation and consequent formation of LB and LN.

Kotzbauer et al. report PET tracers for imaging α-synuclein aggregates in LB and LN (Clinical and Transformation Imaging, 2016, 5, 3-14). 6-[¹⁸F]-Fluoro-L-Dopa is used as a PET tracer to evaluate the function of dopaminergic neurons. The SPECT tracer [¹²³I]-2-[β]-carbomethoxy-3-[β]-(4-iodophenyl)-tropane is used to evaluate the function of the vesicular monoamine transporter. WO2004/075882 discloses an *in vivo* imaging method for diagnosing the presence of abnormally folded or aggregated protein and/or amyloid fibril or amyloid in a subject, where the method comprises administration of a radiolabeled inositol derivative. WO2004/075882 discloses that *in vivo* imaging methods can be applied for the diagnosis of PD. See also WO2010/063701, WO2004/100998 and WO2005/013889.

The above-described *in vivo* imaging techniques target the disease process at a stage when LB and LN are present in the central nervous system (CNS). At this stage, clinical symptoms are evident, and about 80% of striatal dopamine neurons and 50% of nigral neurons are lost. As the neurons of the CNS cannot regenerate on their own after cell death, neuroprotective treatment will only benefit neurons if still alive at the time of diagnosis. It would be advantageous for patients to get treatment to curb disease progression as early as possible. There is therefore a need for a method of identifing PD before significant loss of neurons.

CN2019800317559 discloses a class of styryl benzothiazole derivatives that can be used as *in vivo* imaging agents for the diagnosis of PD or other degenerative disorders or CNS disorders. Among them, compound F0502B 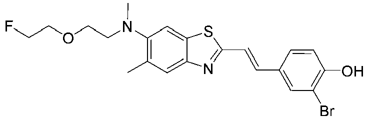 with specific binding to α-synuclein was specifically disclosed.

### SUMMARY

The present disclosure aims to provide a sulfur-containing fused ring compound; a pharmaceutical composition containing same; a preparation method therefor; and the use thereof. The sulfur-containing fused ring compound of the present disclosure can be used as a PET-CT tracer and is used for developing and imaging α-synuclein aggregates in LB and LN, so as to diagnose CNS disorders.

The present disclosure solves the above-mentioned technical problems through the following technical solutions:

The present disclosure aims to provide a sulfur-containing fused ring compound of Formula A, the pharmaceutically acceptable salt thereof, the tautomer thereof, or the radioisotope-labeled derivative thereof;
wherein the sulfur-containing fused ring compound of Formula A is as follows: 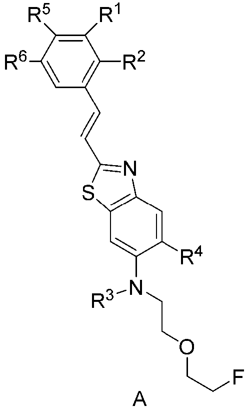
in Formula A,
R¹ is F, Cl, Br, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 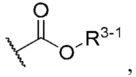 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻³, and R³⁻⁹ are independently halogen, -OH, -COOH, -CN,-NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 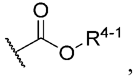 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶ , R⁴⁻⁷, R⁴⁻⁸ , and R⁴⁻⁹ are independently halogen, -OH, -COOH, -CN,-NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
when R⁵ is -OH, then R¹ is F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the compound satisfies one or more of the following conditions:
   Condition (1): R¹ is F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
   Condition (2): R² is -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
   Condition (3): R³ is H, -OH, C₂₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 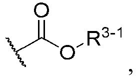 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹.

In one embodiment, in the sulfur-containing fused ring compound represented by Formula A, the pharmaceutically acceptable salt thereof, the tautomer thereof, or the radioisotope-labeled derivative thereof, the definitions of certain groups may be as described below, and the definitions of other groups may be as described in any one of the embodiments of the present disclosure (hereinafter referred to as "in one embodiment"): the sulfur-containing fused ring compound represented by Formula A is represented by Formula A-1:

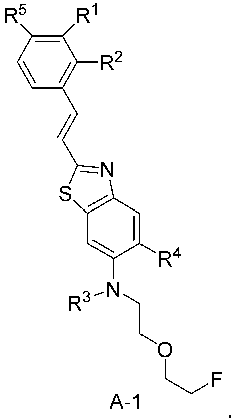

In one embodiment, in Formula A, R¹ is Br, F, Cl, I, -OR¹⁻¹ or -NR¹⁻²R¹⁻³; preferably -OR¹⁻¹or -NR¹⁻²R¹⁻³; more preferably -OH, -OCH₃, -NH₂, or -NHCH₃.

In one embodiment, in Formula A, R² is H, -OH, -OC₁₋₆ alkyl, or -NR²⁻⁰R²⁻¹; preferably -OH, -OC₁₋₆ alkyl, or -NR²⁻⁰R²⁻¹; more preferably -OH, -OCH₃, -NH₂, or -NHCH₃.

In one embodiment, in Formula A, R³ is H, -OH, or C₁₋₆ alkyl; preferably H, -OH, or C₂₋₆ alkyl; more preferably H, -OH, or isopropyl, or, more preferably -OH, ethyl, or isopropyl.

In one embodiment, in Formula A, R⁴ is C₁₋₆ alkyl.

In one embodiment, in Formula A, R¹⁻¹ is H or C₁₋₆ alkyl.

In one embodiment, in Formula A, R¹⁻² and R¹⁻³ are independently H or C₁₋₆ alkyl.

In one embodiment, in Formula A, R²⁻¹ is H.

In one embodiment, in Formula A, R²⁻⁰ is H or C₁₋₆ alkyl.

In one embodiment, in Formula A, R⁵ is -OH, -NH₂, or -NHCH₃; preferably, R⁵ is -OH.

The present disclosure further provides a sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof; wherein,
the sulfur-containing fused ring compound of Formula I-A is as follows: 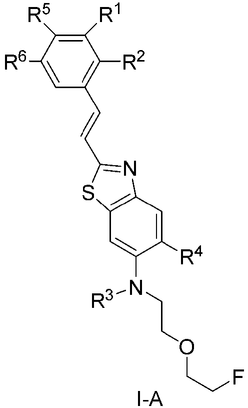
in Formula I-A,
R¹ is F, Cl, Br, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 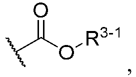 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻³, and R³⁻⁹ are independently halogen, -OH, -COOH, -CN,-NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 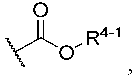 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶ , R⁴⁻⁷, R⁴⁻⁸ , and R⁴⁻⁹ are independently halogen, -OH, -COOH, -CN, - NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻².
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
when R⁵ is -OH, then R¹ is F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶; the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula II-A is as follows: 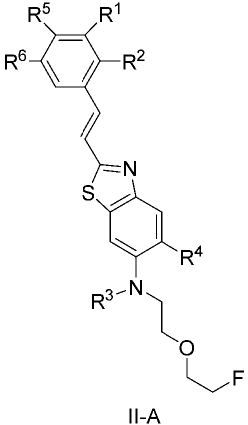
in Formula II-A,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 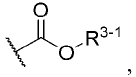 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻³, and R³⁻⁹ are independently halogen, -OH, -COOH, -CN,-NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 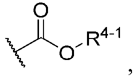 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, - NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula V-A is as follows: 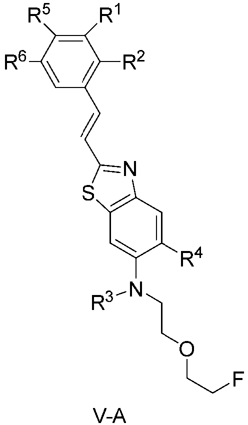
in Formula V-A,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₂₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 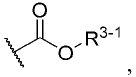 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻³, and R³⁻⁹ are independently halogen, -OH, -COOH, -CN,-NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 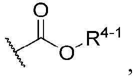 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻³, and R⁴⁻⁹ are independently halogen, -OH, -COOH, -CN,-NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula III-A is as follows: 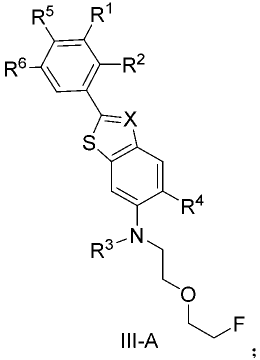
in Formula III-A,
X is N or CR^{a};
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 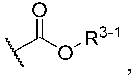 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻³, and R³⁻⁹ are independently halogen, -OH, -COOH, -CN,-NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 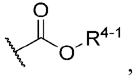 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶ , R⁴⁻⁷, R⁴⁻⁸, and R⁴⁻⁹ are independently halogen, -OH, -COOH, -CN,-NO₂, or -NH₂;
R⁴⁻¹⁻¹ is independently halogen, -OH, -COOH, -CN, -NO₂, or -NH₂;
R^{a} is H or C₁₋₆ alkyl;
or, R^{a} and R² together form -(NH)ₙ-(CH₂)ₘ-(CH=CH)ₚ-; n, m, and p are independently 0, 1, 2, or 3, and n, m, and p are not simultaneously 0;
R⁵ is -OH or -NR⁵⁻¹R⁵⁻².
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula IV-A is as follows: 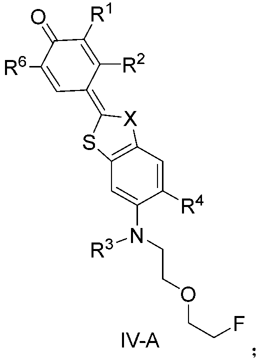
in Formula IV-A,
X is N or CR^{a};
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 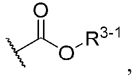 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, - NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 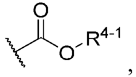 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, - NO₂, or -NH₂;
R⁴⁻¹⁻¹ is independently halogen, -OH, -COOH, -CN, -NO₂, or -NH₂;
R^{a} is H or C₁₋₆ alkyl;
or, R^{a} and R² together form -(NH)ₙ-(CH₂)ₘ-(CH=CH)ₚ-; n, m, and p are independently 0, 1, 2, or 3, and n, m, and p are not simultaneously 0;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula VI-A is as follows: 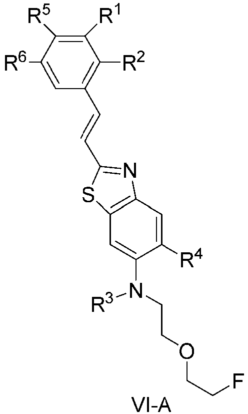
in Formula VI-A,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 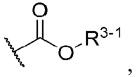 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, - NO₂, or -NH₂;
R³⁻¹⁻¹ is independently halogen, -OH, -COOH, -CN, -NO₂, or -NH₂;
R⁴ is H, C₂₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 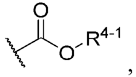 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻³, and R⁴⁻⁹ are independently halogen, -OH, -COOH, -CN, - NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula VII is as follows: 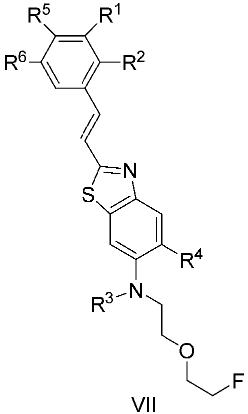
R¹ is F, Cl, Br, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 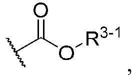 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, - NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 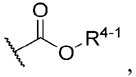 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, - NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 3- to 6-membered heterocycloalkyl has 1, 2, or 3 heteroatoms selected from the group consisting of N, O, and S.

The present disclosure aims to provide a sulfur-containing fused ring compound of Formula I, II, III, IV, V or VI, the pharmaceutically acceptable salt thereof, the tautomer thereof, or the radioisotope-labeled derivative thereof;
wherein, the sulfur-containing fused ring compound of Formula I is as follows: 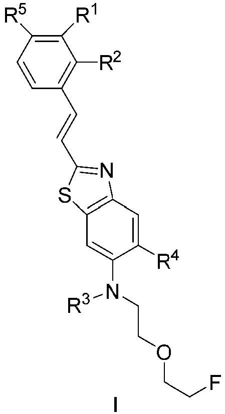
in Formula I,
R¹ is F, Cl, Br, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 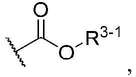 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, - NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 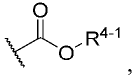 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, - NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
when R⁵ is -OH, then R' is F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula II is as follows: 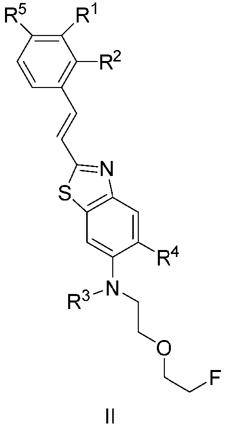
in Formula II,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 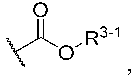 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, - NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 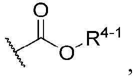 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻³, and R⁴⁻⁹ are independently halogen, -OH, -COOH, -CN, - NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula III is as follows: 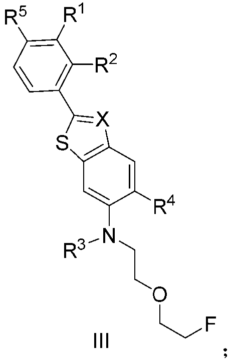
in Formula III,
X is N or CR^{a};
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 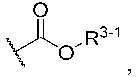 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, - NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 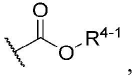 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, - NO₂, or -NH₂;
R⁴⁻¹⁻¹ is independently halogen, -OH, -COOH, -CN, -NO₂, or -NH₂;
R^{a} is H or C₁₋₆ alkyl;
or, R^{a} and R² together form -(NH)ₙ-(CH₂)ₘ-(CH=CH)ₚ-; n, m, and p are independently 0, 1, 2, or 3, and n, m, and p are not simultaneously 0;
R⁵ is -OH or -NR⁵⁻¹R⁵⁻²;
R⁵⁻¹ and R⁵⁻² are independently H or C₁₋₆ alkyl;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula IV is as follows: 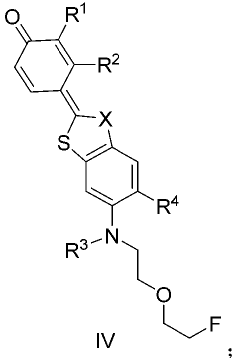
in Formula IV,
X is N or CR^{a};
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁_₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 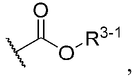 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻³, and R³⁻⁹ are independently halogen, -OH, -COOH, -CN, - NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 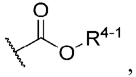 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻³, and R⁴⁻⁹ are independently halogen, -OH, -COOH, -CN, - NO₂, or -NH₂;
R⁴⁻¹⁻¹ is independently halogen, -OH, -COOH, -CN, -NO₂, or -NH₂;
R^{a} is H or C₁₋₆ alkyl;
or, R^{a} and R² together form -(NH)ₙ-(CH₂)ₘ-(CH=CH)ₚ-; n, m, and p are independently 0, 1, 2, or 3, and n, m, and p are not simultaneously 0;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula V is as follows: 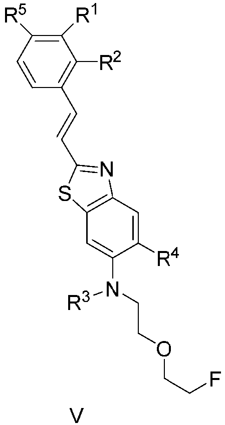
in Formula V,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₂₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 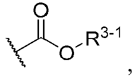 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻³, and R³⁻⁹ are independently halogen, -OH, -COOH, -CN, - NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 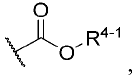 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, - NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻².
R⁵⁻¹ and R⁵⁻² are independently H or C₁₋₆ alkyl;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula VI is as follows: 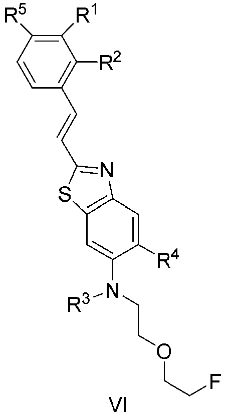
in Formula VI,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 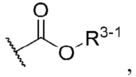 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴ , R³⁻⁵, R³⁻⁶, R³⁻⁷, R³⁻⁸, and R³⁻⁹ are independently halogen, -OH, -COOH, -CN, - NO₂, or -NH₂;
R³⁻¹⁻¹ is independently halogen, -OH, -COOH, -CN, -NO₂, or -NH₂;
R⁴ is H, C₂₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 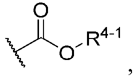 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶ , R⁴⁻⁷, R⁴⁻⁸, and R⁴⁻⁹ are independently halogen, -OH, -COOH, -CN, - NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S.

In one embodiment, in the sulfur-containing fused ring compound of Formula I-A, II-A, III-A, IV-A, V-A, VI-A, VII, I, II, III, IV, V or VI (e.g., I, II, III, IV, V, or VI), the pharmaceutically acceptable salt thereof, the tautomer thereof, or the radioisotope-labeled derivative thereof, the definitions of certain groups may be as described below, and the definitions of other groups may be as described in any one of the embodiments of the present disclosure (hereinafter referred to as "in one embodiment"): in Formula I, R¹ is Br, -OR¹⁻¹ or -NR¹⁻²R¹⁻³; preferably -OR¹⁻¹or -NR¹⁻²R¹⁻³; more preferably -OH, -OCH₃, -NH₂ or -NHCH₃.

In one embodiment, in Formula I-A or Formula I, R² is H, -OH, -OC₁₋₆ alkyl, or -NR²⁻⁰R²⁻¹.

In one embodiment, in Formula I-A or Formula I, R³ is H, -OH, or C₁₋₆ alkyl.

In one embodiment, in Formula I-A or Formula I, R⁴ is C₁₋₆ alkyl.

In one embodiment, in Formula I-A or Formula I, R¹⁻¹ is H or C₁₋₆ alkyl.

In one embodiment, in Formula I-A or Formula I, R¹⁻² and R¹⁻³ are independently H or C₁₋₆ alkyl.

In one embodiment, in Formula I-A or Formula I, R²⁻¹ is H.

In one embodiment, in Formula I-A or Formula I, R²⁻⁰ is H or C₁₋₆ alkyl.

In one embodiment, in Formula I-A or Formula I, R⁵ is -OH, -NH₂, or -NHCH₃; preferably - OH.

In one embodiment, in Formula I-A or Formula I, R¹ is Br, -OH, -OCH₃, -NH₂ or -NHCH₃;
R² is H, -OH, -OCH₃, -NH₂ or -NHCH₃;
R³ is H, -OH, or C₁₋₆ alkyl;
R⁴ is C₁₋₆ alkyl;
R⁵ is -OH, -NH₂, or -NHCH₃.

In one embodiment, in Formula I-A or Formula I, R¹ is Br, -OH, -OCH₃, -NH₂ or -NHCH₃;
R² is H;
R³ is C₁₋₆ alkyl;
R⁴ is C₁₋₆ alkyl;
R⁵ is -OH, -NH₂, or -NHCH₃.

In one embodiment, in Formula II-A or Formula II, R¹ is Br, F, Cl, I, -OR¹⁻¹, or -NR¹⁻²R¹⁻³.

In one embodiment, in Formula II-A or Formula II, R² is -OH, -OC₁₋₆ alkyl, or -NR²⁻⁰R²⁻¹; preferably -OH, -OCH₃, -NH₂, or -NHCH₃.

In one embodiment, in Formula II-A or Formula II, R³ is H, -OH, or C₁₋₆ alkyl.

In one embodiment, in Formula II-A or Formula II, R⁴ is C₁₋₆ alkyl.

In one embodiment, in Formula II-A or Formula II, R¹⁻¹ is H or C₁₋₆ alky.

In one embodiment, in Formula II-A or Formula II, R¹⁻² and R¹⁻³ are independently H or C₁₋₆ alkyl.

In one embodiment, in Formula II-A or Formula II, R²⁻¹ is H.

In one embodiment, in Formula II-A or Formula II, R²⁻⁰ is H or C₁₋₆ alkyl.

In one embodiment, in Formula II-A or Formula II, R⁵ is -OH, -NH₂, or -NHCH₃; preferably -OH.

In one embodiment, in Formula II-A or Formula II, R¹ is Br, F, Cl, I, -OH, -OCH₃, -NH₂ or - NHCH₃;
R² is -OH, -OCH₃, -NH₂, or -NHCH₃;
R³ is H, -OH, or C₁₋₆ alkyl;
R⁴ is C₁₋₆ alkyl;
R⁵ is -OH, -NH₂, or -NHCH₃.
In one embodiment, in Formula II-A or Formula II, R¹ is Br;
R² is -OH, -OCH₃, -NH₂, or -NHCH₃;
R³ is C₁₋₆ alkyl;
R⁴ is C₁₋₆ alkyl;
R⁵ is -OH.

In one embodiment, in Formula III-A or Formula III, R¹ is Br, F, Cl, I, -OR¹⁻¹, or -NR¹⁻²R¹⁻³.

In one embodiment, in Formula III-A or Formula III, R² is H, -OH, -OC₁₋₆ alkyl, or -NR²⁻⁰R²⁻¹; or, R^{a} and R² together form -(NH)ₙ-(CH₂)ₘ-(CH=CH)ₚ-; n, m, and p are independently 0, 1, 2, or 3, and n, m, and p are not simultaneously 0; preferably, R² is H, or R^{a} and R² together form -(NH)- or - (CH=CH)-.

In one embodiment, in Formula III-A or Formula III, R³ is H, -OH, or C₁₋₆ alkyl.

In one embodiment, in Formula III-A or Formula III, R⁴ is C₁₋₆ alkyl.

In one embodiment, in Formula III-A or Formula III, R¹⁻¹ is H or C₁₋₆ alkyl.

In one embodiment, in Formula III-A or Formula III, R¹⁻² and R¹⁻³ are independently H or C₁₋₆ alkyl.

In one embodiment, in Formula III-A or Formula III, R²⁻¹ is H.

In one embodiment, in Formula III-A or Formula III, R²⁻⁰ is H or C₁₋₆ alkyl.

In one embodiment, in Formula III-A or Formula III, R⁵ is -OH, -NH₂, or -NHCH₃; preferably -OH.

In one embodiment, in Formula III-A or Formula III, X is N or CR^{a};
R¹ is Br, F, Cl, I, -OH, -OCH₃, -NH₂ or -NHCH₃;
R² is H; or, R^{a} and R² together form -NH- or -CH=CH-;
R³ is H, -OH, or C₁₋₆ alkyl;
R⁴ is C₁₋₆ alkyl;
R⁵ is -OH, -NH₂, or -NHCH₃.

In one embodiment, in Formula III-A or Formula III, X is N or CR^{a};
R¹ is Br;
R² is H; or, R^{a} and R² together form -NH- or -CH=CH-;
R³ is C₁₋₆ alkyl;
R⁴ is C₁₋₆ alkyl;
R⁵ is -OH.

In one embodiment, in Formula IV-A or Formula IV, R¹ is Br, F, Cl, I, -OR¹⁻¹, or -NR¹⁻² R¹⁻³

In one embodiment, in Formula IV, R² is H, -OH, -OC₁₋₆ alkyl, or -NR²⁻⁰R²⁻¹; or, R^{a} and R² together form -(NH)ₙ-(CH₂)ₘ-(CH=CH)ₚ-; n, m, and p are independently 0, 1, 2, or 3, and n, m, and p are not simultaneously 0; preferably, R² is H, or R^{a} and R² together form -(NH)- or -(CH=CH)-.

In one embodiment, in Formula IV-A or Formula IV, R³ is H, -OH, or C₁₋₆ alkyl.

In one embodiment, in Formula IV-A or Formula IV, R⁴ is C₁₋₆ alkyl.

In one embodiment, in Formula IV-A or Formula IV, R¹⁻¹ is H or C₁₋₆ alkyl.

In one embodiment, in Formula IV-A or Formula IV, R¹⁻² and R¹⁻³ are independently H or C₁₋₆ alkyl.

In one embodiment, in Formula IV-A or Formula IV, R²⁻¹ is H.

In one embodiment, in Formula IV-A or Formula IV, R²⁻⁰ is C₁₋₆ alkyl.

In one embodiment, in Formula IV-A or Formula IV, in Formula IV, X is N or CR^{a};
R¹ is Br, F, Cl, I, -OH, -OCH₃, -NH₂ or -NHCH₃;
R² is H; or, R^{a} and R² together form -NH- or -(CH=CH)-;
R³ is H, -OH or C₁₋₆ alkyl;
R⁴ is C₁₋₆ alkyl.

In one embodiment, in Formula IV-A or Formula IV, X is N or CR^{a};
R¹ is Br;
R² is H; or, R^{a} and R² together form -NH- or -(CH=CH)-;
R³ is C₁₋₆ alkyl;
R⁴ is C₁₋₆ alkyl.

In one embodiment, in Formula V-A or Formula V, R¹ is Br, F, Cl, I, -OR¹⁻¹, or -NR¹⁻²R¹⁻³.

In one embodiment, in Formula V-A or Formula V, R² is H, -OH, -OC₁₋₆ alkyl, or -NR²⁻⁰R²⁻¹.

In one embodiment, in Formula V-A or Formula V, R³ is H, -OH, or C₂₋₆ alkyl; preferably H, -OH, or isopropyl; or preferably -OH, ethyl, or isopropyl; most preferably ethyl.

In one embodiment, in Formula V-A or Formula V, R⁴ is C₁₋₆ alkyl.

In one embodiment, in Formula V-A or Formula V, R¹⁻¹ is H or C₁₋₆ alkyl.

In one embodiment, in Formula V-A or Formula V, R¹⁻² and R¹⁻³ are independently H or C₁₋₆ alkyl.

In one embodiment, in Formula V-A or Formula V, R²⁻¹ is H.

In one embodiment, in Formula V-A or Formula V, R²⁻⁰ is H or C₁₋₆ alkyl.

In one embodiment, in Formula V-A or Formula V, R⁵ is -OH, -NH₂, or -NHCH₃; preferably -OH.

In one embodiment, in Formula V-A or Formula V, R¹ is Br, F, Cl, I, -OH, -OCH₃, -NH₂ or - NHCH₃;
R² is H, -OH, -OCH₃, -NH₂ or -NHCH₃;
R³ is H, -OH, or isopropyl; or R³ is -OH, ethyl, or isopropyl;
R⁴ is C₁₋₆ alkyl;
R⁵ is -OH, -NH₂, or -NHCH₃.

In one embodiment, in Formula V-A or Formula V, R¹ is Br;
R² is H;
R³ is H, -OH, or isopropyl; or R³ is -OH, ethyl, or isopropyl;
R⁴ is C₁₋₆ alkyl;
R⁵ is -OH.

In one embodiment, in Formula VI-A or Formula VI, R¹ is Br, F, Cl, I, -OR¹⁻¹, or -NR¹⁻²R¹⁻³.

In one embodiment, in Formula VI-A or Formula VI, R² is H, -OH, -OC₁₋₆ alkyl, or -NR²⁻⁰R²⁻¹.

In one embodiment, in Formula VI-A or Formula VI, R³ is H, -OH, or C₁₋₆ alkyl.

In one embodiment, in Formula VI-A or Formula VI, R⁴ is C₂₋₆ alkyl; preferably ethyl or isopropyl.

In one embodiment, in Formula VI-A or Formula VI, R¹⁻¹ is H or C₁₋₆ alkyl.

In one embodiment, in Formula VI-A or Formula VI, R¹⁻² and R¹⁻³ are independently H or C₁₋₆ alkyl.

In one embodiment, in Formula VI-A or Formula VI, R²⁻¹ is H.

In one embodiment, in Formula VI-A or Formula VI, R²⁻⁰ is H or C₁₋₆ alkyl.

In one embodiment, in Formula VI-A or Formula VI, R⁵ is -OH, -NH₂, or -NHCH₃; preferably -OH.

In one embodiment, in Formula VI-A or Formula VI, R¹ is Br, F, Cl, I, -OH, -OCH₃, -NH₂ or -NHCH₃;
R² is H, -OH, -OCH₃, -NH₂ or -NHCH₃;
R³ is H, -OH, or C₁₋₆ alkyl;
R⁴ is C₂₋₆ alkyl;
R⁵ is -OH, -NH₂, or -NHCH₃.

In one embodiment, in Formula VI-A or Formula VI, R¹ is Br;
R² is H;
R³ is H, -OH, or C₁₋₆ alkyl;
R⁴ is ethyl or isopropyl;
R⁵ is -OH.

In one embodiment, in Formula VII, R¹ is Br, F, Cl, I, -OR¹⁻¹, or -NR¹⁻²R¹⁻³.

In one embodiment, in Formula VII, R² is H, -OH, -OC₁₋₆ alkyl, or -NR²⁻⁰R²⁻¹

In one embodiment, in Formula VII, R³ is H, -OH, or C₁₋₆ alkyl.

In one embodiment, in Formula VII, R⁴ is C₂₋₆ alkyl.

In one embodiment, in Formula VII, R¹⁻¹ is H or C₁₋₆ alkyl.

In one embodiment, in Formula VII, R¹⁻² and R¹⁻³ are independently H or C₁₋₆ alkyl.

In one embodiment, in Formula VII, R²⁻¹ is H.

In one embodiment, in Formula VII, R²⁻⁰ is H or C₁₋₆ alkyl.

In one embodiment, in Formula VII, R⁵ is -OH, -NH₂, or -NHCH₃; preferably -OH.

In one embodiment, in Formula I-A, II-A, III-A, IV-A, V-A, VI-A, or VII, R⁶ is H or -OC₁₋₆ alkyl; preferably H or methoxy.

In one embodiment, in Formula VII, R¹ is Br, F, Cl, I, -OH, -OCH₃, -NH₂ or -NHCH₃;
R² is H, -OH, -OCH₃, -NH₂ or -NHCH₃;
R³ is H, -OH, or C₁₋₆ alkyl;
R⁴ is C₁₋₆ alkyl;
R⁵ is -OH, -NH₂, or -NHCH₃;
R⁶ is methoxy.

In one embodiment, in Formula VII, R¹ is Br, -OH, -OCH₃, -NH₂ or -NHCH₃;
R² is H;
R³ is C₁₋₆ alkyl;
R⁴ is C₁₋₆ alkyl;
R⁵ is -OH, -NH₂, or -NHCH₃;
R⁶ is methoxy.

In one embodiment, in Formula A, I-A, II-A, III-A, IV-A, V-A, VI-A, VII, I, II, III, IV, V, or VI, the C₁₋₆ alkyl is independently methyl, ethyl, *n*-propyl, isopropyl, *n*-butyl, isobutyl, or *tert-*butyl*;* for example, methyl, ethyl, or isopropyl; and for another example, methyl.

In one embodiment, in Formula A, I-A, II-A, III-A, IV-A, V-A, VI-A, VII, I, II, III, IV, V, or VI, the C₂₋₆ alkenyl is independently vinyl, propenyl, *n*-propenyl, isopropenyl, *n*-butenyl, isobutenyl, *sec*-butenyl, or *tert*-butenyl.

In one embodiment, in Formula A, I-A, II-A, III-A, IV-A, V-A, VI-A, VII, I, II, III, IV, V, or VI, the C₂₋₆ alkynyl is independently ethynyl, *n*-propynyl, isopropynyl, *n*-butynyl, isobutynyl, *sec-*butynyl, or *tert*-butynyl.

In one embodiment, in Formula A, I-A, II-A, III-A, IV-A, V-A, VI-A, VII, I, II, III, IV, V, or VI, the C₃₋₆ cycloalkyl is independently cyclopropyl, cyclobutyl, cyclopropyl, cyclopentyl, or cyclohexyl.

In one embodiment, in Formula A, I-A, II-A, III-A, IV-A, V-A, VI-A, VII, I, II, III, IV, V, or VI, the -O-C₁₋₆ alkyl is independently methoxy, ethoxy, *n*-propoxy, isopropoxy, *n*-butoxy, isobutoxy, or *tert*-butoxy; for example, methoxy.

In one embodiment, in Formula A, I-A, II-A, III-A, IV-A, V-A, VI-A, VII, I, II, III, IV, V, or VI, the halogen is independently F, Cl, Br, or I.

In one embodiment, in Formula A, I-A, II-A, III-A, IV-A, V-A, VI-A, VII, I, II, III, IV, V, or VI, the C₆₋₁₀ aryl is independently phenyl or naphthyl, for example, phenyl.

In one embodiment, in Formula A, V-A, VI-A, V, or VI, the C₂₋₆ alkyl is independently ethyl, *n*-propyl, isopropyl, *n*-butyl, isobutyl, or tert-butyl; for example, ethyl or isopropyl.

In one embodiment, the sulfur-containing fused ring compound of Formula I-A is any one selected from the group consisting of the following compounds:

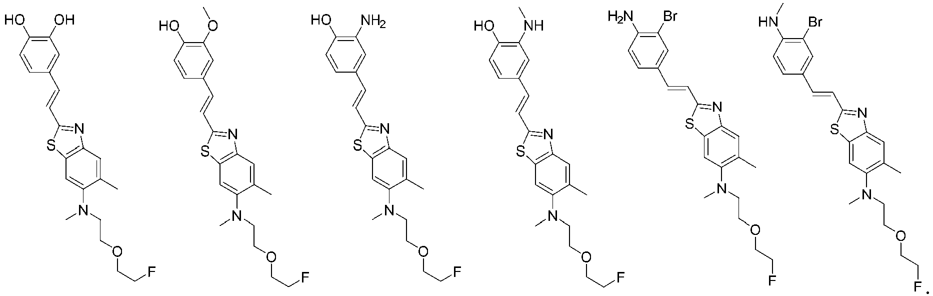

In one embodiment, the sulfur-containing fused ring compound of Formula II-A is any one selected from the group consisting of the following compounds:

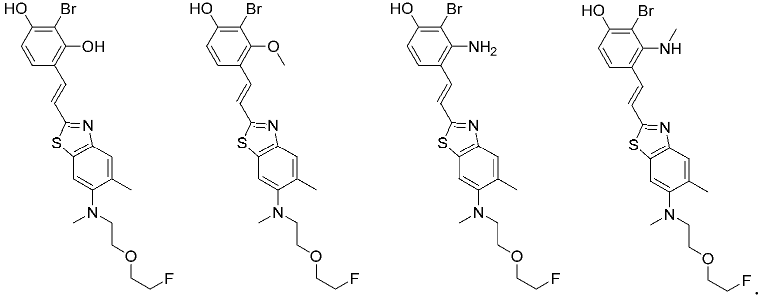

In one embodiment, the sulfur-containing fused ring compound of Formula III-A is any one selected from the group consisting of the following compounds:

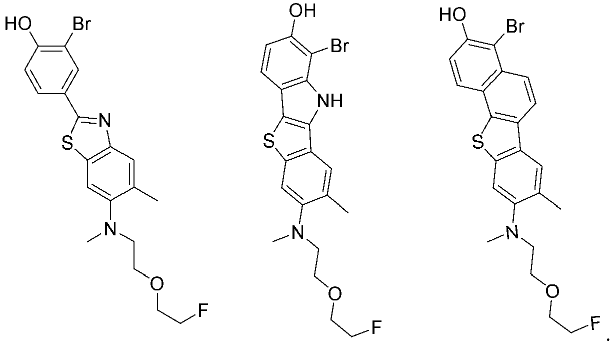

In one embodiment, the sulfur-containing fused ring compound of Formula IV-A is:

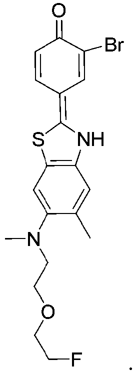

In one embodiment, the sulfur-containing fused ring compound of Formula V-A is any one selected from the group consisting of the following compounds:

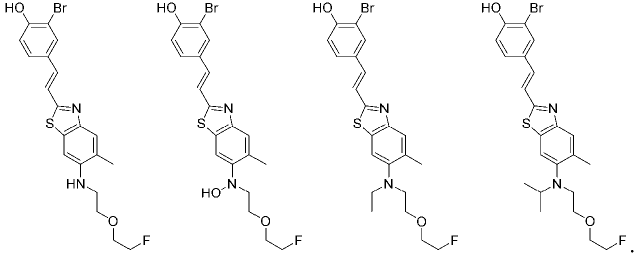

In one embodiment, the sulfur-containing fused ring compound of Formula VI-A is any one selected from the group consisting of the following compounds:

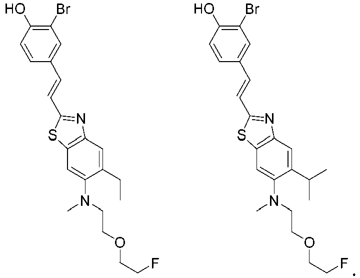

In one embodiment, the sulfur-containing fused ring compound of Formula VII is:

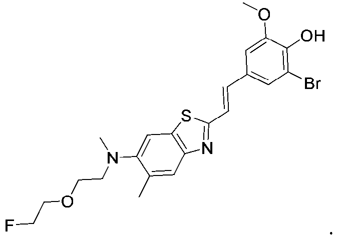

In one embodiment, the radioisotope-labeled derivative is a compound obtained by replacing the F, C, I, or N atom in the sulfur-containing fused ring compound as shown in Formula A, I-A, II-A, III-A, IV-A, V-A, VI-A, or VII, the pharmaceutically acceptable salt thereof, or the tautomer thereof with ¹⁸F, ¹¹C, ¹²⁴I, ¹²³I, or ¹³N; preferably ¹⁸F.

The present disclosure also provides a pharmaceutical composition, comprising the sulfur-containing fused ring compound of Formula A, I-A, II-A, III-A, IV-A, V-A, VI-A, or VII, the pharmaceutically acceptable salt thereof, the tautomer thereof, or the radioisotope-labeled derivative thereof, and a pharmaceutically acceptable excipient.

The present disclosure also provides a kit comprising the sulfur-containing fused ring compound of Formula A, I-A, II-A, III-A, IV-A, V-A, VI-A, or VII, the pharmaceutically acceptable salt thereof, the tautomer thereof, or the radioisotope-labeled derivative thereof, and instructions for use.

The present disclosure provides a use of the sulfur-containing fused ring compound of Formula A, I-A, II-A, III-A, IV-A, V-A, VI-A, or VII, the pharmaceutically acceptable salt thereof, the tautomer thereof, or the radioisotope-labeled derivative thereof, in the manufacture of a PET-CT tracer.

The PET-CT tracer can be used for imaging α-synuclein aggregates in LB and LN; for example, the PET-CT tracer is used for imaging PD, dementia with Lewy bodies (DLB), or multiple system atrophy (MSA).

The PET-CT tracer can be used for diagnostic exclusion of progressive supranuclear palsy or corticobasal degeneration.

The present disclosure further provides a use of the sulfur-containing fused ring compound of Formula A, I-A, II-A, III-A, IV-A, V-A, VI-A or VII, the pharmaceutically acceptable salt thereof, the tautomer thereof, or the radioisotope-labeled derivative thereof, as an *in vivo* imaging agent for diagnosing CNS disorders; wherein the CNS disorders including but not limited to diseases related to α-synuclein expression; for example, diseases related to α-synuclein PFFs expression; further for example, the diseases related to α-synuclein expression are PD, DLB, or MSA.

The present disclosure further provides a use of the sulfur-containing fused ring compound of Formula A, I-A, II-A, III-A, IV-A, V-A, VI-A, or VII, the pharmaceutically acceptable salt thereof, the tautomer thereof, or the radioisotope-labeled derivative thereof, as an *in vivo* imaging agent for diagnosing CNS disorders, and ruling out progressive supranuclear palsy (PSP) or corticobasal degeneration (CBD).

The five aforementioned disorders all fall under the category of parkinsonism and share common clinical manifestations, such as hand tremors and gait disturbances, making their diagnosis challenging. However, three of these disorders-PD, DLB, and MSA-are classified as α-synucleinopathies. In patients with these conditions, there is misfolding and abnormal aggregation of α-synuclein in the brain. When a tracer is administered to such patients, imaging can be achieved, thereby enabling the diagnosis of these diseases. In contrast, in the other two disorders-PSP and CBD-lack α-synuclein aggregates in the patients' brains. Consequently, these disorders exhibit no detectable tracer binding, permitting exclusion of PSP/CBD diagnoses.

The present disclosure further provides a brain imaging method, comprising the following steps:
(1) Administering to a subject the sulfur-containing fused ring compound of Formula A, I-A, II-A, III-A, IV-A, V-A, VI-A, or VII, the pharmaceutically acceptable salt thereof, the tautomer thereof, or the radioisotope-labeled derivative thereof;
(2) Performing PET-CT scanning on the subject's brain.

The present disclosure further provides a method for detecting α-synuclein protofibrils in the brain, comprising the following steps:
(1) Administering to a subject the sulfur-containing fused ring compound of Formula A, I-A, II-A, III-A, IV-A, V-A, VI-A, or VII, the pharmaceutically acceptable salt thereof, the tautomer thereof, or the radioisotope-labeled derivative thereof;
(2) Detecting the sulfur-containing fused ring compound of Formula A, I-A, II-A, III-A, IV-A, V-A, VI-A, or VII, the pharmaceutically acceptable salt thereof, the tautomer thereof, or the radioisotope-labeled derivative thereof.

The present disclosure further provides a method for diagnosing whether a subject has Parkinson's disease or has a potential risk of developing Parkinson's disease, comprising the following steps:
(1) Administering to the subject the sulfur-containing fused ring compound of Formula A, I-A, II-A, III-A, IV-A, V-A, VI-A, or VII, the pharmaceutically acceptable salt thereof, the tautomer thereof, or the radioisotope-labeled derivative thereof;
(2) Detecting the sulfur-containing fused-ring compound of Formula A, I-A, II-A, III-A, IV-A, V-A, VI-A, or VII, the pharmaceutically acceptable salt thereof, the tautomer thereof, or the radioisotope-labeled derivative thereof;
(3) Diagnosing whether the subject has Parkinson's disease or has a potential risk of developing Parkinson's disease based on the detection result of step (2).

The sulfur-containing fused ring compounds of the present disclosure exhibit superior affinity and selectivity for α-Syn aggregates. SAR studies have revealed that: in the sulfur-containing fused ring compounds of Formula A, I-A, II-A, III-A, IV-A, V-A, VI-A, or VII, the R¹ substituent can form a hydrogen bond with the hydroxyl group on T44 of α-Syn; the R² substituent can form a hydrogen bond with the carboxylic acid group of E46 on α-Syn; the R⁵ substituent can form a hydrogen bond with the amide group backbone structure of G41 on α-Syn; and the R³ and R⁴ substituents can augment van der Waals forces through hydrophobic interactions. Additionally, compounds of Formulae I-A, II-A, V-A, or VI-A can span three β-sheet layers of α-Syn protofibrils, while compounds of Formulae III-A and IV-A can span 2 β-sheet layers, thereby achieving superior binding affinity for α-Syn aggregates.

Unless otherwise specified, the following terms shall have the meanings set forth below:

The term "halogen" refers to fluorine, chlorine, bromine, or iodine.

The term "alkyl" refers to a straight or branched-chain hydrocarbon group having a specified number of carbon atoms (e.g., C₁₋₆). Alkyl includes, but is not limited to, methyl, ethyl, *n*-propyl, isopropyl, *n*-butyl, *tert-*butyl*,* isobutyl, *sec*-butyl, *n*-pentyl, and *n*-hexyl.

The term "alkenyl" refers to a straight or branched hydrocarbon chain group having at least one double bond, consisting solely of carbon and hydrogen atoms, containing a specified number of carbon atoms (e.g., C₂₋₆), and attached to the remainder of the molecule through a single bond. Alkenyl includes, but is not limited to: ethenyl, *n*-propenyl, isopropenyl, *n*-butenyl, isobutenyl, *sec*-butenyl, *tert-*butenyl, *n*-pentenyl, 2-methylbutenyl, 2,2-dimethylethenyl, and *n*-hexenyl.

The term "alkynyl" refers to a straight or branched hydrocarbon chain group having at least one carbon-carbon triple bond, consisting solely of carbon and hydrogen atoms, containing a specified number of carbon atoms (e.g., C₂₋₆), and attached to the remainder of the molecule through a single bond. Alkynyl includes, but is not limited to: ethynyl, *n*-propynyl, *iso*-propynyl, *n*-butynyl, *iso-*butynyl, se*c*-butynyl, *tert*-butynyl, *n*-pentynyl, 2-methylbutynyl, 2,2-dimethylpropynyl, or n-hexynyl.

The term "cycloalkyl" refers to a cyclic hydrocarbon group having a specified number of carbon atoms (e.g., C₃₋₆) and consisting solely of carbon atoms. Cycloalkyl includes, but is not limited to: cyclopropyl, cyclobutyl, cyclopentyl, and cyclohexyl.

The term "aryl" refers to a cyclic hydrocarbon group having a specified number of carbon atoms (e.g., C₆₋₁₀) and consisting solely of carbon atoms, which is monocyclic or polycyclic and at least one ring thereof is aromatic (satisfying Hückel's rule). Aryl includes, but is not limited to: phenyl and naphthalenyl.

The term "heteroaryl" refers to a cyclic hydrocarbon group having a specified number of ring carbon atoms (e.g., 5- to 10-membered), containing a specified number of heteroatoms (e.g., 1, 2, or 3) selected from specified heteroatom types (one or more of N, O, and S), wherein the cyclic hydrocarbon group is monocyclic or polycyclic and at least one ring thereof is aromatic (satisfying Hückel's rule). When said heteroaryl is polycyclic and comprises one aromatic ring and one non-aromatic ring, said heteroaryl is attached to other moieties of the molecule via either the aromatic ring or the non-aromatic ring. Heteroaryl includes, but is not limited to, furanyl, pyrrolyl, thienyl, pyrazolyl, imidazolyl, oxazolyl, thiazolyl, pyridinyl, pyrimidinyl, and indolyl.

The term "heterocycloalkyl" refers to a stable, saturated heterocyclic hydrocarbon group (e.g., 3- to 6-membered) comprising carbon atoms and heteroatoms (N, O, or S), including but not limited to, aziridinyl, oxiranyl, oxetanyl, thietanyl, tetrahydrothienyl, or thiazolidinyl.

The dash ("-") at the terminus of a group denotes the bonding site through which said group is attached to other moieties of the molecule. For example, "-OCH₃" refers to a methoxy group wherein the oxygen atom is attached to the rest of the molecule.

The term " 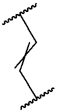 " refers to a combination of cis and trans isomers.

When any variable (e.g., group R¹⁻³) appears more than once in the definition of a compound, each occurrence of said variable is defined independently and without influence from any other occurrence. For example, "a C₁₋₆ alkyl group substituted by 3 R¹⁻³" means the C₁₋₆ alkyl group is substituted by 3 R¹⁻³ substituents, wherein the definition of each R¹⁻³ is independent of the others, and each substituent may be identical to or different from the others.

The term "pharmaceutically acceptable salt" refers to a salt formed by the reaction of a compound with an acid or base that is pharmaceutically acceptable (safe and suitable for patient use). When a compound contains a relatively acidic functional group, a base addition salt may be obtained by contacting the free form of the compound with a sufficient amount of a pharmaceutically acceptable base in a suitable inert solvent. Pharmaceutically acceptable base addition salts include, but are not limited to, salts derived from sodium, potassium, calcium, aluminum, magnesium, bismuth, and ammonium. When a compound contains a relatively basic functional group, an acid addition salt may be obtained by contacting the free form of the compound with a sufficient amount of a pharmaceutically acceptable acid in a suitable inert solvent. Pharmaceutically acceptable acid addition salts include, but are not limited to, hydrochloride, sulfate, and methanesulfonate salts. For further details, see Handbook of Pharmaceutical Salts: Properties, Selection, and Use (P. Heinrich Stahl, 2002).

The term "Positron Emission Tomography (PET)" refers to an imaging technique that produces images (e.g., three-dimensional images) by detecting paired γ rays indirectly emitted by a positron-emitting radionuclide tracer. Images of tracer concentration within the area are then constructed by computer analysis. A radioactive tracer is administered to a subject, e.g., into blood circulation. Typically, there is a waiting period while tracer becomes concentrated in areas of interest; then the subject is placed in the imaging scanner. As the radionuclide undergoes positron emission decay, it emits a positron (an antiparticle of the electron with opposite charge), until it decelerates to a point where it can interact with electrons, producing a pair of (γ) photons moving in approximately opposite directions. These are detected in a scanning device. The technique typically utilizes simultaneous or coincident detection of the pair of photons moving in approximately opposite directions (the scanner typically has a built-in slight direction-error tolerance). Photons that do not arrive in pairs (i.e., within a timing-window) are typically ignored. One typically localizes the source of the photons along a straight line of coincidence (also termed the Line of Response or LOR). This data is used to generate an image.

The term "radioisotope-labeled derivative" refers to a compound in which one or more atoms within the molecule have been replaced by a radioactive isotope, thereby enabling its identification and use as a tracer. It possesses the same chemical and biological properties as its corresponding unlabeled compound, differing only in its radioactivity, which allows it to be detected and/or tracked using radiometric detection techniques (e.g., replacement of atoms with ¹⁸F, ¹¹C, or ¹³C). In the present disclosure, a radioisotope-labeled derivative refers to a radioisotope-labeled compound selected from: a sulfur-containing fused ring compound of Formula A, I-A, II-A, III-A, IV-A, V-A, VI-A, or VII; a pharmaceutically acceptable salt of a sulfur-containing fused ring compound of Formula A, I-A, II-A, III-A, IV-A, V-A, VI-A, or VII; or a tautomer of a sulfur-containing fused ring compound of Formula A, I-A, II-A, III-A, IV-A, V-A, VI-A, or VII.

Following administration to a subject of an *in vivo* imaging moiety-labeled sulfur-containing fused ring compound as disclosed herein, the subject is imaged. Methods for detecting said sulfur-containing fused ring compound of Formula A, I-A, II-A, III-A, IV-A, V-A, VI-A, or VII, or a pharmaceutically acceptable salt thereof, or a tautomer thereof, or a radioisotope-labeled derivative thereof, are conventional in the art. The sulfur-containing fused ring compound may be administered at any suitable dose. Imaging of the subject may be performed using any suitable imaging apparatus (e.g., an apparatus capable of acquiring magnetic resonance images (MRI), positron emission tomography images (PET scans), or computed tomography images (CT scans)).

The term "α-synuclein deposits" refers to an insoluble proteinaceous inclusions comprising the protein α-synuclein. Lewy bodies (LB) and Lewy neurites (LN) are well-known insoluble proteinaceous inclusions wherein α-synuclein is the main component, and have been reported in the central nervous system (CNS) as well as the autonomic nervous system (ANS) in Parkinson's disease (PD). However, PD is conventionally considered as a disorder of the CNS, and is known *in vivo* imaging methods for the detection of PD target α-synuclein deposits present in the CNS.

The term "central nervous system (CNS)" refers to the part of the nervous system in vertebrates consisting of the brain and the spinal cord. In the CNS, endothelial cells are packed together more tightly than in the rest of the body by means of "tight junctions", which are multifunctional complexes that form a seal between adjacent epithelial cells, thereby preventing the passage of most dissolved molecules from one side of the epithelial sheet to the other. This forms the blood-brain barrier (BBB), which blocks the transit of all molecules except those that cross cell membranes by means of lipid solubility (e.g., oxygen, carbon dioxide, ethanol, and steroid hormones) and those that are allowed in by specific transport systems (e.g., sugars and certain amino acids). Generally, substances with molecular weights more than 500 Daltons (e.g., antibodies) cannot traverse the BBB by passive diffusion, whereas smaller molecules typically can. In order for an *in vivo* imaging agent to access targets in the CNS, its chemical structure has to be modified for passage across the BBB, or alternatively, the *in vivo* imaging agent has to be administered directly into the CNS using relatively invasive procedures.

The term "pharmaceutical excipient" refers to excipients and additives used in the manufacture of pharmaceutical products and formulation prescriptions. It encompasses all substances included in a pharmaceutical formulation other than the active pharmaceutical ingredient (API). As specified in the Pharmacopoeia of the People's Republic of China (2020 Editi*on)* and the Handbook of Pharmaceutical Excipients (Rowe et al., 2009*).* Pharmaceutical excipients primarily serve to: provide a safe, stable, and functional drug composition; enable controlled dissolution of the API at a desired rate post-administration; and facilitate effective absorption of the API following administration. Pharmaceutical excipients may function as inert fillers or confer specific functional properties, including but not limited to: stabilizing the bulk pH of the composition; or preventing degradation of the API. The pharmaceutical excipients may comprise one or more members selected from the group consisting of: binders, suspending agents, emulsifiers, diluents, fillers, granulating agents, adhesives, disintegrants, lubricants, anti-adherents, glidants, wetting agents, gelling agents, absorption delay agents, dissolution inhibitors, enhancers, adsorbents, buffers, chelating agents, preservatives, colorants, flavoring agents, and sweeteners.

The "detection" step of the disclosed method involves the detection of signals either externally to the human body, or via use of detectors designed for use *in vivo* (such as intravascular radiation), or optical detectors (such as endoscopes) (e.g. suitable for detection of signals in the gut), or radiation detectors designed for intra-operative use. This detection step can also be understood as the acquisition signal data.

The *"in vivo* imaging method" selected for detection of signals emitted by said *in vivo* imaging moiety depends on the nature of the signals. Therefore, where the signals come from a paramagnetic metal ion, magnetic resonance imaging (MRI) is used, where the signals are γ rays, single-photon emission tomography (SPECT) is used, and where the signals are positrons, positron emission tomography (PET) is used; and where the signals are optically active, optical imaging is used. All are suitable for use in the method of the present disclosure, with PET and SPECT are preferred, as they are least likely to suffer from background and therefore are diagnostically useful.

The pharmaceutical composition of the present disclosure may be prepared by any method known to those skilled in the art based on the disclosed contents, including but not limited to, conventional mixing; dissolution; granulation; emulsification; micronization; encapsulation; embedding; or lyophilization processes.

When used as a pharmaceutical, the sulfur-containing fused ring compounds represented by Formula A, I-A, II-A, III-A, IV-A, V-A, VI-A, or VII, their pharmaceutically acceptable salts, tautomers, or radioisotopically labeled derivatives thereof, can be administered in any dosage form of pharmaceutical composition. These compositions can be prepared according to methods well known in the pharmaceutical arts and can be administered via various routes, depending on the need for local or systemic treatment and the target region. Administration may be in the form of topical (including epidermal and transdermal, ocular and mucosal, including delivery such as intranasal, vaginal, and rectal), pulmonary (e.g., via powder or aerosol inhalation or insufflation, including nebulization; tracheal or intranasal), oral (both solid and liquid formulations), or parenteral administration. Examples of solid oral formulations include, but are not limited to, powders, capsules, caplets, soft capsules, and tablets. Examples of liquid formulations for oral or mucosal administration include, but are not limited to, suspensions, emulsions, elixirs, and solutions. Examples of topical formulations include, but are not limited to, emulsions, gels, ointments, creams, patches, pastes, foams, lotions, drops, or serum formulations. Examples of formulations for parenteral administration include, but are not limited to, solutions for injection, dry formulations that can be dissolved or suspended in a pharmaceutically acceptable carrier, suspensions for injection, and emulsions for injection. Pharmaceutical compositions and formulations for external administration may include transdermal patches, ointments, emulsions, creams, gels, drops, suppositories, sprays, liquids, and powders. Examples of other suitable formulations of the described pharmaceutical compositions include, but are not limited to, eye drops and other ophthalmic formulations; aerosols such as nasal sprays or inhalants. Oral administration may include dosage forms formulated for once-daily or twice-daily (BID) administration. Parenteral administration includes intravenous, intraarterial, subcutaneous, intraperitoneal, intramuscular injection or infusion; or intracranial administration such as intrathecal or intraventricular administration. Parenteral administration can be in the form of a single bolus dose, or it can be via a continuous infusion pump. Conventional pharmaceutical carriers, water, powdered or oily bases, thickeners, and the like may be necessary or required. The pharmaceutical compositions of the present disclosure may also be in controlled-release or delayed-release dosage forms (e.g., liposomes or microspheres).

The term "treatment" refers to any of the following: (1) alleviation of one or more biological manifestations of a disease; (2) interference with one or more points in the biological cascade that induces the disease; or (3) retardation of the progression of one or more biological manifestations of the disease.

The term "prevention" refers to reduction of the risk of developing a disease.

The term "subject" refers to any animal that has received or will receive treatment, preferably a mammal, most preferably a human. Mammals include but are not limited to cattle, horses, sheep, pigs, cats, dogs, mice, rats, rabbits, guinea pigs, monkeys, humans, and the like.

All preferred embodiments disclosed herein may be combined in any manner without departing from the common knowledge in the art, thereby providing various preferred instances of the present disclosure.

Reagents and raw materials used in the present disclosure are commercially available.

The present disclosure provides significant advantages in that the sulfur-containing fused ring compound can be used as a PET-CT tracer and is used for developing and imaging α-synuclein aggregates in LB and LN, so as to diagnose CNS disorders.

### BRIEF DESCRIPTION OF THE DRAWINGS

FIG. 1 shows the binding specificity of the compounds to α-synuclein aggregates in a cellular model, Scale bar: 40 µm.
FIG. 2 demonstrates the binding specificity of the compounds to brain tissue sections from *SNCA* Tg mice treated with rotenone, Scale bar: 40 µm.
FIG. 3 depicts the binding specificity of the compounds to brain tissue sections of human patients with DLB, Scale bar: 40 µm.

### DETAILED DESCRIPTION OF THE PREFERRED EMBODIMENT

The present disclosure is further illustrated by the following examples, which are not to be construed as limiting the scope of the present disclosure. Experimental methods in the following examples for which no specific conditions are indicated are conducted under conventional conditions or in accordance with the manufacturer's instructions.

### Example 1: Synthesis of Compound 1

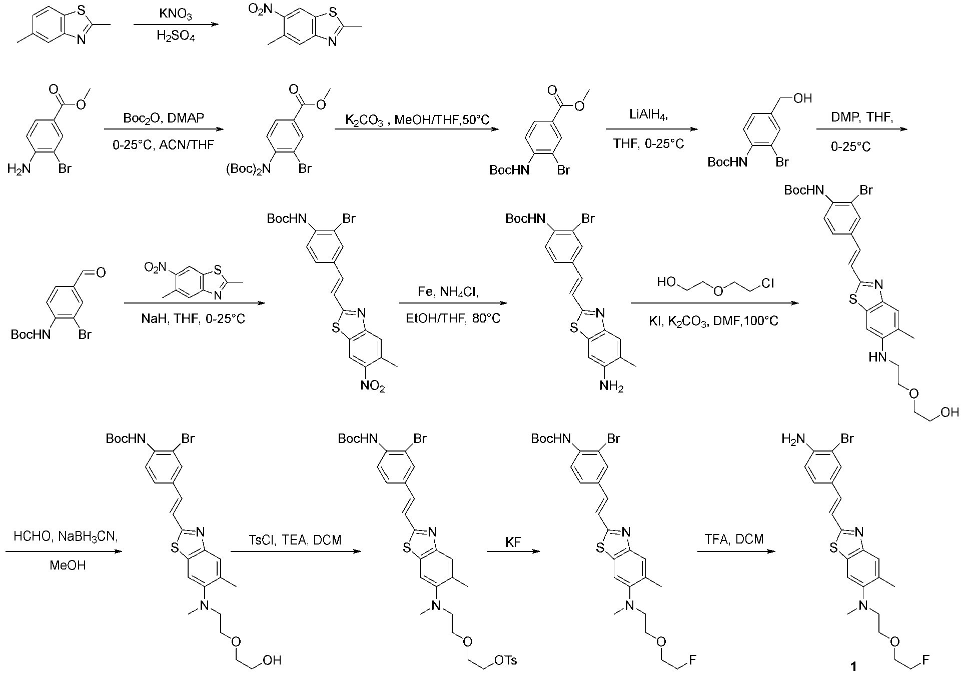

### Synthesis of 2-(4-amino-3-bromostyryl)-N-(2-fluoroethoxy)ethyl-N,5-dimethylbenzo[d]thiazol-6-amine

### Step 1: Synthesis of 2,5-dimethyl-6-nitrobenzo[d]thiazole

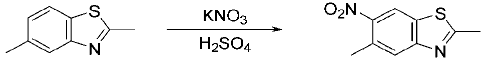

To a solution of 2,5-dimethylbenzo[*d*]thiazole (16 g, 98.15 mmol) in conc. H₂SO₄ (200 mL) was added KNO₃ (10.9 g, 107.96 mmol) portionwise at 0 °C. The resulting mixture was stirred at room temperature for 3 h. After the reaction was complete, the reaction mixture was poured into ice-water (2.0 L) and a yellow solid precipitated. The mixture was filtered, and the filter cake was washed with saturated sodium carbonate solution and water to afford the crude product, which was purified by column chromatography on silica gel (PE:EA = 0-15%) to give the desired product (7.4 g, 36% yield) as yellow solid. LC-MS (ESI): [M+H]⁺ = 209.0; ¹HNMR (400 MHz, CDCl₃) *δ* 8.43 (s, 1H), 7.76 (s, 1H), 2.81 (s, 3H), 2.64 (s, 3H).

### Step 2: Synthesis of methyl 4-(bis(tert-butoxycarbonyl)amino)-3-bromobenzoate

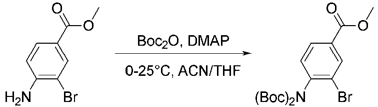

To a solution of methyl 4-amino-3-bromobenzoate (25 g, 108.7 mmol) and DMAP (13.27 g, 108.7 mmol) in THF and ACN (800 mL, v/v=1/1) was added Boc₂O (75 mL, 326.1 mmol) dropwise at 0 °C. The mixture was stirred at room temperature for 16 h, then the solvent was removed. The residue was added citric acid (10%, w/w) and extracted with EtOAc. The combined organic layers were dried over anhydrous Na₂SO₄, filtered, and concentrated to give the desired product (46 g, 98.6% yield) as yellow solid. LC-MS (ESI): [M+Na]⁺ = 454.0.

### Step 3: Synthesis of methyl 3-bromo-4-((tert-butoxycarbonyl)amino)benzoate

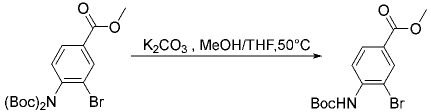

To a solution of methyl 4-(bis(tert-butoxycarbonyl)amino)-3-bromobenzoate (46 g, 107.2 mmol) in MeOH (700 mL) was added K₂CO₃ (22.5 g, 163.0 mmol). The reaction mixture was stirred at 50 °C for 12 h, then filtered. The filter cake was washed with DCM/MeOH=20:1, the filtrate was concentrated under reduced pressure and purified by column chromatography on silica gel (PE:EA=20:1) to give the desired product (22 g, 62.3% yield). LC-MS (ESI): [M-56+H]⁺ = 273.9.

### Step 4: Synthesis of tert-butyl (2-bromo-4-(hydroxymethyl)phenyl)carbamate

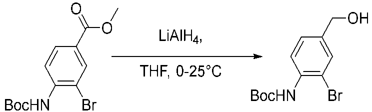

To a suspension of LiAlH₄ (5 g, 33.7 mmol) in anhydrous THF (200 mL) was added a solution of 3-bromo-4-(*tert*-butoxycarbonylamino)benzoate (22 g, 66.8 mmol) in THF (60 mL) at 0 °C. The mixture was stirred at 50 °C for 12 h. The mixture was quenched by H₂O (5 mL), 15% NaOH (5 mL) and H₂O (15 mL). Then filtered, the filtrate was concentrated under reduced pressure, and purified by column chromatography on silica gel (PE:EA=2:1) to give the desired product (14 g, 69.6% yield) as colorless oil. LC-MS (ESI): [M-56-17+H]⁺ = 229.9.

### Step 5: Synthesis of tert-butyl (2-bromo-4-formylphenyl)carbamate

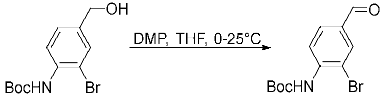

To a solution of *tert-*butyl (2-bromo-4-(hydroxymethyl)phenyl)carbamate (14 g, 46.5 mmol) in DCM (150 mL) was added DMP (29.58 g, 69.77 mmol) in batches at 0 °C. Then the mixture was stirred at room temperature for 2 h. The mixture was extracted with water. The combined organic layers were purified by column chromatography on silica gel (PE:EA=0-70%) to give the desired product (13 g, 93.4% yield) as yellow solid. LC-MS (ESI): [M+H-56]⁺ = 245.9.

### Step 6: Synthesis of tert-butyl (E)-(2-bromo-4-(2-(5-methyl-6-nitrobenzo[d]thiazol-2-yl)vinyl)phenyl)carbamate

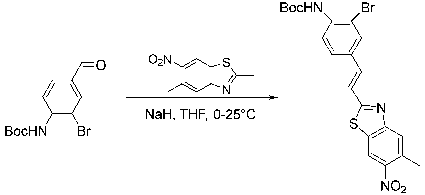

To a solution of 2,5-dimethyl-6-nitrobenzothiazole (2.78 g, 13.4 mmol) in THF (60 mL) was added NaH (1.6 g, 40.13 mmol, wt: 60%) at 0 °C. After being stirred at room temperature for 30 min, *tert*-butyl (2-bromo-4-formylphenyl)carbamate (4 g, 13.38 mmol) was added. The mixture was stirred for 14 h at room temperature. The mixture was quenched by water and extracted with EtOAc. The combined organic layers were dried over anhydrous Na₂SO₄, filtered, and concentrated. The residue was purified by column chromatography on silica gel (DCM:EA=100:1) to give the desired product (2.8 g, 42.8% yield) as black solid. LC-MS (ESI): [M+H]⁺ = 492.0; ¹H NMR (400 MHz, CDCl₃) *δ* 8.47(s, 1H), 8.18 (d, *J* = 12 Hz, 1H), 7.80 (s, 1H), 7.68 (d, *J* = 2.0 Hz, 1H), 7.46-7.43 (m, 1H), 7.41 (s, 1H), 7.21 (d, *J* = 8.0 Hz, 1H), 7.07 (s, 1H), 2.66 (s, 3H), 1.48 (s, 9H).

### Step 7: Synthesis of tert-butyl (E)-(4-(2-(6-amino-5-methylbenzo[d]thiazol-2-yl)vinyl)-2-bromophenyl)carbamate

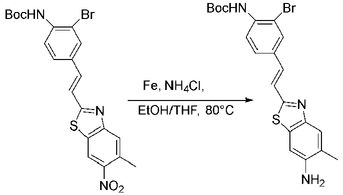

To a solution of *tert*-butyl (*E*)-(2-bromo-4-(2-(5-methyl-6-nitrobenzo[*d*]thiazol-2-yl)vinyl)phenyl) carbamate (2.80 g, 5.77 mmol) in THF and EtOH (80 mL, v/v=1:1) was added aqueous solution of ammonium chloride (3.1 g, 57.3 mmol, 15 mL) and iron powder (3.2 g, 57.3 mmol) at room temperature. The resulting mixture was stirred at 80 °C for 2 h. After being cooled to room temperature, the mixture was filtered. The filtrate was concentrated to give the desired product (2.6 g, 99.0% yield) as red solid. LC-MS (ESI): [M+H]⁺ = 462.1.

### Step 8: Synthesis of tert-butyl (E)-(2-bromo-4-(2-(6-((2-(2-hydroxyethoxy)ethyl)amino)-5-methylbenzo[d]thiazol-2-yl)vinyl)phenyl)carbamate

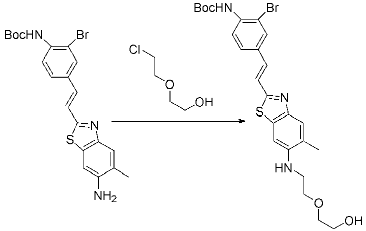

To a solution of *tert-*butyl (*E*)-(4-(2-(6-amino-5-methylbenzo[*d*]thiazol-2-yl)vinyl)-2-bromophenyl)carbamate (2.6 g, 5.66 mmol) in DMF (30 mL) was added K₂CO₃ (2.34 g, 16.99 mmol), KI (940 mg, 5.66 mmol), and 2-(2-chloroethoxy)ethan-1-ol (3.54 g, 28.32 mmol). The resulting mixture was stirred at 100 °C for 48 h. The mixture was added water and extracted with EtOAc. The combined organic layers were dried over anhydrous Na₂SO₄, and filtered. The filtrate was concentrated to give the residue, which was purified by column chromatography on silica gel (DCM: EA=3:1) to give the desired product (1.4 g, 36.7% yield) as red oil. LC-MS (ESI): [M+H]⁺ =550.1.

### Step 9: Synthesis of tert-butyl (E)-(2-bromo-4-(2-(6-((2-(2-hydroxyethoxy)ethyl)(methyl)amino)-5-methylbenzo[d]thiazol-2-yl)vinyl)phenyl)carbamate

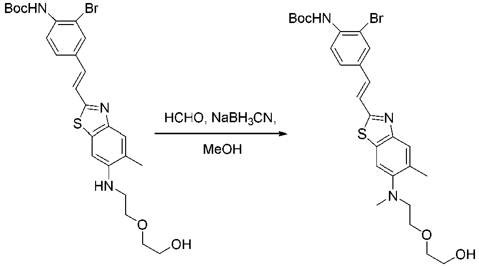

To a solution of *tert*-butyl (*E*)-(2-bromo-4-(2-(6-((2-(2-hydroxyethoxy)ethyl)amino)-5-methylbenzo[d]thiazol-2-yl)vinyl)phenyl)carbamate (1.4 g, 2.56 mmol) in MeOH (15 mL) was added aqueous formaldehyde (30% wt, 0.3 mL). After being stirred at 0 °C for 30 min, NaBH₃CN (242 mg, 3.84 mmol) was added. The resulting mixture was stirred at room temperature for 1 h. The solvent was removed. The residue was purified by column chromatography on silica gel (DCM: EA=3:1) to give the desired product (1.4 g, 97.2% yield) as red oil. LC-MS (ESI): [M+H]⁺ = 564.1.

### Step 10: Synthesis of (E)-2-(2-((2-(3-bromo-4-((tert-butoxycarbonyl)amino)styryl)-5-methylbenzo[d] thiazol-6-yl)(methyl)amino)ethoxy)ethyl 4-methylbenzenesulfonate

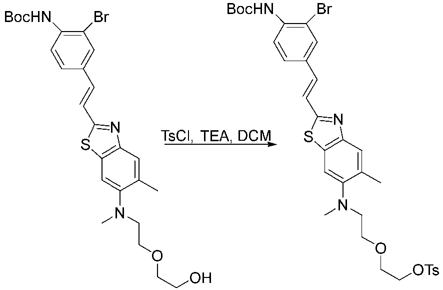

To a solution of *tert-*butyl (*E*)-(2-bromo-4-(2-(6-((2-(2-hydroxyethoxy)ethyl)(methyl)amino)-5-methylbenzo[*d*]thiazol-2-yl)vinyl)phenyl)carbamate (1.4 g, 2.49 mmol) and TEA (1.4 mL) in DCM (15 mL) was added TsCl (948 mg, 4.99 mmol) at 0 °C. The resulting mixture was stirred at room temperature for 16 h. The mixture was quenched by H₂O and extracted with EtOAc. The organic layer was concentrated, and purified by column chromatography on silica gel (PE: EA = 5: 1) to give the desired product (1.7 g, 95.5% yield) as light green solid. LC-MS(ESI): [M+H]⁺ = 718.1.

### Step 11: Synthesis of tert-butyl (E)-(2-bromo-4-(2-(6-((2-(2-fluoroethoxy)ethyl)(methyl)amino)-5-methylbenzo[d]thiazol-2-yl)vinyl)phenyl)carbamate

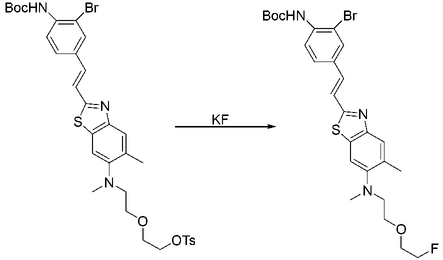

A solution of (*E*)-2-(2-((2-(3-bromo-4-((*tert*-butoxycarbonyl)amino)styryl)-5-methylbenzo[*d*]thiazol-6-yl)(methyl)amino)ethoxy)ethyl 4-methylbenzenesulfonate (1.7 g, 2.38 mmol), Kryptofix 222 (2.24 g, 5.94 mmol), and KF (1.38 g, 23.77 mmol) in ACN (15 mL) was stirred at 60 °C for 2 h. After being cooled to room temperature, the solvent was removed. The residue was purified by column chromatography on silica gel (PE: EA=4:1) to give the desired product (450 mg, 33.6% yield) as light green oil. LC-MS (ESI): [M+H]⁺ =566.1; ¹H NMR (400 MHz, CDCl₃) *δ* 8.14 (d,*J* = 8.0 Hz, 1H), 7.70 - 7.65 (m, 2H), 7.45 - 7.41 (m, 2H), 7.21 - 7.17 (m, 2H), 7.03 (s, 1H), 4.53 - 4.51 (m, 1H), 4.41 - 4.39 (m, 1H), 3.66 - 3.56(m, 4H), 3.14 (t, *J* =8.0 Hz, 2H), 2.76 (s, 3H), 2.56 (s, 3H), 1.48 (s, 9H).

### Step 12: Synthesis of 2-(4-amino-3-bromostyryl)-N-(2-(2-fluoroethoxy)ethyl)-N,5-dimethylbenzo[d] thiazol-6-amine

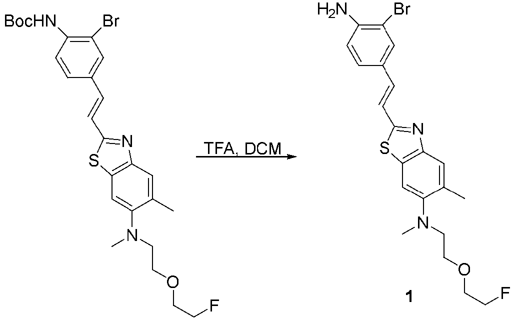

To a solution of *tert-*butyl (*E*)-(2-bromo-4-(2-(6-((2-(2-fluoroethoxy)ethyl)(methyl)amino)-5-methylbenzo[*d*]thiazol-2-yl)vinyl)phenyl)carbamate (200 mg, 0.35 mmol) in DCM (2.5 mL) was added TFA (1 mL). The resulting mixture was stirred at room temperature for 2 h. Then the solvent was removed. The residue was adjusted to pH 8 with saturated sodium carbonate solution. The mixture was extracted with EtOAc. The combined organic layers were dried over anhydrous Na₂SO₄, filtered and concentrated to give the crude product, which was purified by Prep-HPLC (10 mM ammonium bicarbonate) to give compound 1 (93 mg, 57.3% yield) as yellow oil. LC-MS (ESI): [M+H]⁺ = 464.1, 466.1; ¹H NMR (400 MHz, DMSO-*d₆*) *δ* 7.77 (d, *J* = 2.0 Hz, 1H), 7.71 (d, *J* = 12.3, 2H), 7.48 (dd,*J* = 8.4, 2.0 Hz, 1H), 7.38 - 7.25 (m, 2H), 6.81 (d, *J* = 8.4 Hz, 1H), 5.80 (s, 2H), 4.57 - 4.55 (m, 1H), 4.45 - 4.43 (m, 1H), 3.68 - 3.66 (m, 1H), 3.62 - 3.58 (m, 3H), 3.10 (t, *J* = 6.0 Hz, 2H), 2.77 - 2.75 (m, 3H), 2.38 - 2.36 (m, 3H).

### Example 2: Synthesis of 2-(3-bromo-4-(methylamino)styryl)-N-(2-(2-fluoroethoxy)ethyl)-N,5-dimethylbenzo[d]thiazol-6-amine

T*ert*-butyl (*E*)-(2-bromo-4-(2-(6-((2-(2-fluoroethoxy)ethyl)(methyl)amino)-5-methylbenzo[*d*]thiazol-2-yl)vinyl)phenyl)carbamate was synthesized following the procedure of Example 1.

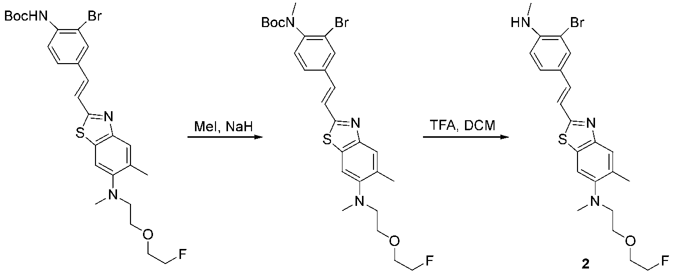

### Step 1 : Synthesis of tert-butyl (E)-(2-bromo-4-(2-(6-((2-(2-fluoroethoxy)ethyl)(methyl)amino)-5-methylbenzo[d]thiazol-2-yl)vinyl)phenyl)(methyl)carbamate

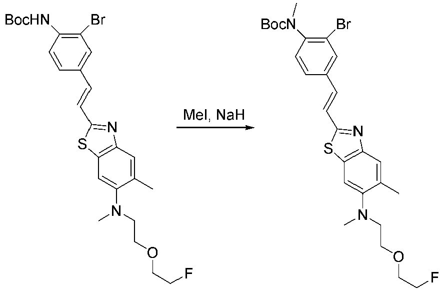

To a solution of *tert-*butyl (*E*)-(2-bromo-4-(2-(6-((2-(2-fluoroethoxy)ethyl)(methyl)amino)-5-methylbenzo[*d*]thiazol-2-yl)vinyl)phenyl)carbamate (250 mg, 0.44 mmol) in DMF (3 mL) was added NaH (26 mg, 0.66 mmol, wt: 60%) at 0 °C. After being stirred for 20 min, CH₃I (75 mg, 0.53 mmol) was added. The resulting mixture was stirred at room temperature for 1 h. The reaction mixture was quenched by water and extracted with EtOAc. The combined organic layers were dried over anhydrous Na₂SO₄, and filtered. The filtrate was concentrated to give the desired product (210 mg, 85.6% yield) as yellow oil. LC-MS (ESI): [M+H]⁺ = 580.2.

### Step 2: Synthesis of 2-(3-bromo-4-(methylamino)styryl)-N-(2-fluoroethoxy)ethyl)-N,5-dimethylbenzo[d]thiazol-6-amine

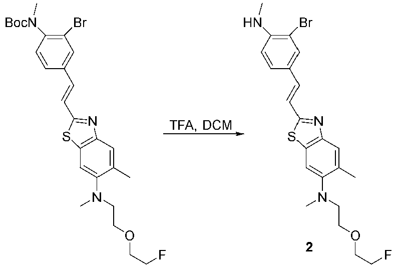

To a solution of *tert-*butyl (*E*)-(2-bromo-4-(2-(6-((2-(2-fluoroethoxy)ethyl)(methyl)amino)-5-methylbenzo[*d*]thiazol-2-yl)vinyl)phenyl)(methyl)carbamate (210 mg, 0.36 mmol) in DCM (2.5 mL) was added TFA (1 mL). The resulting mixture was stirred at room temperature for 2 h. Then the solvent was removed. The residue was adjusted to pH 8 with saturated sodium carbonate solution and extracted with EtOAc. The combined organic layers were dried over anhydrous Na₂SO₄, filtered and concentrated to give the crude product, which was purified by Prep-HPLC (10 mM ammonium bicarbonate) to give compound 2 (57 mg, 33.1% yield) as yellow oil. LC-MS (ESI): [M+H]⁺ =478.1, 480.1; ¹H NMR (400 MHz, DMSO-*d₆*) *δ* 7.86 (d, *J* = 2.0 Hz, 1H), 7.74 - 7.69 (m, 2H), 7.62-7.59 (m, 1H), 7.41 - 7.28 (m, 2H), 6.66-6.63 (m, 1H), 5.84 - 5.77 (m, 1H), 4.57 - 4.55 (m, 1H), 4.45 - 4.43 (m, 1H), 3.68 - 3.66 (m, 1H), 3.62-3.58 (m, 3H), 3.10 (t, *J =* 6.0 Hz, 2H), 2.81 (d, *J* = 4.8 Hz, 3H), 2.76 - 2.74 (m, 3H), 2.39 - 2.37 (m, 3H).

### Example 3: Synthesis of Compound A and Compound B

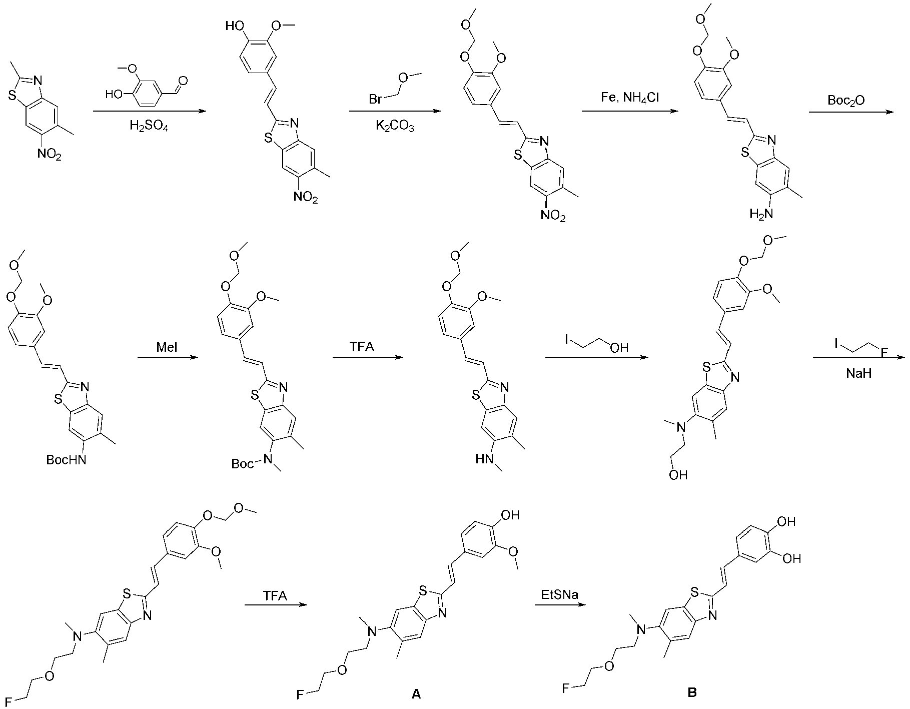

Compound A and Compound B were synthesized following the procedure of Example 1 and Example 2, respectively.

Compound A: LC-MS (ESI): [M+H]⁺ = 417.2.

Compound B: LC-MS (ESI): [M+H]⁺ = 403.2.

### Another Method for Preparing Compound A

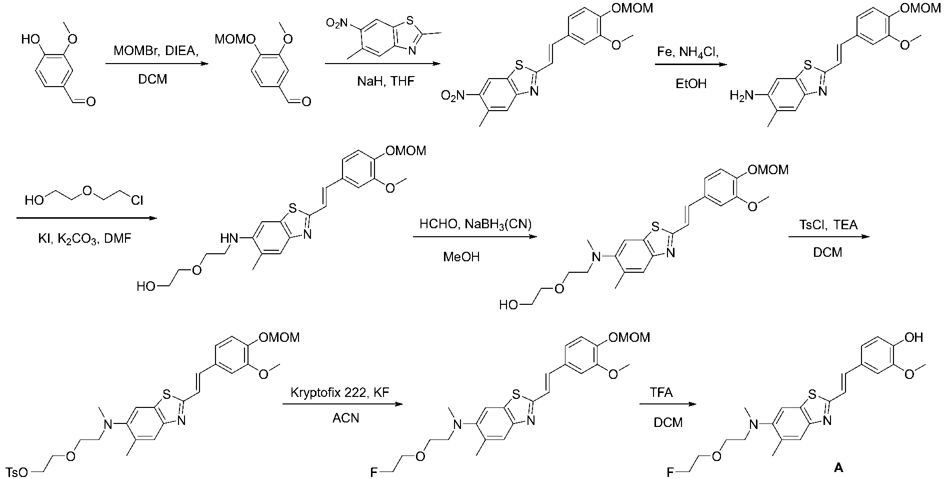

### Synthesis of (E)-4-(2-(6-((2-(2-fluoroethoxy)ethyl)(methyl)amino)-5-methylbenzo[d]thiazol-2-yl)vinyl)-2-methoxyphenol

### Step 1: Synthesis of 3-methoxy-4-(methoxymethoxy)benzaldehyde

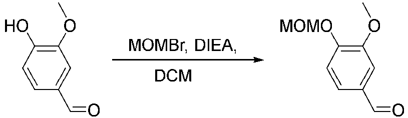

To a solution of 4-hydroxy-3-methoxybenzaldehyde (3 g, 19.73 mmol) and DIEA (5.09 g, 39.47 mmol) in DCM (30 mL) was added MOMBr (1.9 mL, 23.68 mmol, density 1.54 g/cm³) dropwise at 0 °C. The resulting mixture was stirred at room temperature for 12 h under N₂. After the reaction was complete, the solvent was removed. The residue was purified by column chromatography on silica gel (PE: EA=3:1) to give the desired product (3.3 g, 85% yield) as colorless oil. LC-MS (ESI): [M+H]⁺ = 197.2; ¹H NMR (400 MHz, CDCl₃) δ 9.87 (s, 1H), 7.49 - 7.40 (m, 2H), 7.28-7.26 (m, 1H), 5.33 (s, 2H), 3.95 (s, 3H), 3.52 (s, 3H).

### Step 2: Synthesis of (E)-2-(3-methoxy-4-(methoxymethoxy)styryl)-5-methyl-6-nitrobenzo[d]thiazole

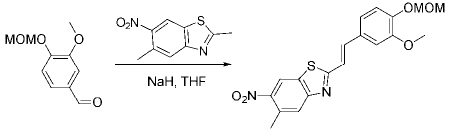

To a solution of 2,5-dimethyl-6-nitrobenzo[d]thiazole (1.9 g, 9.27 mmol) in THF (40 mL) was added NaH (742 mg, 18.55 mmol, wt: 60%) at 0 °C. The resulting mixture was stirred at 0 °C for 40 min under N₂. Then a solution of 3-methoxy-4-(methoxymethoxy)benzaldehyde (2 g, 10.20 mmol) in THF (5 mL) was added to the mixture. After being stirred at room temperature for 4 h under N₂, the mixture was quenched by saturated aqueous NH₄Cl (10 mL), and filtered. The filter cake was washed with EtOAc (20 mL) and dried to give the desired product (2.9 g, 81% yield) as orange solid. LC-MS (ESI): [M+H]⁺ =387.3; ¹H NMR (400 MHz, DMSO-*d₆*) δ 8.90 (s, 1H), 8.01 (s, 1H), 7.73 (d, *J =* 16.0 Hz, 1H), 7.63 (d, *J* = 16.0 Hz, 1H), 7.51 (d, *J* = 2.0 Hz, 1H), 7.32 (dd, *J* = 8.4, 2.0 Hz, 1H), 7.12 (d,*J* = 8.4 Hz, 1H), 5.23 (s, 2H), 3.87 (s, 3H), 3.40 (s, 3H), 2.65 (s, 3H).

### Step 3: Synthesis of (E)-2-(3-methoxy-4-(methoxymethoxy)styryl)-5-methylbenzo[d]thiazol-6-amine

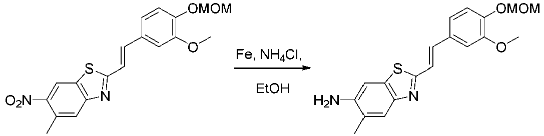

To a solution of (*E*)-2-(3-methoxy-4-(methoxymethoxy)styryl)-5-methyl-6-nitrobenzo[*d*]thiazole (2.90 g, 7.51 mmol) in EtOH (50 mL) was added a solution of NH₄Cl (3.98 g, 75.12 mmol, 5 mL) and iron powder (2.10 g, 37.56 mmol) at room temperature. The resulting mixture was stirred at 80 °C for 2 h. After being cooled to room temperature, the mixture was filtered, and the filtrate was concentrated. The residue was dissolved in H₂O (20 mL) and extracted with DCM/MeOH (20/1 v/v, 20 mL x 3). The combined organic layers were concentrated to give the desired product (2.1 g, 78% yield) as orange solid. LC-MS (ESI): [M+H]⁺ = 357.3; ¹H NMR (400 MHz, CDCl₃) δ 7.78 (s, 1H), 7.55-7.30 (m, 2H), 7.18 - 7.10 (m, 4H), 5.28 (s, 2H), 3.95 (s, 3H), 3.54 (s, 3H), 2.33 (s, 3H).

### Step 4: Synthesis of (E)-2-(2-((2-(3-methoxy-4-(methoxymethoxy)styryl)-5-methylbenzo[d]thiazol-6-yl)amino)ethoxy)ethan-1-ol

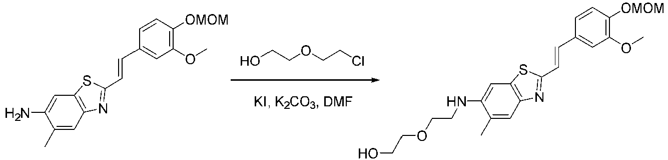

To a solution of (*E*)-2-(3-methoxy-4-(methoxymethoxy)styryl)-5-methylbenzo[*d*]thiazol-6-amine (1.6 g, 4.49 mmol) in DMF (20 mL) was added K₂CO₃ (1.86 g, 13.48 mmol), KI (746 mg, 4.49 mmol), and 2-(2-chloroethoxy)ethan-1-ol (2.80 g, 22.47 mmol). The resulting mixture was stirred at 100 °C for 48 h under N₂. After being cooled to room temperature, the solvent was removed. The residue was purified by column chromatography on silica gel (DCM: EA=1:1) to give the desired product (1.0 g, 38% yield) as red oil. LC-MS (ESI): [M+H]⁺ = 445.3; ¹H NMR (400 MHz, CDCl₃) δ 7.70 (s, 1H), 7.31 (d, *J* = 4.8 Hz, 2H), 7.15 (d, *J* = 8.4 Hz, 1H), 7.12 (d, *J =* 1.6 Hz, 1H), 7.09-7.08 (m, 1H), 7.03 (s, 1H), 5.27 (s, 2H), 3.93 (s, 3H), 3.86 - 3.76 (m, 4H), 3.68 - 3.63 (m, 2H), 3.53 (s, 3H), 3.43 (t, *J =* 5.2 Hz, 2H), 2.31 (s, 3H).

### Step 5: Synthesis of (E)-2-(2-((2-(3-methoxy-4-(methoxymethoxy)styryl)-5-methylbenzo[d]thiazol-6-yl)(methyl)amino)ethoxy)ethan-1-ol

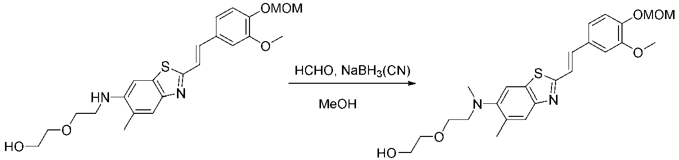

A solution of (*E*)-2-(2-((2-(3-methoxy-4-(methoxymethoxy)styryl)-5-methylbenzo[d]thiazol-6-yl)amino)ethoxy)ethan-1-ol (900 mg, 2.03 mmol), aqueous formaldehyde (40% wt, 304 mg, 2.03 mmol), and glacial acetic acid (0.2 mL) in MeOH (10 mL) was stirred at 0 °C for 30 min. Then NaBH₃CN (255 mg, 4.05 mmol) was added, the resulting mixture was stirred at room temperature for 12 h. The solvent was removed. The residue was purified by column chromatography on silica gel (DCM: EA=1:1) to give the desired product (600 mg, 60% yield) as yellow solid. LC-MS (ESI): [M+H]⁺ = 459.4; ¹H NMR (400 MHz, CDCl₃) δ 7.85 (s, 1H), 7.52-7.46 (m, 1H), 7.31-7.28 (m, 2H), 7.20-7.18 (m, 1H), 7.14-7.12 (m, 2H), 5.28 (s, 2H), 3.95 (s, 3H), 3.73-3.68 (m, 4H), 3.54 (s, 3H), 3.53 (s, 3H), 3.35-2.95 (m, 4H), 2.64 (s, 3H).

### Step 6: Synthesis of (E)-2-(2-((2-(3-methoxy-4-(methoxymethoxy)styryl)-5-methylbenzo[d]thiazol-6-yl)(methyl)amino)ethoxy)ethyl 4-methylbenzenesulfonate

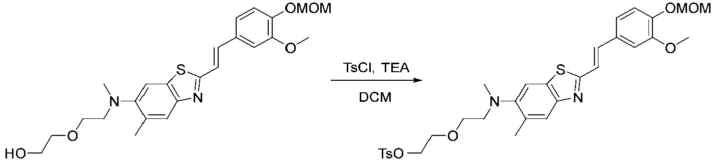

To a solution of (*E*)-2-(2-((2-(3-methoxy-4-(methoxymethoxy)styryl)-5-methylbenzo[d]thiazol-6-yl)(methyl)amino)ethoxy)ethan-1-ol (550 mg, 1.20 mmol) and TEA (485 mg, 4.80 mmol) in DCM (6 mL) was added TsCl (456 mg, 2.40 mmol) at 0 °C. The resulting mixture was stirred at room temperature for 16 h under N₂. The solvent was removed. The residue was purified by column chromatography on silica gel (PE: EA= 3: 1) to give the desired product (650 mg, 88% yield) as yellow solid. LC-MS (ESI): [M+H]⁺ =613.3; ¹H NMR (400 MHz, DMSO-*d₆*) δ 7.77 (d, *J* = 8.4 Hz, 2H), 7.71 (d, *J* = 6.4 Hz, 2H), 7.50 (d, *J* = 4.0 Hz, 2H), 7.45 (dd, *J* = 5.2, 2.8 Hz, 3H), 7.25 (dd, *J* = 8.4, 1.6 Hz, 1H), 7.09 (d, *J =* 8.4 Hz, 1H), 5.20 (s, 2H), 4.14 - 4.07 (m, 2H), 3.86 (s, 3H), 3.58 - 3.52 (m, 2H), 3.49 (t, *J* = 6.0 Hz, 2H), 3.40 (s, 3H), 3.00 (t, *J =* 6.0 Hz, 2H), 2.70 (s, 3H), 2.53 - 2.47 (m, 2H), 2.38 (s, 3H), 2.33 (s, 3H).

### Step 7: Synthesis of (E)-N-(2-(2-fluoroethoxy)ethyl)-2-(3-methoxy-4-(methoxymethoxy)styryl)-N,5-dimethylbenzo[d]thiazol-6-amine

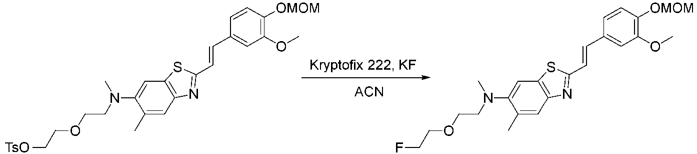

A solution of (*E*)-2-(2-((2-(3-methoxy-4-(methoxymethoxy)styryl)-5-methylbenzo[*d*]thiazol-6-yl)(methyl)amino)ethoxy)ethyl 4-methylbenzenesulfonate (300 mg, 0.49 mmol), Kryptofix 222 (462 mg, 1.22 mmol), and KF (284 mg, 4.90 mmol) in ACN (3 mL) was stirred at 60 °C for 2 h under N₂. After being cooled to room temperature, the solvent was removed. The residue was purified by column chromatography on silica gel (PE: EA=5:1) to give the desired product (112 mg, 50% yield) as yellow oil. LC-MS (ESI): [M+H]⁺ =461.3; ¹H NMR (400 MHz, CDCl₃) δ 7.76 (s, 1H), 7.53 (s, 1H), 7.38 (d, *J =* 16. Hz, 1H), 7.29-7.25 (m, 1H), 7.17 (d, *J =* 8.0 Hz, 1H), 7.13 (d, *J =* 1.8 Hz, 1H), 7.12 - 7.08 (m, 1H), 5.27 (s, 2H), 4.64 - 4.53 (m, 1H), 4.53 - 4.40 (m, 1H), 3.94 (s, 3H), 3.72 - 3.62 (m, 4H), 3.52 (s, 3H), 3.22 (t, *J* = 6.0 Hz, 2H), 2.84 (s, 3H), 2.44 (s, 3H).

### Step 8: Synthesis of (E)-4-(2-(6-((2-(2-fluoroethoxy)ethyl)(methyl)amino)-5-methylbenzo[d]thiazol-2-yl)vinyl)-2-methoxyphenol

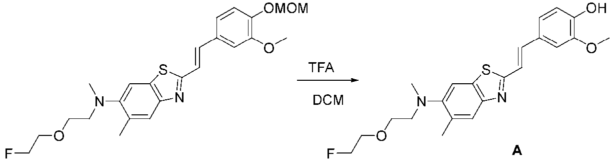

To a solution of (*E*)-*N*-(2-(2-fluoroethoxy)ethyl)-2-(3-methoxy-4-(methoxymethoxy)styryl)-*N*,5-dimethylbenzo[d]thiazol-6-amine (112 mg, 0.24 mmol) in DCM (2.5 mL) was added TFA (0.5 mL). The resulting mixture was stirred at room temperature for 2 h. Then the solvent was removed. The residue was dissolved in MeOH and purified by MPLC (ACN/Water) to give compound A (48 mg, 47.5% yield) as yellow oil. LC-MS (ESI): [M+H]⁺ = 417.3; ¹H NMR (400 MHz, DMSO-*d₆*) δ 9.45 (s, 1H), 7.72 (d, *J* = 11.2 Hz, 2H), 7.41 (m, 2H), 7.37 (d, *J =* 1.6 Hz, 1H), 7.14 (dd, *J* = 8.0, 2.0 Hz, 1H), 6.80 (d, *J* = 8.0 Hz, 1H), 4.61 - 4.53 (m, 1H), 4.48 - 4.41 (m, 1H), 3.85 (s, 3H), 3.69 - 3.65 (m, 1H), 3.63 - 3.56 (m, 3H), 3.10 (t, *J* = 6.0 Hz, 2H), 2.75 (s, 3H), 2.37 (s, 3H); ¹⁹F NMR (376 MHz, DMSO-*d₆*) δ - 221.35.

### Another Method for Preparing Compound B

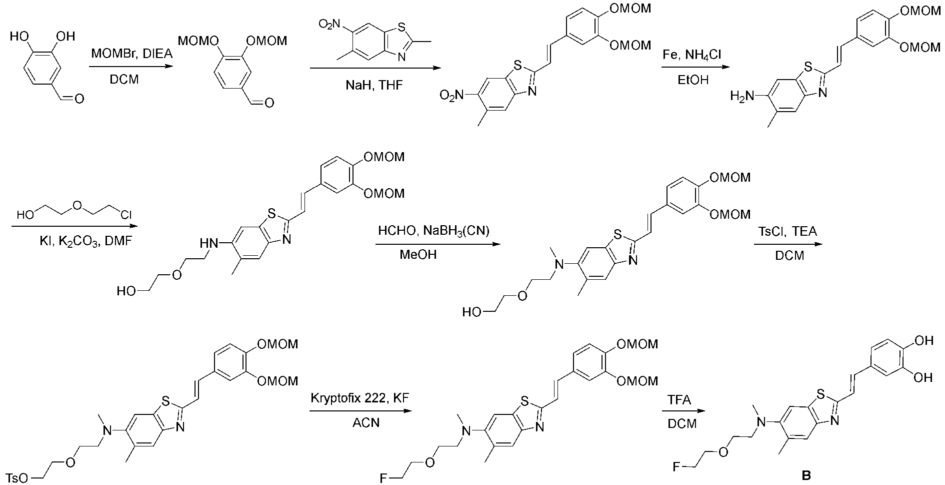

### Synthesis of (E)-4-(2-(6-((2-(2-fluoroethoxy)ethyl)(methyl)amino)-5-methylbenzo[d]thiazol-2-yl)vinyl)benzene-1,2-diol

### Step 1: Synthesis of 3,4-bis(methoxymethoxy)benzaldehyde

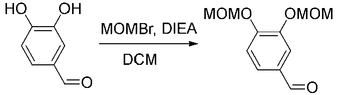

To a solution of 3,4-dihydroxybenzaldehyde (3 g, 27.74 mmol) and DIEA (11.2 g, 86.95 mmol) in DCM (40 mL) was added MOMBr (4.2 mL, 52.17 mmol, density 1.54 g/cm³) dropwise at 0 °C. The resulting mixture was stirred at room temperature for 16 h under N₂. After the reaction was complete, the solvent was removed. The residue was purified by column chromatography on silica gel (PE: EA=3:1) to give the desired product (4.4 g, 89% yield) as yellow oil. LC-MS (ESI): [M+H]⁺ =227.2; ¹H NMR (400 MHz, CDCl₃) δ 9.87 (s, 1H), 7.68 (d, *J =* 2.0 Hz, 1H), 7.51 (dd, *J =* 8.4, 2.0 Hz, 1H), 7.29 (d, *J* = 8.4 Hz, 1H), 5.33 (s, 2H), 5.30 (s, 2H), 3.53 (s, 3H), 3.52 (s, 3H).

### Step 2: Synthesis of (E)-2-(3,4-bis(methoxymethoxy)styryl)-5-methyl-6-nitrobenzo[d]thiazole

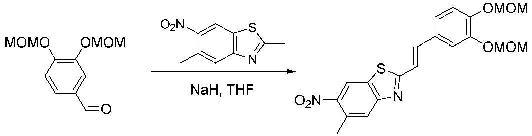

To a solution of 2,5-dimethyl-6-nitrobenzo[d]thiazole (1.67 g, 8.04 mmol) in THF (40 mL) was added NaH (643 mg, 16.09 mmol, wt: 60%) at 0 °C. The resulting mixture was stirred at 0 °C for 40 min under N₂. Then a solution of 3,4-bis(methoxymethoxy)benzaldehyde (2 g, 8.84 mmol) in THF (5 mL) was added to the mixture. After being stirred at room temperature for 4 h under N₂, the mixture was quenched by saturated aqueous NH₄Cl, and filtered. The filter cake was washed with EtOAc (20 mL) and dried to give the desired product (2.7 g, 80% yield) as orange solid. LC-MS (ESI): [M+H]⁺ =417.3; ¹H NMR (400 MHz, DMSO-*d₆*) δ 8.89 (s, 1H), 8.01 (s, 1H), 7.71 (d, *J =* 16 Hz, 1H), 7.56 (d, *J* = 16 Hz, 2H), 7.42-7.41 (m, 1H), 7.17 (d, *J* = 7.6 Hz, 1H), 5.27 (d, *J =* 6.4 Hz, 4H), 3.44 (s, 3H), 3.41 (s, 3H), 2.64 (s, 3H).

### Step 3: Synthesis of (E)-2-(3,4-bis(methoxymethoxy)styryl)-5-methylbenzo[d]thiazol-6-amine

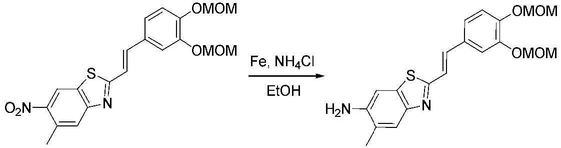

To a solution of (*E*)-2-(3,4-bis(methoxymethoxy)styryl)-5-methyl-6-nitrobenzo[*d*]thiazole (2.70 g, 6.49 mmol) in EtOH (50 mL) was added a solution of NH₄Cl (3.44 g, 64.90 mmol, 5 mL) and iron powder (1.8 g, 32.45 mmol) at room temperature. The resulting mixture was stirred at 80 °C for 2 h. After being cooled to room temperature, the mixture was filtered, and the filtrate was concentrated. The residue was dissolved in H₂O (20 mL) and extracted with DCM/MeOH = 20/1 (v/v, 20 mL x 3). The combined organic layers were concentrated to give the desired product (2.0 g, 80% yield) as orange solid. LC-MS (ESI): [M+H]⁺ =387.3; ¹H NMR (400 MHz, CDCl₃) δ 7.75 (s, 1H), 7.40-7.30 (m, 2H), 7.26-7.18 (m, 4H), 5.28 (d*, J =* 2.4 Hz, 4H), 3.53 (s, 6H), 2.34 (s, 3H).

### Step 4: Synthesis of (E)-2-(2-((2-(3,4-bis(methoxymethoxy)styryl)-5-methylbenzo[d]thiazol-6-yl)amino)ethoxy)ethan-1-ol

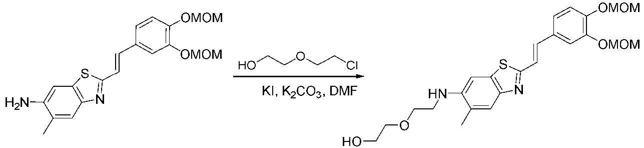

To a solution of (*E*)-2-(3,4-bis(methoxymethoxy)styryl)-5-methylbenzo[*d*]thiazol-6-amine (1.8 g, 4.66 mmol) in DMF (20 mL) was added K₂CO₃ (1.93 g, 13.98 mmol), KI (774 mg, 4.66 mmol), and 2-(2-chloroethoxy)ethan-1-ol (2.91 g, 23.31 mmol). The resulting mixture was stirred at 100 °C for 48 h under N₂. After being cooled to room temperature, the solvent was removed. The residue was purified by column chromatography on silica gel (DCM: EA=1:1) to give the desired product (1.1 g, 45% yield) as red oil. LC-MS (ESI): [M+H]⁺ =475.3; ¹H NMR (400 MHz, CDCl₃) δ 7.66 (s, 1H), 7.40 (s, 1H), 7.27 (S, 2H), 7.16 (s, 2H), 6.99 (s, 1H), 5.28 (d, *J* = 2.4 Hz, 4H), 3.85 - 3.76 (m, 4H), 3.66 - 3.63 (m, 2H), 3.54 (s, 3H), 3.53 (s, 3H), 3.42 (t, *J =* 5.2 Hz, 2H), 2.28 (s, 3H).

### Step 5: Synthesis of (E)-2-(2-((2-(3,4-bis(methoxymethoxy)styryl)-5-methylbenzo[d]thiazol-6-yl)(methyl)amino)ethoxy)ethan-1-ol

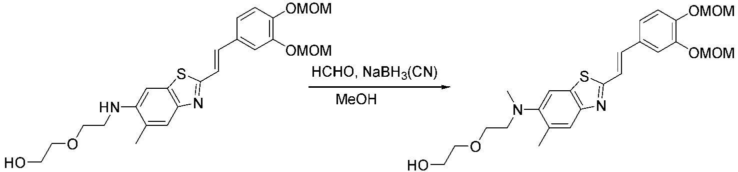

A solution of (*E*)-2-(2-((2-(3,4-bis(methoxymethoxy)styryl)-5-methylbenzo[d]thiazol-6-yl)amino)ethoxy)ethan-1-ol (1 g, 2.10 mmol), aqueous formaldehyde (316 mg, 2.10 mmol, 40% wt), and glacial acetic acid (0.2 mL) in MeOH (10 mL) was stirred at 0 °C for 30 min. Then NaBH₃CN (265 mg, 4.21 mmol) was added, the resulting mixture was stirred at room temperature for 12 h. The solvent was removed. The residue was purified by column chromatography on silica gel (DCM: EA=1:1) to give the desired product (700 mg, 63% yield) as yellow solid. LC-MS (ESI): [M+H]⁺ = 489.4; ¹H NMR (400 MHz, CDCl₃) δ 7.81 (s, 1H), 7.45-7.38 (m, 2H), 7.30-7.26 (m, 2H), 7.19 (s, 2H), 5.28 (d, *J =* 2.4 Hz, 4H), 3.78 - 3.63 (m, 4H), 3.54 (m, 9H), 3.38-2.75 (m, 4H), 2.52 (s, 3H).

### Step 6: Synthesis of (E)-2-(2-((2-(3,4-bis(methoxymethoxy)styryl)-5-methylbenzo[d]thiazol-6-yl)(methyl)amino)ethoxy)ethyl 4-methylbenzenesulfonate

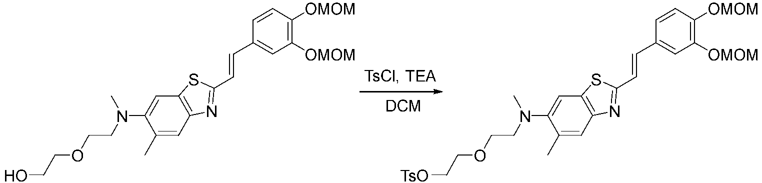

To a solution of (*E*)-2-(2-((2-(3,4-bis(methoxymethoxy)styryl)-5-methylbenzo[*d*]thiazol-6-yl)(methyl)amino)ethoxy)ethan-1-ol (566 mg, 1.14 mmol) and TEA (463 mg, 4.59 mmol) in DCM (6 mL) was added TsCl (436 mg, 2.29 mmol) at 0 °C. The resulting mixture was stirred at room temperature for 16 h under N₂. The solvent was removed. The residue was purified by column chromatography on silica gel (PE:EA=3:1) to give the desired product (650 mg, 88% yield) as yellow solid. LC-MS (ESI): [M+H]⁺ = 643.3; ¹H NMR (400 MHz, DMSO-*d₆*) δ 7.77 (d, *J* = 8.0 Hz, 2H), 7.71 (d, *J* = 6.4 Hz, 2H), 7.52 (d, *J* = 1.6 Hz, 1H), 7.50 - 7.41 (m, 4H), 7.35 (dd, *J* = 8.4, 1.6 Hz, 1H), 7.14 (d, *J* = 8.4 Hz, 1H), 5.26 (d, *J* = 13.2 Hz, 4H), 4.15 - 4.05 (m, 2H), 3.59 - 3.53 (m, 2H), 3.49 (t, *J* = 6.0 Hz, 2H), 3.44 (s, 3H), 3.41 (s, 3H), 3.00 (t, *J =* 6.0 Hz, 2H), 2.70 (s, 3H), 2.38 (s, 3H), 2.33 (s, 3H).

### Step 7: Synthesis of (E)-2-(3,4-bis(methoxymethoxy)styryl)-N-(2-(2-fluoroethoxy)ethyl)-N,5-dimethylbenzo[d]thiazol-6-amine

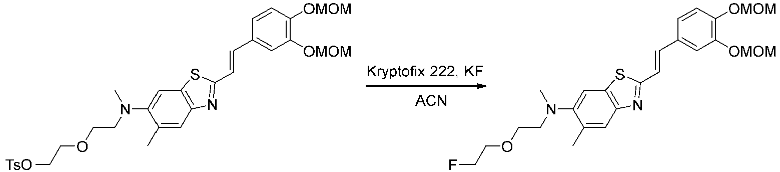

A solution of (*E*)-2-(2-((2-(3,4-bis(methoxymethoxy)styryl)-5-methylbenzo[d]thiazol-6-yl)(methyl)amino)ethoxy)ethyl 4-methylbenzenesulfonate (300 mg, 0.46 mmol), Kryptofix 222 (440 mg, 1.17 mmol), and KF (271 mg, 4.67 mmol) in ACN (3 mL) was stirred at 60 °C for 2 h under N₂. After being cooled to room temperature, the solvent was removed. The residue was purified by column chromatography on silica gel (PE: EA=5: 1) to give the desired product (450 mg, 52% yield) as yellow oil. LC-MS (ESI): [M+H]⁺ = 491.4; ¹H NMR (400 MHz, CDCl₃) δ 7.72 (s, 1H), 7.50 (s, 1H), 7.38 (s, 1H), 7.33 (d, *J =* 16.0 Hz, 1H), 7.26-7.22 (m, 1H), 7.14 (s, 2H), 5.24 (d, *J =* 3.6 Hz, 4H), 4.60 - 4.50 (m, 1H), 4.47 - 4.39 (m, 1H), 3.69 - 3.58 (m, 4H), 3.51 (s, 3H), 3.49 (s, 3H), 3.20-3.14 (m, 2H), 2.81 (s, 3H), 2.41 (s, 3H).

### Step 8: Synthesis of (E)-4-(2-(6-((2-(2-fluoroethoxy)ethyl)(methyl)amino)-5-methylbenzo[d]thiazol-2-yl)vinyl)benzene-1,2-diol

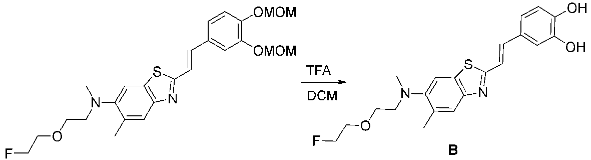

To a solution of (*E*)-2-(3,4-bis(methoxymethoxy)styryl)-*N*-(2-(2-fluoroethoxy)ethyl)-*N*,5-dimethylbenzo[*d*]thiazol-6-amine (120 mg, 0.24 mmol) in DCM (2.5 mL) was added TFA (0.5 mL). The resulting mixture was stirred at room temperature for 2 h. Then the solvent was removed. The residue was purified by MPLC(ACN/Water) to give compound B (47 mg, 47.9% yield) as yellow oil. LC-MS (ESI): [M+H]⁺ = 403.3;¹H NMR (400 MHz, DMSO-*d₆*) δ 9.42 (s, 1H), 9.11 (s, 1H), 7.71 (d, *J* = 8.8 Hz, 2H), 7.35 (d, *J =* 16.0 Hz, 1H), 7.18 (d, *J* = 16.0 Hz, 1H), 7.09 (d, *J =* 2.0 Hz, 1H), 7.02 (dd, *J* = 8.0, 2.0 Hz, 1H), 6.77 (d, *J =* 8.0 Hz, 1H), 4.61 - 4.51 (m, 1H), 4.47 - 4.41 (m, 1H), 3.71 - 3.64 (m, 1H), 3.63 - 3.56 (m, 3H), 3.10 (t, *J =* 6.0 Hz, 2H), 2.75 (s, 3H), 2.36 (s, 3H); ¹⁹F NMR (376 MHz, DMSO-*d₆*) δ -221.35.

### Example 4: Synthesis of Compound C

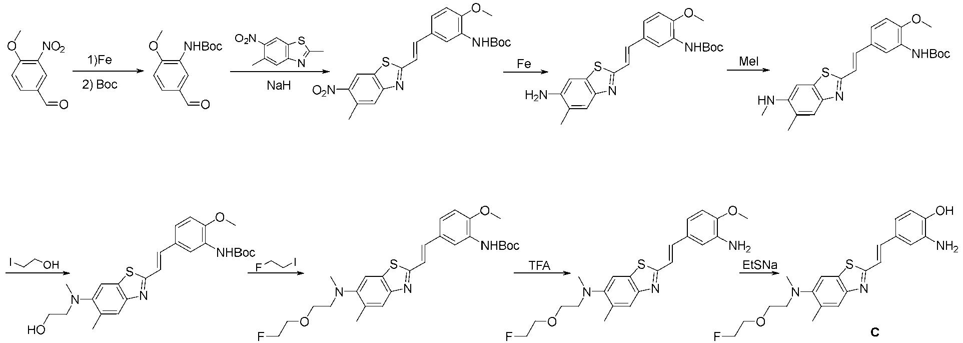

Compound C was synthesized following the procedure of Example 1 and Example 2.

Compound C: LC-MS (ESI): [M+H]⁺ = 402.2.

### Another Method for Preparing Compound C

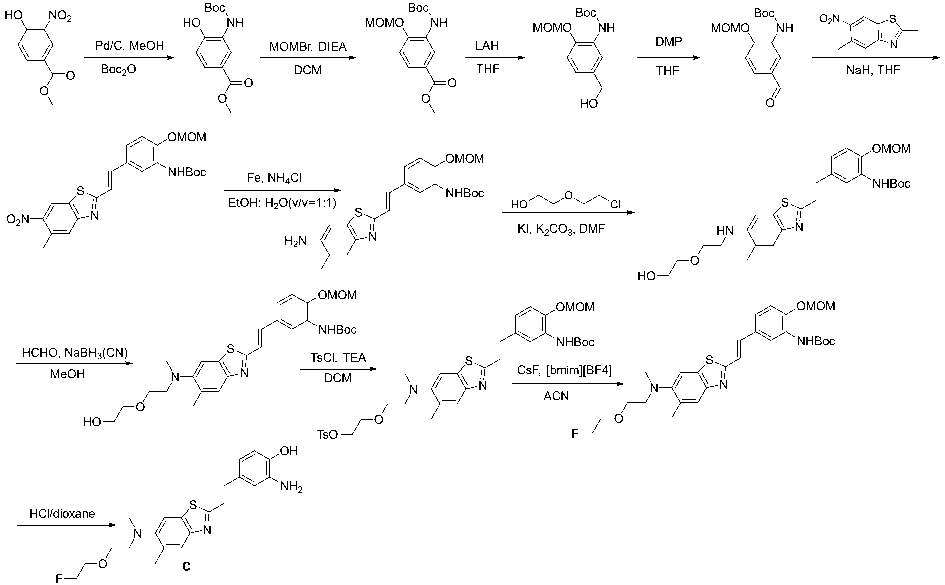

### Synthesis of (E)-2-amino-4-(2-(6-((2-(2-fluoroethoxy)ethyl)(methyl)amino)-5-methylbenzo[d]thiazol-2-yl)vinyl)phenol

### Step 1: Synthesis of methyl 3-((tert-butoxycarbonyl)amino)-4-hydroxybenzoate

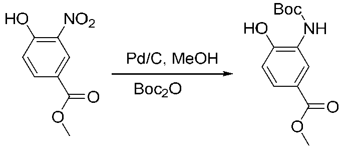

To a solution of methyl 4-hydroxy-3-nitrobenzoate (10 g, 50.76 mmol) in MeOH (60 mL) was added Pd/C (1 g) and Boc₂O (16.6 g, 76.14 mmol). The resulting mixture was stirred at room temperature for 16 h under H₂. After the reaction was complete, the mixture was filtered, and the filtrate was concentrated to give the desired product (13.1 g, 97% yield) as yellow oil. LC-MS (ESI): [M+H-56]⁺ = 212.1.

### Step 2: Synthesis of methyl 3-((tert-butoxycarbonyl)amino)-4-(methoxymethoxy)benzoate

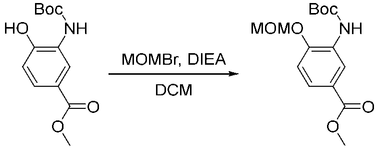

To a solution of methyl 3-((*tert*-butoxycarbonyl)amino)-4-hydroxybenzoate (13.1 g, 49.06 mmol) and DIEA (12.8 mL, 73.59 mmol) in DCM (100 mL) was added MOMBr (4.81 mL, 58.88 mmol, density 1.54 g/cm³) dropwise at 0 °C. The resulting mixture was stirred at room temperature for 2 h under N₂. After the reaction was complete, the mixture was washed with H₂O (30 mL) and extracted with DCM (100 mL x 3). The combined organic layers were dried over anhydrous Na₂SO₄ and filtered. The filtrate was concentrated to give the residue, which was purified by column chromatography on silica gel (PE: EA=5:1) to give the desired product (13.5 g, 88.2% yield) as white solid. LC-MS (ESI): [M+H-56]⁺ =256.1; ¹H NMR (400 MHz, DMSO-*d₆*) δ 8.39 (d, *J =* 2.0 Hz, 1H), 8.25 (s, 1H), 7.65 (dd, *J =* 8.8, 2.4 Hz, 1H), 7.19 (d, *J =* 8.4 Hz, 1H), 5.30 (s, 2H), 3.83 (s, 3H), 3.42 (s, 3H), 1.49 (s, 9H).

### Step 3: Synthesis of tert-butyl (5-(hydroxymethyl)-2-(methoxymethoxy)phenyl)carbamate

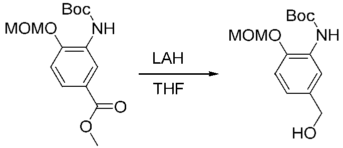

LiAlH₄ (3.3 g, 86.82 mmol) was added in portions to a stirred solution of methyl 3-((*tert-*butoxycarbonyl)amino)-4-(methoxymethoxy)benzoate (13.5 g, 43.41 mmol) in THF (200 mL) at 0 °C. The mixture was stirred at room temperature for 3 h. The mixture was quenched by H₂O (50 mL), and extracted with DCM (30 mL x 3). The combined organic layers were dried over anhydrous Na₂SO₄ and filtered. The filtrate was concentrated to give the residue, which was purified by column chromatography on silica gel (PE: EA=5: 1) to give the desired product (8.87 g, 72.2% yield) as yellow oil. LC-MS (ESI): [M+H-56]⁺ =228.1; ¹H NMR (400 MHz, DMSO-*d₆*) δ 7.97 (s, 1H), 7.69 (s, 1H), 7.04 (d, *J =* 8.4 Hz, 1H), 6.93 (dd, *J* = 8.4, 2.4 Hz, 1H), 5.17 (s, 2H), 5.09 (t, *J* = 5.6 Hz, 1H), 4.40 (d, *J* = 5.6 Hz, 2H), 3.40 (s, 3H), 1.47 (s, 9H).

### Step 4: Synthesis of tert-butyl (5-formyl-2-(methoxymethoxy)phenyl)carbamate

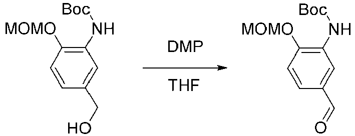

To a solution of *tert-*butyl (5-(hydroxymethyl)-2-(methoxymethoxy)phenyl)carbamate (8.87 g, 35.06 mmol) in DCM (100 mL) was added DMP (29.73 g, 70.12 mmol) in batches at 0 °C. Then the mixture was stirred at room temperature for 20 h. The mixture was washed with saturated aqueous NaHCO₃ (50 mL), and extracted with DCM (100 mL x 3). The combined organic layers were dried over anhydrous Na₂SO₄ and filtered. The filtrate was concentrated to give the residue, which was purified by column chromatography on silica gel (PE: EA=5:1) to give the desired product (5.8 g, 65.9% yield) as yellow solid. LC-MS (ESI): [M+H-56]⁺ =226.1; ¹H NMR (400 MHz, DMSO-*d₆*) δ 9.86 (s, 1H), 8.34 (s, 1H), 8.29 (d, *J* = 2.4 Hz, 1H), 7.61 (dd, *J* = 8.4, 2.0 Hz, 1H), 7.28 (d, *J* = 8.4 Hz, 1H), 5.34 (s, 2H), 3.43 (s, 3H), 1.49 (s, 9H).

### Step 5: Synthesis of tert-butyl (E)-(2-(methoxymethoxy)-5-(2-(5-methyl-6-nitrobenzo[d]thiazol-2-yl)vinyl)phenyl)carbamate

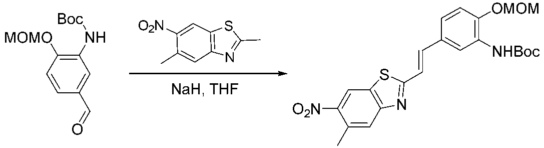

To a solution of 2,5-dimethyl-6-nitrobenzothiazole (5.46 g, 26.20 mmol) in THF (100 mL) was added NaH (2.38 g, 59.61 mmol, wt: 60%) at 0 °C. After being stirred at room temperature for 30 min, *tert*-butyl (5-formyl-2-(methoxymethoxy)phenyl)carbamate (6.7 g, 23.82 mmol) was added. The mixture was stirred for 4 h at room temperature. The mixture was quenched by water (50 mL) and extracted with EtOAc (100 mL x 3). The combined organic layers were dried over anhydrous Na₂SO₄, filtered, and concentrated. The residue was purified by column chromatography on silica gel (PE:EA=1:1) to give the desired product (6.8 g, 60.5% yield) as black solid. LC-MS (ESI): [M+H-56]⁺ =472.3; ¹H NMR (400 MHz, DMSO-*d₆*) δ 8.89 (s, 1H), 8.20 (s, 1H), 8.09 (s, 1H), 7.73 (d, *J* = 16.0 Hz, 1H), 7.51 (dd, *J =* 8.4, 2.0 Hz, 1H), 7.46 (d, *J* = 16.0 Hz, 1H), 7.17 (d, *J =* 8.8 Hz, 1H), 5.28 (s, 2H), 3.43 (s, 3H), 2.65 (s, 3H), 1.50 (s, 9H).

### Step 6: Synthesis of tert-butyl (E)-(5-(2-(6-amino-5-methylbenzo[d]thiazol-2-yl)vinyl)-2-(methoxymethoxy)phenyl)carbamate

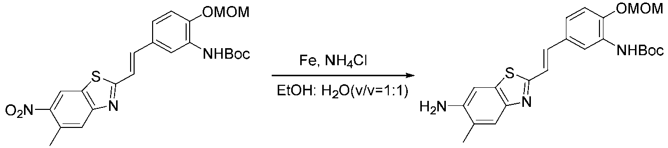

To a solution of *tert*-butyl (*E*)-(2-(methoxymethoxy)-5-(2-(5-methyl-6-nitrobenzo[*d*]thiazol-2-yl)vinyl)phenyl)carbamate (3 g, 6.37 mmol) in EtOH and H₂O (40 mL, v/v=1:1) was added NH₄Cl (3.44 g, 63.69 mmol) and iron powder (3.57 g, 63.69 mmol) at room temperature. The resulting mixture was stirred at 80 °C for 20 h. After being cooled to room temperature, the mixture was filtered. The filtrate was concentrated to give the desired product (1.39 g, 49.5% yield) as brown solid. LC-MS (ESI): [M+H-56]⁺ = 442.4; ¹H NMR (400 MHz, DMSO-*d₆*) δ 8.15 (s, 1H), 7.99 (s, 1H), 7.53 (s, 1H), 7.39 (dd, *J =* 8.8, 2.4 Hz, 1H), 7.28 (d, *J* = 7.6 Hz, 2H), 7.13 (d, *J* = 8.4 Hz, 1H), 7.10 (s, 1H), 5.25 (s, 2H), 3.42 (s, 3H), 2.19 (s, 3H), 1.50 (s, 9H).

### Step 7: Synthesis of tert-butyl (E)-(5-(2-(6-((2-(2-hydroxyethoxy)ethyl)amino)-5-methylbenzo[d]thiazol-2-yl)vinyl)-2-(methoxymethoxy)phenyl)carbamate

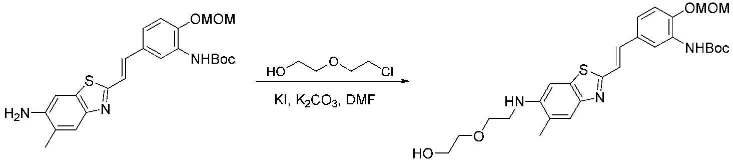

To a solution of *tert-*butyl (*E*)-(5-(2-(6-amino-5-methylbenzo[*d*]thiazol-2-yl)vinyl)-2-(methoxymethoxy)phenyl)carbamate (1.18 g, 2.67 mmol) in DMF (16 mL) was added K₂CO₃ (1.11 g, 8.03 mmol), KI (0.44 g, 2.67 mmol), and 2-(2-chloroethoxy)ethan-1-ol (3.32 g, 26.76 mmol). The resulting mixture was stirred at 100 °C for 48 h. After being cooled to room temperature, H₂O (50 mL) was added. The mixture was extracted with EtOAc (50 mL x 3). The combined organic layers were dried over anhydrous Na₂SO₄, filtered, concentrated, and purified by column chromatography on silica gel (PE:EA=1:2) to give the desired product (0.65 g, 45.9% yield) as yellow oil. LC-MS (ESI): [M+H]⁺ = 530.4; ¹H NMR (400 MHz, DMSO-*d₆*) δ 8.15 (s, 1H), 8.00 (s, 1H), 7.96 (s, 1H), 7.58 (s, 1H), 7.40 (dd, *J* = 8.8, 2.4 Hz, 1H), 7.30 (d, *J* = 9.6 Hz, 1H), 7.13 (t, *J =* 5.2 Hz, 2H), 5.25 (s, 2H), 5.14 (t, *J* = 5.6 Hz, 1H), 4.65 (t, *J =* 5.2 Hz, 1H), 3.73 - 3.58 (m, 4H), 3.54 - 3.48 (m, 4H), 3.43 (s, 3H), 2.21 (s, 3H), 1.50 (s, 9H).

### Step 8: Synthesis of tert-butyl (E)-(5-(2-(6-((2-(2-hydroxyethoxy)ethyl)(methyl)amino)-5-methylbenzo[d]thiazol-2-yl)vinyl)-2-(methoxymethoxy)phenyl)carbamate

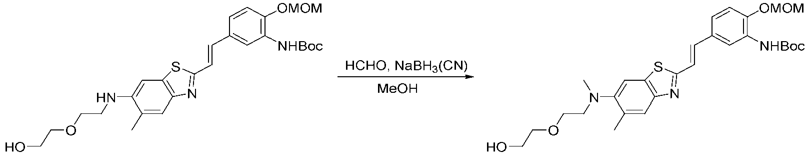

To a solution of *tert*-butyl (*E*)-(5-(2-(6-((2-(2-hydroxyethoxy)ethyl)amino)-5-methylbenzo[*d*]thiazol-2-yl)vinyl)-2-(methoxymethoxy)phenyl)carbamate (0.65 g, 1.23 mmol) in MeOH (15 mL) was added aqueous formaldehyde (73.7 mg, 2.46 mmol, 30% wt). After being stirred at 0 °C for 30 min, NaBH₃CN (2.08 g, 9.83 mmol) was added. The resulting mixture was stirred at room temperature for 20 h. The solvent was removed. The residue was purified by column chromatography on silica gel (PE:EA=1:2) to give the desired product (0.48 g, 71% yield) as yellow oil. LC-MS (ESI): [M+H]⁺ =544.4; ¹H NMR (400 MHz, DMSO-*d₆*) δ 8.16 (s, 1H), 8.05 (s, 1H), 7.73 (d, *J* = 3.2 Hz, 2H), 7.51 - 7.40 (m, 2H), 7.33 (d, *J =* 16.4 Hz, 1H), 7.14 (d, *J* = 8.8 Hz, 1H), 5.26 (s, 2H), 4.57 (t, *J =* 5.6 Hz, 1H), 3.58 (t, *J =* 6.0 Hz, 2H), 3.48 (q, *J =* 5.2 Hz, 2H), 3.45 - 3.38 (m, 5H), 3.09 (t, *J* = 6.0 Hz, 2H), 2.76 (s, 3H), 2.37 (s, 3H), 1.50 (s, 9H).

### Step 9: Synthesis of (E)-2-(2-((2-(3-((tert-butoxycarbonyl)amino)-4-(methoxymethoxy)styryl)-5-methylbenzo[d]thiazol-6-yl)(methyl)amino)ethoxy)ethyl 4-methylbenzenesulfonate

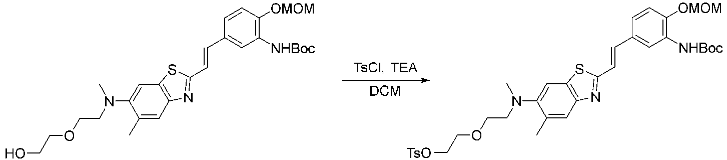

To a solution of *tert-*butyl (*E*)-(5-(2-(6-((2-(2-hydroxyethoxy)ethyl)(methyl)amino)-5-methylbenzo[*d*]thiazol-2-yl)vinyl)-2-(methoxymethoxy)phenyl)carbamate (0.48 g, 0.88 mmol) and TEA (356.5 mg, 3.53 mmol) in DCM (10 mL) was added TsCl (335.3 mg, 1.76 mmol) at 0 °C. The resulting mixture was stirred at room temperature for 20 h. The mixture was quenched by H₂O (50 mL) and extracted with EtOAc (100 mL x 3). The combined organic layers were concentrated, and purified by column chromatography on silica gel (PE:EA=1:2) to give the desired product (0.43 g, 58.6% yield) as yellow solid. LC-MS (ESI): [M+H]⁺ = 698.3; ¹H NMR (400 MHz, DMSO-*d₆*) δ 8.18 (s, 1H), 8.04 (s, 1H), 7.81 - 7.67 (m, 4H), 7.52 - 7.41 (m, 4H), 7.34 (d, *J =* 16.0 Hz, 1H), 7.15 (d, *J* = 8.4 Hz, 1H), 5.27 (s, 2H), 4.14 - 4.07 (m, 2H), 3.61 - 3.45 (m, 4H), 3.43 (s, 3H), 3.01 (t, *J =* 6.0 Hz, 2H), 2.71 (s, 3H), 2.40 (s, 3H), 2.33 (s, 3H), 1.50 (s, 9H).

### Step 10: Synthesis of tert-butyl (E)-(5-(2-(6-((2-(2-fluoroethoxy)ethyl)(methyl)amino)-5-methylbenzo[d]thiazol-2-yl)vinyl)-2-(methoxymethoxy)phenyl)carbamate

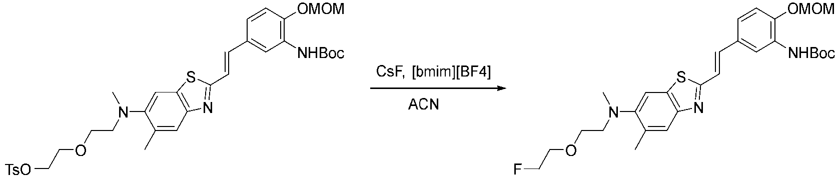

A solution of (*E*)-2-(2-((2-(3-((*tert*-butoxycarbonyl)amino)-4-(methoxymethoxy)styryl)-5-methylbenzo[*d*]thiazol-6-yl)(methyl)amino)ethoxy)ethyl 4-methylbenzenesulfonate (0.33 g, 0.47 mmol), [bmim][BF₄] (6 mL), and KF (359.8 mg, 2.37 mmol) in ACN (6 mL) was stirred at 100 °C for 5 h. After being cooled to room temperature, the solvent was removed. The residue was purified by column chromatography on silica gel (PE:EA=1:1) to give the desired product (160 mg, 62% yield) as yellow oil. LC-MS (ESI): [M+H]⁺ =546.4; ¹H NMR (400 MHz, DMSO-*d₆*) δ 8.17 (s, 1H), 8.03 (s, 1H), 7.74 (d, *J* = 8.8 Hz, 2H), 7.51 - 7.41 (m, 2H), 7.33 (d, *J =* 16.0 Hz, 1H), 7.15 (d, *J =* 8.4 Hz, 1H), 5.26 (s, 2H), 4.60 - 4.52 (m, 1H), 4.48 - 4.40 (m, 1H), 3.71 - 3.58 (m, 4H), 3.43 (s, 3H), 3.11 (t, *J =* 6.0 Hz, 2H), 2.76 (s, 3H), 2.38 (s, 3H), 1.50 (s, 9H).

### Step 11: Synthesis of (E)-2-amino-4-(2-(6-((2-(2-fluoroethoxy)ethyl)(methyl)amino)-5-methylbenzo[d]thiazol-2-yl)vinyl)phenol

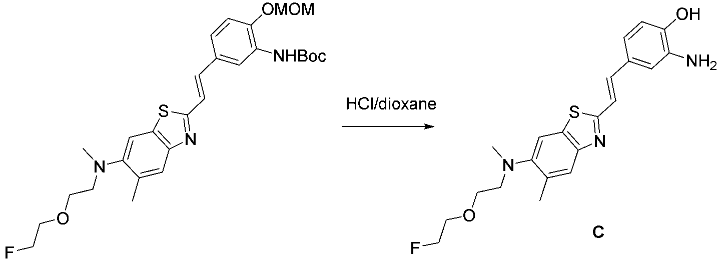

To a solution of *tert*-butyl (*E*)-(5-(2-(6-((2-(2-fluoroethoxy)ethyl)(methyl)amino)-5-methylbenzo[*d*]thiazol-2-yl)vinyl)-2-(methoxymethoxy)phenyl)carbamate (100 mg, 0.18 mmol) in 1,4-dioxane (1 mL) was added HCl/1,4-dioxane (4 M, 4 mL). The resulting mixture was stirred at room temperature for 4 h. The precipitated solid was filtered off, washed successively with EtOAc (20 ml) and Et₂O (10 mL) to give compound C (34.1 mg, 46.3% yield) as orange solid. LC-MS (ESI): [M+H]⁺ = 402.2; ¹H NMR (400 MHz, CD₃OD) δ 8.45 (s, 1H), 7.96 (s, 1H), 7.73 - 7.65 (m, 3H), 7.40 (d, *J =* 16.0 Hz, 1H), 7.09 (d, *J =* 8.0 Hz, 1H), 4.57 - 4.48 (m, 1H), 4.45 - 4.38 (m, 1H), 4.01 (t, *J =* 4.8 Hz, 2H), 3.65 - 3.44 (m, 4H), 3.40 (s, 3H), 2.68 (s, 3H); ¹⁹F NMR (376 MHz, CD₃OD) δ -221.33.

### Example 5: Synthesis of Compound D

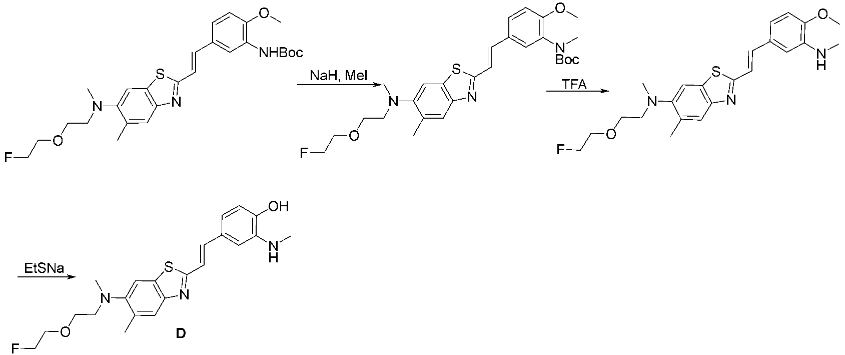

Compound D was synthesized following the procedure of Example 1 and Example 2.

Compound D: LC-MS (ESI): [M+H]⁺ = 416.2

### Another Method for Preparing Compound D

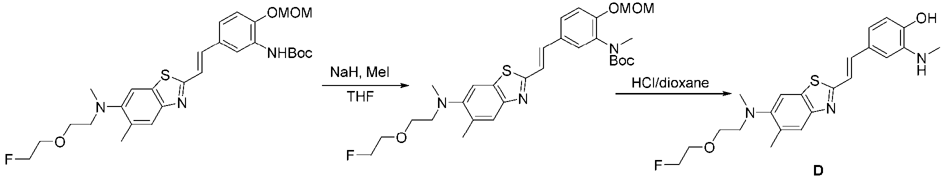

### Synthesis of (E)-4-(2-(6-((2-(2-fluoroethoxy)ethyl)(methyl)amino)-5-methylbenzo[d]thiazol-2-yl)vinyl)-2-(methylamino)phenol

### Step 1: Synthesis of tert-butyl (E)-(5-(2-(6-((2-(2-fluoroethoxy)ethyl)(methyl)amino)-5-methylbenzo[d]thiazol-2-yl)vinyl)-2-(methoxymethoxy)phenyl)(methyl)carbamate

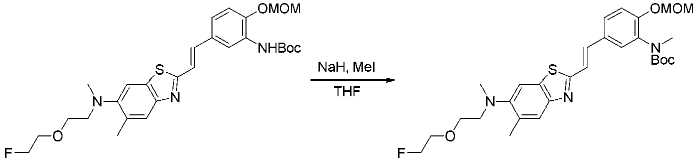

To a solution of *tert*-butyl (*E*)-(5-(2-(6-((2-(2-fluoroethoxy)ethyl)(methyl)amino)-5-methylbenzo[*d*]thiazol-2-yl)vinyl)-2-(methoxymethoxy)phenyl)carbamate (120 mg, 0.22 mmol) in DMF (3 mL) was added NaH (17.6 mg, 0.44 mmol, wt: 60%) at 0 °C. After being stirred for 20 min, CH₃I (62.6 mg, 0.44 mmol) was added. The resulting mixture was stirred at room temperature for 16 h. After the reaction was complete, the mixture was diluted with H₂O (30 mL) and extracted with EtOAc (20 mL x 3). The combined organic layers were dried over anhydrous Na₂SO₄ and filtered. The filtrate was concentrated to give the residue, which was purified by column chromatography on silica gel (PE: EA=5: 1) to give the desired product (89 mg, 72.3% yield) as white solid. LC-MS (ESI): [M+H]⁺ =560.4; ¹H NMR (400 MHz, DMSO-*d₆*) δ 7.75 (s, 1H), 7.74 (s, 1H), 7.70 - 7.58 (m, 2H), 7.48 (d, *J =* 2.4 Hz, 2H), 7.19 (d, *J =* 8.8 Hz, 1H), 5.28 (s, 2H), 4.61 - 4.38 (m, 2H), 3.74 - 3.56 (m, 4H), 3.41 (s, 3H), 3.19 - 2.95 (m, 5H), 2.76 (s, 3H), 2.38 (s, 3H), 1.49 (s, 9H).

### Step 2: Synthesis of (E)-4-(2-(6-((2-(2-fluoroethoxy)ethyl)(methyl)amino)-5-methylbenzo[d]thiazol-2-yl)vinyl)-2-(methylamino)phenol

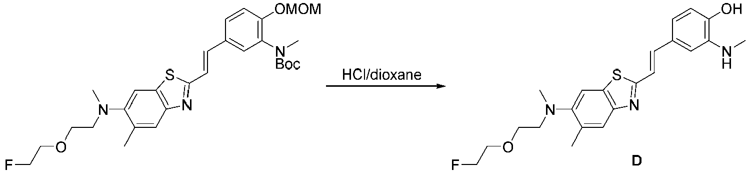

To a solution of *tert*-butyl (*E*)-(5-(2-(6-((2-(2-fluoroethoxy)ethyl)(methyl)amino)-5-methylbenzo[*d*]thiazol-2-yl)vinyl)-2-(methoxymethoxy)phenyl)(methyl)carbamate (89 mg, 0.16 mmol) in 1,4-dioxane (0.5 mL) was added HCl/1,4-dioxane (4 M, 2 mL). The resulting mixture was stirred at room temperature for 5 h. The precipitated solid was filtered off, washed successively with EtOAc (20 ml) and Et₂O (10 mL) to give compound D (34.4 mg, 52.1% yield) as orange solid. LC-MS (ESI): [M+H]⁺ = 416.2; ¹H NMR (400 MHz, CD₃OD) δ 8.44 (s, 1H), 7.96 (s, 1H), 7.78 - 7.66 (m, 3H), 7.44 (d*, J =* 16.4 Hz, 1H), 7.12 (d, *J =* 8.8 Hz, 1H), 4.57 - 4.37 (m, 2H), 4.01 (t*, J =* 5.0 Hz, 2H), 3.65 - 3.47 (m, 4H), 3.40 (s, 3H), 3.10 (s, 3H), 2.68 (s, 3H); ¹⁹F NMR (376 MHz, DMSO) δ -221.32.

### Example 6: Synthesis of Compound E and Compound F

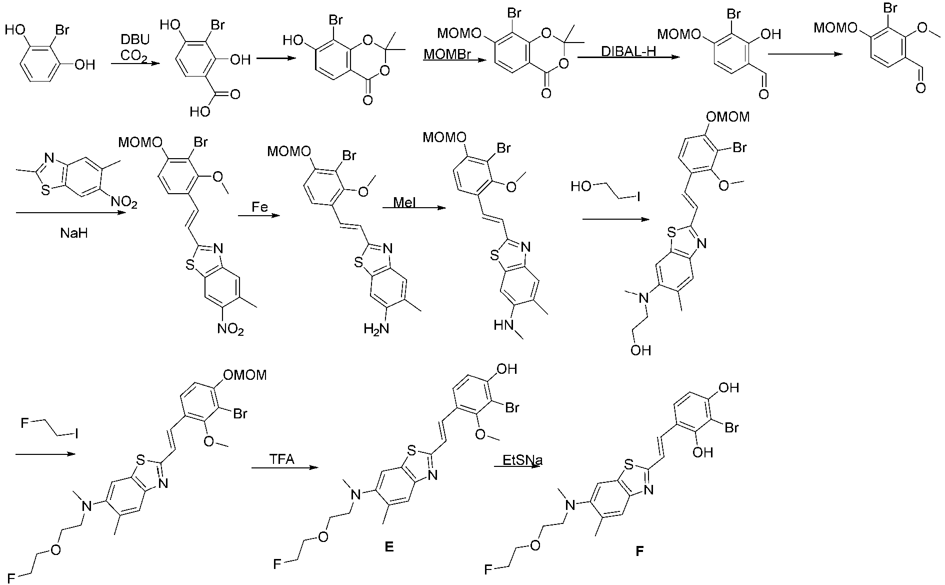

Compound E and Compound F were synthesized following the procedure of Example 1 and Example 2, respectively.

Compound E: LC-MS (ESI): [M+H]⁺ = 495.1, 497.1.

Compound F: LC-MS (ESI): [M+H]⁺ = 481.0, 483.0.

### Another Method for Preparing Compound F

### Synthesis of (E)-2-bromo-4-(2-(6-((2-(2-fluoroethoxy)ethyl)(methyl)amino)-5-methylbenzo[d]thiazol-2-yl)vinyl)-3-methoxyphenol

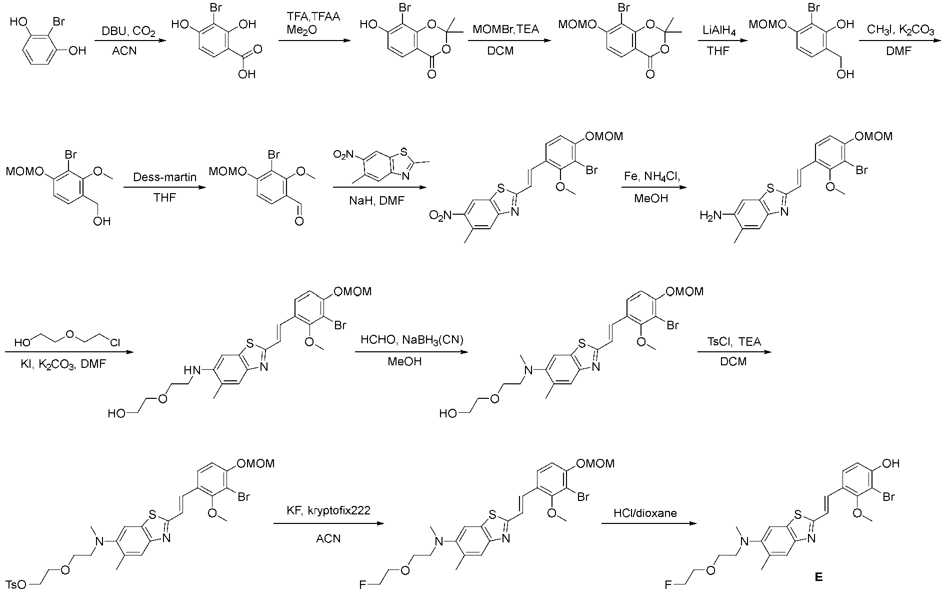

### Step 1: Synthesis of 3-bromo-2,4-dihydroxybenzoic acid

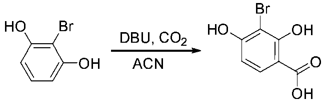

To a solution of 2-bromobenzene-1,3-diol (10.0 g, 52.91 mmol) in anhydrous ACN (200 mL) was added DBU (24.2 g, 158.73 mmol). Carbon dioxide gas was bubbled through the reaction mixture at room temperature for 24 h. After the reaction was complete, excess CO₂ was purged under a nitrogen stream. The mixture was adjusted to pH 5-6 with HCl (2 N), diluted with H₂O (200 mL), and extracted with EtOAc (100 mL x 3). The combined organic layers were washed with saturated brine (100 mL), dried over anhydrous Na₂SO₄, and filtered. The filtrate was concentrated. The residue was purified by column chromatography on silica gel (EtOAc: AcOH = 100:1) to give the desired product (11.2 g, 91% yield) as pink solid. ¹H NMR (400 MHz, DMSO-*d₆*) δ 13.81 (s, 1H), 12.28 (s, 1H), 11.21 (s, 1H), 7.64 (d, *J* = 8.8 Hz, 1H), 6.55 (d, *J* = 8.8 Hz, 1H).

### Step 2: Synthesis of 8-bromo-7-hydroxy-2,2-dimethyl-4H-benzo[d][1,3]dioxin-4-one

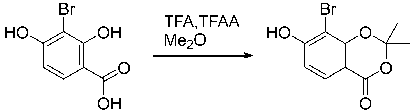

To a solution of 3-bromo-2,4-dihydroxybenzoic acid (5.0 g, 21.46 mmol) in TFA (39.1 g, 343.4 mmol) was added TFAA (23.0 g, 107.3 mmol) dropwise over 20 min at 0 °C, then acetone (6.2 g, 0.73 mmol) was added dropwise over 30 min at room temperature. After being stirred at 90 °C for 36 h under N₂, the solvent was removed. The residue was triturated with toluene (20 mL) and evaporated to dryness under vacuum. This trituration procedure was repeated three times. The residue was purified by column chromatography on silica gel (PE:EA = 22:78) to give the desired product (2.3 g, 39% yield) as white solid. LC-MS (ESI): [M-H]⁻ =271.0, 273.0; ¹H NMR (400 MHz, DMSO-*d₆*) δ 11.69 (s, 1H), 7.71 (d, *J =* 8.8 Hz, 1H), 6.79 (d, *J* = 8.4 Hz, 1H), 1.69 (s, 6H).

### Step 3: Synthesis of 8-bromo-7-(methoxymethoxy)-2,2-dimethyl-4H-benzo[d][1,3]dioxin-4-one

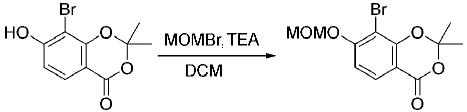

To a solution of 8-bromo-7-hydroxy-2,2-dimethyl-4*H*-benzo[*d*][1,3]dioxin-4-one (2.3 g, 8.42 mmol) and TEA (2.6 g, 25.26 mmol) in DCM (20 mL) was added MOMBr (2.1 g, 16.84 mmol) dropwise at 0 °C. After being stirred at room temperature for 16 h, the reaction mixture was diluted with water (50 mL) and extracted with EtOAc (50 mL x 3). The combined organic layers were washed with saturated brine (100 mL), dried over anhydrous Na₂SO₄, and filtered. The filtrate was concentrated. The residue was purified by column chromatography on silica gel (PE:MTBE = 87:13) to give the desired product (2.1 g, 78.6% yield) as white solid. ¹H NMR (400 MHz, DMSO-*d₆*) δ 7.86 (d, *J =* 8.8 Hz, 1H), 7.05 (d, *J =* 8.8 Hz, 1H), 5.42 (s, 2H), 3.43 (s, 3H), 1.72 (s, 6H).

### Step 4: Synthesis of 2-bromo-6-(hydroxymethyl)-3-(methoxymethoxy)phenol

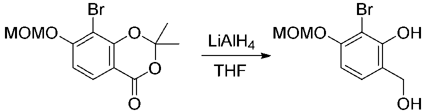

To a solution of 8-bromo-7-(methoxymethoxy)-2,2-dimethyl-4*H*-benzo[*d*][1,3]dioxin-4-one (2.1 g, 6.62 mmol) in THF (20 mL) was added LAH (503 mg, 13.24 mmol) at 0 °C. The mixture was stirred at 0 °C for 15 min, then warmed to room temperature and stirred for 2 h. After the reaction was complete, THF (20 mL) was added to the mixture, then water (0.5 mL) was added dropwise over 5 min, followed by 15% aqueous NaOH (0.5 mL). After stirring for 15 min, water (1.5 mL) was added dropwise, and stirring continued for 10 min. The mixture was adjusted to pH 6 with 2 N HCl and filtered. The filtrate was extracted with EtOAc (50 mL x 3). The combined organic layers were washed with saturated brine (100 mL), dried over anhydrous Na₂SO₄, filtered, and concentrated to give desired product (1.4 g) as yellow oil, which was used directly in the next step without purification. LC-MS (ESI): [M-H]⁻ =261.1, 263.0.

### Step 5: Synthesis of (3-bromo-2-methoxy-4-(methoxymethoxy)phenyl)methanol

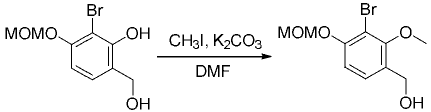

To a mixture of 2-bromo-6-(hydroxymethyl)-3-(methoxymethoxy)phenol (1.6 g, 6.08 mmol) and K₂CO₃ (2.5 g, 18.24 mmol) in DMF (20 mL) was added CH₃I (1.7 g, 12.16 mmol). The reaction mixture was stirred at room temperature for 16 h. After dilution with water (100 mL), the mixture was extracted with EtOAc (50 mL x 3). The combined organic layers were washed with saturated brine (60 mL), dried over anhydrous Na₂SO₄, filtered, and concentrated under reduced pressure. The residue was purified by column chromatography on silica gel (PE:MTBE = 82:18) to give the desired product (1.8 g, 95% yield) as colorless oil. ¹H NMR (400 MHz, DMSO-*d₆*) δ 7.34 (d, *J* = 8.4 Hz, 1H), 6.99 (d, *J =* 8.8 Hz, 1H), 5.26 (s, 2H), 5.12 (t, *J =* 5.6, 1H), 4.50 - 4.49 (m, 2H), 3.75 (s, 3H), 3.40 (s, 3H). Step 6: Synthesis of 3-bromo-2-methoxy-4-(methoxymethoxy)benzaldehyde

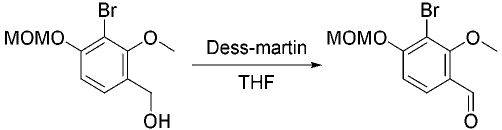

To a solution of (3-bromo-2-methoxy-4-(methoxymethoxy)phenyl)methanol (1.6 g, 5.77 mmol) in DCM (20 mL) was added DMP (4.9 g, 11.55 mmol) at 0 °C portionwise over 5 min. The reaction mixture was warmed to room temperature and stirred for 2 h. After the reaction was complete, the solvent was removed. The residue was purified by column chromatography on silica gel (PE:MTBE = 89:11) to give the desired product (1.5 g, 94% yield) as colorless oil. ¹H NMR (400 MHz, DMSO-*d₆*) δ 10.12 (s, 1H), 7.77 (d, *J =* 8.8 Hz, 1H), 7.16 (d, *J* = 8.8 Hz, 1H), 5.41 (s, 2H), 3.92 (s, 3H), 3.42 (s, 3H).

### Step 7: Synthesis of (E)-2-(3-bromo-2-methoxy-4-(methoxymethoxy)styryl)-5-methyl-6-nitrobenzo[d]thiazole

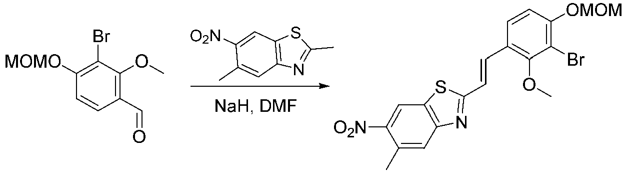

To a solution of 2,5-dimethyl-6-nitrobenzo[*d*]thiazole (1.48 g, 6.97 mmol) in THF (20 mL) was added NaH (429 mg, 10.72 mmol, wt: 60%) at 0 °C. The resulting mixture was stirred at 0 °C for 30 min. Then 3-bromo-2-methoxy-4-(methoxymethoxy)benzaldehyde (1.50 g, 5.36 mmol) was added to the mixture. After stirred at room temperature for 16 h, the mixture was quenched by saturated aqueous NH₄Cl (10 mL), and extracted with EtOAc (50 mL x 3). The combined organic layers were washed with saturated brine (60 mL), dried over anhydrous Na₂SO₄, filtered, and concentrated under reduced pressure. The residue was purified by column chromatography on silica gel (PE : DCM = 0~100%) to give the desired product (1.47 g, 58.9% yield) as yellow solid. LC-MS (ESI): [M+H]⁺ = 465.2, 467.1; ¹H NMR (400 MHz, CDCl₃) δ 8.55 (s, 1H), 7.92 (s, 1H), 7.84 (d, *J =* 16.4 Hz, 1H), 7.58 (d, *J* = 8.8 Hz, 1H), 7.43 (d, *J* = 16.8 Hz, 1H), 7.03 (d, *J* = 8.8 Hz, 1H), 5.31 (s, 2H), 3.93 (s, 3H), 3.54 (s, 3H), 2.75 (s, 3H).

### Step 8: Synthesis of (E)-2-(3-bromo-2-methoxy-4-(methoxymethoxy)styryl)-5-methylbenzo[d]thiazol-6-amine

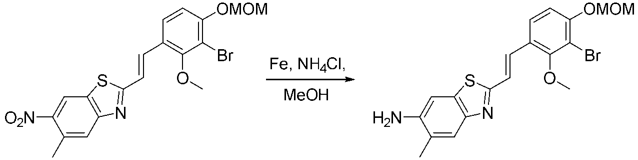

To a solution of (*E*)-2-(3-bromo-2-methoxy-4-(methoxymethoxy)styryl)-5-methyl-6-nitrobenzo[*d*]thiazole (1.42 g, 3.05 mmol) in MeOH (20 mL) was added NH₄Cl (1.63 g, 30.50 mmol) and iron powder (854 mg, 15.25 mmol) at room temperature. The resulting mixture was stirred at 70 °C for 16 h. After cooled to room temperature, the mixture was filtered, and the filtrate was concentrated. The residue was purified by column chromatography on silica gel (DCM:MeOH = 96:4) to give the desired product (1.27 g, 95.6% yield) as yellow solid. LC-MS (ESI): [M+H]⁺ =435.1, 437.1; ¹H NMR (400 MHz, DMSO-*d₆*) δ 7.84 (d, *J* = 8.8 Hz, 1H), 7.56 (s, 1H), 7.49 - 7.39 (m, 2H), 7.10 - 7.06 (m, 2H), 5.34 (s, 2H), 5.30 (s, 2H), 3.81 (s, 3H), 3.42 (s, 3H), 2.18 (s, 3H).

### Step 9: Synthesis of (E)-2-(2-((2-(3-bromo-2-methoxy-4-(methoxymethoxy)styryl)-5-methylbenzo[d]thiazol-6-yl)amino)ethoxy)ethan-1-ol

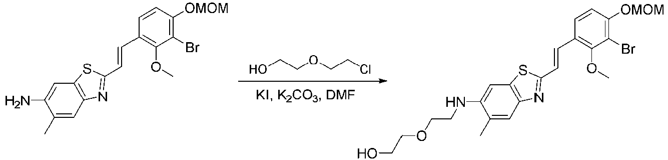

To a solution of (*E*)-2-(3-bromo-2-methoxy-4-(methoxymethoxy)styryl)-5-methylbenzo[*d*]thiazol-6-amine (1.25 g, 2.87 mmol) in DMF (10 mL) was added K₂CO₃ (1.19 g, 8.61 mmol), KI (94 mg, 0.57 mmol), and 2-(2-chloroethoxy)ethan-1-ol (1.07 g, 8.61 mmol). The resulting mixture was stirred at 100 °C for 24 h under N₂. After being cooled to room temperature, the mixture was diluted with ice water (30 mL) and extracted with EtOAc (30 mL x 3). The combined organic layers were washed with saturated brine (60 mL), dried over anhydrous Na₂SO₄, filtered, and the filtrate was concentrated under reduced pressure. The residue was purified by column chromatography on silica gel (DCM:MeOH = 95:5) to give the desired product (580 mg, 39% yield) as yellow oil. LC-MS (ESI): [M+H]⁺ =523.2, 525.2; ¹H NMR (400 MHz, DMSO-*d₆*) δ 7.85 (d, *J* = 9.2 Hz, 1H), 7.61 (s, 1H), 7.50 - 7.41 (m, 2H), 7.13 (s, 1H), 7.08 (d, *J =* 8.8 Hz, 1H), 5.34 (s, 2H), 5.17 (t, *J* = 6.4 Hz, 1H), 4.64 (t, *J =* 5.2 Hz, 1H), 3.81 (s, 3H), 3.67 (t, *J =* 6.0 Hz, 2H), 3.55 - 3.51 (m, 2H), 3.50 - 3.47 (m, 2H), 3.43 (s, 3H), 3.36 - 3.35 (m, 2H), 2.21 (s, 3H).

### Step 10: Synthesis of (E)-2-(2-((2-(3-bromo-2-methoxy-4-(methoxymethoxy)styryl)-5-methylbenzo[d]thiazol-6-yl)(methyl)amino)ethoxy)ethan-1-ol

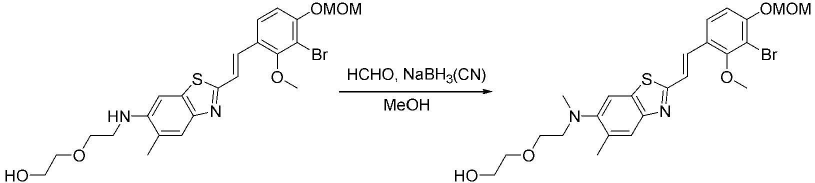

To a solution of (*E*)-2-(2-((2-(3-bromo-2-methoxy-4-(methoxymethoxy)styryl)-5-methylbenzo[*d*]thiazol-6-yl)amino)ethoxy)ethan-1-ol (580 mg, 1.11 mmol) in MeOH (4 mL) was added aqueous formaldehyde (333 mg, 11.10mmol, 40% wt) at 0 °C. After stirring for 15 min at this temperature, NaBH₃CN (255 mg, 4.05 mmol) was added. The resulting mixture was warmed to room temperature and stirred for 16 h. The reaction was quenched with H₂O (20 mL) and extracted with EtOAc (30 mL x 3). The combined organic layers were washed with saturated brine (60 mL), dried over anhydrous Na₂SO₄, filtered, and the filtrate was concentrated under reduced pressure. The residue was purified by column chromatography on silica gel (DCM:MeOH = 95:5) to give the desired product (460 mg, 77.5% yield) as yellow solid. LC-MS (ESI): [M+H]⁺ =537.2, 539.2; ¹H NMR (400 MHz, DMSO-*d₆*) δ 7.89 (d*, J =* 8.8 Hz, 1H), 7.75 (d, *J* = 8.4 Hz, 2H), 7.61 - 7.51 (m, 2H), 7.09 (d, *J =* 8.8 Hz, 1H), 5.36 (s, 2H), 4.56 (t, *J =* 5.6 Hz, 1H), 3.83 (s, 3H), 3.58 (t, *J =* 6.0 Hz, 2H), 3.48 - 3.45 (m, 2H), 3.43 (s, 3H), 3.42 - 3.39 (m, 2H), 3.09 (t, *J =* 6.0 Hz, 2H), 2.76 (s, 3H), 2.37 (s, 3H).

### Step 11: Synthesis of (E)-2-(2-((2-(3-bromo-2-methoxy-4-(methoxymethoxy)styryl)-5-methylbenzo[d]thiazol-6-yl)(methyl)amino)ethoxy)ethyl 4-methylbenzenesulfonate

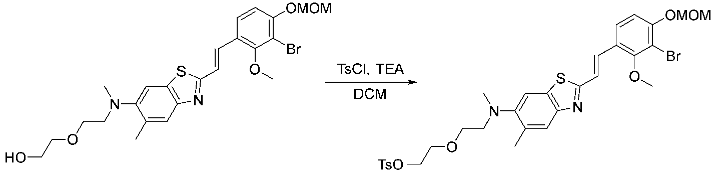

A solution of (*E*)-2-(2-((2-(3-bromo-2-methoxy-4-(methoxymethoxy)styryl)-5-methylbenzo[*d*]thiazol-6-yl)(methyl)amino)ethoxy)ethan-1-ol (410 mg, 0.76 mmol), TEA (307 mg, 3.04 mmol) and TsCl (291 mg, 1.53 mmol) in DCM (3 mL) was stirred at room temperature for 16 h under N₂. The reaction mixture was diluted with H₂O (20 mL) and extracted with EtOAc (30 mL x 3). The combined organic layers were washed with brine (60 mL), dried over anhydrous Na₂SO₄, filtered, and concentrated under reduced pressure. The residue was purified by column chromatography on silica gel (PE:EA = 70:30) to give the desired product (460mg, 78.8% yield) as yellow solid. LC-MS (ESI): [M+H]⁺ =691.2, 693.2; ¹H NMR (400 MHz, DMSO-*d₆*) δ 7.89 (d, *J* = 8.8 Hz, 1H), 7.77 (d, *J* = 8.4 Hz, 3H), 7.70 (s, 1H), 7.61 - 7.51 (m, 2H), 7.45 (d, *J =* 8.4 Hz, 2H), 7.09 (d, *J =* 8.8 Hz, 1H), 5.36 (s, 2H), 4.10 (t, *J* = 4.4 Hz, 2H), 3.83 (s, 3H), 3.55 (t, *J* = 4.4 Hz, 2H), 3.49 (t, *J* = 6.0 Hz, 2H), 3.43 (s, 3H), 3.01 (t, *J* = 5.6 Hz, 2H), 2.71 (s, 3H), 2.39 (s, 3H), 2.33 (s, 3H).

### Step 12: Synthesis of (E)-2-(3-bromo-2-methoxy-4-(methoxymethoxy)styryl)-N-(2-(2-fluoroethoxy)ethyl)-N,5-dimethylbenzo[d]thiazol-6-amine

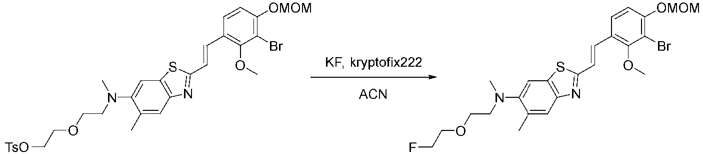

To solution of (*E*)-2-(2-((2-(3-bromo-2-methoxy-4-(methoxymethoxy)styryl)-5-methylbenzo[*d*]thiazol-6-yl)(methyl)amino)ethoxy)ethyl 4-methylbenzenesulfonate (300 mg, 0.43 mmol) in ACN (3 mL) was added CsF (327 mg, 2.15 mmol), [bmim][BF₄] (6 mL, 20 v). The resulting mixture was stirred at 100 °C for 2 h under N₂. The reaction mixture was quenched with H₂O (20 mL) and extracted with EtOAc (30 mL x 3). The combined organic layers were washed with brine (60 mL), dried over anhydrous Na₂SO₄, filtered, and concentrated under reduced pressure. The residue was purified by column chromatography on silica gel (PE:EA = 82:18) to give the desired product (180 mg, 77.6% yield) as yellow solid. LC-MS (ESI): [M+H]⁺ =539.2, 541.2; ¹H NMR (400 MHz, DMSO-*d₆*) δ 7.89 (d, *J =* 9.2 Hz, 1H), 7.76 (d, *J* = 8.0 Hz, 2H), 7.61 - 7.51 (m, 2H), 7.09 (d, *J* = 8.8 Hz, 1H), 5.36 (s, 2H), 4.56 (t, *J* = 4 Hz, 1H), 4.44 (t, *J =* 4 Hz, 1H), 3.83 (s, 3H), 3.67 (t, *J* = 4.4 Hz, 1H), 3.63 - 3.58 (m, 3H), 3.43 (s, 3H), 3.11 (t, *J =* 6.4 Hz, 2H), 2.76 (s, 3H), 2.38 (s, 3H).

### Step 13: Synthesis of (E)-2-bromo-4-(2-(6-((2-(2-fluoroethoxy)ethyl)(methyl)amino)-5-methylbenzo[d]thiazol-2-yl)vinyl)-3-methoxyphenol

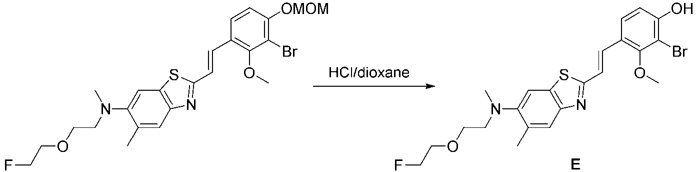

To a solution of (*E*)-2-(3-bromo-2-methoxy-4-(methoxymethoxy)styryl)-*N*-(2-(2-fluoroethoxy)ethyl)-*N*,5-dimethylbenzo[*d*]thiazol-6-amine (80 mg, 0.15 mmol) in 1,4-dioxane (1.5 mL) was added HCl/1,4-dioxane (4 M, 1.5 mL). The resulting mixture was stirred at room temperature for 2 h. After the reaction was complete, EtOAc (6 mL) was added. The precipitated solid was filtered off, washed successively with EtOAc (20 ml) and Et₂O (10 mL) to give compound E (65.3 mg, 88% yield) as orange solid. LC-MS (ESI): [M+H]⁺ =495.1, 497.1; ¹⁹F NMR (376 MHz, DMSO) δ -221.33; ¹H NMR (400 MHz, DMSO-*d₆*) δ 11.06 (s, 1H), 8.31 (s, 1H), 7.90 (s, 1H), 7.75 (d, *J* = 8.8 Hz, 1H), 7.69 - 7.65 ( d, *J* = 16.0 Hz, 1H), 7.51 - 7.47 (d, *J* = 16.0 Hz, 1H), 6.91 (d, *J* = 8.8 Hz, 1H), 4.52 (t,*J* = 8.0 Hz,1H), 4.41 - 4.39 (m, 1H), 3.81 (s, 3H), 3.62 - 3.52 (m, 6H), 3.11 (s, 3H), 2.55 (s, 3H).

### Another Method for Preparing Compound F

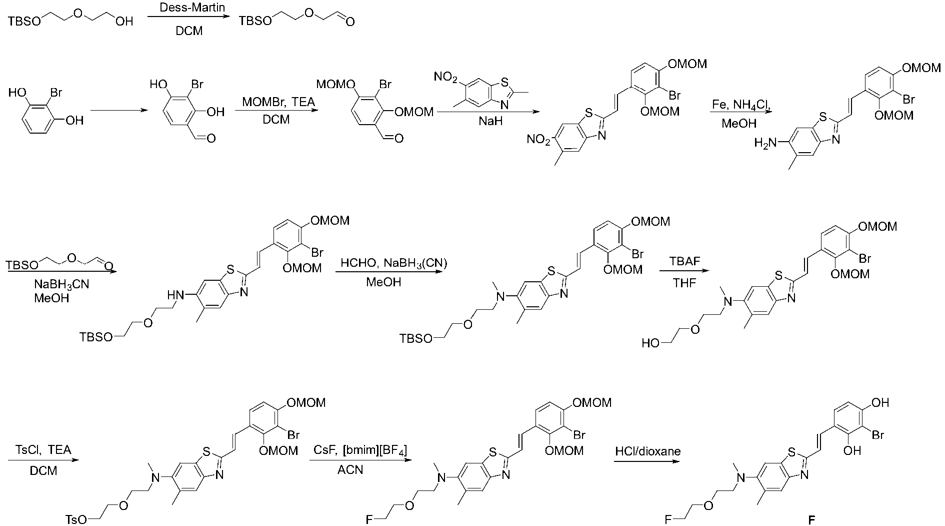

### Synthesis of (E)-2-bromo-4-(2-(6-((2-(2-fluoroethoxy)ethyl)(methyl)amino)-5-methylbenzo[d]thiazol-2-yl)vinyl)benzene-1,3-diol

### Step 1: Synthesis of 2-(2-((tert-butyldimethylsilyl)oxy)ethoxy)acetaldehyde

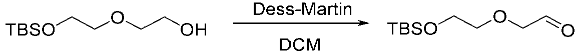

To a solution of 2-(2-((*tert*-butyldimethylsilyl)oxy)ethoxy)ethan-1-ol (551 mg, 2.50 mmol) in anhydrous DCM (5 mL) was added DMP (1.4 g, 3.25 mmol) at 0 °C. The reaction mixture was stirred at room temperature for 4 h. The solvent was removed. The residue was triturated with MTBE (20 mL) and filtered. The filtrate was concentrated under reduced pressure to give the crude product (500 mg) as colorless solid, which was used into the next step directly without further purification.

### Step 2: Synthesis of 3-bromo-2,4-dihydroxybenzaldehyde

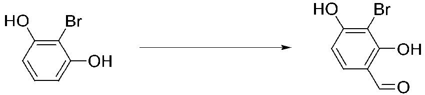

POCl₃ (40.6 g, 264.5 mmol, 25 mL) was added dropwise to anhydrous DMF (50 mL) at 0 °C under N₂. The mixture was stirred at room temperature for 1.5 h. A solution of 2-bromo-1,3-benzenediol (5.0 g, 26.45 mmol) in anhydrous DMF (25 mL) was then added dropwise while maintaining the internal temperature below 30 °C. After stirring at room temperature for 16 h, the reaction mixture was poured into ice-water (200 mL) and adjusted to pH 2-3 with saturated aqueous NaHCO₃. The aqueous layer was extracted with EtOAc (200 mL x 3). The combined organic layers were washed with saturated brine (200 mL), dried over anhydrous Na₂SO₄, and filtered. The filtrate was concentrated to give the residue, which was purified by column chromatography on silica gel (PE:MTBE = 75:25) to give the desired product (450 mg, 7.8% yield) as white solid. ¹H NMR (400 MHz, CDCl₃) δ 12.16 (s, 1H), 9.70 (s, 1H), 7.45 (d, *J =* 8.4 Hz, 1H), 6.73 (d, *J =* 8.8 Hz, 1H), 6.31 (s, 1H).

### Step 3: Synthesis of 3-bromo-2,4-bis(methoxymethoxy)benzaldehyde

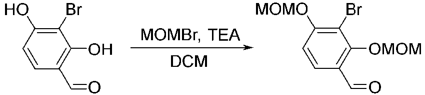

To a solution of 3-bromo-2,4-dihydroxybenzaldehyde (950 mg, 4.38 mmol) and TEA (2.21 g, 21.90 mmol) in DCM (20 mL) was added MOMBr (1.64 g, 13.14 mmol) dropwise at 0 °C. The resulting mixture was stirred at room temperature for 16 h. After the reaction was complete, the mixture was diluted with H₂O (30 mL) and extracted with DCM (30 mL x 3). The combined organic layers were washed with saturated brine (60 mL), dried over anhydrous Na₂SO₄ and filtered. The filtrate was concentrated to give the residue, which was purified by column chromatography on silica gel (PE:MTBE = 87:13) to give the desired product (670 mg, 50.1% yield) as white solid. ¹H NMR (400 MHz, CDCl₃) δ 10.23 (s, 1H), 7.81 *(d, J=* 8.8 Hz, 1H), 7.07 *(d, J=* 8.8 Hz, 1H), 5.33 (s, 2H), 5.21 (s, 2H), 3.64 (s, 3H), 3.52 (s, 3H).

### Step 4: Synthesis of (E)-2-(3-bromo-2,4-bis(methoxymethoxy)styryl)-5-methyl-6-nitrobenzo[d]thiazole

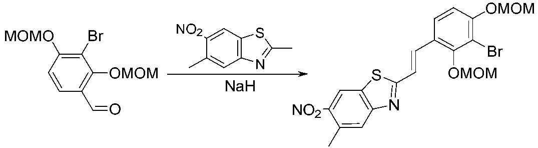

To a solution of 2,5-dimethyl-6-nitrobenzothiazole (595 mg, 2.86 mmol) in THF (10 mL) was added NaH (205 mg, 5.12 mmol, wt: 60%) at 0 °C. After being stirred at room temperature for 30 min, 3-bromo-2,4-bis(methoxymethoxy)benzaldehyde (670 mg, 2.20 mmol) was added. The mixture was stirred for 16 h at room temperature. The mixture was quenched by aqueous NH₄Cl (50 mL) and extracted with EtOAc (30 mL x 3). The combined organic layers were washed with brine (60 mL), dried over anhydrous Na₂SO₄, filtered. The filtrate was concentrated to give the residue, which was purified by column chromatography on silica gel (PE:DCM = 0~100%) to give the desired product (620 mg, 56% yield) as yellow solid. LC-MS (ESI): [M+H]⁺ =495.2, 497.1; ¹H NMR (400 MHz, CDCl₃) δ 8.55(s, 1H), 7.99 (d, *J* =16.4 Hz, 1H), 7.91 (s, 1H), 7.64 (d, *J* = 8.8 Hz, 1H), 7.38 (d, *J* = 8.4 Hz, 1H), 7.06 (d, *J* = 8.8 Hz, 1H), 5.31 (s, 2H), 5.19 (s, 2H), 3.75 (s, 3H), 3.54 (s, 3H), 2.74 (s, 3H).

### Step 5: Synthesis of (E)-2-(3-bromo-2,4-bis(methoxymethoxy)styryl)-5-methylbenzo[d]thiazol-6-amine

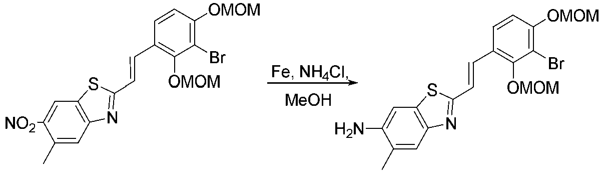

To a solution of (*E*)-2-(3-bromo-2,4-bis(methoxymethoxy)styryl)-5-methyl-6-nitrobenzo[*d*]thiazole (620 mg, 1.25 mmol) in MeOH (10 mL) was added NH₄Cl (669 mg, 12.5 mmol) and iron powder (350 mg, 6.26 mmol) at room temperature. The resulting mixture was stirred at 80 °C for 16 h. After being cooled to room temperature, the mixture was filtered. The filtrate was concentrated to give the residue, which was purified by column chromatography on silica gel (DCM:MeOH = 98:2) to give the desired product (530 mg, 91.0% yield) as red solid. LC-MS (ESI): [M+H]⁺ = 465.1, 467.1; ¹HNMR (400 MHz, CDCl₃) δ 7.75 - 7.68(m, 2H), 7.60 - 7.58 (m, 1H), 7.36 - 7.32 (m, 1H), 7.12 (s, 1H), 7.03 - 7.01 (m, 1H), 5.29 (s, 2H), 5.17 (s, 2H), 3.74 (s, 3H), 3.53 (s, 3H), 2.32 (s, 3H).

### Step 6: Synthesis of (E)-2-(3-bromo-2,4-bis(methoxymethoxy)styryl)-N-(2-(2-((tertbutyldimethylsilyl)oxy)ethoxy)ethyl)-5-methylbenzo[d]thiazol-6-amine

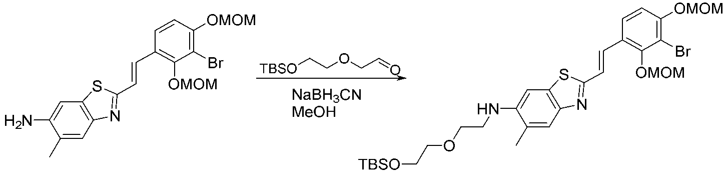

To a solution of 2-(2-((*tert*-butyldimethylsilyl)oxy)ethoxy)acetaldehyde (500 mg, 2.29 mmol) in MeOH (3 mL) was added (*E*)-2-(3-bromo-2,4-bis(methoxymethoxy)styryl)-5-methylbenzo[*d*]thiazol-6-amine (380 mg, 0.82 mmol). After stirring for 30 min at room temperature, NaBH₃CN (103 mg, 1.64 mmol) was added. The resulting mixture was stirred at room temperature for 16 h. The reaction was quenched with H₂O (20 mL) and extracted with EtOAc (30 mL x 3). The combined organic layers were washed with brine (60 mL), dried over anhydrous Na₂SO₄, filtered, and the filtrate was concentrated under reduced pressure. The residue was purified by column chromatography on silica gel (PE : EA = 82 : 18) to give the desired product (330 mg, 60.2% yield) as yellow oil. LC-MS (ESI): [M+H]⁺ = 667.3, 669.3; ¹H NMR (400 MHz, DMSO-*d₆*) δ 7.88 (d, *J* = 8.8 Hz, 1H), 7.59 - 7.54(m, 2H), 7.43 (d, *J =* 16.4 Hz, 1H), 7.11 - 7.08 (m, 2H), 5.35 (s, 2H), 5.13 (s, 3H), 3.73 - 3.69 (m, 2H), 3.68 - 3.66 (m, 2H), 3.65 (s, 3H), 3.51 (t, *J* = 6.4 Hz, 2H), 3.43 (s, 3H), 3.36 - 3.32 (m, 2H), 3.20 (s, 3H), 0.86 (s, 9H), 0.04 (s, 6H).

### Step 7: Synthesis of (E)-2-(3-bromo-2,4-bis(methoxymethoxy)styryl)-N-(2-(2-((tertbutyldimethylsilyl)oxy)ethoxy)ethyl)-N,5-dimethylbenzo[d]thiazol-6-amine

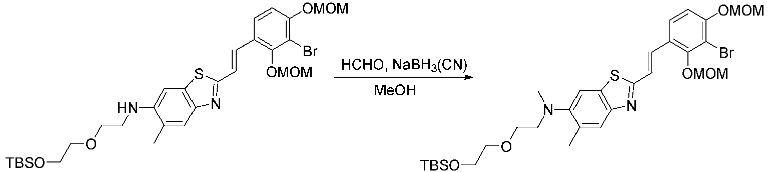

To a solution of (*E*)-2-(3-bromo-2,4-bis(methoxymethoxy)styryl)-*N*-(2-(2-((*tert*butyldimethylsilyl)oxy)ethoxy)ethyl)-5-methylbenzo[d]thiazol-6-amine (300 mg, 0.45 mmol) in MeOH (2 mL) was added aqueous formaldehyde (0.3 mL, 30% wt) at 0 °C. After stirring for 30 min at this temperature, NaBH₃CN (85 mg, 1.35 mmol) was added. The resulting mixture was warmed to room temperature and stirred for 16 h. The solvent was removed. The residue was purified by column chromatography on silica gel (PE:EA = 83:17) to give the desired product (260 mg, 84.7% yield) as yellow oil. LC-MS (ESI): [M+H]⁺ = 681.3, 683.3; ¹H NMR (400 MHz, DMSO-*d₆*) δ 7.92 (d,*J* = 8.0 Hz, 1H), 7.74 - 7.68 (m, 3H), 7.51 - 7.47 (m, 1H), 7.11 (d, *J =* 8.8 Hz, 1H), 5.36 (s, 2H), 5.14 (s, 2H), 3.66 - 3.63 (m, 5H), 3.58 (t, *J =* 6 Hz, 2H), 3.44 - 3.41 (m, 5H), 3.09 (t, *J* = 6.0 Hz, 2H), 2.76 (s, 3H), 2.36 (s, 3H), 0.84 (s, 9H), 0.01 (s, 6H).

### Step 8: Synthesis of (E)-2-(2-((2-(3-bromo-2,4-bis(methoxymethoxy)styryl)-5-methylbenzo[d]thiazol-6-yl)(methyl)amino)ethoxy)ethan-1-ol

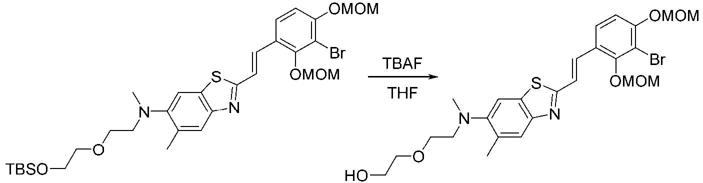

To a solution of (*E*)-2-(3-bromo-2,4-bis(methoxymethoxy)styryl)-*N*-(2-(2-((*tert*butyldimethylsilyl)oxy)ethoxy)ethyl)-*N*,5-dimethylbenzo[*d*]thiazol-6-amine (260 mg, 0.38 mmol) in THF (3 mL) was added TBAF (120 mg, 0.76 mmol). The resulting mixture was stirred at room temperature for 2 h. The reaction was quenched with aqueous NH₄Cl (20 mL) and extracted with EtOAc (30 mL x 3). The combined organic layers were washed with saturated brine (60 mL), dried over anhydrous Na₂SO₄, filtered, and the filtrate was concentrated under reduced pressure. The residue was purified by column chromatography on silica gel (PE:MTBE = 0-30%, DCM:MeOH = 0-6%) to give the desired product (200 mg, 92% yield) as yellow oil. LC-MS (ESI): [M+H]⁺ = 567.2, 569.1; ¹H NMR (400 MHz, DMSO-*d₆*) δ 7.91 (d, *J* = 9.2 Hz, 1H), 7.74(s, 2H), 7.70(d, *J* = 16.4 Hz, 1H), 7.49(d, *J* = 16 Hz, 1H), 7.11(d, *J* =8.4 Hz, 1H), 5.36(s, 2H), 5.14(s, 2H), 4.56(t, *J* = 5.2 Hz, 1H), 3.65(s, 3H), 3.57(t, *J* = 6.0 Hz, 2H), 3.46(t, *J* = 5.2 Hz, 2H), 3.43(s, 3H), 3.42 - 3.39(m, 2H), 3.09 - 3.07(m, 2H), 2.76(s,3H), 2.37(s, 3H).

### Step 9: Synthesis of (E)-2-(2-((2-(3-bromo-2,4-bis(methoxymethoxy)styryl)-5-methylbenzo[d]thiazol-6-yl)(methyl)amino)ethoxy)ethyl 4-methylbenzenesulfonate

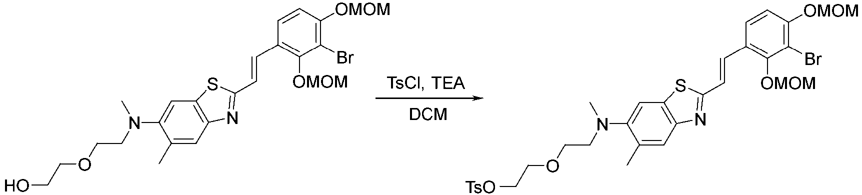

To a solution of (*E*)-2-(2-((2-(3-bromo-2,4-bis(methoxymethoxy)styryl)-5-methylbenzo[*d*]thiazol-6-yl)(methyl)amino)ethoxy)ethan-1-ol (190 mg, 0.33 mmol) and TEA (133 mg, 1.32 mmol) in DCM (2 mL) was added TsCl (126 mg, 0.66 mmol) at 0 °C. The resulting mixture was stirred at room temperature for 16 h. The reaction mixture was quenched with H₂O (20 mL) and extracted with EtOAc (30 mL x 3). The combined organic layers were washed with brine (60 mL), dried over anhydrous Na₂SO₄, filtered, and concentrated under reduced pressure. The residue was purified by column chromatography on silica gel (PE:EA = 70:30) to give the desired product (180 mg, 76% yield) as yellow solid. LC-MS (ESI): [M+H]⁺ =721.2, 723.2; ¹H NMR (400 MHz, DMSO-*d₆*) δ 7.92 (d, *J* = 8.8 Hz, 1H), 7.78 - 7.69(m, 5H), 7.52(s, 1H), 7.48 - 7.44(m, 2H), 7.11(d, *J =* 8.8 Hz, 1H), 5.36(s, 2H), 5.14(s, 2H), 4.10(t, *J* = 4.4 Hz, 2H), 3.65(s, 3H), 3.55(t, *J* = 4.4 Hz, 2H), 3.49(t, *J* = 2.0 Hz, 2H), 3.43(s, 3H), 3.00(t, *J =* 6 Hz, 2H), 2.70(s, 3H), 2.39(s, 3H), 2.33(s, 3H).

### Step 10: Synthesis of (E)-2-(3-bromo-2,4-bis(methoxymethoxy)styryl)-N-(2-(2-fluoroethoxy)ethyl)-N,5-dimethylbenzo[d]thiazol-6-amine

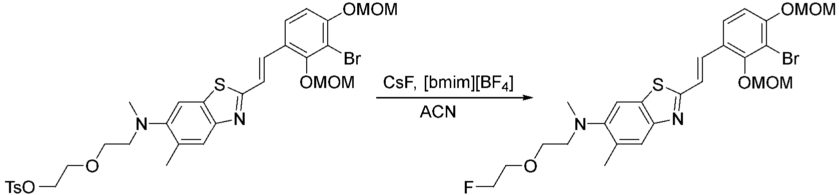

To solution of (*E*)-2-(2-((2-(3-bromo-2,4-bis(methoxymethoxy)styryl)-5-methylbenzo[*d*]thiazol-6-yl)(methyl)amino)ethoxy)ethyl 4-methylbenzenesulfonate (100 mg, 0.14 mmol) in ACN (2 mL, 20 v) was added CsF (106 mg, 0.70 mmol), [bmim][BF₄] (2 mL, 20 v). The resulting mixture was stirred at 100 °C for 2 h. The reaction mixture was quenched with H₂O (20 mL) and extracted with EtOAc (30 mL x 3). The combined organic layers were washed with brine (60 mL), dried over anhydrous Na₂SO₄, filtered, and concentrated under reduced pressure. The residue was purified by column chromatography on silica gel (DCM : MeOH = 97:3) to give the desired product (38 mg, 47.7% yield) as yellow solid. LC-MS (ESI): [M+H]⁺ =569.2, 571.2; ¹H NMR (400 MHz, DMSO-*d₆*) δ 7.92 (d, *J =* 8.8 Hz, 1H), 7.75-7.68 (m, 3H), 7.51 - 7.47(d, *J* = 16 Hz, 1H), 7.11(d, *J* = 9.2 Hz, 1H), 5.36(s, 2H), 5.14(s, 2H), 4.55(t, *J* = 4.0 Hz, 1H), 4.43(t, *J =* 4.0 Hz, 1H), 3.68-3.65(m, 4H), 3.62 - 3.58(m, 3H), 3.43(s, 3H), 3.11(t, *J* = 6 Hz, 2H), 2.76(s, 3H), 2.37(s, 3H).

### Step 11: Synthesis of (E)-2-bromo-4-(2-(6-((2-(2-fluoroethoxy)ethyl)(methyl)amino)-5-methylbenzo[d]thiazol-2-yl)vinyl)benzene-1,3-diol

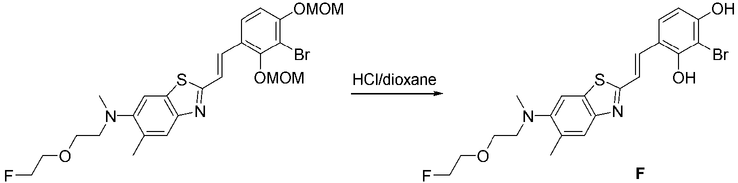

To a solution of (*E*)-2-(3-bromo-2,4-bis(methoxymethoxy)styryl)-*N*-(2-(2-fluoroethoxy)ethyl)-*N*,5-dimethylbenzo[*d*]thiazol-6-amine (38 mg, 0.07 mmol) in 1,4-dioxane (1 mL) was added HCl/1,4-dioxane (4 M, 1 mL). The resulting mixture was stirred at room temperature for 16 h. EtOAc (5 ml) was added to the reaction mixture. The precipitated solid was filtered off, washed successively with EtOAc (20 ml) and Et₂O (10 mL) to give compound F (21.1 mg, 63% yield) as orange solid. LC-MS (ESI): [M+H]⁺ =481.1, 483.1; ¹⁹F NMR (376 MHz, DMSO-*d₆*) δ -221.32; ¹H NMR (400 MHz, CD₃OD) δ 8.42(s, 1H), 8.00 (d, *J =* 8.4 Hz, 1H), 7.90 (s, 1H), 7.52 - 7.47 (m, 2H), 6.56 (d, *J* = 8.4 Hz, 1H), 4.56 - 4.54 (m, 1H), 4.44 - 4.42 (m, 1H), 4.00(t, *J* = 0.8 Hz, 2H), 3.63 (t, *J =* 4.0 Hz, 1H), 3.57 - 3.55 (m, 3H), 3.40 (s, 3H), 2.68 (s, 3H).

### Example 7: Synthesis of Compound G

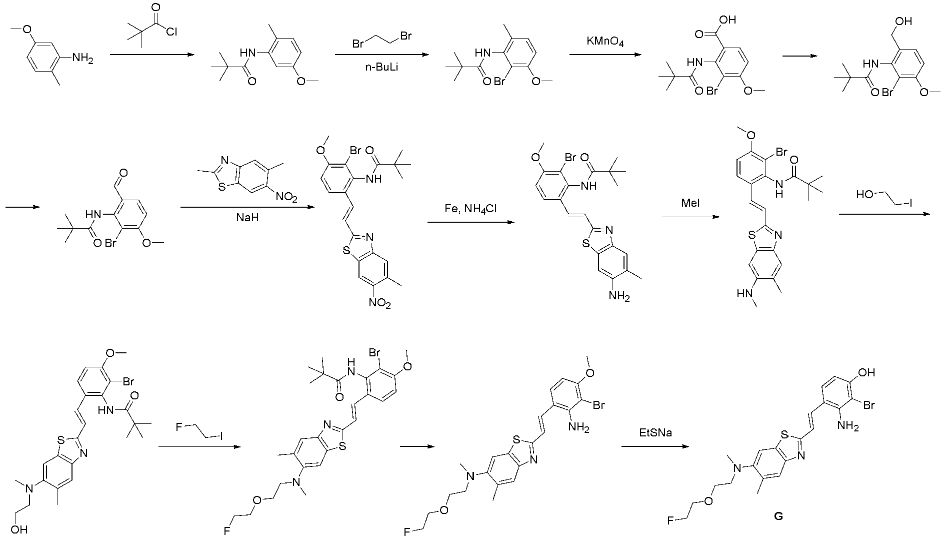

Compound G was synthesized following the procedure of Example 1 and Example 2.

Compound G: LC-MS (ESI): [M+H]⁺ = 480.1, 482.1.

### Another Method for Preparing Compound G

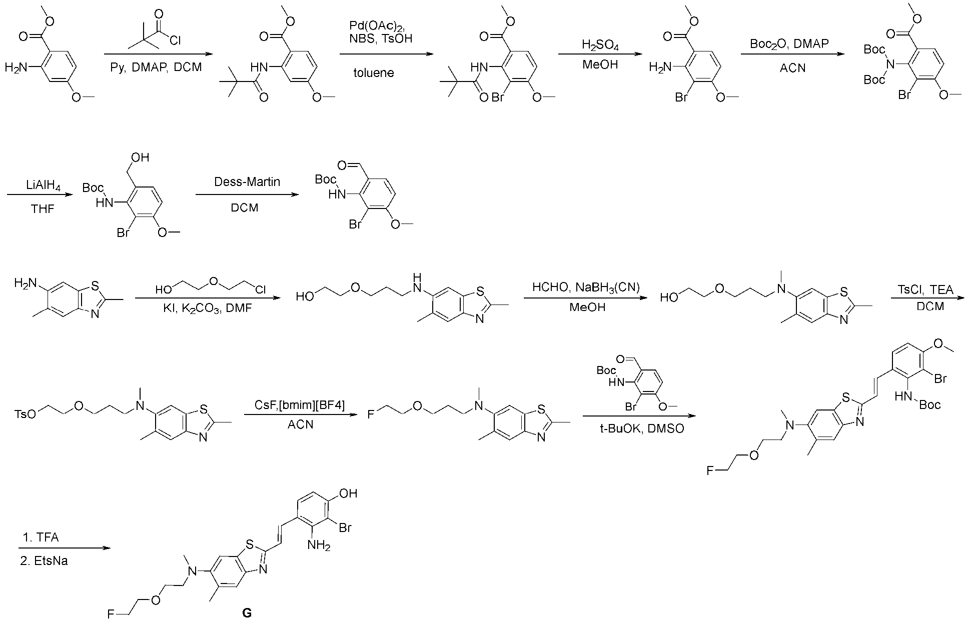

### Step 1: Synthesis of methyl 4-methoxy-2-pivalamidobenzoate

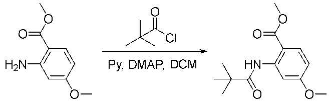

To a solution of methyl 2-amino-4-methoxybenzoate (5.0 g, 27.6 mmol), DMAP (337 mg, 2.76 mmol) and pyridine (4.36 g, 55.2 mmol) in DCM (50 mL) was added pivaloyl chloride (4.97 g, 41.4 mmol) dropwise at 0 °C. The reaction mixture was stirred at 0 °C for 2 h. The mixture was quenched with 1N HCl (30 mL) and water (50 mL). The mixture was extracted with DCM (50 mL x 3). The combined organic layers were washed with brine (100 mL), dried over anhydrous Na₂SO₄, filtered, and concentrated under reduced pressure. The residue was purified by column chromatography on silica gel (PE:EA=10:1) to give the desired product (7.0 g, 95% yield) as white solid. ¹H NMR (400 MHz, CDCl₃) δ 11.54 (s, 1H), 8.49 (d, *J* = 2.8 Hz, 1H), 7.95 (d, *J* = 9.2 Hz, 1H), 6.58 (dd, *J* = 8.8, 2.8 Hz, 1H), 3.89 (s, 3H), 3.87 (s, 3H), 1.35 (s, 9H).

### Step 2: Synthesis of methyl 3-bromo-4-methoxy-2-pivalamidobenzoate

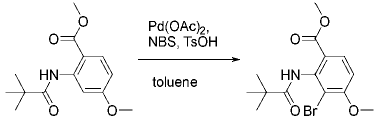

To a solution of methyl 4-methoxy-2-pivalamidobenzoate (2 g, 7.54 mmol) and NBS (2.95 g, 16.6 mmol) in toluene (20 mL) was added TsCl (2.59 g, 15.1 mmol) and Pd(OAc)₂ (341 mg, 1.51 mmol). The reaction mixture was stirred at room temperature under N₂ for 16 h. After the reaction was complete, the reaction mixture was poured into ice-water (30 mL) and filtered. The filtrate was extracted with EtOAc (20 mL x 3). The combined organic layers were washed with brine (30 mL), dried over anhydrous Na₂SO₄, filtered, and concentrated under reduced pressure. The residue was purified by column chromatography on silica gel PE:EA=1:1) to give the desired product (0.2 g, 7.7% yield) as colorless oil. ¹H NMR (400 MHz, CDCl₃) δ 8.68 (s, 1H), 7.88 (d, *J =* 8.8 Hz, 1H), 6.77 (d, *J* = 9.2 Hz, 1H), 3.95 (s, 3H), 3.85 (s, 3H), 1.36 (s, 9H).

### Step 3: Synthesis of methyl 2-amino-3-bromo-4-methoxybenzoate

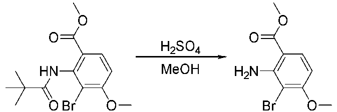

To a solution of methyl 3-bromo-4-methoxy-2-pivalamidobenzoate (200 mg, 0.58 mmol) in MeOH (2 mL) was added H₂SO₄ (0.4 mL). The reaction mixture was stirred at 70 °C under N₂ for 16 h. After cooling to room temperature, the mixture was poured into ice-water (10 mL) and extracted with EtOAc (20 mL x 3). The combined organic layers were washed with brine (10 mL), dried over anhydrous Na₂SO₄, filtered, and concentrated under reduced pressure. The residue was purified by column chromatography on silica gel (PE:EA=1:1) to give the desired product (0.14 g, 93% yield) as white solid. LC-MS (ESI): [M+H]⁺ = 259.9, 261.9.

### Step 4: Synthesis of methyl 2-(bis(tert-butoxycarbonyl)amino)-3-bromo-4-methoxybenzoate

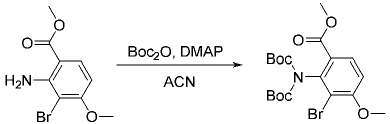

To a solution of methyl 2-amino-3-bromo-4-methoxybenzoate (130 mg, 0.5 mmol) and DMAP (184 mg, 1.5 mmol) inACN (40 mL) was added Boc₂O (547 mg, 2.51 mmol). The reaction mixture was stirred at 80 °C for 1 h. After cooling to room temperature, the solvent was removed. The residue was purified by column chromatography on silica gel (PE:EA=4:1) to give the desired product (0.18 g, 78% yield) as white solid. LC-MS (ESI): [M+Na]⁺ = 482.0, 484.0; ¹H NMR (400 MHz, CDCl₃) δ 8.01 (d, *J* = 8.8 Hz, 1H), 6.90 (d, *J =* 9.2 Hz, 1H), 3.98 (s, 3H), 3.86 (s, 3H), 1.36 (s, 18H).

### Step 5: Synthesis of tert-butyl (2-bromo-6-(hydroxymethyl)-3-methoxyphenyl)carbamate

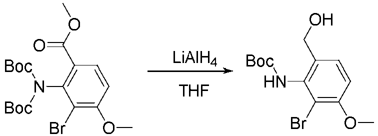

To a solution of methyl 2-(bis(*tert*-butoxycarbonyl)amino)-3-bromo-4-methoxybenzoate (150 mg, 0.326 mmol) in THF (4 mL) was added LiAlH₄ (25 mg, 0.653 mmol) at 0 °C. The reaction mixture was stirred at 0 °C for 2 h. The reaction was carefully quenched by successive addition of H₂O (0.25 mL), 15% aqueous NaOH solution (0.25 mL), and H₂O (0.75 mL) at 0 °C. The mixture was filtered through a pad of Celite, and the filter cake was washed with EtOAc (5 mL x 3). The combined filtrates were concentrated under reduced pressure to afford the crude product, which was purified by column chromatography on silica gel (PE:EA = 4:1) to give the desired product (70 mg, 64% yield) as white solid. LC-MS (ESI): [M+Na]⁺ = 354.0, 356.0; ¹H NMR (400 MHz, CDCl₃) δ 7.43 (d, *J =* 8.4 Hz, 1H), 6.85 (d, *J* = 8.4 Hz, 1H), 6.38 (s, 1H), 4.55 (s, 2H), 3.91 (s, 3H), 1.52 (s, 9H).

### Step 6: Synthesis of tert-butyl (2-bromo-6-formyl-3-methoxyphenyl)carbamate

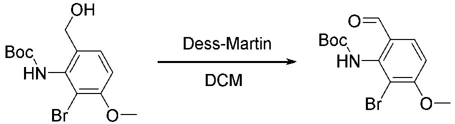

To a solution of *tert*-butyl (2-bromo-6-(hydroxymethyl)-3-methoxyphenyl)carbamate (70 mg, 0.21 mmol) in DCM (3 mL) was added DMP (134 mg, 0.32 mL) at 0 °C. Then the mixture was stirred at room temperature for 1 h. The mixture was sequentially quenched with aqueous Na₂S₂O₄ (2 mL), and saturated aqueous NaHCO₃ (3 mL). The mixture was extracted with DCM (3 mL x 3). The combined organic layers were washed with brine (5 mL), dried over anhydrous Na₂SO₄ and filtered. The filtrate was concentrated to give the residue, which was purified by column chromatography on silica gel (PE: EA=3:1) to give the desired product (60 mg, 86% yield) as white solid. LC-MS (ESI): [M+Na]⁺ =352.0, 354.0; ¹H NMR (400 MHz, CDCl₃) δ 9.90 (s, 1H), 7.86 (d, *J* = 8.8 Hz, 1H), 7.06 (s, 1H), 6.87 (d, *J =* 8.8 Hz, 1H), 3.98 (s, 3H), 1.50 (s, 9H).

### Step 7: Synthesis of 2-(2-((2,5-dimethylbenzo[d]thiazol-6-yl)amino)ethoxy)ethan-1-ol

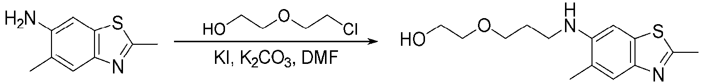

A solution of 2,5-dimethylbenzo[*d*]thiazol-6-amine (2.4 g, 13.5 mmol), 2-(2-chloroethoxy)ethan-1-ol (12.0 g, 67.5 mmol), K₂CO₃ (5.6 g, 40.5 mmol) and KI (2.3 g, 13.5 mmol) in DMF (30 mL) was stirred at 100 °C for 16 h under N₂. After the reaction was complete, the solvent was removed. The residue was purified by column chromatography on silica gel (DCM:MeOH=10:1) to give the desired product (2.0 g, 56.3% yield) as yellow solid. LC-MS (ESI): [M+H]⁺ =267.2; ¹H NMR (400 MHz, CDCl₃) δ 7.62 (s, 1H), 6.97 (s, 1H), 3.82-3.77 (m, 4H), 3.64-3.62 (m, 2H), 3.38 (t, *J* = 4.0Hz, 2H), 2.75 (s, 3H), 2.26 (s, 3H).

### Step 8: Synthesis of 2-(2-((2,5-dimethylbenzo[d]thiazol-6-yl)(methyl)amino)ethoxy)ethan-1-ol

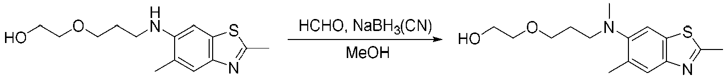

To a solution of 2-(2-((2,5-dimethylbenzo[*d*]thiazol-6-yl)amino)ethoxy)ethan-1-ol (2.0 g, 7.5 mmol) in MeOH (30 mL) was added aqueous formaldehyde (1.25 g, 15.0 mmol, 36% wt) at 0 °C. After stirring for 30 min at this temperature, NaBH₃CN (0.95 g, 15.0 mmol) was added. The resulting mixture was warmed to room temperature and stirred for 2 h. After the reaction was complete, the mixture was diluted with H₂O (20 mL) and extracted with DCM (30 mL x 3). The combined organic layers were washed with saturated brine (60 mL), dried over anhydrous Na₂SO₄, filtered, and the filtrate was concentrated under reduced pressure. The residue was purified by column chromatography on silica gel (DCM:MeOH = 92:8) to give the desired product (1.6 g, 76.2% yield) as yellow oil. LC-MS (ESI): [M+H]⁺ = 281.2; ¹H NMR (400 MHz, CDCl₃) δ 7.73 (s, 1H), 7.52 (s, 1H), 3.69 (t, *J =* 4.0Hz, 2H), 3.63 (t, *J =* 4.0Hz, 2H), 3.54 (t, *J =* 4.0Hz, 2H), 3.22-3.21(m, 2H), 2.82 (s, 3H), 2.79 (s, 3H), 2.45 (s, 3H).

### Step 9: Synthesis of 2-(2-((2,5-dimethylbenzo[d]thiazol-6-yl)(methyl)amino)ethoxy)ethyl 4-methylbenzenesulfonate

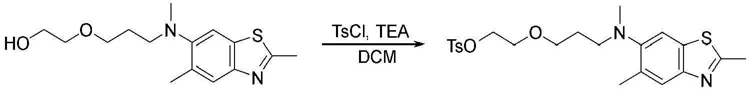

To a solution of 2-(2-((2,5-dimethylbenzo[*d*]thiazol-6-yl)(methyl)amino)ethoxy)ethan-1-ol (1.6 g, 5.7 mmol) and TEA (2.3 g, 22.8 mmol) in DCM (20 mL) was added TsCl (2.2 g, 11.4 mmol) at 0 °C. The resulting mixture was stirred at room temperature for 16 h. The mixture was quenched by H₂O and extracted with DCM (20 mL x 3). The combined organic layers were dried over anhydrous Na₂SO₄, filtered and concentrated to give the crude product, which was purified by column chromatography on silica gel (PE: EA = 3: 1) to give the desired product (1.9 g, 76.6% yield) as yellow oil. LC-MS (ESI): [M+H]⁺ = 435.2; ¹H NMR (400 MHz, CDCl₃) δ 7.79-7.77 (m, 3H), 7.34-7.26 (m, 3H), 4.11-4.10 (m, 2H), 3.59-3.56 (m, 4H), 3.26-3.24 (m, 2H), 2.84-2.80 (m, 6H), 2.44-2.40 (m, 6H).

### Step 10: Synthesis of N-(2-(2-fluoroethoxy)ethyl)-N,2,5-trimethylbenzo[d]thiazol-6-amine

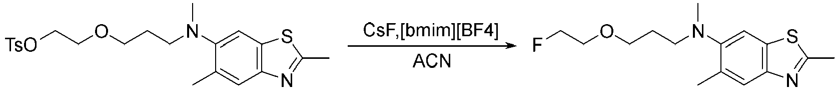

A solution of 2-(2-((2,5-dimethylbenzo[*d*]thiazol-6-yl)(methyl)amino)ethoxy)ethyl 4-methylbenzenesulfonate (1.9 g, 4.37 mmol), [bmim][BF₄] (40 mL), and CsF (3.3 g, 21.8 mmol) in ACN (40 mL) was stirred at 100 °C for 2 h. After being cooled to room temperature, the mixture was diluted with H₂O (20 mL) and extracted with EtOAc (20 mL x 3). The combined organic layers were dried over anhydrous Na₂SO₄, filtered and concentrated to give the crude product, which was purified by column chromatography on silica gel (PE: EA = 3: 1) to give the desired product (1.04 g, 86.7% yield) as colorless oil. LC-MS (ESI): [M+H]⁺ =283.2; ¹H NMR (400 MHz, DMSO-*d₆*) δ 7.70 (s, 1H), 7.68 (s, 1H), 4.55 (t, *J =* 4.0Hz, 1H), 4.43 (t, *J =* 4.0Hz, 1H), 3.65 (t, *J =* 4.0Hz, 1H), 3.60-3.75 (m, 3H), 3.07 (t, *J =* 4.0Hz, 1H), 2.73 (s, 3H), 2.72 (s, 3H), 2.35 (s, 3H).

### Step 11: Synthesis of tert-butyl (E)-(2-bromo-6-(2-(6-((2-(2-fluoroethoxy)ethyl)(methyl)amino)-5-methylbenzo[d]thiazol-2-yl)vinyl)-3-methoxyphenyl)carbamate

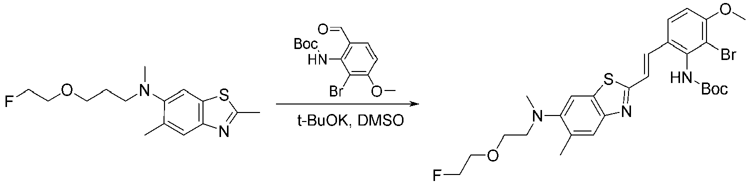

To a solution of N-(2-(2-fluoroethoxy)ethyl)-N,2,5-trimethylbenzo[d]thiazol-6-amine (240 mg, 0.80 mmol) and t-BuOK (180 mg, 1.6mmol) in DMSO (1 mL) was added tert-butyl (2-bromo-6-formyl-3-methoxyphenyl)carbamate (264 mg, 0.8 mmol) at 10 °C. The resulting mixture was stirred at room temperature for 3 h. The reaction mixture was quenched with H₂O (20 mL) and extracted with EtOAc (30 mL x 3). The combined organic phases were washed with brine (60 mL), dried over anhydrous Na₂SO₄, filtered, and concentrated under reduced pressure. The crude product was purified by column chromatography on silica gel (PE:EA = 60:40) to give the desired product (100 mg, 21% yield) as yellow oil. LC-MS (ESI): [M+H]⁺ = 594.1, 596.1; ¹H NMR (400 MHz, DMSO-*d₆*) δ 7.89 (d, *J* = 9.2 Hz, 1H), 7.61 - 7.51 (m, 2H), 6.90 - 6.82 (m, 3H), 4.56 (t, *J =* 4 Hz, 1H), 4.44 (t, *J* = 4 Hz, 3H), 3.83 (s, 3H), 3.67 (t, *J* = 4.4 Hz, 1H), 3.63 - 3.58 (m, 3H), 2.76 (s, 3H), 2.38 (s, 3H), 1.49 (s, 9H).

### Step 12: Synthesis of (E)-3-amino-2-bromo-4-(2-(6-((2-(2-fluoroethoxy)ethyl)(methyl)amino)-5-methylbenzo[d]thiazol-2-yl)vinyl)phenol

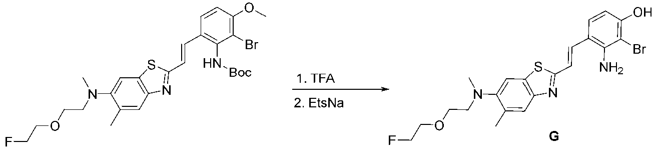

To a solution of *tert*-butyl (*E*)-(2-bromo-6-(2-(6-((2-(2-fluoroethoxy)ethyl)(methyl)amino)-5-methylbenzo[*d*]thiazol-2-yl)vinyl)-3-methoxyphenyl)carbamate (50 mg, 0.08 mmol) in anhydrous DCM (2 mL) was added TFA (0.5 mL). The reaction mixture was stirred at 20 °C for 0.5 h. After the reaction was complete, the solvent was removed. The crude product was dissolved in DMF (2.5 mL), followed by addition of EtSNa (34 mg, 0.4 mmol). The resulting mixture was stirred at 100 °C for 0.5 h. After cooling, the mixture was filtered. The filtrate was purified by prep-HPLC to give Compound G (10 mg, yield 23%) as brown solid. LC-MS (ESI): [M+H]⁺ = 480.1, 482.1; ¹H NMR (400 MHz, CD₃OD) δ 8.42(s, 1H), 8.00-7.90 (m, 2H), 7.52 - 7.47 (m, 2H), 6.56 (d, *J =* 8.4 Hz, 1H), 4.56 - 4.54 (m, 1H), 4.44 - 4.42 (m, 1H), 4.00(t, *J =* 0.8 Hz, 2H), 3.63 (t, *J =* 4.0 Hz, 1H), 3.57 - 3.55 (m, 3H), 3.40 (s, 3H), 2.68 (s, 3H).

### Example 8: Synthesis of Compound H

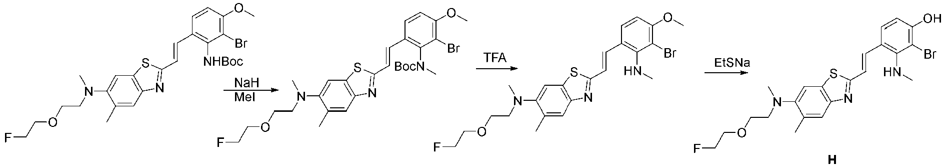

Compound H was synthesized following the procedure of Example 1 and Example 2.

Compound H: LC-MS (ESI): [M+H]⁺ = 494.1, 496.1.

### Step 1: Synthesis of tert-butyl (E)-(2-bromo-6-(2-(6-((2-(2-fluoroethoxy)ethyl)(methyl)amino)-5-methylbenzo[d]thiazol-2-yl)vinyl)-3-methoxyphenyl)(methyl)carbamate

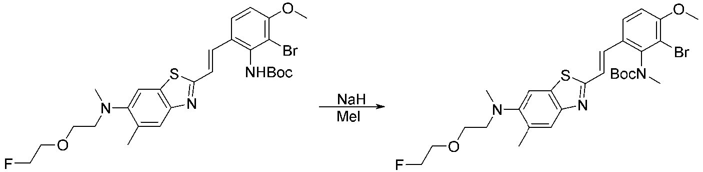

To a solution of *tert*-butyl (*E*)-(2-bromo-6-(2-(6-((2-(2-fluoroethoxy)ethyl)(methyl)amino)-5-methylbenzo[*d*]thiazol-2-yl)vinyl)-3-methoxyphenyl)carbamate (150 mg, 0.25 mmol) in DMF (3 mL) was added NaH (20 mg, 0.5 mmol, wt: 60%) at 0 °C. After being stirred at 0 °C for 0.5 h, CH₃I (70 mg, 0.5 mmol) was added. The resulting mixture was stirred at room temperature for 16 h. After the reaction was complete, the reaction mixture was quenched by H₂O (30 mL) and extracted with EtOAc (20 mL x 3). The combined organic layers were dried over anhydrous Na₂SO₄, filtered and concentrated under reduced pressure. The crude product was purified by column chromatography on silica gel (PE:EA=5:1) to give the desired product (90 mg, 59% yield) as yellow solid. LC-MS (ESI): [M+H]⁺ = 608.1, 610.1; ¹H NMR (400 MHz, DMSO-*d₆*) δ 7.89 (d, *J* = 9.2 Hz, 1H), 7.61 - 7.51 (m, 2H), 6.90 - 6.82 (m, 3H), 4.56 (t, *J* = 4 Hz, 1H), 4.44 (t, *J* = 4 Hz, 3H), 3.83 (s, 3H), 3.41 (s, 3H), 3.67 (t, *J* = 4.4 Hz, 1H), 3.63 - 3.58 (m, 3H), 2.76 (s, 3H), 2.38 (s, 3H), 1.49 (s, 9H).

### Step 2: Synthesis of (E)-2-bromo-4-(2-(6-((2-(2-fluoroethoxy)ethyl)(methyl)amino)-5-methylbenzo[d]thiazol-2-yl)vinyl)-3-(methylamino)phenol

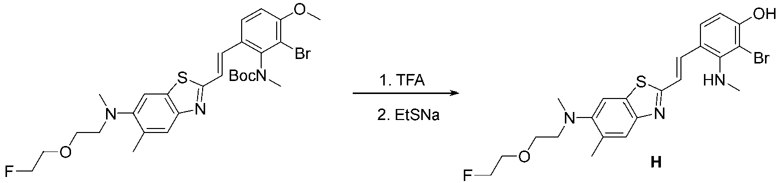

To a solution of *tert*-butyl (*E*)-(2-bromo-6-(2-(6-((2-(2-fluoroethoxy)ethyl)(methyl)amino)-5-methylbenzo[d]thiazol-2-yl)vinyl)-3-methoxyphenyl)(methyl)carbamate (90 mg, 0.15 mmol) in anhydrous DCM (5 mL) was added TFA (1 mL). The reaction mixture was stirred at 20 °C for 0.5 h. After the reaction was complete, the solvent was removed. The crude product was dissolved in DMF (3 mL), followed by addition of EtSNa (63 mg, 0.75 mmol). The resulting mixture was stirred at 100 °C for 0.5 h. After cooling, the mixture was filtered. The filtrate was purified by prep-HPLC to give Compound H (24 mg, yield 32%) as brown solid. LC-MS (ESI): [M+H]⁺ = 494.1, 496.1; ¹H NMR (400 MHz, CD₃OD) δ 8.42 (s, 1H), 8.00-7.90 (m, 2H), 7.52 - 7.47 (m, 2H), 6.56 (d, *J =* 8.4 Hz, 1H), 4.56 - 4.54 (m, 1H), 4.44 - 4.42 (m, 1H), 4.00 (t, *J* = 0.8 Hz, 2H), 3.63 (t, *J* = 4.0 Hz, 1H), 3.57 - 3.55 (m, 3H), 3.40 (s, 3H), 3.10 (s, 3H), 2.68 (s, 3H).

### Example 9: Synthesis of Compound I and Compound J

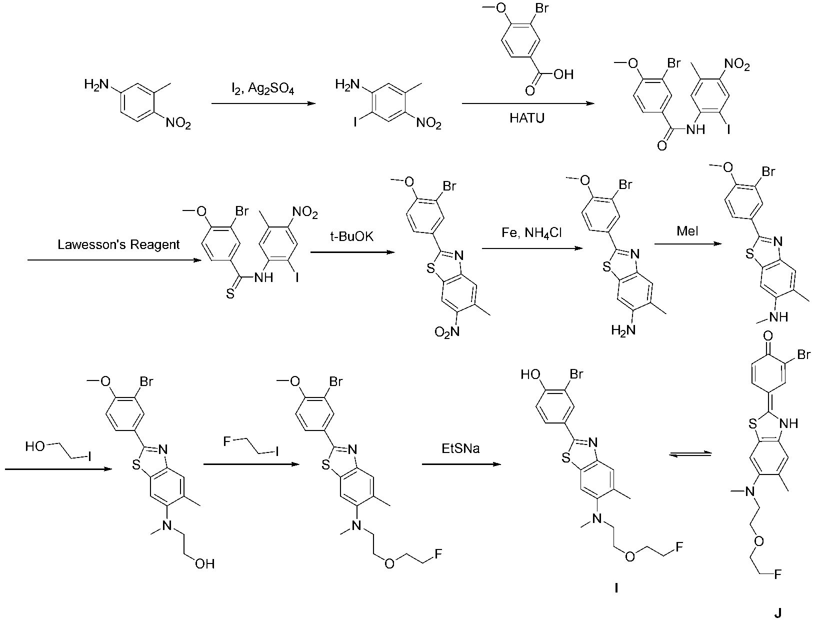

Compound I and Compound J were synthesized following the procedure of Example 1 and Example 2, respectively.

Compound I: LC-MS (ESI): [M+H]⁺ = 439.0, 441.0.

Compound J: LC-MS (ESI): [M+H]⁺ = 439.0, 441.0.

### Another Method for Preparing Compound I and Compound J

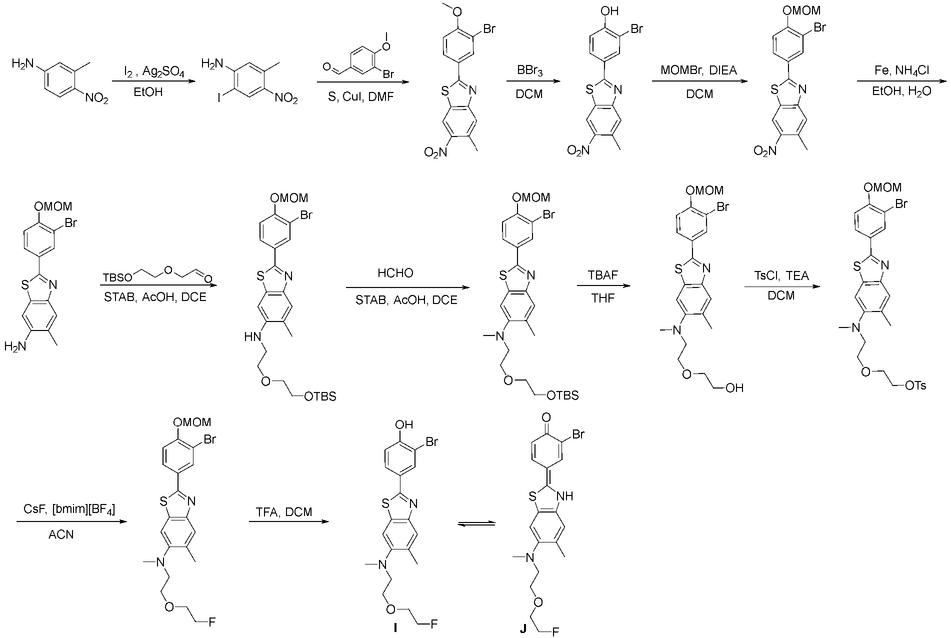

### Synthesis of 2-bromo-4-(6-((2-(2-fluoroethoxy)ethyl)(methyl)amino)-5-methylbenzo[d]thiazol-2-yl)phenol

### Step 1: Synthesis of 2-iodo-5-methyl-4-nitroaniline

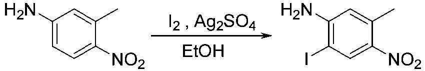

To a solution of I₂ (43.4 g, 17.11 mmol) in EtOH (400 mL) was added Ag₂SO₄ (41.1 g, 13.16 mmol) and 3-methyl-4-nitroaniline (20 g, 13.16 mmol). The mixture was stirred at room temperature for 4 h. After the reaction was complete, the solvent was removed. The residue was dissolved in DCM (100 mL) and saturated sodium sulfite solution (100 mL). The mixture was extracted with DCM (100 mL x 3). The combined organic layers were dried over anhydrous Na₂SO₄ and filtered. The filtrate was concentrated to give the crude product, which was purified by column chromatography on silica gel (PE:EA=3:1) to give the desired product (14.2 g, 38.9% yield) as yellow solid. LC-MS (ESI): [M+H]⁺=278.9; ¹H NMR (400 MHz, CDCl₃) δ 8.48 (s, 1H), 6.54 (s, 1H), 4.66 (s, 2H), 2.55 (s, 3H).

### Step 2: Synthesis of 2-(3-bromo-4-methoxyphenyl)-5-methyl-6-nitrobenzo[d]thiazole

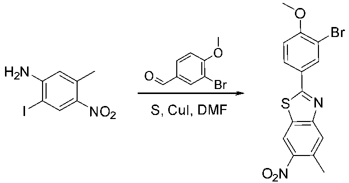

A mixture of 2-iodo-5-methyl-4-nitroaniline (14.2 g, 51.08 mmol), 3-bromo-4-methoxybenzaldehyde (10.98 g, 51.08 mmol), sulfur (49.04 g, 153.2 mmol), and CuI (972 mg, 5.1 mmol) in DMF (300 mL) was stirred at 130 °C for 48 h. After the reaction was complete, the mixture was filtered. The filter cake was washed with DCM (60 mL). The filtrate was concentrated under reduced pressure. The residue was purified by column chromatography on silica gel (PE:EA=2:1) to give the desired product (2.87 g, 14.8% yield) as yellow solid. LC-MS (ESI): [M+H]⁺ =379.1, 381.1; ¹H NMR (400 MHz, CDCl₃) δ 8.58 (s, 1H), 8.32 (d, *J* = 2.4 Hz, 1H), 8.01 (dd, *J =* 2.4 Hz, 2.0 Hz, 1H), 7.93 (s, 1H), 7.01 (d, *J =* 8.4 Hz, 1H), 4.00 (s, 3H), 2.75 (s, 3H).

### Step 3: Synthesis of 2-bromo-4-(5-methyl-6-nitrobenzo[d]thiazol-2-yl)phenol

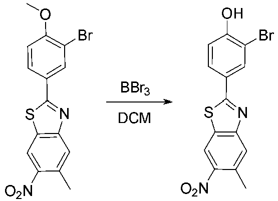

To a solution of 2-(3-bromo-4-methoxyphenyl)-5-methyl-6-nitrobenzo[d]thiazole (2.87 g, 7.57 mmol) in DCM (60 mL) was added BBr₃ (5.69 g, 22.71 mmol) dropwise at -70 °C. The reaction mixture was stirred at room temperature for 2 h. After the reaction was complete, the mixture was quenched with saturated aqueous NaHCO₃ at 0 °C. The aqueous layer was extracted with DCM (50 mL × 3). The combined organic layers were dried over anhydrous Na₂SO₄ and filtered. The filtrate was concentrated under reduced pressure. The residue was purified by column chromatography on silica gel (PE:EA = 1:1) to give the desired product (2.18 g, 78.9% yield) as yellow solid. LC-MS (ESI): [M+H]⁺=365.1, 367.1; ¹H NMR (400 MHz, DMSO-d₆) δ 11.3 (s, 1H), 8.92 (s, 1H), 8.23 (d, *J* = 2.4 Hz, 1H), 8.08 (s, 1H), 7.97 (dd, *J =* 2.0 Hz, 1H), 7.13 (d, *J =* 8.4 Hz, 1H), 2.65 (s, 3H).

### Step 4: Synthesis of 2-(3-bromo-4-(methoxymethoxy)phenyl)-5-methyl-6-nitrobenzo[d]thiazole

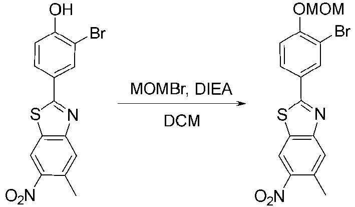

To a solution of 2-bromo-4-(5-methyl-6-nitrobenzo[d]thiazol-2-yl)phenol (2.18 g, 5.97 mmol) and DIEA (2.31 g, 17.91 mmol) in DCM (40 mL) was added MOMBr (1.11 g, 8.96 mmol) at 0 °C. The reaction mixture was stirred at room temperature for 2 h. After the reaction was complete, the mixture was diluted with H₂O (30 mL) and extracted with DCM (30 mL x 3). The combined organic layers were dried over anhydrous Na₂SO₄ and filtered. The filtrate was concentrated under reduced pressure. The residue was purified by column chromatography on silica gel (PE:EA = 3:1) to give the desired product (1.95 g, 79.92% yield) as yellow solid. LC-MS (ESI): [M+H]⁺ =411.1, 413.1; ¹H NMR (400 MHz, DMSO-*d₆*) δ 8.93 (s, 1H), 8.29 (d, *J =* 1.6 Hz, 1H), 8.08-8.06 (m, 2H), 7.39 (d, *J =* 8.8 Hz, 1H), 5.43 (s, 2H), 3.44 (s, 3H), 2.64 (s, 3H).

### Step 5: Synthesis of 2-(3-bromo-4-(methoxymethoxy)phenyl)-5-methylbenzo[d]thiazol-6-amine

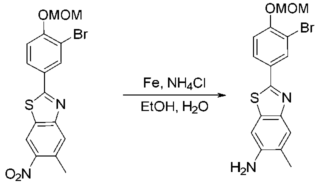

To a solution of 2-(3-bromo-4-(methoxymethoxy)phenyl)-5-methyl-6-nitrobenzo[*d*]thiazole (1.95 g, 4.77 mmol) and NH₄Cl (2.58 g, 47.7 mmol) in EtOH and H₂O (300 mL, v:v=2:1) was added iron powder (1.34 g, 23.85 mmol). The resulting mixture was stirred at 80 °C for 16 h. After the reaction was complete, the mixture was filtered. The filter cake was washed with MeOH. The filtrate was concentrated under reduced pressure. The residue was diluted with water (10 mL) and extracted with DCM (20 mL x 3). The combined organic layers were dried over anhydrous Na₂SO₄ and filtered. The filtrate was concentrated under reduced pressure to give the desired product (1.63 g, 90.0% yield) as yellow solid. LC-MS (ESI): [M+H-56]⁺= 379.1, 381.1; ¹H NMR (400 MHz, DMSO-*d₆*) δ 8.13 (d, *J* = 2.4 Hz, 1H), 7.87 (dd, *J =* 2.0 Hz, 1H), 7.61 (s, 1H), 7.32 (d, *J* = 8.8 Hz, 1H), 7.13 (s, 1H), 5.37 (s, 2H), 5.27 (s, 2H), 3.43 (s, 3H), 2.19 (s, 3H).

### Step 6: Synthesis of 2-(3-bromo-4-(methoxymethoxy)phenyl)-N-(2-(2-((tertbutyldimethylsilyl)oxy)ethoxy)ethyl)-5-methylbenzo[d]thiazol-6-amine

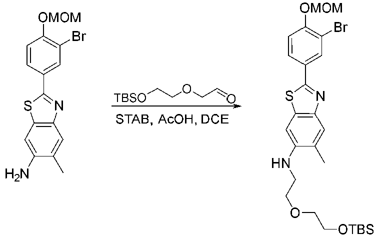

To a solution of 2-(3-bromo-4-(methoxymethoxy)phenyl)-5-methylbenzo[*d*]thiazol-6-amine (1.25 g, 3.3 mmol) and 2-(2-((*tert*-butyldimethylsilyl)oxy)ethoxy)acetaldehyde (800 mg, 3.63 mmol) in 1,2-dichloroethane (20 mL) was added AcOH (18 mg, 0.3 mmol) and STAB (1.4 g, 6.6 mmol). The resulting mixture was stirred at room temperature for 2 h. After the reaction was complete, the mixture was quenched with H₂O and extracted with DCM (20 mL x 3). The combined organic layers were dried over anhydrous Na₂SO₄, and filtered. The residue was purified by column chromatography on silica gel (PE:EA = 4:1) to give the desired product (560 mg, 29.3% yield) as yellow oil. LC-MS (ESI): [M+H]⁺=581.3, 583.3; ¹H NMR (400 MHz, DMSO-*d₆*) δ 8.14 (d, *J* = 2.0 Hz, 1H), 7.89 (dd, *J =* 2.0 Hz, 1H), 7.66 (s, 1H), 7.32 (d, *J* = 8.4 Hz, 1H), 7.15 (s, 1H), 5.37 (s, 2H), 5.10 (t, *J =* 5.6 Hz, 1H), 3.73-3.66 (m, 4H), 3.51 (t, *J =* 4.8 Hz, 2H), 3.43 (s, 3H), 3.35-3.32 (m, 2H), 2.21 (s, 3H), 0.85 (s, 9H), 0.04 (s, 6H).

### Step 7: Synthesis of 2-(3-bromo-4-(methoxymethoxy)phenyl)-N-(2-(2-((tertbutyldimethylsilyl)oxy)ethoxy)ethyl)-N,5-dimethylbenzo[d]thiazol-6-amine

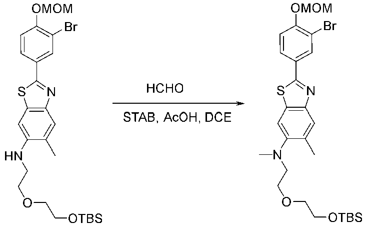

To a solution of 2-(3-bromo-4-(methoxymethoxy)phenyl)-*N*-(2-(2-((*tert*butyldimethylsilyl)oxy)ethoxy)ethyl)-5-methylbenzo[*d*]thiazol-6-amine (560 mg, 0.96 mmol) and formaldehyde (120 mg, 1.44 mmol, 36%wt) in 1,2-dichloroethane (20 mL) was added AcOH (6 mg, 0.1 mmol) and STAB (407 mg, 1.92 mmol). The resulting mixture was stirred at room temperature for 2 h. After the reaction was complete, the mixture was quenched with H₂O and extracted with DCM (20 mL x 3). The combined organic layers were dried over anhydrous Na₂SO₄, and filtered. The residue was purified by column chromatography on silica gel (PE:EA = 4:1) to give the desired product (530 mg, 92.5%) as yellow oil. LC-MS (ESI): [M+H]⁺ = 595.3, 597.3; ¹H NMR (400 MHz, CDCl₃) δ 8.28 (d, *J* = 2.0 Hz, 1H), 7.89 (dd, *J* = 2.4 Hz, 2.0 Hz, 1H), 7.83 (s, 1H), 7.55 (s, 1H), 7.22 (d, *J =* 8.8 Hz, 1H), 5.32 (s, 2H), 3.73 (t, *J* = 5.2 Hz, 2H), 3.65 (t, *J =* 6.0 Hz, 2H), 3.54 (s, 3H), 3.50 (t, *J* = 5.2 Hz, 2H), 3.19 (t, *J =* 6.0 Hz, 2H), 2.84 (s, 3H), 2.45 (s, 3H), 0.89 (s, 9H), 0.04 (s, 6H).

### Step 8: Synthesis of 2-(2-((2-(3-bromo-4-(methoxymethoxy)phenyl)-5-methylbenzo[d]thiazol-6-yl)(methyl)amino)ethoxy)ethan-1-ol

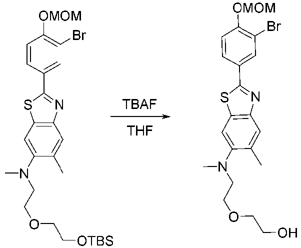

To a solution of 2-(3-bromo-4-(methoxymethoxy)phenyl)-*N*-(2-(2-((*tert*butyldimethylsilyl)oxy)ethoxy) ethyl)-*N*,5-dimethylbenzo[*d*]thiazol-6-amine (530 mg, 0.89 mmol) in anhydrous THF (10 mL) was added TBAF (1.78 mL, 1.78 mmol, 1M in THF). The reaction mixture was stirred at room temperature for 2 h. After the reaction was complete, the reaction was quenched with saturated NH₄Cl (10 mL) and extracted with EtOAc (20 mL x 3). The combined organic layers were dried over anhydrous Na₂SO₄ and filtered. The filtrate was concentrated to give the residue, which was purified by column chromatography on silica gel (PE:EA = 2:1) to give the desired product (380 mg, 88.7% yield) as yellow oil. LC-MS (ESI): [M+H]⁺ =481.2, 483.2; ¹H NMR (400 MHz, CDCl₃) δ 8.28 (d, *J* = 2.0 Hz, 1H), 7.89 (dd, *J* = 2.0 Hz,2.4 Hz, 1H), 7.84 (s, 1H), 7.56 (s, 1H), 7.22 (d, *J =* 8.8 Hz, 1H), 5.32 (s, 2H), 3.70 (t, *J =* 4.4 Hz, 2H), 3.66 (t, *J =* 6.0 Hz, 2H), 3.56 (t, *J =* 4.4 Hz, 2H), 3.54 (s, 3H), 3.22 (t, *J* = 6.0 Hz, 2H), 2.84 (s, 3H), 2.47 (s, 3H).

### Step 9: Synthesis of 2-(2-((2-(3-bromo-4-(methoxymethoxy)phenyl)-5-methylbenzo[d]thiazol-6-yl)(methyl)amino)ethoxy)ethyl 4-methylbenzenesulfonate

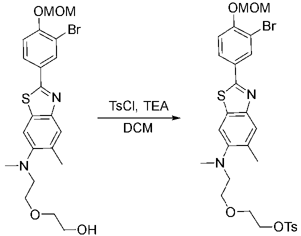

To a solution of 2-(2-((2-(3-bromo-4-(methoxymethoxy)phenyl)-5-methylbenzo[d]thiazol-6-yl)(methyl)amino)ethoxy)ethan-1-ol (380 mg, 0.79 mmol) and TEA (319 mg, 3.16 mmol) in DCM (10 mL) was added TsCl (301 mg, 1.58 mmol) at 0 °C. The reaction mixture was stirred at room temperature for 16 h. After the reaction was complete, the reaction was quenched with H₂O (10 mL) and extracted with DCM (20 mL x 3). The combined organic layers were dried over anhydrous Na₂SO₄ and filtered. The filtrate was concentrated to give the residue, which was purified by column chromatography on silica gel (PE:EA= 5:1) to give the desired product (390 mg, 77.8% yield) as yellow oil. LC-MS (ESI): [M+H]⁺ = 635.2, 637.2; ¹H NMR (400 MHz, DMSO-*d₆*) δ 8.22 (d, *J* = 2.4 Hz, 1H), 7.97 (dd, *J* = 2.0 Hz, 2.4 Hz, 1H), 7.81 (s, 1H), 7.78-7.76 (m, 3H), 7.46-7.44 (m, 2H), 7.36 (d, *J* = 8.8 Hz, 1H), 5.40 (s, 2H), 4.10 (t, *J =* 4.4 Hz, 2H), 3.55 (t, *J =* 4.4 Hz, 2H), 3.49 (t, *J* = 6.0 Hz, 2H), 3.44 (s, 3H), 3.01 (t, *J =* 6.0 Hz, 2H), 2.71 (s, 3H), 2.39 (s, 3H), 2.35 (s, 3H).

### Step 10: Synthesis of 2-(3-bromo-4-(methoxymethoxy)phenyl)-N-(2-(2-fluoroethoxy)ethyl)-N,5-dimethylbenzo[d]thiazol-6-amine

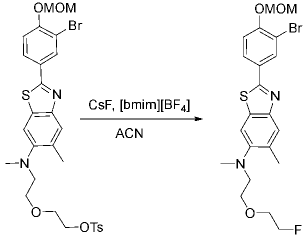

A solution of 2-(2-((2-(3-bromo-4-(methoxymethoxy)phenyl)-5-methylbenzo[*d*]thiazol-6-yl)(methyl)amino)ethoxy)ethyl 4-methylbenzenesulfonate (300 mg, 0.47 mmol), [bmim][BF₄] (6 mL), and CsF (357 mg, 2.35 mmol) in ACN (6 mL) was stirred at 100 °C for 2 h under N₂. After cooling to room temperature, the reaction mixture was diluted with H₂O (10 mL) and extracted with EtOAc (20 mL x 3). The combined organic layers were dried over anhydrous Na₂SO₄ and filtered. The filtrate was concentrated to give the residue, which was purified by column chromatography on silica gel (PE:EA = 4:1) to give the desired product (175 mg, 76.7% yield) as yellow oil. LC-MS (ESI): [M+H]⁺ = 483.2, 485.2; ¹H NMR (400 MHz, DMSO-*d₆*) δ 8.21 (d, *J* = 2.0 Hz, 1H), 7.97 (dd, *J* = 2.4 Hz, 2.0 Hz, 1H), 7.81-7.79 (m, 2H), 7.36 (d, *J =* 8.8 Hz, 1H), 5.39 (s, 2H), 4.55 (t, *J =* 4.0 Hz, 1H), 4.43 (t, *J* = 4.0 Hz, 1H), 3.67 (t, *J* = 4.0 Hz, 1H), 3.62-3.58 (m, 3H), 3.44 (s, 3H), 3.11 (t, *J* = 6.0 Hz, 2H), 2.76 (s, 3H), 2.38 (s, 3H).

### Step 11: Synthesis of 2-bromo-4-(6-((2-(2-fluoroethoxy)ethyl)(methyl)amino)-5-methylbenzo[d]thiazol-2-yl)phenol

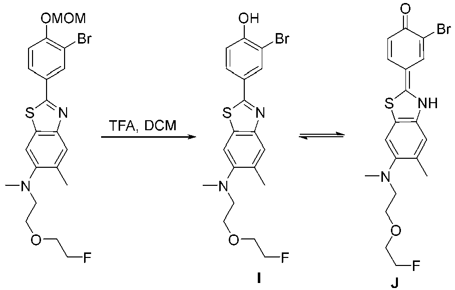

To a solution of 2-(3-bromo-4-(methoxymethoxy)phenyl)-N-(2-(2-fluoroethoxy)ethyl)-N,5-dimethylbenzo[*d*]thiazol-6-amine (100 mg, 0.21 mmol) in DCM (2 mL) was added TFA (0.5 mL). After being stirred for 2 h at room temperature, the solvent was removed. The residue was adjusted to pH 8 with saturated sodium carbonate solution and extracted with EtOAc (10 mL x 3). The combined organic layers were dried over anhydrous Na₂SO₄, filtered and concentrated to give the crude product, which was purified by MPLC to give compound I (30.5 mg, 33.9% yield) as white solid. LC-MS (ESI): [M+H]⁺ = 439.1, 441.1; ¹⁹F NMR (376 MHz, DMSO) δ -221.35; ¹H NMR (400 MHz, DMSO-*d₆*) δ 11.02 (s, 1H), 8.12 (d, *J* = 2.4 Hz, 1H), 7.86 (dd, *J* = 2.0, 2.0 Hz, 1H), 7.79 - 7.78 (m, 2H), 7.10 (d, *J* = 8.4 Hz, 1H), 4.56 (t, *J* = 4.0 Hz, 1H), 4.44 (t, *J* = 4.0 Hz, 1H), 3.67 (t, *J* = 4.0 Hz, 1H), 3.63 - 3.58 (m, 3H), 3.11 (t, *J* = 6.0 Hz, 2H), 2.76 (s, 3H), 2.38 (s, 3H).

### Example 10: Synthesis of Compound K

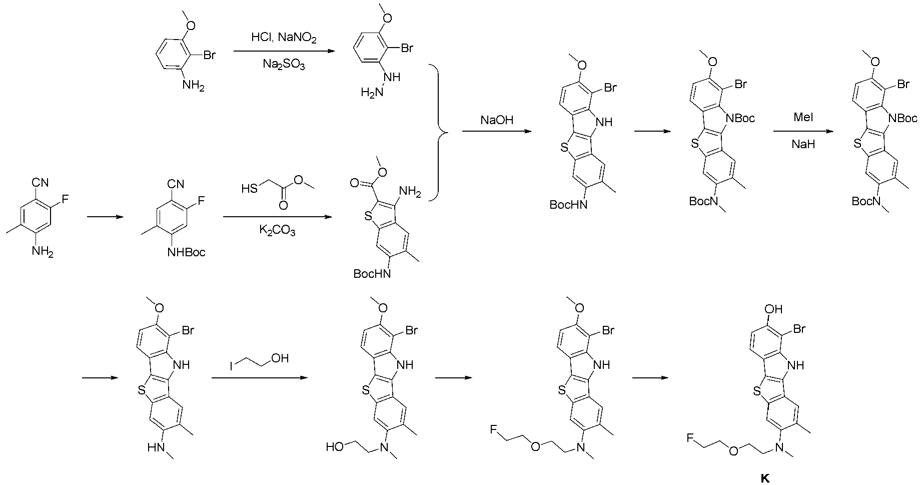

Compound K was synthesized following the procedure of Example 1 and Example 2.

Compound K: LC-MS (ESI): [M+H]⁺ = 451.0, 453.0.

### Another Method for Preparing Compound K.

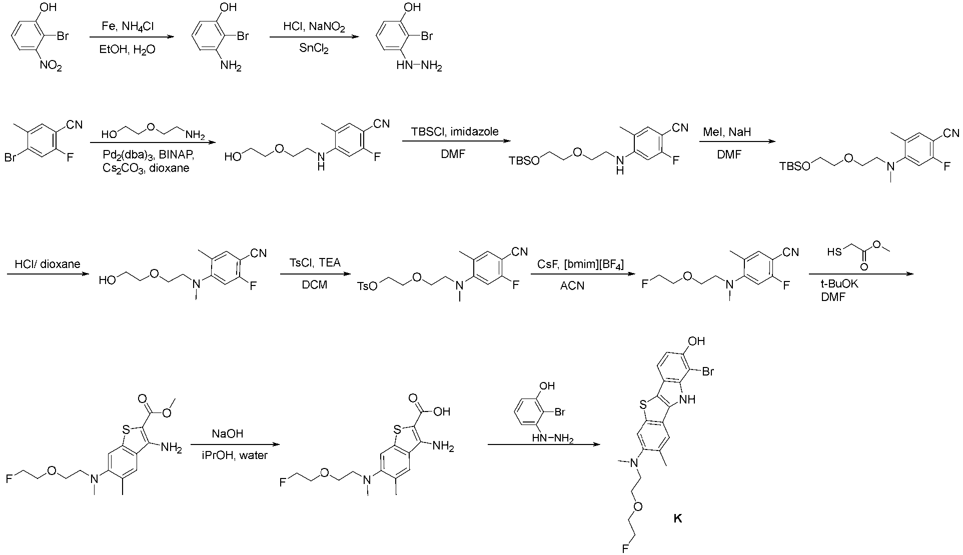

### 1-Bromo-7-((2-(2-fluoroethoxy)ethyl)(methyl)amino)-8-methyl-10H-benzo[4,5]thieno[3,2-b]indol-2-ol

### Step 1: Synthesis of 3-amino-2-bromophenol

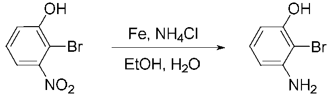

To a solution of 2-bromo-3-nitrophenol (2.0 g, 9.17 mmol) in EtOH and H₂O (30 mL, v:v=2: 1) was added NH₄Cl (4.908 g, 91.7 mmol) and iron powder (2.564 g, 45.9 mmol). The resulting mixture was stirred at 80 °C for 4 h. After the reaction was complete, the mixture was filtered. The filtrate was concentrated under reduced pressure. The residue was purified by column chromatography on silica gel (PE:EA=0-60%) to give the desired product (1.242 g, 72.0% yield) as black solid. LC-MS (ESI): [M+H]⁺=188.0, 190.0; ¹H NMR (400 MHz, CD₃OD) δ 6.86 (t, *J =* 8.0 Hz, 1H), 6.32 (dd, *J =* 8.0, 1.6 Hz, 1H), 6.22 (dd, *J =* 8.0, 1.2 Hz, 1H).

### Step 2: Synthesis of 2-bromo-3-hydrazineylphenol

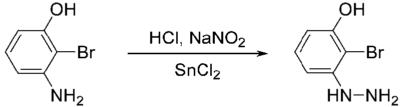

To a solution of 3-amino-2-bromophenol (200 mg, 1.06 mmol) in aqueous hydrochloric acid (6 M, 3 mL) was added a solution of NaNO₂ (147 mg, 2.12 mmol) in water (6 mL) dropwise at 0 °C. After being stirred at 0 °C for 30 min, the resulting solution was added dropwise to a solution of tin(II) chloride dihydrate (480 mg, 2.12 mmol) in aqueous hydrochloric acid (6 M, 3 mL). The mixture was stirred at 0 °C for 2 h. After the reaction was complete, the precipitated solid was collected by filtration. The filter cake was washed with TBME (20 mL) and dried under reduced pressure to give the desired product (83 mg, 38.2% yield) as tan solid. LC-MS (ESI): [M+H]⁺=203.0, 205.0; ¹H NMR (400 MHz, DMSO-d6 ) δ 7.15 (t, *J* = 8.0 Hz, 1H), 6.59 (d, *J =* 8.0 Hz, 1H), 6.46 (d, *J* = 8.8 Hz, 1H).

### Step 3: Synthesis of 2-fluoro-4-((2-(2-hydroxyethoxy)ethyl)amino)-5-methylbenzonitrile

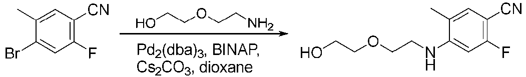

A mixture of 4-bromo-2-fluoro-5-methylbenzonitrile (9.0 g, 42.0 mmol), 2-(2-aminoethoxy)ethan-1-ol (8.84 g, 84.0 mmol), Cs₂CO₃ (27.38 g, 84.0 mmol), BINAP (2.62 g, 4.2 mmol), and Pd₂(dba)₃ (2.02 g, 2.1 mmol) in 1,4-dioxane (100 mL) was stirred at 100 °C for 16 h. After the reaction was complete, the reaction mixture was filtered. The filtrate was concentrated under reduced pressure. The residue was purified by column chromatography on silica gel (PE:EA=0-60%) to give the desired product (5.3 g, 53.0% yield) as colorless oil. LC-MS (ESI): [M+H]⁺ =239.3; ¹H NMR (400 MHz, DMSO-d6) δ 7.34 (d, *J* = 8.0 Hz, 1H), 6.58 (d, *J* = 13.2 Hz, 1H), 6.18-6.15 (m, 1H), 4.62 (t, *J* = 5.2 Hz, 1H), 3.59 (t, *J =* 6.0 Hz, 2H), 3.51-3.44 (m, 4H), 3.38-3.3 (m, 2H), 2.05 (s, 3H).

### Step 4: Synthesis of 4-((2-(2-((tert-butyldimethylsilyl)oxy)ethoxy)ethyl)amino)-2-fluoro-5-methylbenzonitrile

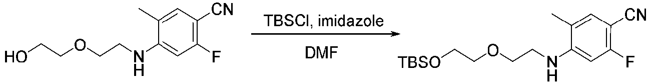

To a solution of 2-fluoro-4-((2-(2-hydroxyethoxy)ethyl)amino)-5-methylbenzonitrile (3.15 g, 13.65 mmol) and imidazole (1.355 g, 18.80 mmol) in DMF (40 mL) was added TBSCl (2.385 g, 15.90 mmol) at 0 °C. The reaction mixture was stirred for 16 h at room temperature. After the reaction was complete, the mixture was quenched with water (120 mL), and extracted with EtOAc (30 mL x 3). The combined organic layers were dried over anhydrous Na₂SO₄ and filtered. The filtrate was concentrated to give the residue, which was purified by column chromatography on silica gel (PE:EA=0-15%) to give the desired product (3.58 g, 76.7% yield) as colorless oil. LC-MS (ESI): [M+H]⁺ =353.4; ¹H NMR (400 MHz, CDCl₃) δ 7.17 (d, *J =* 7.2 Hz, 1H), 6.29 (d, *J =* 12.0 Hz, 1H), 3.80-3.75 (m, 4H), 3.59-3.57 (m, 2H), 3.33 (t, *J* = 5.2 Hz, 2H), 2.08 (s, 3H), 0.89 (s, 9H), 0.06 (s, 6H).

### Step 5: Synthesis of 4-((2-(2-((tert-butyldimethylsilyl)oxy)ethoxy)ethyl)(methyl)amino)-2-fluoro-5-methylbenzonitrile

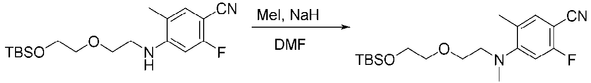

To a solution of 4-((2-(2-((*tert-*butyldimethylsilyl)oxy)ethoxy)ethyl)amino)-2-fluoro-5-methylbenzonitrile (3.58 g, 9.13 mmol) in DMF (40 mL) was added NaH (1.55 g, 13.70 mmol, wt: 60%) at 0 °C. After being stirred at 0 °C for 30 min, MeI (1.68 g, 11.83 mmol) was added. The resulting mixture was stirred at room temperature for 2 h. After the reaction was complete, the mixture was quenched with saturated NH₄Cl solution (10 mL), and extracted with EtOAc (30 mL x 3). The combined organic layers were dried over anhydrous Na₂SO₄ and filtered. The filtrate was concentrated to give the residue, which was purified by column chromatography on silica gel (PE:EA=0-15%) to give the desired product (2.912 g, 80.0% yield) as colorless oil. LC-MS (ESI): [M+H]⁺ =367.3; ¹H NMR (400 MHz, CDCl₃) δ 7.28 (d, *J =* 7.6 Hz, 1H), 6.74 (d, *J =* 11.6 Hz, 1H), 3.72-3.69 (m, 2H), 3.64 (t, *J =* 5.6 Hz, 2H), 3.48-3.45 (m, 2H), 3.25 (t, *J =* 5.6 Hz, 2H), 2.87 (s, 3H), 2.25 (s, 3H), 0.88 (s, 9H), 0.05 (s, 6H).

### Step 6: Synthesis of 2-fluoro-4-((2-(2-hydroxyethoxy)ethyl)(methyl)amino)-5-methylbenzonitrile

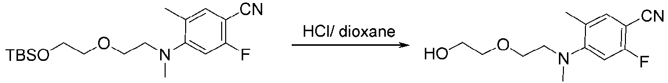

To a solution of 4-((2-(2-((*tert*-butyldimethylsilyl)oxy)ethoxy)ethyl)(methyl)amino)-2-fluoro-5-methylbenzonitrile (2.0 g, 5.4 mmol) in DCM (20 mL) was added HCl/1,4-dioxane (4 M, 5.31 mmol). After being stirred at room temperature for 1 h, the solvent was removed. The residue was purified by column chromatography on silica gel (PE:EA=0-60%) to give the desired product (1.27 g, 90.0% yield) as colorless oil. LC-MS (ESI): [M+H]⁺ =253.1; ¹H NMR (400 MHz, DMSO-d6) δ 7.55 (d, *J* = 8.8 Hz, 1H), 6.97 (d, *J =* 12.4 Hz, 1H), 4.54 (t, *J =* 5.6 Hz, 1H), 3.58 (t, *J =* 5.6 Hz, 2H), 3.45 - 3.41 (m, 2H), 3.39 - 3.35 (m, 2H), 3.25 (t, *J* = 5.6 Hz, 2H), 2.84 (s, 3H), 2.22 (s, 3H).

### Step 7: Synthesis of 2-(2-((4-cyano-5-fluoro-2-methylphenyl)(methyl)amino)ethoxy)ethyl 4-methylbenzenesulfonate

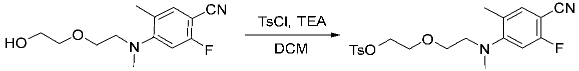

To a solution of 2-fluoro-4-((2-(2-hydroxyethoxy)ethyl)(methyl)amino)-5-methylbenzonitrile (1.0 g, 3.96 mmol) and TEA (1.604 g, 15.85 mmol) in DCM (15 mL) was added TsCl (1.510 g, 7.93 mmol) at 0 °C. After being stirred at room temperature for 16 h, the solvent was removed. The residue was purified by column chromatography on silica gel (PE:EA=0-30%) to give the desired product (1.30 g, 80.7% yield) as colorless oil. LC-MS (ESI): [M+H]⁺ =407.2; ¹H NMR (400 MHz, CDCl₃) δ 7.79 - 7.77 (m, 2H), 7.34 (d, *J =* 8.0 Hz, 2H), 7.28 (d, *J* = 7.6 Hz, 2H), 6.73 (d, *J =* 11.6 Hz, 1H), 4.11 - 4.08 (m, 2H), 3.61 - 3.57 (m, 4H), 3.22 (t, *J* = 5.6 Hz, 2H), 2.83 (s, 3H), 2.45 (s, 3H), 2.23 (d, *J* = 0.9 Hz, 3H).

### Step 8: Synthesis of 2-fluoro-4-((2-(2-fluoroethoxy)ethyl)(methyl)amino)-5-methylbenzonitrile

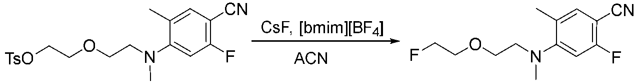

To solution of 2-(2-((4-cyano-5-fluoro-2-methylphenyl)(methyl)amino)ethoxy)ethyl 4-methylbenzenesulfonate (1.30 mg, 3.2 mmol) and [bmim][BF₄] (20 mL) in ACN (20 mL) was added CsF (2.433 g, 16.0 mmol). After being stirred at 100 °C for 16 h, the reaction mixture was quenched with H₂O (20 mL) and extracted with EtOAc (30 mL x 3). The combined organic layers were washed with brine (60 mL), dried over anhydrous Na₂SO₄, and filtered. The filtrate was concentrated to give the residue, which was purified by column chromatography on silica gel (PE:EA=0-30%) to give the desired product (793 mg, 97.5% yield) as colorless oil. LC-MS (ESI): [M+H]⁺=255.2; ¹H NMR (400 MHz, CDCl₃) δ 7.30 (d, *J* = 7.6 Hz, 1H), 6.76 (d, *J* = 11.6 Hz, 1H), 4.58 - 4.56 (m, 1H), 4.46 - 4.44 (m, 1H), 3.70 - 3.65 (m, 3H), 3.62 - 3.60 (m, 1H), 3.28 (t, *J* = 5.6 Hz, 2H), 2.88 (s, 3H), 2.26 (s, 3H).

### Step 9: Synthesis of methyl 3-amino-6-((2-(2-fluoroethoxy)ethyl)(methyl)amino)-5-methylbenzo[b]thiophene-2-carboxylate

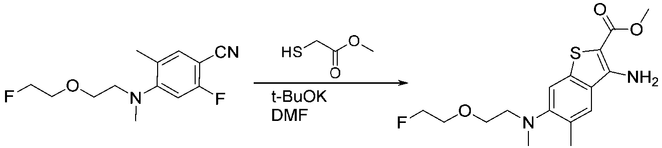

To a solution of 2-fluoro-4-((2-(2-fluoroethoxy)ethyl)(methyl)amino)-5-methylbenzonitrile (793 mg, 3.12 mmol) in DMF (10 mL) was added methyl 2-mercaptoacetate (1.019 g, 9.37 mmol) and *t*-BuOk (1.075 g, 9.37 mmol). After being stirred at 30 °C for 5 h, the mixture was quenched with H₂O (30 mL) and extracted with EtOAc (30 mL x 3). The combined organic layers were dried over anhydrous Na₂SO₄ and filtered. The filtrate was concentrated to give the residue, which was purified by column chromatography on silica gel (PE:EA=0-60 %) to give the desired product (300 mg, 27.6% yield) as yellow oil. LC-MS (ESI): [M+H]⁺=341.3; ¹H NMR (400 MHz, CD₃OD) δ 7.68 (s, 1H), 7.37 (s, 1H), 4.53 - 4.51 (m, 1H), 4.41 - 4.39 (m, 1H), 3.81 (s, 3H), 3.69 - 3.60 (m, 4H), 3.19 (t, *J* = 6.0 Hz, 2H), 2.81 (s, 3H), 2.40 (s, 3H).

### Step 10: Synthesis of 1-bromo-7-((2-(2-fluoroethoxy)ethyl)(methyl)amino)-8-methyl-10H-benzo[4,5]thieno[3,2-b]indol-2-ol

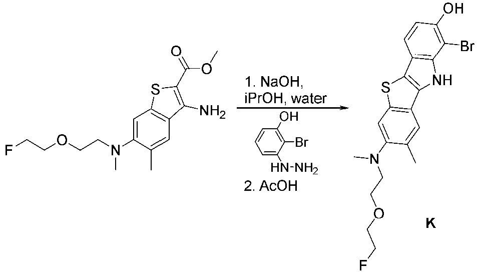

A mixture of 3-amino-6-((2-(2-fluoroethoxy)ethyl)(methyl)amino)-5-methylbenzo[b]thiophene-2-carboxylate (200 mg, 0.59 mmol) and NaOH (95 mg, 2.36 mmol) in isopropanol (3 mL) and H₂O (0.5 mL) was stirred at 80 °C for 3 h. After being cooled to room temperature, the solvent was removed. The residue was dissolved in acetic acid (3 mL), and 2-bromo-3-hydrazinylphenol (132 mg, 0.70 mmol) was added. The resulting mixture was stirred at 80 °C for 30 min. After the reaction was complete, the mixture was purified by pre-HPLC to give compound K (30.4 mg, 11.3% yield) as light brown solid. LC-MS (ESI): [M+H]⁺ = 451.1, 453.1; ¹⁹F NMR (376 MHz, DMSO-*d*₆): δ -221.44; ¹H NMR (400 MHz, DMSO-*d₆*) δ 11.71 (s, 1H), 9.96 (s, 1H), 8.16 (s, 0.5H), 8.03 (s, 1H), 7.68 (s, 1H), 7.50 (d, *J =* 8.4 Hz, 1H), 6.82 (d, *J* = 8.4 Hz, 1H), 4.57 - 4.55 (m, 1H), 4.45 - 4.43 (m, 1H), 3.68 - 3.66 (m, 1H), 3.63 - 3.59 (m, 3H), 3.11 (t, *J =* 6.0 Hz, 2H), 2.76 (s, 3H), 2.40 (s, 3H).

### Example 11: Synthesis of Compound L

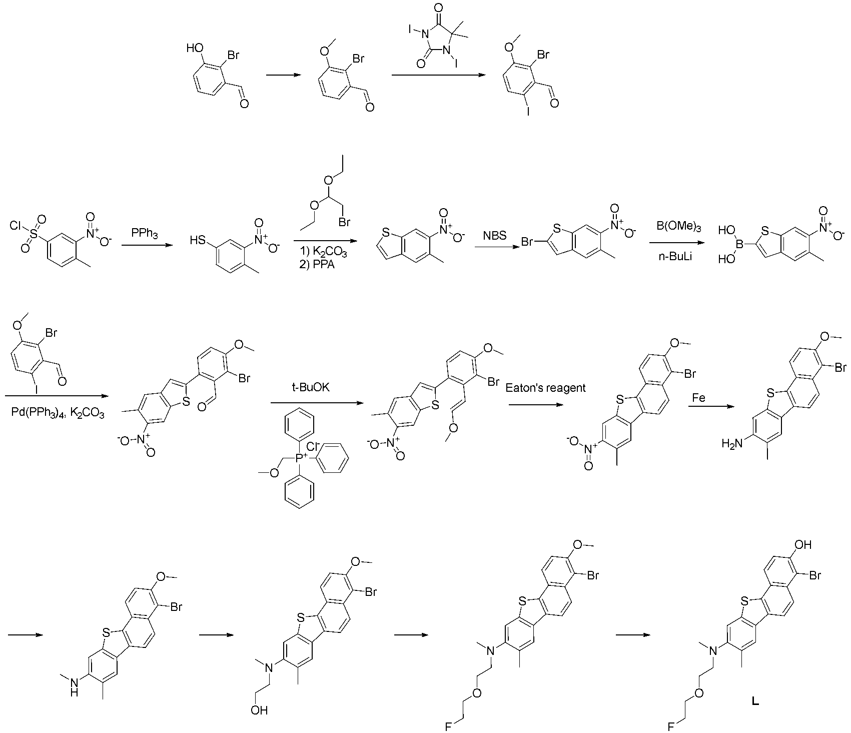

Compound L was synthesized following the procedure of Example 1 and Example 2.

Compound L: LC-MS (ESI): [M+H]⁺ = 462.1, 464.1.

### Example 12: Synthesis of Compound M

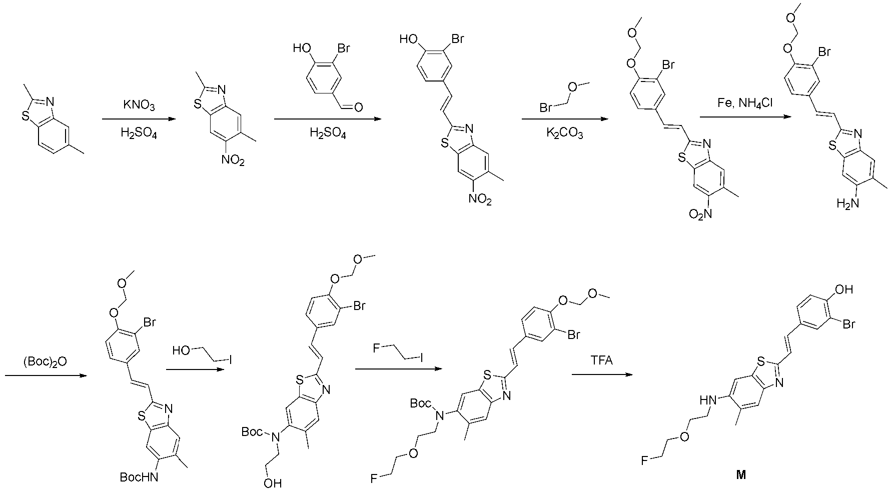

Compound M was synthesized following the procedure of Example 1 and Example 2.

Compound M: LC-MS (ESI): [M+H]⁺ = 451.0, 453.0.

### Another Method for Preparing Compound M

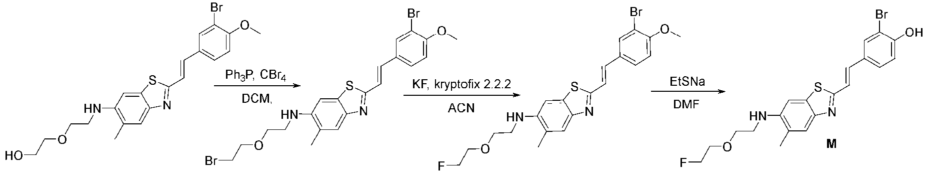

### Step 1: Synthesis of (E)-2-(3-bromo-4-methoxystyryl)-N-(2-(2-bromoethoxy)ethyl)-5-methylbenzo[d]thiazol-6-amine

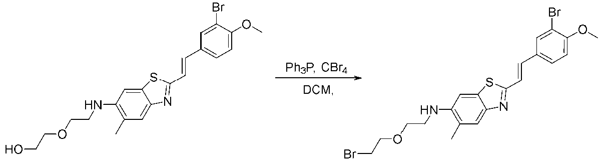

To a solution of (*E*)-2-(2-((2-(3-bromo-4-methoxystyryl)-5-methylbenzo[d]thiazol-6-yl)amino)ethoxy)ethan-1-ol (2.1 g, 4.5 mmol) and CBr₄ (6.0 g, 18.2 mmol) in anhydrous DCM (100 mL) was added PPh₃ (4.76 g, 18.2 mmol) at 0 °C. After being stirred at 0 °C for 3 h, the reaction mixture was poured into water (50 mL) and extracted with DCM (100 mL). The organic layer was dried over anhydrous Na₂SO₄ and filtered. The filtrate was concentrated to give the residue, which was purified by column chromatography on silica gel (PE:EA = 2:1) to give the desired product (2.1 g, 88% yield) as yellow solid. LC-MS (ESI): [M+H]⁺ = 524.9, 526.9; ¹H NMR (400 MHz, DMSO-*d*₆): δ 8.04 (s, 1 H), 7.76 - 7.74 (m, 2 H), 7.71 (s, 1 H), 7.50 (s, 2 H), 7.18-7.16 (d, *J =* 8.8 Hz, 1 H), 3.90 (s, 3 H), 3.78-3.75 (m, 4 H), 2.92-2.89 (m, 4 H), 2.39 (s, 3 H).

### Step 2: Synthesis of (E)-2-(3-bromo-4-methoxystyryl)-N-(2-(2-fluoroethoxy)ethyl)-5-methylbenzo[d]thiazol-6-amine

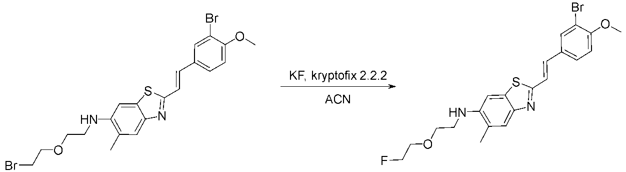

To a solution of (*E*)-2-(3-bromo-4-methoxystyryl)-*N*-(2-(2-bromoethoxy)ethyl)-5-methylbenzo[*d*]thiazol-6-amine (1.6 g, 3.0 mmol) in ACN (16 mL) was added CsF (2.65 g, 46.0 mmol) and kryptofix 2.2.2 (3.44 g, 9.2 mmol). After being stirred at 60 °C for 1 h under N₂, the reaction mixture was quenched with H₂O (30 mL) and extracted with EtOAc (40 mL x 2). The combined organic layers were washed with brine (60 mL), dried over anhydrous Na₂SO₄ and filtered. The filtrate was concentrated to give the residue, which was purified by column chromatography on silica gel (PE:EA=0%-30%) to give the desired product (240 mg, 17% yield) as yellow solid. LC-MS (ESI): [M+H]⁺ = 465.0, 467.0; ¹H NMR (400 MHz, DMSO-*d*₆): δ 7.99 (s, 1 H), 7.73-7.71 (d, *J* = 8.4 Hz, 1 H), 7.58 (s, 1 H), 7.44-7.31 (m, 2 H), 7.16-7.13 (m, 2 H), 5.17 (brs, 1 H), 4.61 (d, *J =* 4.0 Hz, 1 H), 4.49 (d, *J =* 4.0 Hz, 1 H), 3.89 (s, 3 H), 3.75-3,65 (m, 4 H), 3.38 (m, 2 H), 2.20 (s, 3 H).

### Step 3: Synthesis of (E)-2-bromo-4-(2-(6-((2-(2-fluoroethoxy)ethyl)amino)-5-methylbenzo[d]thiazol-2-yl)vinyl)phenol

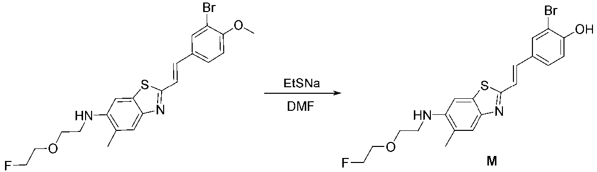

To a solution of (*E*)-2-(3-bromo-4-methoxystyryl)-*N*-(2-(2-fluoroethoxy)ethyl)-5-methylbenzo[*d*]thiazol-6-amine (240 mg, 0.52 mmol) in DMF (2.5 mL) was added EtSNa (222 mg, 2.59 mmol). After being stirred at 100 °C for 0.5 h, the mixture was quenched by water (20 mL) and extracted with EtOAc (30 mL x 2). The combined organic layers were washed with brine (20 mL), dried over anhydrous Na₂SO₄, and filtered. The filtrate was concentrated to give the residue, which was purified by column chromatography on silica gel (PE:EA=0%-35%) to give Compound M (30 mg, 13% yield) as yellow solid. LC-MS (ESI): [M+H]⁺ = 451.0, 453.0; ¹H NMR (400 MHz, DMSO-*d*₆): δ 10.59 (s, 1 H), 7.87 (s, 1 H), 7.58 - 7.56 (m, 2 H), 7.35-7.26 (m, 2 H), 7.12 (s, 1 H), 6.98-6.96 (d,*J* = 8.4 Hz, 1 H) 5.15 (m, 1 H), 4.62-4.59 (m, 1 H), 4.51-4.47 (d, *J* = 4.0 Hz, 1 H), 3.75-3.66 (m, 4 H), 3.37 (m, 2 H), 2.20 (s, 3 H).

### Example 13: Synthesis of Compound N

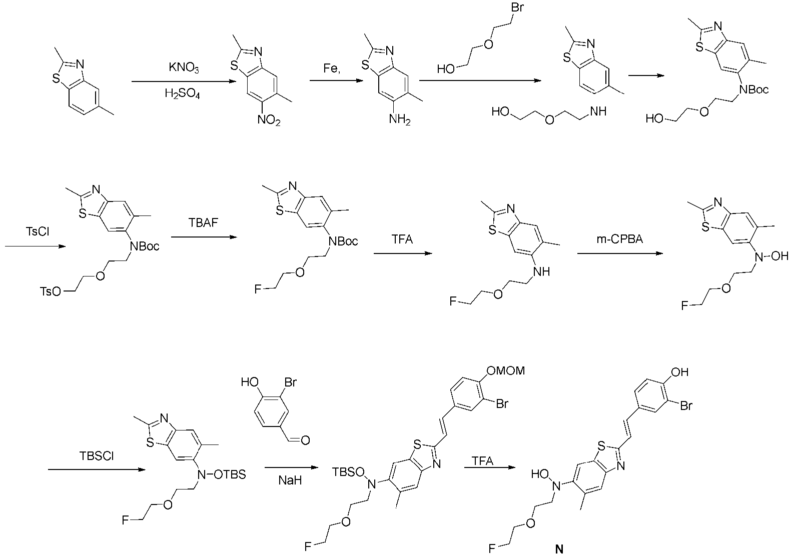

Compound N was synthesized following the procedure of Example 1 and Example 2.

Compound N: LC-MS (ESI): [M+H]⁺ = 467.0, 469.0.

### Example 14: Synthesis of Compound O

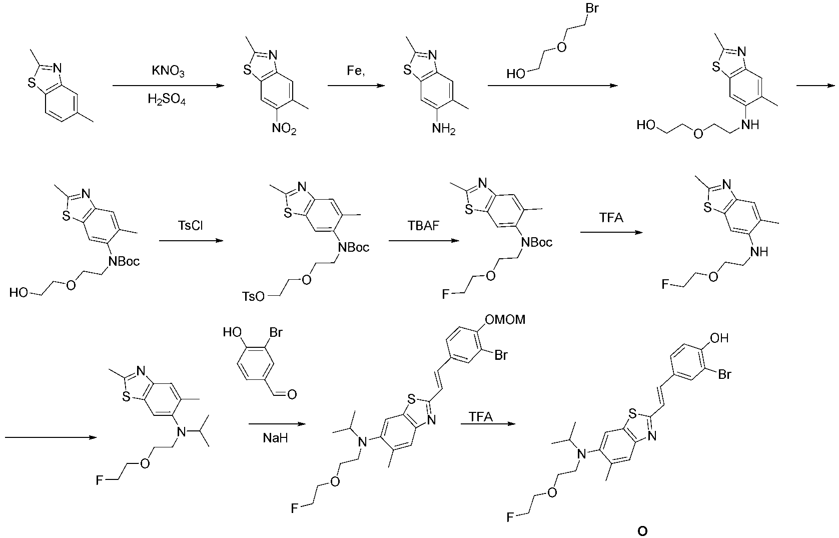

Compound O was synthesized following the procedure of Example 1 and Example 2.

Compound O: LC-MS (ESI): [M+H]⁺ = 493.1, 495.1.

### Another Method for Preparing Compound O

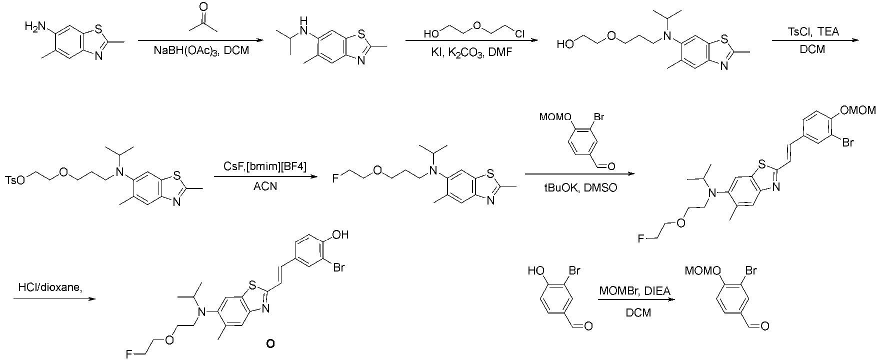

### Synthesis of (E)-2-bromo-4-(2-(6-((2-(2-fluoroethoxy)ethyl)(isopropyl)amino)-5-methylbenzo[d]thiazol-2-yl)vinyl)phenol

### Step 1: Synthesis of 3-bromo-4-(methoxymethoxy)benzaldehyde

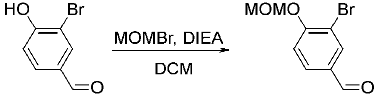

To a solution of 3-bromo-4-hydroxybenzaldehyde (1.0 g, 4.9 mmol) and DIEA (1.28 g, 9.9 mmol) in DCM (20 mL) was added MOMBr (4.7 g, 37.5 mmol) at room temperature. After being stirred at room temperature for 4 h, the solvent was removed. The residue was purified by column chromatography on silica gel (PE:EA=10:1) to give the desired product (1.02 g, 83.6% yield) as white solid. ¹H NMR (400 MHz, DMSO-*d₆*): δ 9.87 (s, 1H), 8.13 (d, *J* = 2.0 Hz, 1H), 7.90 (dd, *J* = 8.4, 2.0 Hz, 1H), 7.39 (d, *J* = 8.4 Hz, 1H), 5.43 (s, 2H), 3.43 (s, 3H).

### Step 2: Synthesis of N-isopropyl-2,5-dimethylbenzo[d]thiazol-6-amine

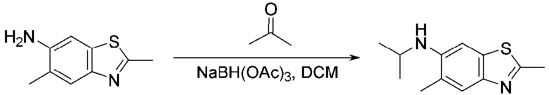

To a solution of 2,5-dimethylbenzo[*d*]thiazol-6-amine (700 mg, 3.9 mmol) in DCM (20 mL) was added acetone (2.3 g, 39 mmol) and NaBH(OAc)₃ (2.5 g, 11.8 mmol). The resulting mixture was stirred at room temperature for 16 h. After the reaction was complete, the solvent was removed. The residue was purified by column chromatography on silica gel (PE:EA=4:1) to give the desired product (680 mg, 78.6%) as yellow solid. LC-MS (ESI): [M+H]⁺ = 221.2; ¹H NMR (400 MHz, DMSO-*d₆*) δ 7.51 (s, 1H), 7.04 (s, 1H), 4.50 (d, *J* = 7.9 Hz, 1H), 3.69-3.61 (m, 1H), 2.66 (s, 3H), 2.18 (s, 3H), 1.21 (d, *J* = 6.3 Hz, 6H).

### Step 3: Synthesis of 2-(2-((2,5-dimethylbenzo[d]thiazol-6-yl)(isopropyl)amino)ethoxy)ethan-1-ol

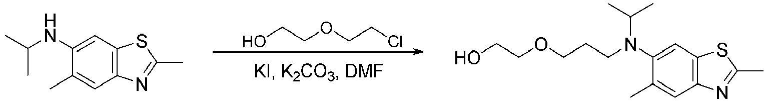

To a solution of *N*-isopropyl-2,5-dimethylbenzo[*d*]thiazol-6-amine (680 mg, 3 mmol) in DMF (10 mL) was added 2-(2-chloroethoxy)ethan-1-ol (1.9 g, 15 mmol), K₂CO₃ (854 mg, 6 mmol), and KI (1 g, 6 mmol). The resulting mixture was stirred at 100 °C for 24 h. After cooling to room temperature, water (50 mL) was added. The mixture was extracted with EtOAc (50 mL x 3). The combined organic layers were washed with saturated brine (60 mL), dried over anhydrous Na₂SO₄ and filtered. The filtrate was concentrated to give the residue, which was purified by column chromatography on silica gel (PE:EA= 1:1) to give the desired product (150 mg, 15.7% yield) as yellow oil. LC-MS (ESI): [M+H]⁺ = 309.4.

### Step 4: Synthesis of 2-(2-((2,5-dimethylbenzo[d]thiazol-6-yl)(isopropyl)amino)ethoxy)ethyl 4-methylbenzenesulfonate

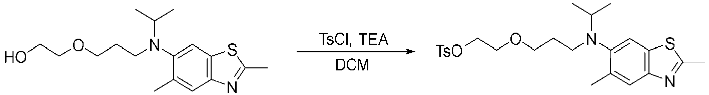

To a solution of 2-(2-((2,5-dimethylbenzo[*d*]thiazol-6-yl)(isopropyl)amino)ethoxy)ethan-1-ol (150 mg, 0.48 mmol) and TEA (98 mg, 0.97 mL) in DCM (5 mL) was added TsCl (139 mg, 0.73 mmol) at room temperature. After being stirred at room temperature for 4 h, the solvent was removed. The residue was purified by column chromatography on silica gel (PE:EA=3: 1) to give the desired product (180 mg, 80% yield) as brown solid. LC-MS (ESI): [M+H]⁺ = 463.4; ¹H NMR (400 MHz, DMSO-*d₆*) δ 7.79 (s, 1H), 7.77 - 7.66 (m, 3H), 7.44 (d, *J* = 8.0 Hz, 2H), 4.11 - 3.94 (m, 2H), 3.51 - 3.39 (m, 2H), 3.21 - 3.07 (m, 4H), 3.04 (q, *J* = 6.4 Hz, 1H), 2.74 (s, 3H), 2.41 (s, 3H), 2.29 (d, *J =* 0.8 Hz, 3H), 1.03 (d, *J* = 6.4 Hz, 6H).

### Step 5: Synthesis of N-(2-(2-fluoroethoxy)ethyl)-N-isopropyl-2,5-dimethylbenzo[d]thiazol-6-amine

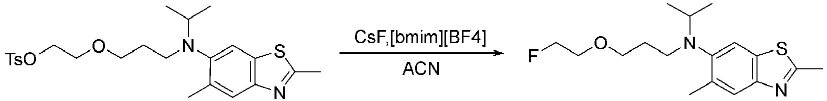

To solution of 2-(2-((2,5-dimethylbenzo[*d*]thiazol-6-yl)(isopropyl)amino)ethoxy)ethyl 4-methylbenzenesulfonate (150 mg, 0.32 mmol) in ACN (3 mL) was added [bmim][BF₄] (3 mL) and CsF (245 mg, 1.6 mmol). The resulting mixture was stirred at 100 °C for 2 h under N₂. The reaction mixture was quenched with H₂O (20 mL) and extracted with EtOAc (20 mL x 3). The combined organic layers were washed with brine (20 mL), dried over anhydrous Na₂SO₄ and filtered. The filtrate was concentrated under reduced pressure to give the residue, which was purified by column chromatography on silica gel (PE:EA=4:1) to give the desired product (90 mg, 74% yield) as brown oil. LC-MS (ESI): [M+H]⁺ = 311.4; ¹H NMR (400 MHz, CDCl₃) δ 7.71 (s, 1H), 7.53 (s, 1H), 4.44-4.32 (m, 2H), 3.53-3.09 (m, 7H), 2.74 (s, 3H), 2.34 (s, 3H), 1.07 (s, 6H).

### Step 6: Synthesis of (E)-2-(3-bromo-4-(methoxymethoxy)styryl)-N-(2-(2-fluoroethoxy)ethyl)-N-isopropyl-5-methylbenzo[d]thiazol-6-amine

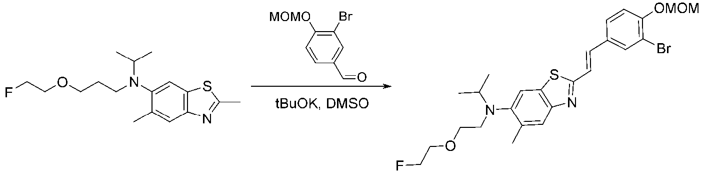

To a solution of *N*-(2-(2-fluoroethoxy)ethyl)-*N*-isopropyl-2,5-dimethylbenzo[*d*]thiazol-6-amine (70 mg, 0.22 mmol) in DMSO (2 mL) was added t-BuOK (50 mg, 0.45 mmol) and 3-bromo-4-(methoxymethoxy)benzaldehyde (55 mg, 0.22 mmol) at room temperature. After being stirred at room temperature for 2 h, the reaction mixture was diluted with water (20 mL) and extracted with EtOAc (20 mL x 3). The combined organic layers were washed with brine (30 mL), dried over anhydrous Na₂SO₄ and filtered. The filtrate was concentrated to give the residue, which was purified by column chromatography on silica gel (PE:EA=5:1) to give the desired product (100 mg, 64.2% yield) as brown solid. LC-MS (ESI): [M+H]⁺ = 537.4, 539.4; ¹H NMR (400 MHz, CDCl₃) δ 7.82 - 7.65 (m, 2H), 7.55 (s, 1H), 7.40 (dd, *J* = 8.6, 2.1 Hz, 1H), 7.30 (d, *J* = 16.3 Hz, 1H), 7.21 (s, 1H), 7.11 (d, *J* = 8.6 Hz, 1H), 5.22 (s, 2H), 4.46 (s, 1H), 4.34 (s, 1H), 3.46 (s, 5H), 3.23 (d, *J =* 90.9 Hz, 5H), 2.35 (s, 3H), 1.09 (s, 6H).

### Step 7: Synthesis of (E)-2-bromo-4-(2-(6-((2-(2-fluoroethoxy)ethyl)(isopropyl)amino)-5-methylbenzo[d]thiazol-2-yl)vinyl)phenol

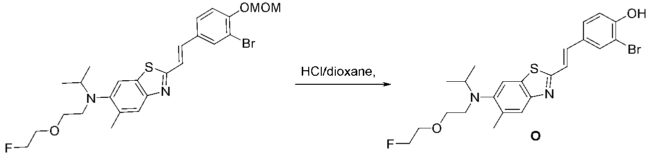

To a solution of (*E*)-2-(3-bromo-4-(methoxymethoxy)styryl)-*N*-(2-(2-fluoroethoxy)ethyl)-*N-*isopropyl-5-methylbenzo[*d*]thiazol-6-amine (80 mg, 0.14 mmol) in DCM (2 mL) was added HCl/1,4-dioxane (4 M, 2 mL). The resulting mixture was stirred at room temperature for 3 h. The precipitated solid was filtered off and dried *in vacuo* to give compound O (22.4 mg, 24.4% yield) as brown solid. LC-MS (ESI): [M+H]⁺ =493.1, 495.1; ¹H NMR (400 MHz, CD₃OD) δ 8.31 (s, 1H), 7.95 (s, 1H), 7.85 (d, *J* = 2.0 Hz, 1H), 7.69 - 7.48 (m, 2H), 7.35 (d, *J =* 16.0 Hz, 1H), 6.96 (d, *J =* 8.4 Hz, 1H), 4.49 (s, 1H), 4.37 (s, 1H), 4.12-4.04 (m, 3H), 3.69 (s, 1H), 3.48-3.47(m, 2H) 3.18 (s, 1H), 2.67 (s, 3H), 1.53-1.33 (m, 6H); ¹⁹FNMR (400 MHz, DMSO-*d*₆): δ -221.15.

### Example 15: Synthesis of Compound P

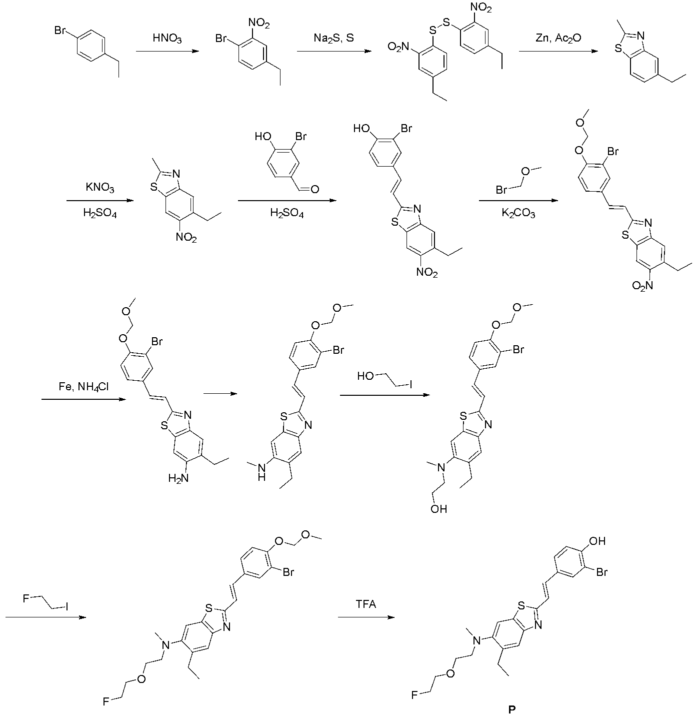

Compound P was synthesized following the procedure of Example 1 and Example 2.

Compound P: LC-MS (ESI): [M+H]⁺ = 479.1, 481.1.

### Another Method for Preparing Compound P

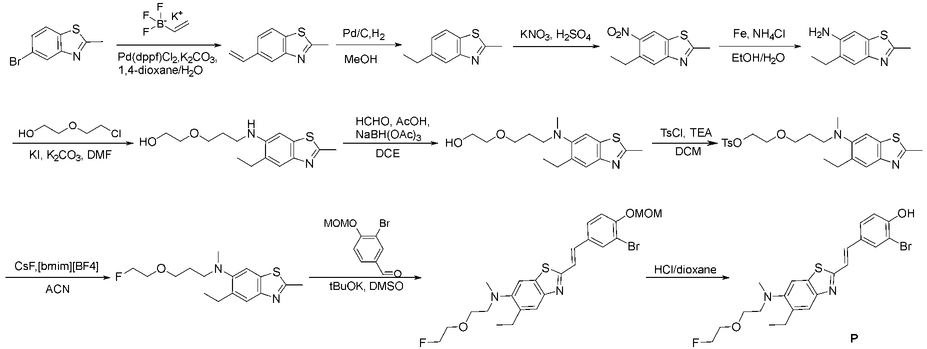

### Synthesis of (E)-2-bromo-4-(2-(5-ethyl-6-((2-(2-fluoroethoxy)ethyl)(methyl)amino)benzo[d]thiazol-2-yl)vinyl)phenol

### Step 1: Synthesis of 2-methyl-5-vinylbenzo[d]thiazole

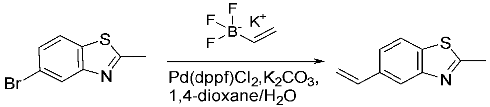

A mixture of 5-bromo-2-methylbenzo[*d*]thiazole (11 g, 48.2 mmol), potassium vinyltrifluoroborate (9.7 g, 72.3 mmol), K₂CO₃ (20 g, 144.6 mmol) and Pd(dppf)Cl₂ (1.76 g, 2.4 1mmol) in 1,4-dioxane/H₂O (110 mL, v/v=5/1) was stirred at 80 °C for 16 h under N₂. After the reaction was complete, the mixture was filtered, the filtrate was concentrated to give the residue, which was purified by column chromatography on silica gel (PE:EA=20:1) to give the desired product (6.87 g, 82% yield) as yellow oil. LC-MS (ESI): [M+H]⁺ =176.1; ¹H NMR (400 MHz, CDCl₃) δ 7.95 (d, *J* = 1.2 Hz, 1H), 7.75 (d, *J* = 8.4 Hz, 1H), 7.43 (dd, *J* = 8.4, 1.6 Hz, 1H), 6.83 (dd, *J* = 17.6, 10.8 Hz, 1H), 5.83 (dd, *J =* 17.6, 0.8 Hz, 1H), 5.31 (d, *J =* 10.8 Hz, 1H), 2.83 (s, 3H).

### Step 2: Synthesis of 5-ethyl-2-methylbenzo[d]thiazole

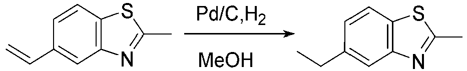

A mixture of 2-methyl-5-vinylbenzo[*d*]thiazole (6.87 g, 39 mmol) and Pd/C (10%, 0.7 g) in MeOH (70 mL) was stirred at room temperature for 16 h under H₂. After the reaction was complete, the mixture was filtered, the filtrate was concentrated to give the desired product (6.74 g, 97.1% yield) as yellow oil. LC-MS (ESI): [M+H]⁺ =178.2; ¹H NMR (400 MHz, CDCl₃) δ 7.78 (d, *J* = 0.8 Hz, 1H), 7.71 (d, *J =* 8.4 Hz, 1H), 7.20 (dd, *J =* 8.4, 1.6 Hz, 1H), 2.82 (s, 3H), 2.78 (dd, *J* = 15.2, 7.6 Hz, 2H), 1.30 (t, *J* = 7.6 Hz, 3H).

### Step 3: Synthesis of 5-ethyl-2-methyl-6-nitrobenzo[d]thiazole

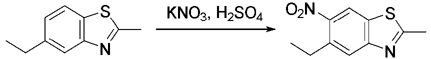

5-Ethyl-2-methylbenzo[d]thiazole (6.74 g, 38.02 mmol) was added to con.H₂SO₄ (70 mL) at 0 °C, then KNO₃ (4.23 g, 41.82 mmol) was added portionwise. After being stirred at room temperature for 2 h, the mixture was poured into ice-water (200 mL). The mixture was adjusted to pH 8 with saturated sodium carbonate solution and extracted with EtOAc (200 mL x 3). The combined organic layers were dried over anhydrous Na₂SO₄ and filtered. The filtrate was concentrated to give the residue, which was purified by column chromatography on silica gel (PE:EA = 20: 1) to give the desired product (4.2 g, 49.7% yield) as yellow solid. LC-MS (ESI): [M+H]⁺ =223.1; ¹H NMR (400 MHz, CDCl₃) δ 8.43 (s, 1H), 7.90 (s, 1H), 3.05 (q, *J* = 7.6 Hz, 2H), 2.89 (s, 3H), 1.35 (t, *J* = 7.6 Hz, 3H).

### Step 4: Synthesis of 5-ethyl-2-methylbenzo[d]thiazol-6-amine

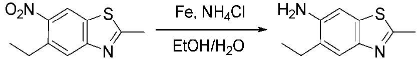

To a solution of 5-ethyl-2-methyl-6-nitrobenzo[*d*]thiazole(3 g, 13.5 mmol) in EtOH/H₂O (60 mL, v/v=5/1) was added NH₄Cl (7.2 g, 135 mmol) and iron powder (3.77 g, 67.5 mmol) at room temperature. The resulting mixture was stirred at 80 °C for 2 h. After being cooled to room temperature, the mixture was filtered. The filtrate was concentrated to give the residue, which was purified by column chromatography on silica gel (PE:EA = 5:1) to give the desired product (2.28 g, 87.9% yield) as yellow solid. LC-MS (ESI): [M+H]⁺ =193.1; ¹H NMR (400 MHz, CDCl₃) δ 7.67 (s, 1H), 7.11 (s, 1H), 2.76 (s, 3H), 2.63 (q, *J* = 7.6 Hz, 2H), 1.33 (t, *J* = 7.6 Hz, 3H).

### Step 5: Synthesis of 2-(2-((5-ethyl-2-methylbenzo[d]thiazol-6-yl)amino)ethoxy)ethan-1-ol

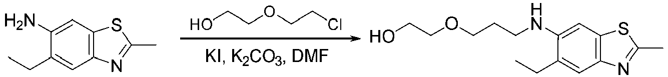

A solution of 5-ethyl-2-methylbenzo[*d*]thiazol-6-amine (2.28 g, 11.88 mmol), 2-(2-chloroethoxy)ethan-1-ol (7.4 g, 59.38 mmol), K₂CO₃ (4.93 g, 35.64 mmol) and KI (1.97 g, 11.88 mmol) in DMF (30 mL) was stirred at 100 °C for 16 h. After the reaction was complete, the solvent was removed. The residue was purified by column chromatography on silica gel (PE:EA=2:1) to give the desired product (2.25 g, 67.6% yield) as yellow oil. LC-MS (ESI): [M+H]⁺ =281.2; ¹H NMR (400 MHz, DMSO-d₆) δ 7.50 (s, 1H), 7.08 (s, 1H), 4.98 (t, *J* = 5.6 Hz, 1H), 4.63 (t, *J* = 5.2 Hz, 1H), 3.65 (t, *J* = 6.0 Hz, 2H), 3.57 - 3.43 (m, 4H), 3.30 (dd, *J* = 11.6, 6.0 Hz, 2H), 2.67 (s, 3H), 2.56 (q, *J =* 7.2 Hz, 2H), 1.20 (t, *J =* 7.2 Hz, 3H).

### Step 6: Synthesis of 2-(2-((5-ethyl-2-methylbenzo[d]thiazol-6-yl)(methyl)amino)ethoxy)ethan-1-ol

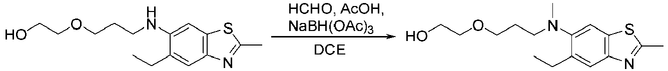

To a solution of 2-(2-((5-ethyl-2-methylbenzo[*d*]thiazol-6-yl)amino)ethoxy)ethan-1-ol (2.25 g, 8.04 mmol) in 1,2-dichloroethane (23 mL) was added formaldehyde (1.3 g, 16.08mmol, 36%wt) and AcOH (483.0 mg, 8.04 mmol). After being stirred at room temperature for 30 min, STAB (5.11g, 24.12 mmol) was added. After being stirred at room temperature for 1 h, the solvent was removed. The residue was purified by column chromatography on silica gel (PE:EA=3:2) to give the desired product (1.89 g, 80.0% yield) as colorless oil. LC-MS (ESI): [M+H]⁺ =295.2; ¹H NMR (400 MHz, DMSO-d₆) δ 7.78 (s, 1H), 7.70 (s, 1H), 4.55 (t, *J* = 5.4 Hz, 1H), 3.52 (t, *J =* 6.0 Hz, 2H), 3.47 (dd, *J* = 10.4, 5.2 Hz, 2H), 3.39 (t, *J =* 5.2 Hz, 2H), 3.04 (t, *J =* 6.0 Hz, 2H), 2.81 - 2.75 (m, 2H), 2.74 (s, 3H), 2.70 (s, 3H), 1.22 (t, *J =* 7.6 Hz, 3H).

### Step 7: Synthesis of 2-(2-((5-ethyl-2-methylbenzo[d]thiazol-6-yl)(methyl)amino)ethoxy)ethyl 4-methylbenzenesulfonate

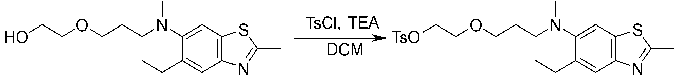

To a solution of 2-(2-((5-ethyl-2-methylbenzo[*d*]thiazol-6-yl)(methyl)amino)ethoxy)ethan-1-ol (0.5 g, 1.7 mmol) and TEA (687 mg, 6.8 mmol) in DCM (5 mL) was added TsCl (324 mg, 3.4 mmol) at 0 °C. After being stirred at room temperature for 16 h, the solvent was removed. The residue was purified by column chromatography on silica gel (PE: EA = 4: 1) to give the desired product (0.53 g, 69.6% yield) as colorless oil. LC-MS (ESI): [M+H]⁺ =449.2; ¹H NMR (400 MHz, CDCl₃) δ 7.86 - 7.70 (m, 3H), 7.52 (s, 1H), 7.32 (d, *J* = 8.0 Hz, 2H), 4.15 - 4.10 (m, 2H), 3.63 - 3.58 (m, 2H), 3.53 (s, 2H), 3.08 (s, 2H), 2.98-2.65(m, 8H), 2.43 (s, 3H), 1.26 (t, *J* = 7.2 Hz, 3H).

### Step 8: Synthesis of 5-ethyl-N-(2-(2-fluoroethoxy)ethyl)-N,2-dimethylbenzo[d]thiazol-6-amine

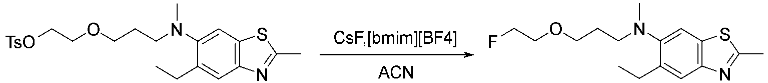

To a solution of 2-(2-((5-ethyl-2-methylbenzo[*d*]thiazol-6-yl)(methyl)amino)ethoxy)ethyl 4-methylbenzenesulfonate (430 mg, 0.96 mmol) in ACN (8 mL) was added [bmim][BF₄] (8 mL) and CsF (327 mg, 2.15 mmol). After being stirred at 100 °C for 4 h, the mixture was quenched with H₂O (20 mL) and extracted with EtOAc (30 mL x 3). The combined organic layers were washed with brine (60 mL), dried over anhydrous Na₂SO₄ and filtered. The filtrate was concentrated to give the residue, which was purified by column chromatography on silica gel (PE:EA=4:1) to give the desired product (0.2 g, 70.4% yield) as yellow oil. LC-MS (ESI): [M+H]⁺ =297.2; ¹H NMR (400 MHz, CDCl₃) δ 7.79 (s, 1H), 7.56 (s, 1H), 4.75 - 4.31 (m, 2H), 3.73 - 3.67 (m, 1H), 3.66 - 3.56 (m, 3H), 3.18 (s, 2H), 2.89 - 2.70 (m, 8H), 1.28 (t, *J =* 7.6 Hz, 3H).

### Step 9: Synthesis of (E)-2-(3-bromo-4-(methoxymethoxy)styryl)-5-ethyl-N-(2-(2-fluoroethoxy)ethyl)-N-methylbenzo[d]thiazol-6-amine

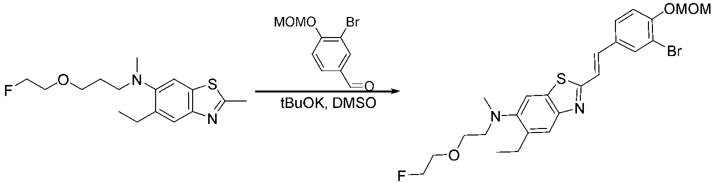

To a solution of 5-ethyl-N-(2-(2-fluoroethoxy)ethyl)-N,2-dimethylbenzo[d]thiazol-6-amine (0.2 g, 0.68 mmol) in DMSO (4 mL) was added t-BuOK (84 mg, 0.75 mmol) and 3-bromo-4-(methoxymethoxy)benzaldehyde (174 mg, 0.71 mmol) at room temperature. After being stirred at room temperature for 1 h, the reaction mixture was diluted with water (20 mL) and extracted with EtOAc (20 mL x 3). The combined organic layers were washed with brine (30 mL), dried over anhydrous Na₂SO₄ and filtered. The filtrate was concentrated to give the residue, which was purified by column chromatography on silica gel (PE:EA=4:1) to give the desired product (170 mg, 47.8% yield) as yellow oil. LC-MS (ESI): [M+H]⁺ =523.2, 525.2; ¹H NMR (400 MHz, CDCl₃) δ 7.84 (s, 1H), 7.78 (d, *J* = 2.0 Hz, 1H), 7.59 (s, 1H), 7.46 (dd, *J* = 8.4, 2.0 Hz, 1H), 7.37 (d, *J* = 16.0 Hz, 1H), 7.25 (d, *J =* 16.0 Hz, 1H), 7.17 (d, *J =* 8.4 Hz, 1H), 5.29 (s, 2H), 4.67 - 4.37 (m, 2H), 3.79 - 3.59 (m, 4H), 3.53 (s, 3H), 3.23 (s, 2H), 2.93 - 2.70 (m, 5H), 1.31 (t, *J* = 7.6 Hz, 3H).

### Step 10: Synthesis of (E)-2-bromo-4-(2-(5-ethyl-6-((2-(2-fluoroethoxy)ethyl)(methyl)amino)benzo[d]thiazol-2-yl)vinyl)phenol

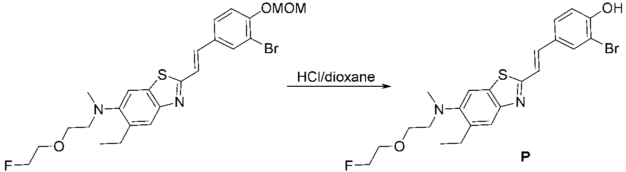

To a solution of (*E*)-2-(3-bromo-4-(methoxymethoxy)styryl)-5-ethyl-*N*-(2-(2-fluoroethoxy)ethyl)-*N*-methylbenzo[d]thiazol-6-amine (70 mg, 0.13 mmol) in 1,4-dioxane (2 mL) was added HCl/1,4-dioxane (4 M, 4 mL). The resulting mixture was stirred at room temperature for 20 h. The precipitated solid was filtered off, washed successively with EtOAc (20 ml) and Et₂O (10 mL) to give compound P (49.6 mg, 72.1% yield) as yellow solid. LC-MS (ESI): [M+H]⁺ =479.1, 481.1; ¹⁹F NMR (376 MHz, MeOD) δ -221.40; ¹H NMR (400 MHz, MeOD) δ 8.50 (s, 1H), 7.97 (s, 1H), 7.87 (d, *J* = 1.6 Hz, 1H), 7.72 (d, *J* = 16.4 Hz, 1H), 7.58 (dd, *J =* 8.4, 2.0 Hz, 1H), 7.38 (d, *J =* 16.0 Hz, 1H), 6.96 (d, *J* = 8.4 Hz, 1H), 4.57 - 4.37 (m, 2H), 4.03 (t, *J* = 4.4 Hz, 2H), 3.64 - 3.47 (m, 4H), 3.43 (s, 3H), 3.05 (q, *J* = 7.2 Hz, 2H), 1.43 (t, *J =* 7.2 Hz, 3H).

### Example 16: Synthesis of Compound Q

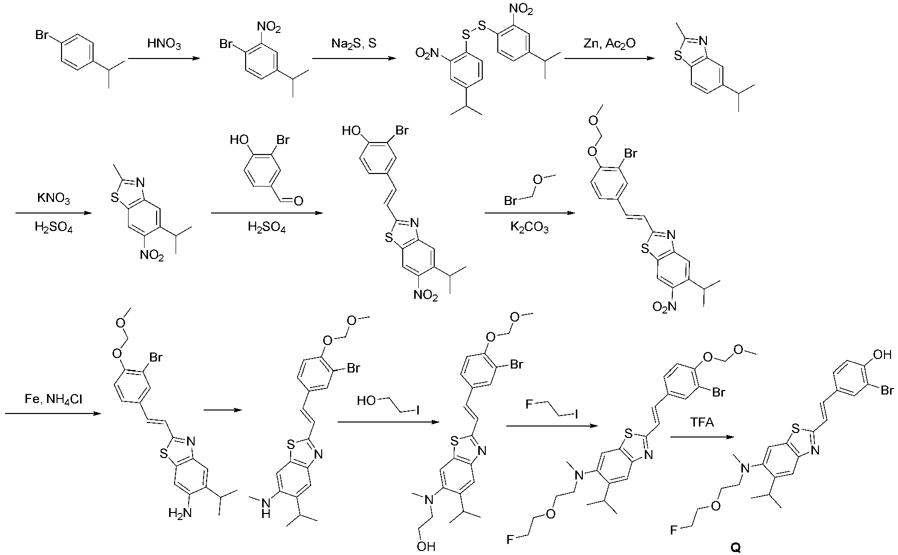

Compound Q was synthesized following the procedure of Example 1 and Example 2.

Compound Q: LC-MS (ESI): [M+H]⁺ = 493.1, 495.1.

### Another Method for Preparing Compound Q

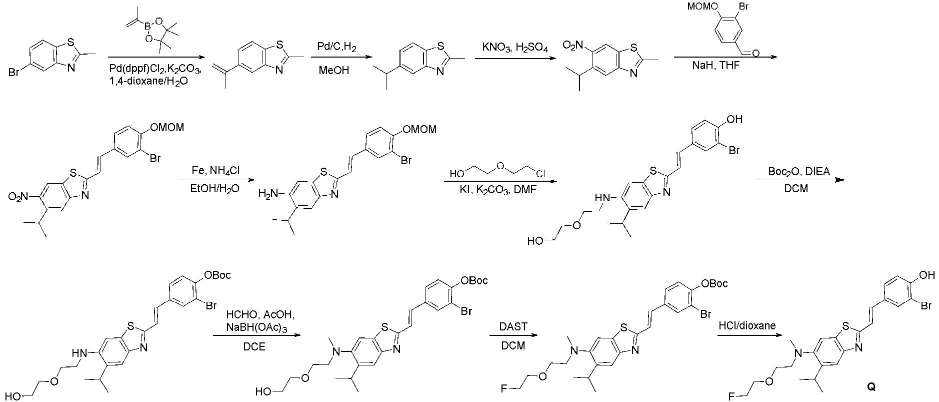

### Synthesis of (E)-2-bromo-4-(2-(6-((2-(2-fluoroethoxy)ethyl)(methyl)amino)-5-isopropylbenzo[d]thiazol-2-yl)vinyl)phenol

### Step 1: Synthesis of 2-methyl-5-(prop-1-en-2-yl)benzo[d]thiazole

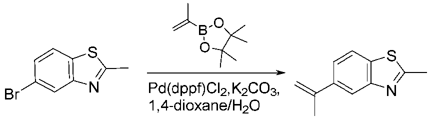

A mixture of 5-bromo-2-methylbenzo[*d*]thiazole (11 g, 48.2 mmol), 4,4,5,5-tetramethyl-2-(prop-1-en-2-yl)-1,3,2-dioxaborolane (12.1 g, 72.3 mmol), K₂CO₃ (20 g, 144.6 mmol) and Pd(dppf)Cl₂ (1.76 g, 2.4 1mmol) in 1,4-dioxane/H₂O (110 mL, v/v=5/1) was stirred at 70 °C for 16 h under N₂. After the reaction was complete, the mixture was filtered, the filtrate was concentrated to give the residue, which was purified by column chromatography on silica gel (PE:EA=20:1) to give the desired product (7.2 g, 79.1% yield) as yellow oil. LC-MS (ESI): [M+H]⁺=190.2; ¹H NMR (400 MHz, CDCl₃) δ 8.03 (d, *J* = 1.6 Hz, 1H), 7.75 (d, *J =* 8.4 Hz, 1H), 7.51 (dd, *J* = 8.4, 2.0 Hz, 1H), 5.46 (s, 1H), 5.19 - 5.12 (m, 1H), 2.84 (s, 3H), 2.22 (s, 3H).

### Step 2: Synthesis of 5-isopropyl-2-methylbenzo[d]thiazole

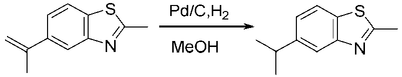

A mixture of 2-methyl-5-(prop-1-en-2-yl)benzo[*d*]thiazole (7.2 g, 38 mmol) and Pd/C (10%, 0.72 g) in MeOH (72 mL) was stirred at room temperature for 16 h under H₂. After the reaction was complete, the mixture was filtered, the filtrate was concentrated to give the desired product (7.1 g, 97.7% yield) as yellow oil. LC-MS (ESI): [M+H]⁺ =192.2.

### Step 3: Synthesis of 5-isopropyl-2-methyl-6-nitrobenzo[d]thiazole

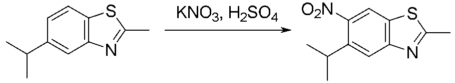

5-Isopropyl-2-methyl-6-nitrobenzo[*d*]thiazole(7.1 g, 37.11 mmol) was dissolved in con.H₂SO₄ (70 mL) at 0 °C, then KNO₃ (4.12 g, 40.82 mmol) was added portionwise. After being stirred at room temperature for 2 h, the mixture was poured into ice-water (200 mL). The mixture was adjusted to pH 8 with saturated sodium carbonate solution and extracted with EtOAc (300 mL x 3). The combined organic layers were dried over anhydrous Na₂SO₄ and filtered. The filtrate was concentrated to give the residue, which was purified by column chromatography on silica gel (PE:EA = 20:1) to give the desired product (4.7 g, 53.6% yield) as yellow solid. LC-MS (ESI): [M+H]⁺ = 237.2; ¹H NMR (400 MHz, CDCl₃) δ 8.25 (s, 1H), 8.03 (s, 1H), 3.64 - 3.44 (m, 1H), 2.89 (s, 3H), 1.36 (d, *J* = 6.8 Hz, 6H).

### Step 4: Synthesis of (E)-2-(3-bromo-4-(methoxymethoxy)styryl)-5-isopropyl-6-nitrobenzo[d]thiazole

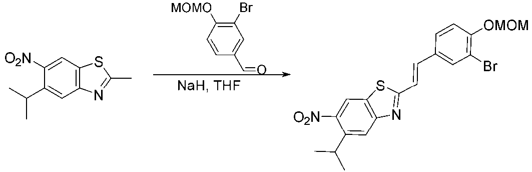

To a solution of 5-isopropyl-2-methyl-6-nitrobenzo[d]thiazole (4.7 g, 19.9 mmol) in THF (47 mL) was added NaH (1.6 g, 39.8 mmol, wt: 60%) at 0 °C. After being stirred at 0 °C for 30 min, 3-bromo-4-(methoxymethoxy)benzaldehyde (5.12 g, 20.9 mmol) was added. The mixture was stirred for 16 h at room temperature. The mixture was quenched by water (20 mL) and extracted with DCM (50 mL x 3). The combined organic layers were dried over anhydrous Na₂SO₄ and filtered. The filtrate was concentrated to give the residue, which was purified by column chromatography on silica gel (100% DCM) to give the desired product (4.7 g, 50.9% yield) as yellow solid. LC-MS (ESI): [M+H]⁺ =463.2, 465.2; ¹H NMR (400 MHz, CDCl₃) δ 8.28 (s, 1H), 8.06 (s, 1H), 7.83 (d, *J =* 2.0 Hz, 1H), 7.55 (d, *J* = 16.0 Hz, 1H), 7.50 (dd, *J* = 8.8, 2.0 Hz, 1H), 7.30 (s, 1H), 7.20 (d, *J* = 8.4 Hz, 1H), 5.31 (s, 2H), 3.58 - 3.50 (m, 4H), 1.38 (d, *J* = 6.8 Hz, 6H).

### Step 5: Synthesis of (E)-2-(3-bromo-4-(methoxymethoxy)styryl)-5-isopropylbenzo[d]thiazol-6-amine

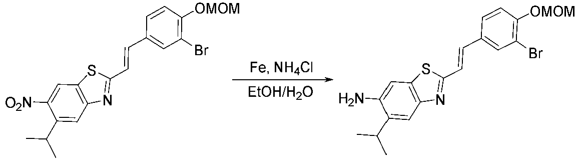

To a solution of (*E*)-2-(3-bromo-4-(methoxymethoxy)styryl)-5-isopropyl-6-nitrobenzo[d]thiazole (4.7 g, 10.14 mmol) in EtOH/H₂O (60 mL, v/v=5:1) was added NH₄Cl (5.42 g, 101.4 mmol) and iron powder (2.83 g, 50.7 mmol) at room temperature. The resulting mixture was stirred at 80 °C for 16 h. After being cooled to room temperature, the mixture was filtered. The filtrate was concentrated to give the residue, which was purified by column chromatography on silica gel to give the desired product (3.85 g, 87.5% yield) as yellow solid. LC-MS (ESI): [M+H]⁺ =433.2, 435.2; ¹H NMR (400 MHz, DMSO-d₆) δ 8.06 (d, *J* = 2.0 Hz, 1H), 7.76 (dd, *J* = 8.8, 2.0 Hz, 1H), 7.63 (s, 1H), 7.49 (d, *J* = 16.0 Hz, 1H), 7.37 (d, *J* = 16.0 Hz, 1H), 7.29 (d, *J* = 8.8 Hz, 1H), 7.16 (d, *J* = 4.8 Hz, 1H), 5.39 (s, 4H), 3.48 (s, 3H), 3.18-3.05 (m, 1H), 1.28 (d, *J* = 6.8 Hz, 6H).

### Step 6: Synthesis of (E)-2-bromo-4-(2-(6-((2-(2-hydroxyethoxy)ethyl)amino)-5-isopropylbenzo[d]thiazol-2-yl)vinyl)phenol

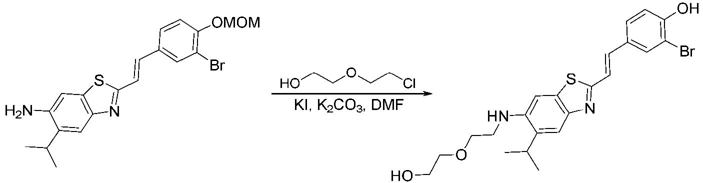

To a solution of (*E*)-2-(3-bromo-4-(methoxymethoxy)styryl)-5-isopropylbenzo[*d*]thiazol-6-amine (3.7 g, 8.54 mmol) in DMF (50 mL) was added K₂CO₃ (3.58 g, 25.62 mmol), KI (1.42 g, 8.54 mmol), and 2-(2-chloroethoxy)ethan-1-ol (10.6 g, 85.4 mmol). The resulting mixture was stirred at 100 °C for 16 h. After the reaction was complete, the solvent was removed. The residue was purified by column chromatography on silica gel (DCM:MeOH=20:1) to give the desired product (1.6 g, 20% yield) as yellow solid. LC-MS (ESI): [M+H]⁺ =477.2, 479.2.

### Step 7: Synthesis of (E)-2-bromo-4-(2-(6-((2-(2-hydroxyethoxy)ethyl)amino)-5-isopropylbenzo[d]thiazol-2-yl)vinyl)phenyl tert-butyl carbonate

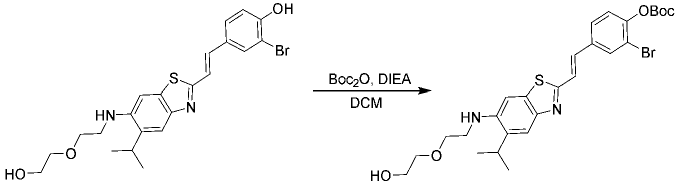

To a solution (*E*)-2-bromo-4-(2-(6-((2-(2-hydroxyethoxy)ethyl)amino)-5-isopropylbenzo[d]thiazol-2-yl)vinyl)phenol (1.6 g, 3.35 mmol) and DIEA (1.3 g, 10.05 mmol) in DCM (32 mL) was added Boc₂O (0.73 g, 3.35 mmol). After being stirred at 40 °C for 16 h, the solvent was removed. The residue was purified by column chromatography on silica gel (PE:EA = 1:1) to give the desired product (0.33 g, 17% yield) as yellow oil. LC-MS (ESI): [M+H]⁺ =577.3, 579.3; ¹H NMR (400 MHz, DMSO-d₆) δ 8.06 (d, *J =* 2.0 Hz, 1H), 7.76 (dd, *J =* 8.8, 2.0 Hz, 1H), 7.63 (s, 1H), 7.49 (d, *J* = 16.0 Hz, 1H), 7.37 (d, *J* = 16.0 Hz, 1H), 7.29 (d, *J* = 8.8 Hz, 1H), 7.16 (d, *J* = 4.8 Hz, 1H), 5.39 (s, 4H), 3.48 (s, 3H), 3.12-3.02 (m, 1H), 1.28 (d, *J =* 6.8 Hz, 6H).

### Step 8: Synthesis of (E)-2-bromo-4-(2-(6-((2-(2-hydroxyethoxy)ethyl)(methyl)amino)-5-isopropylbenzo[d]thiazol-2-yl)vinyl)phenyl tert-butyl carbonate

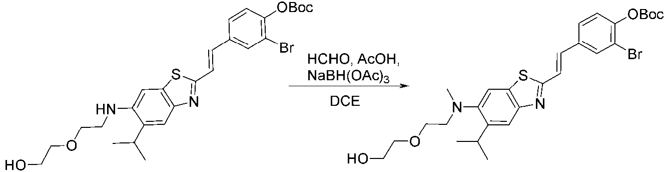

To a solution of (*E*)-2-bromo-4-(2-(6-((2-(2-hydroxyethoxy)ethyl)amino)-5-isopropylbenzo[*d*]thiazol-2-yl)vinyl)phenyl *tert*-butyl *c*arbonate (236 mg, 0.41 mmol) in DCE (4 mL) was added aqueous formaldehyde (67.0 mg, 0.82 mmol, 30% wt) and AcOH (25.0 mg, 0.41 mmol). After being stirred at room temperature for 30 min, STAB (260.0 mg, 1.23 mmol) was added. The resulting mixture was stirred at room temperature for 0.5 h. The solvent was removed. The residue was purified by column chromatography on silica gel (PE:EA=2:1) to give the desired product (0.17 g, 70.2% yield) as yellow oil. LC-MS (ESI): [M+H]⁺=591.2, 593.2; ¹H NMR (400 MHz, DMSO-d₆) δ 8.18 (d, *J =* 1.6 Hz, 1H), 7.89 (s, 1H), 7.86 - 7.76 (m, 2H), 7.62 (q, *J =* 16.0 Hz, 2H), 7.43 (d, *J =* 8.4 Hz, 1H), 4.56 (t, *J* = 5.2 Hz, 1H), 3.66 - 3.57 (m, 1H), 3.53 (t, *J* = 6.0 Hz, 2H), 3.48 (dd, *J* = 10.4, 5.2 Hz, 2H), 3.40 (t, *J =* 5.2 Hz, 2H), 3.08 (t, *J =* 6.0 Hz, 2H), 2.72 (s, 3H), 1.52 (s, 9H), 1.23 (d, *J =* 6.8 Hz, 6H).

### Step 9: Synthesis of (E)-2-bromo-4-(2-(6-((2-(2-fluoroethoxy)ethyl)(methyl)amino)-5-isopropylbenzo[d]thiazol-2-yl)vinyl)phenyl tert-butyl carbonate

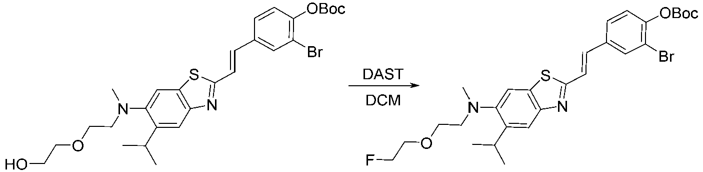

To a solution of (*E*)-2-bromo-4-(2-(6-((2-(2-hydroxyethoxy)ethyl)(methyl)amino)-5-isopropylbenzo[*d*]thiazol-2-yl)vinyl)phenyl *tert*-butyl carbonate (0.17 g, 0.29 mmol) in DCM (3 mL) was added DAST (92 mg, 0.57 mmol). After being stirred at room temperature for 2 h, the mixture was quenched with saturated aqueous sodium bicarbonate solution and extracted with DCM. The combined organic layers were dried over anhydrous Na₂SO₄ and filtered. The filtrate was concentrated to give the residue, which was purified by prep-HPLC to give the desired product (49 mg, 28% yield) as yellow oil. LC-MS (ESI): [M+H]⁺ =593.2, 595.2; ¹H NMR (400 MHz, CDCl₃) δ 7.90 (s, 1H), 7.80 (d, *J* = 2.0 Hz, 1H), 7.60 (s, 1H), 7.52 (dd, *J* = 8.4, 2.0 Hz, 1H), 7.40 (d, *J* = 16.0 Hz, 1H), 7.30 (d, *J* = 16.0 Hz, 1H), 7.24 (d, *J =* 8.4 Hz, 1H), 4.68 - 4.37 (m, 2H), 3.74 - 3.57 (m, 5H), 3.20 (t, *J* = 6.0 Hz, 2H), 2.79 (s, 3H), 1.58 (s, 9H), 1.27 (d, *J* = 6.8 Hz, 6H).

### Step 10: Synthesis of (E)-2-bromo-4-(2-(6-((2-(2-fluoroethoxy)ethyl)(methyl)amino)-5-isopropylbenzo[d]thiazol-2-yl)vinyl)phenol

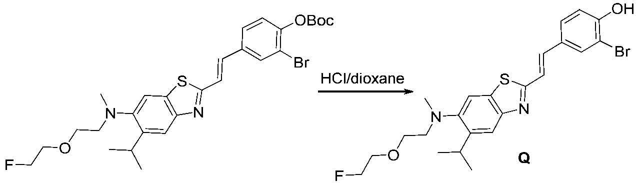

To a solution of (*E*)-2-bromo-4-(2-(6-((2-(2-fluoroethoxy)ethyl)(methyl)amino)-5-isopropylbenzo[*d*]thiazol-2-yl)vinyl)phenyl *tert-*butyl carbonate (45 mg, 0.08 mmol) in 1,4-dioxane (2 mL) was added HCl/1,4-dioxane (4 M, 8 mL). The resulting mixture was stirred at room temperature for 20 h. The precipitated solid was filtered off, washed successively with EtOAc (20 ml) and Et₂O (10 mL) to give compound Q (29.7 mg, 74.2% yield) as yellow solid. LC-MS (ESI): [M+H]⁺ =493.1, 495.1; ¹⁹F NMR (376 MHz, CD₃OD) δ -224.14; ¹H NMR (400 MHz, CD₃OD) δ 8.43 (s, 1H), 8.06 (s, 1H), 7.86 (d, *J* = 2.0 Hz, 1H), 7.65 (d, *J* = 16.0 Hz, 1H), 7.56 (dd, *J* = 8.4, 2.0 Hz, 1H), 7.36 (d, *J* = 16.0 Hz, 1H), 6.97 (d, *J =* 8.4 Hz, 1H), 4.59 - 4.42 (m, 2H), 4.01 (t, *J =* 4.8 Hz, 2H), 3.70 - 3.51 (m, 4H), 3.51 - 3.45 (m, 1H), 3.43 (s, 3H), 1.42 (d, *J =* 6.8 Hz, 6H).

### Example 17: Synthesis of Compound R

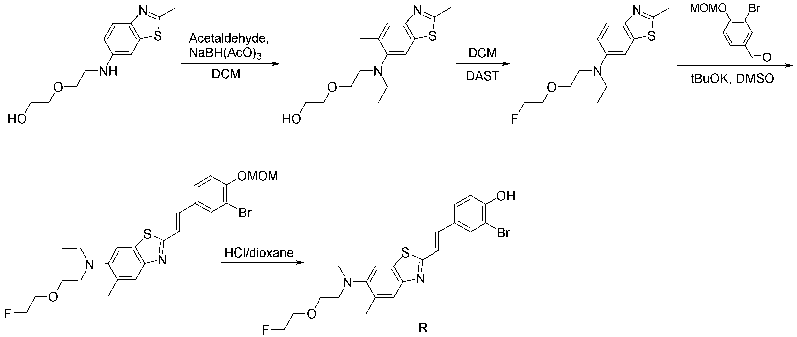

### Synthesis of (E)-2-bromo-4-(2-(6-(ethyl(2-(2-fluoroethoxy)ethyl)amino)-5-methylbenzo[d]thiazol-2-yl)vinyl)phenol

### Step 1: Synthesis of 2-(2-((2,5-dimethylbenzo[d]thiazol-6-yl)(ethyl)amino)ethoxy)ethan-1-ol

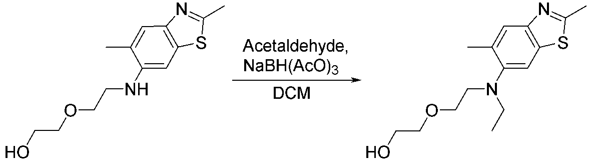

To a solution of 2-(2-((2,5-dimethylbenzo[*d*]thiazol-6-yl)amino)ethoxy)ethan-1-ol (500 mg, 1.87 mmol) in DCM (50 mL) was added acetaldehyde (827 mg, 18.7 mmol) and NaBH(OAc)₃ (1.19 g, 5.64 mmol). The resulting mixture was stirred at room temperature for 2 h. After the reaction was complete, the solvent was removed. The residue was purified by column chromatography on silica gel (PE:EA=4:1) to give the desired product (450 mg, 81% yield) as brown solid. LC-MS (ESI): [M+H]⁺ =295.3; ¹H NMR (400 MHz, CDCl₃) δ 7.68 (s, 1H), 7.47 (s, 1H), 3.64 - 3.52 (m, 2H), 3.50 - 3.31 (m, 4H), 3.17 (t, *J* = 6.0 Hz, 2H), 3.00 (d, *J* = 8.4 Hz, 2H), 2.72 (s, 3H), 2.37 (d, *J =* 13.2 Hz, 3H), 0.94 (t, *J* = 7.2 Hz, 3H).

### Step 2: Synthesis of N-ethyl-N-(2-(2-fluoroethoxy)ethyl)-2,5-dimethylbenzo[d]thiazol-6-amine

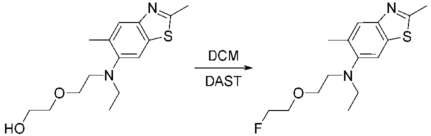

To a solution of 2-(2-((2,5-dimethylbenzo[*d*]thiazol-6-yl)(ethyl)amino)ethoxy)ethan-1-ol (350 mg, 1.2 mmol) in DCM (20 mL) was added DAST (383 g, 2.4 mmol) at 0 °C. After being stirred at 0 °C for 4 h, the mixture was quenched with H₂O (50 mL) and extracted with DCM (40 mL x 3). The combined organic layers were washed with saturated brine (60 mL), dried over anhydrous Na₂SO₄ and filtered. The filtrate was concentrated to give the residue, which was purified by column chromatography on silica gel (PE:EA = 4:1) to give the desired product (120 mg, 26.5% yield) as brown solid. LC-MS (ESI): [M+H]⁺ =297.3; ¹H NMR (400 MHz, CDCl₃) δ 7.68 (s, 1H), 7.48 (s, 1H), 4.47 (s, 1H), 4.35 (s, 1H), 3.66 - 3.37 (m, 4H), 3.19 (d, *J =* 6.8 Hz, 2H), 3.09 - 2.88 (m, 2H), 2.73 (s, 3H), 2.34 (s, 3H), 0.94 (t, *J* = 7.2 Hz, 3H).

### Step 3: Synthesis of (E)-2-(3-bromo-4-(methoxymethoxy)styryl)-N-ethyl-N-(2-(2-fluoroethoxy)ethyl)-5-methylbenzo[d]thiazol-6-amine

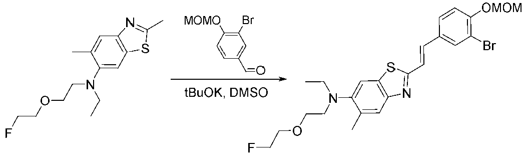

To a solution of *N*-ethyl-*N*-(2-(2-fluoroethoxy)ethyl)-2,5-dimethylbenzo[d]thiazol-6-amine (90 mg, 0.3 mmol) and *t*-BuOK (68 mg, 0.6 mmol) in DMSO (10 mL) was added 3-bromo-4-(methoxymethoxy)benzaldehyde (74 mg, 0.3 mmol) at room temperature. The resulting mixture was stirred at room temperature for 4 h. The reaction mixture was quenched with H₂O (50 mL) and extracted with EtOAc (50 mL x 3). The combined organic phases were washed with saturated brine (100 mL), dried over anhydrous Na₂SO₄, filtered, and concentrated under reduced pressure. The crude product was purified by column chromatography on silica gel (PE:EA = 4:1) to give the desired product (90 mg, 42.5% yield) as brown solid. LC-MS (ESI): [M+H]⁺ =523.4, 525.4; ¹H NMR (400 MHz, DMSO-*d*₆) δ 8.07 (d, *J =* 2.1 Hz, 1H), 7.84 (s, 1H), 7.80 - 7.73 (m, 2H), 7.52 (d, *J =* 2.8 Hz, 2H), 7.26 (d, *J* = 8.8 Hz, 1H), 5.35 (s, 2H), 4.56 - 4.46 (m, 1H), 4.45 - 4.34 (m, 1H), 3.67 - 3.59 (m, 1H), 3.56 - 3.52 (m, 1H), 3.48 (t, *J =* 6.0 Hz, 2H), 3.43 (s, 3H), 3.19 (t, *J =* 6.0 Hz, 2H), 3.09 (q, *J =* 7.2 Hz, 2H), 2.37 (s, 3H), 0.96 (t, *J* = 7.2 Hz, 3H).

### Step 4: Synthesis of (E)-2-bromo-4-(2-(6-(ethyl(2-(2-fluoroethoxy)ethyl)amino)-5-methylbenzo[d]thiazol-2-yl)vinyl)phenol

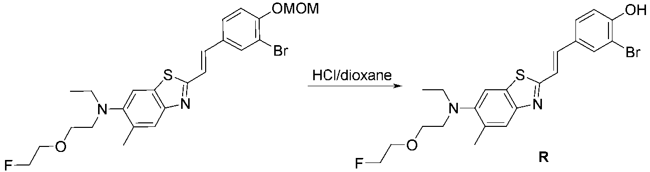

To a solution of (*E*)-2-(3-bromo-4-(methoxymethoxy)styryl)-*N*-ethyl-*N*-(2-(2-fluoroethoxy)ethyl)-5-methylbenzo[*d*]thiazol-6-amine (70 mg, 0.13 mmol) in DCM (2 mL) was added HCl/1,4-dioxane (4 M, 2 mL). The resulting mixture was stirred at room temperature for 4 h. The precipitated solid was filtered off and dried *in vacuo* to give compound R (26.8 mg, 32.5% yield) as brown solid. LC-MS (ESI): [M+H]⁺=479.3/481.3; ¹H NMR (400 MHz, CD₃OD) δ 8.36 (s, 1H), 7.98 (s, 1H), 7.88 (d, *J =* 2.4 Hz, 1H), 7.64 (d, *J =* 16.4 Hz, 1H), 7.57 (dd, *J =* 8.4, 2.0 Hz, 1H), 7.37 (d, *J* = 16.0 Hz, 1H), 6.98 (d, *J =* 8.4 Hz, 1H), 4.55 (s, 1H), 4.43 (s, 1H), 4.01 (d, *J* = 5.2 Hz, 2H), 3.87 - 3.74 (m, 2H), 3.61-3.53 (m, 4H), 2.73 - 2.61 (m, 3H), 1.16 (t, *J* = 7.1 Hz, 3H); ¹⁹F NMR (376 MHz, DMSO-*d*₆): δ -221.19.

### Example 18: Synthesis of Compound S

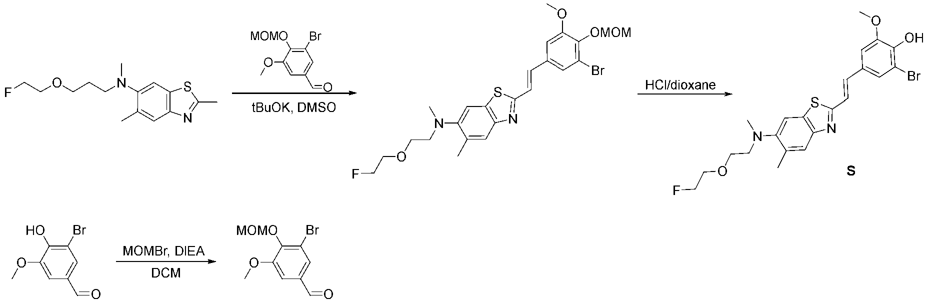

### Synthesis of (E)-2-bromo-4-(2-(6-((2-(2-fluoroethoxy)ethyl)(methyl)amino)-5-methylbenzo[d]thiazol-2-yl)vinyl)-6-methoxyphenol

### Step 1: Synthesis of 3-bromo-5-methoxy-4-(methoxymethoxy)benzaldehyde

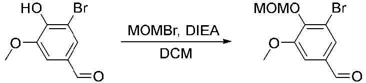

To a solution of 3-bromo-4-hydroxy-5-methoxybenzaldehyde (2.0 g, 8.66 mmol) and Et₃N (4.5 g, 34.64 mmol) in DCM(20 mL) was added MOMBr (2.7 g, 21.65 mmol) at 0 °C. After being stirred at room temperature for 3 h, the reaction mixture was extracted with EtOAc (50 mL x 3). The combined organic layers were washed with saturated brine (100 mL), dried over anhydrous Na₂SO₄ and filtered. The filtrate was concentrated to give the residue, which was purified by column chromatography on silica gel (PE:MTBE=75:25) to give the desired product (1.3 g, 55% yield) as white solid. LC-MS (ESI): [M+H]⁺ =275.1, 277.1; ¹H NMR (400 MHz, DMSO-*d₆*) δ 9.85 (s, 1H), 7.67 (d, *J* = 2.0 Hz, 1H), 7.39 (d, *J* = 2.0 Hz, 1H), 5.30 (s, 2H), 3.92 (s, 3H), 3.65 (s, 3H).

### Step 2: Synthesis of (E)-2-(3-bromo-5-methoxy-4-(methoxymethoxy)styryl)-N-(2-(2-fluoroethoxy)ethyl)-N,5-dimethylbenzo[d]thiazol-6-amine

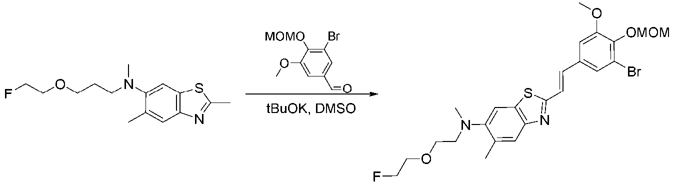

To a solution of *N*-(2-(2-fluoroethoxy)ethyl)-*N*,2,5-trimethylbenzo[*d*]thiazol-6-amine (62 mg, 0.22mmol) and *t*-BuOK (49 mg, 0.44mmol) in DMSO (1 mL) was added 3-bromo-5-methoxy-4-(methoxymethoxy)benzaldehyde (60 mg, 0.22mmol). The resulting mixture was stirred at room temperature for 3 h. The reaction mixture was quenched with H₂O (10 mL) and extracted with EtOAc (30 mL x 3). The combined organic phases were washed with saturated brine (60 mL), dried over anhydrous Na₂SO₄, filtered, and concentrated under reduced pressure. The crude product was purified by column chromatography on silica gel (PE:EA=65:35) to give the desired product (32 mg, 27% yield) as yellow oil. LC-MS (ESI): [M+H]⁺ =539.1, 541.1; ¹H NMR (400 MHz, DMSO-*d*₆) δ 7.76 (d,*J* = 5.6 Hz, 2H), 7.66 - 7.60 (m, 2H), 7.53 - 7.47 (m, 2H), 5.15 (s, 2H), 4.57 - 4.55 (m, 1H), 4.45 - 4.43 (m, 1H), 3.90 (s, 3H), 3.68 - 3.66 (m, 1H), 3.63 - 3.58(m, 3H), 3.54 (s, 3H), 3.11 (t, *J =* 6.0 Hz, 2H), 2.76 (s, 3H), 2.38 (s, 3H).

### Step 3: Synthesis of (E)-2-bromo-4-(2-(6-((2-(2-fluoroethoxy)ethyl)(methyl)amino)-5-methylbenzo[d]thiazol-2-yl)vinyl)-6-methoxyphenol

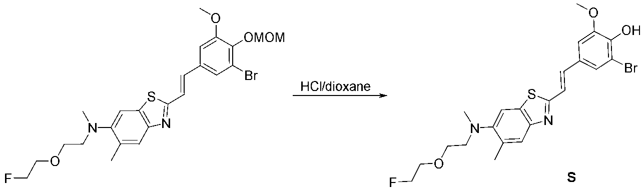

To a solution of (*E*)-2-(3-bromo-5-methoxy-4-(methoxymethoxy)styryl)-*N*-(2-(2-fluoroethoxy)ethyl)-*N*,5-dimethylbenzo[d]thiazol-6-amine (32 g, 0.06 mmol) in 1,4-dioxane (1 mL) was added HCl/1,4-dioxane (4 M, 1 mL). The resulting mixture was stirred at room temperature for 1 h. The precipitated solid was suspended in EtOAc (5mL) and then filtered off to give compound S (24.4 mg, 83% yield) as orange solid. LC-MS (ESI): [M+H]⁺ = 495.2, 497.1; ¹⁹F NMR (376 MHz, DMSO-*d*₆) δ -223.91; ¹H NMR (400 MHz, CD₃OD) δ 8.37 (s, 1H), 7.92 (s, 1H), 7.59 (d, *J =* 16.0 Hz, 1H), 7.42 - 7.27 (m, 3H), 4.56 - 4.54 (m, 1H), 4.43 - 4.42 (m, 1H), 3.99 - 3.98 (m, 2H), 3.96 (s, 3H), 3.63 - 3.56 (m, 4H), 3.39 (s, 3H), 2.67 (s, 3H).

### EXAMPLES DEMONSTRATING EFFECTS

### Effect Example 1:

First, the compound was a final concentration of 5 µM by dissolved in 50 mM Tris-buffer (pH 7.0) with 0.0025% (v/v) DMSO. Then, Aβ, α-Synuclein, and Tau aggregates were added, with their final concentrations being 0.02, 0.04, 0.08, 0.16, 0.32, 0.64, and 1.28 µM, respectively. Subsequently, the fluorescence measurements were detected using a fluorescence spectrophotometer (Horiba, Fluorolog3).

Aβ PFF: Aβ (1-42) peptide (rPeptide; catalog No.: A-1163-1) was resolved in 9 volumes of ice-cold distilled water and vortexed occasionally. After 30 min, one volume of 10X fibril-forming buffer (0.2 M NaPi, 1.5 M NaCl, 0.2% NaN₃, pH 7.5) was added. The final concentration of peptide was 1 mg/ml. The solution was incubated at 37 °C for one week.

α-synuclein Aggregates (Proteos Inc; catalog No.: RP-002).

Tau aggregates: The cDNA sequence of Tau was inserted into a plasmid carrying a GST tag, which was then transfected into BL21 bacteria for protein expression. Bacterial pellets were resuspended in high-salt buffer (0.75 M NaCl, 50 mM Tris, pH 7.4, 1 mM EDTA), sonicated, and centrifuged at 70,000 x g for 30 min. The supernatants were applied onto a Glutathione sepharose beads column (GE Healthcare) and purified by GST tag. Thrombin was used to remove the GST tag. The protein was dialyzed with FPLC buffer (50 mM NaCl, 50 mM Tris- HCl pH 7.4). The protein was filtered through a 0.22 µm syringe filter. The concentration of Tau was adjusted to 3 mg/mL, 12.5 µM heparin was added, and the mixture was shaken at 37 °C and 300 rpm for 7 days to prepare Tau aggregates.
Incubation Concentrations: 0.02, 0.04, 0.08, 0.16, 0.32, 0.64, and 1.28 µM;
Incubation Temperature: 37°C;
Incubation Duration: 5 min;
Detection Wavelengths:

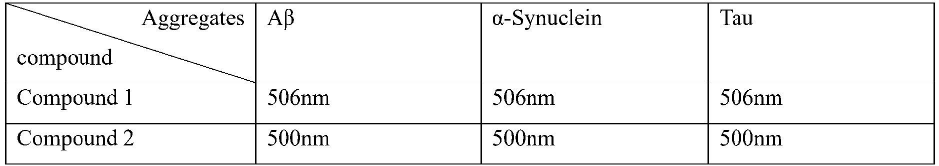

Statistical analysis was performed using GraphPad Prism^{®} version 8.2.1.

### Experimental Results:

The compounds of the present disclosure exhibit superior binding activity and selectivity toward α-Synuclein aggregates. For example, the Kd values for α-Synuclein aggregates are from 1 to 100 nM. The ratio of the Kd of the compounds of the present disclosure for Aβ aggregates to the Kd thereof for α-Synuclein aggregates is (2-100):1. The ratio of the Kd of the compounds of the present disclosure for Tau aggregates to the Kd thereof for α-Synuclein aggregates is (2-100):1.

| Compound C | Kd (nM) | Ratio / α-Synuclein |
|---|---|---|
| α-Synuclein | 313.90 | |
| Aβ | 1506.06 | 4.80 |
| Tau | 625.52 | 2.00 |

| Compound E | Kd (nM) | Ratio / α-Synuclein |
|---|---|---|
| α-Synuclein | 15.61 | |
| Aβ | 87.19 | 5.59 |
| Tau | 36.45 | 2.34 |

| Compound R | Kd (nM) | Ratio / α-Synuclein |
|---|---|---|
| α-Synuclein | 80.91 | |
| Aβ | 1273.38 | 15.74 |
| Tau | 2599.25 | 32.13 |

### Effect Example 2:

Primary cultured neurons were infected with adeno-associated virus (AAV) encoding a-Syn for 7 days and then treated with a-Syn PFFs. The candidate compounds were added to the primary neurons with the indicated aggregates and analyzed using fluorescence microscopy. The candidate compounds selectively yielded positive signals in α-Syn aggregated neurons.

Primary hippocampal neurons were isolated from E16-17 pregnant rats. The Sprague-Dawley rats were used at embryonic E18 days. The cortical tissue was isolated, dissociated, and incubated with 5 ml of D-Hanks containing 0.25% trypsin for 5 min at 37 °C in a humidified atmosphere of 5% CO₂, centrifuged at 1000 x g for 5 min after addition of 4 ml of the neuronal plating medium containing DMEM/F12 with 10% fetal bovine serum. The 1 × 10⁵ cells were plated in 6-well plates for further process. DIV7 neurons were infected with AAV α-Syn FL virus and at DIV12, the neurons were transduced with 100 ng/ml α-Syn PFF, α-Syn 1-140 were incubated with the cultured primary neurons in sterile PBS to a final concentration of 0.1 mg/ml. Sonicated PFFs, or an equivalent volume of the monomer as a control, were transferred into pre-warmed neuronal medium at a final concentration of 100 ng/ml. The samples were immune-stained with anti-p-α-Syn S129 (Abcam; Catalog No.: ab59264) (pseudo-colors were converted to red) to detect a-Syn aggregates and then stained with ThT (Sigma; Catalog No.: S96200) or compounds (0.1 mM).

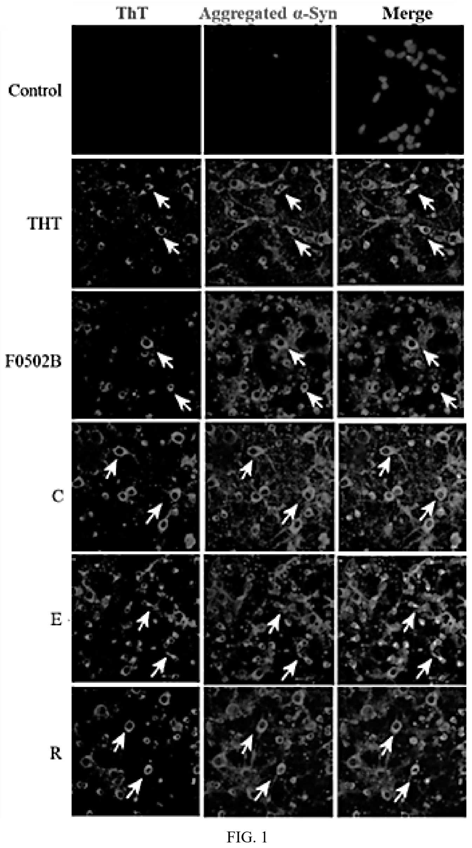

Of these tested compounds, Compound C, Compound E and Compound R selectively yielded positive signals in α-Syn aggregated neurons (Fig. 1).

### Effect Example 3:

To further explore whether the positive compounds selectively bind to Lewy bodies, brain slices from mice were used to test the binding specificity assays.

Staining with a-Synuclein protein coding gene (*SNCA*) transgenic mice (the Jackson Laboratory; stock numbers: 017682) were treated with rotenone (Oral gavage of rotenone under the following protocol: Mice were treated with 0.1 mL/25 g of vehicle containing 1% methylcellulose (Sigma, Catalog No.: M0512) and 1.25% chloroform (Sigma, Catalog No.: 2432) or a solution containing 0.625 mg/mL of rotenone (ULTRA Scientific, Catalog No.: PST-890) with 1% methylcellulose and 1.25% chloroform. We prepared rotenone solution by dissolving rotenone in chloroform and then diluting into 1% methylcellulose solution while mixing vigorously. Rotenone or vehicle control was orally administered with a 1.2 x 60 mm gavage (Unimed, Switzerland) once a day for 5 days/week, consecutively for 3 months.), which contains abundant LB-like inclusions. Furthermore, brain slices from NFT abundant Tau P301S mice and Aβ-enriched 5 x FAD mice were also used separately.

The brain slices were fixed and incubated for 12 h at 4 °C with primary antibodies followed by incubation with Alexa Fluor 568-, Alexa Fluor 488-, or Alexa Fluor 647-conjugated secondary antibodies (Invitrogen) for 3 h at room temperature. Then stained with ThS (Sigma, Catalog No.: T1892) or candidate compounds at room temperature, 4',6-diamidino-2-phenylindole (DAPI, 1 µg/ml) (Sigma-Aldrich) was used for the nuclear staining. The slides were imaged with Olympus FluoView FV1000 confocal laser scanning microscope using Olympus FluoView software to acquire data.

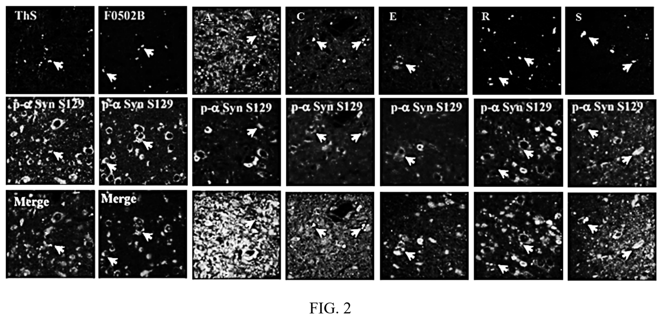

The α-Syn deposits were labeled with p-α-Syn S129 antibody, as a positive control, thioflavin-S (ThS) co-localized with all of these aggregates in the brain sections. Compound A, Compound C, Compound E, Compound R and Compound S sensitively captured a-Syn aggregates (Fig. 2). Notably, none of the tested compounds were reactive with NFTs.

### Effect Example 4:

The *ex vivo* screening was extended into human DLB, and AD patient samples. Neurodegenerative diseases of DLB belong to synucleino-pathies with well-defined Lewy bodies. Therefore, the positive compounds should show robust signals with LBs and LNs.

For immunohistochemical staining, deparaffinized and antigen-retrieved 20 µm-thick human brain sections were treated with 0.3% H₂O₂ at room temperature for 10 min. Subsequently, the sections were washed in PBS with 0.3% Triton X-100 3 times and then blocked with 1% BSA for 30 min, followed by overnight incubation with anti-Aβ/AT8/α-Syn pS129 antibody at 4 °C. The signal was developed using a Histostain-SP kit (Invitrogen, Catalog No.: 95-9943). After washing, the sections were covered with a glass cover using mounting solution.

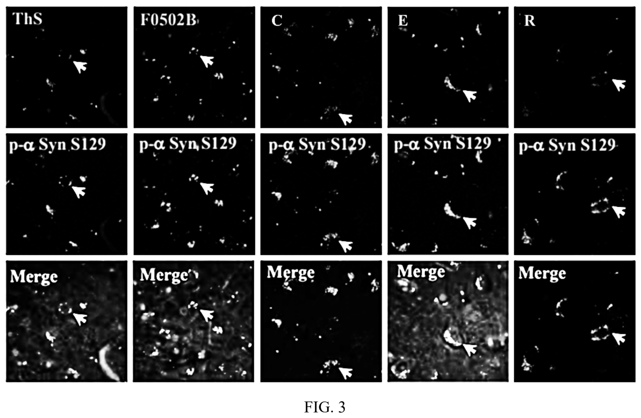

The compounds of the present disclosure, such as Compound C, Compound E, and Compound R, costained with p-α-Syn S129 in DLB patient brain slides, but did not stain Aβ plaques or AT8-positive NFTs in AD brains. Thus, compared to Aβ, these compounds selectively binding to α-Syn aggregates (Fig. 3).

### Effect Example 5:

To test whether these compounds are brain permeable *in vivo, in vivo* PK profiling was conducted to determain brain exposure.

An *in vivo* PK study was performed in male CD-1 (ICR) mice. Weighed a known amount of test compound and dissolved in vehicle (5% NMP + 15% Solutol + 80% PBS) to make a formulation (1 mg/mL). Then administered 5 mg/kg compound via tail vein injection into mice. The experiment included five groups (sampling time points), each group consisting of three male CD-1 (ICR) mice (body weight range: 29.1-30.6 g). At designated time points (0, 1, 5, 15, and 30 minutes post-injection), serial venous blood samples (approximately 30 µL each) and brain tissue samples were harvested from each animal. Blood samples were collected into Vacutainer IM tubes containing EDTA-K2 anticoagulant, inverted several times, and immediately placed on wet ice pending plasma centrifugation. For brain tissue samples, PBS (100 mM, pH 7.4) at 5 times the weight of the tissue (1:4, w/v) was added for homogenization. The concentrations of the test compound in plasma and brain tissue homogenates were measured by the LC/MS/MS analysis, with the specific operations as follows: Aliquots of 10 µL plasma sample or 50 µL brain homogenate sample was transferred to a 96-well plate, mixed with 200 µL protein precipitation solvent (methanol/acetonitrile, 1:1 v/v, containing 5 ng/mL Terfenadine). After protein precipitation, the supernatant was collected and diluted 3-fold with methanol/water (1:1, v/v, containing 0.1% FA). 1 µL of plasma samples or 3 µL of brain tissue homogenate samples was taken for LC/MS/MS analysis.

It was found that:
The plasma half-life of Compound C was 1.14 hours, which was 10-fold extension compared to F0502B. In addition, the brain/plasma ratio of Compound C was 24% at 1 minute and reached a maximum of 129% at 15 minutes.

The brain/plasma ratio of Compound E was 12% at 1 minute, then continued to increase, reaching a maximum of 337% at 15 minutes.

The plasma half-life of Compound R was 0.54 hours, which was 4.7-fold extension compared to F0502B. Moreover, the brain/plasma ratio of Compound R was 5% at 1 minute and reached a maximum of 255% at 30 minutes.

The compounds of the present disclosure exhibit favorable PK properties, with high brain permeability, enabling rapid entry into the brain (their brain/plasma ratios increase in a time-dependent manner), having a optimal *in vivo* half-life and being capable of rapid clearance from plasma.

### Effect Example 6:

*In vivo* validation of the compounds was performed using 8-month-old wild-type mice (Jiangsu Aniphe Biolaboratory Inc), 12-month-old 5xFAD mice (The Jackson Laboratory; Catalog No.: 034840), 6-month-old Tau P301S mice (The Jackson Laboratory; Catalog No.: 008169), and rotenone-treated α-Syn A53T mice (The Jackson Laboratory; Catalog No.: 017682). All mice were stratified by sex and randomly divided into groups, and the compounds were administered via tail vein injection at a dose of 2 mg/kg. Tissues were harvested and mouse brain sections were prepared 0.5 hours after administration, followed by staining with anti-Aβ (Biolegend; Catalog No.: SIG-39300) or AT8 (Thermo Fisher; Catalog No.: MN1020) together with the compounds, respectively. The results showed that the compounds of the present disclosure, such as Compound C, Compound E, and Compound R, exhibited no specific binding to Aβ or AT8, but co-localized with α-Syn-p129 or α-Syn aggregates.

While specific embodiments of the present disclosure have been described above, it shall be understood by those skilled in the art that these are merely illustrative and not restrictive. Various modifications or alterations may be made to these embodiments without departing from the spirit and scope of the invention. Accordingly, the protection scope of the invention is defined by the appended claims.

## Claims

1. A sulfur-containing fused ring compound of Formula A, a pharmaceutically acceptable salt thereof, a tautomer thereof, or a radioisotope-labeled derivative thereof;
wherein the sulfur-containing fused ring compound of Formula A is as follows: 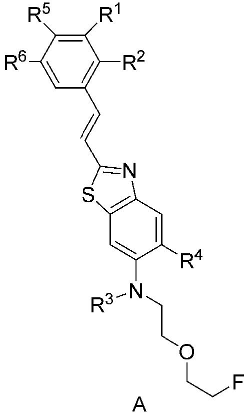
in Formula A,
R¹ is F, Cl, Br, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 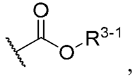 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 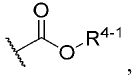 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻³, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
when R⁵ is -OH, then R¹ is F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the compound satisfies one or more of the following conditions:
Condition (1): R¹ is F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
Condition (2): R² is -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
Condition (3): R³ is H, -OH, C₂₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 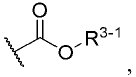 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹.

2. The sulfur-containing fused ring compound of Formula A, the pharmaceutically acceptable salt thereof, the tautomer thereof, or the radioisotope-labeled derivative thereof according to claim 1, wherein the sulfur-containing fused ring compound of Formula A is as shown in Formula A-1:

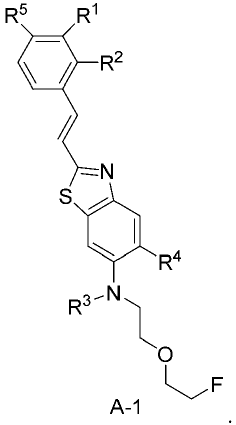

3. The sulfur-containing fused ring compound of Formula A, the pharmaceutically acceptable salt thereof, the tautomer thereof, or the radioisotope-labeled derivative thereof according to claim 1, wherein the sulfur-containing fused ring compound of Formula A, the pharmaceutically acceptable salt thereof, the tautomer thereof, or the radioisotope-labeled derivative thereof satisfies one or more of the following conditions:
Condition (1): in Formula A, R¹ is Br, F, Cl, I, -OR¹⁻¹, or -NR¹⁻²R¹⁻³; preferably -OR¹⁻¹ or -NR¹⁻²R¹⁻³; more preferably -OH, -OCH₃, -NH₂, or -NHCH₃;
Condition (2): in Formula A, R² is H, -OH, -OC₁₋₆ alkyl, or -NR²⁻⁰R²⁻¹; preferably -OH, -OC₁₋₆ alkyl, or -NR²⁻⁰R²⁻¹; more preferably -OH, -OCH₃, -NH₂, or -NHCH₃;
Condition (3): in Formula A, R³ is H, -OH, or C₁₋₆ alkyl; preferably H, -OH, or C₂₋₆ alkyl; more preferably H, -OH, or isopropyl, or, more preferably -OH, ethyl, or isopropyl;
Condition (4): in Formula A, R⁴ is C₁₋₆ alkyl;
Condition (5): in Formula A, R¹⁻¹ is H or C₁₋₆ alkyl;
Condition (6): in Formula A, R¹⁻² and R¹⁻³ are independently H or C₁₋₆ alkyl;
Condition (7): in Formula A, R²⁻¹ is H;
Condition (8): in Formula A, R²⁻⁰ is H or C₁₋₆ alkyl; and
Condition (9): in Formula A, R⁵ is -OH, -NH₂, or -NHCH₃; preferably, R⁵ is -OH.

4. A sulfur-containing fused ring compound of Formula I-A, II-A, III-A, IV-A, V-A, VI-A, or VII, a pharmaceutically acceptable salt thereof, a tautomer thereof, or a radioisotope-labeled derivative thereof, wherein
the sulfur-containing fused ring compound of Formula I-A is as follows: 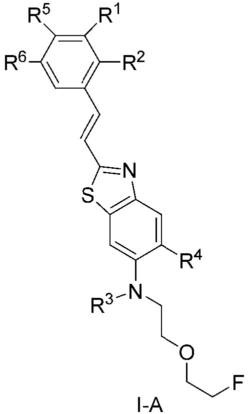
in Formula I-A,
R¹ is F, Cl, Br, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 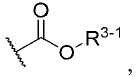 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 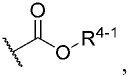 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻³, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
when R⁵ is -OH, then R¹ is F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶; the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula II-A is as follows: 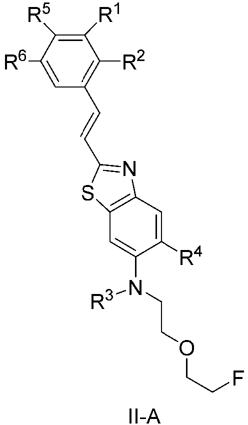
in Formula II-A,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 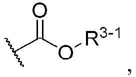 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 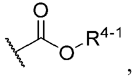 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻³, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula V-A is as follows: 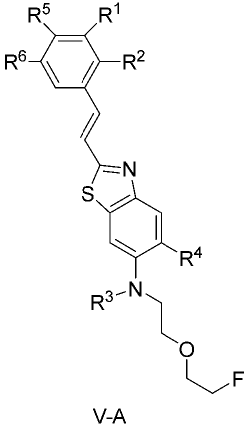
in Formula V-A,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₂₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 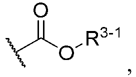 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 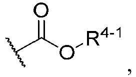 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻³, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula III-A is as follows: 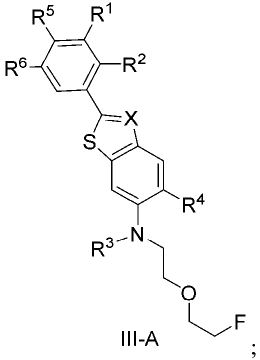
in Formula III-A,
X is N or CR^{a};
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 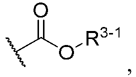 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 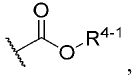 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻³, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R^{a} is H or C₁₋₆ alkyl;
or, R^{a} and R² together form -(NH)ₙ-(CH₂)ₘ-(CH=CH)ₚ-; n, m, and p are independently 0, 1, 2, or 3, and n, m, and p are not simultaneously 0;
R⁵ is -OH or -NR⁵⁻¹R⁵⁻²;
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula IV-A is as follows: 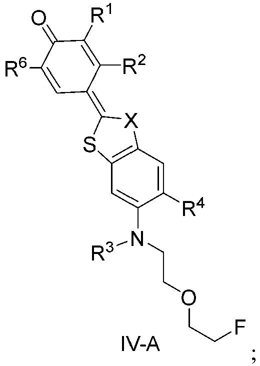
in Formula IV-A,
X is N or CR^{a};
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 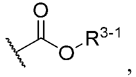 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 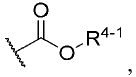 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻³, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R^{a} is H or C₁₋₆ alkyl;
or, R^{a} and R² together form -(NH)ₙ-(CH₂)ₘ-(CH=CH)ₚ-; n, m, and p are independently 0, 1, 2, or 3, and n, m, and p are not simultaneously 0;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula VI-A is as follows: 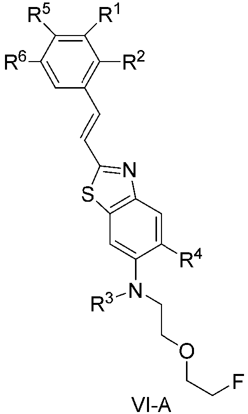
in Formula VI-A,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴ , R¹⁻⁵, and R¹⁻⁶ are independently -OH or -NR¹⁻⁴⁻¹R¹⁻⁴⁻²
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 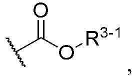 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₂₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 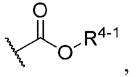 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻³, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula VII is as follows: 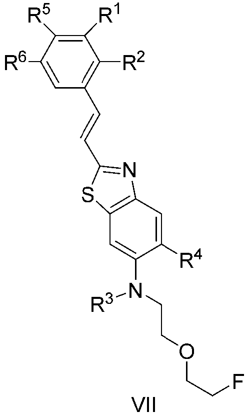
R¹ is F, Cl, Br, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 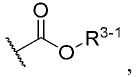 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 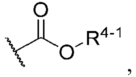 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 3- to 6-membered heterocycloalkyl has 1, 2, or 3 heteroatoms selected from the group consisting of N, O, and S.

5. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4, wherein
the sulfur-containing fused ring compound of Formula I-A is shown in the following Formula I: 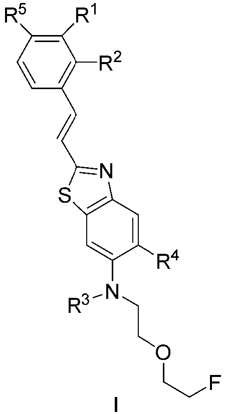
in Formula **I,**
R¹ is F, Cl, Br, I, -OR¹⁻¹, -NR^{1 2}R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 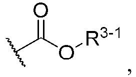 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 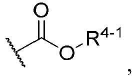 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
when R⁵ is -OH, then R¹ is F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶; the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula II-A is shown in the following Formula II: 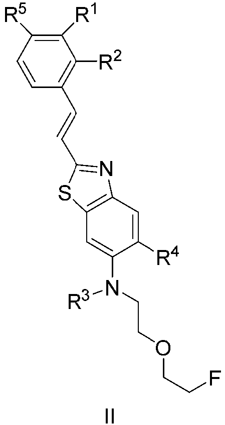
in Formula II,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 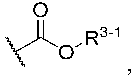 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 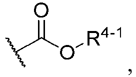 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula V-A is shown in the following Formula V: 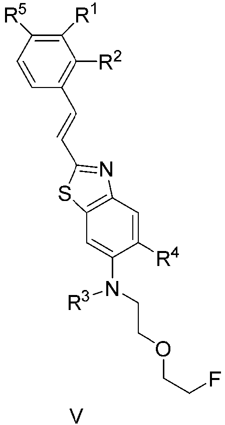
in Formula V,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₂₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 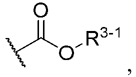 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 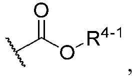 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula III-A is shown in the following Formula III: 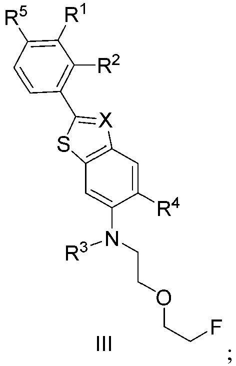
in Formula III,
X is N or CR^{a};
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁻⁴⁻²;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 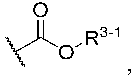 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 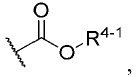 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R^{a} is H or C₁₋₆ alkyl;
or, R^{a} and R² together form -(NH)ₙ-(CH₂)ₘ-(CH=CH)ₚ-; n, m, and p are independently 0, 1, 2, or 3, and n, m, and p are not simultaneously 0;
R⁵ is -OH or -NR⁵⁻¹R⁵⁻²;
R⁵⁻¹ and R⁵⁻² are independently H or C₁₋₆ alkyl;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula IV-A is shown in the following Formula IV: 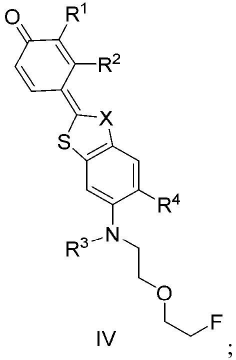
in Formula IV,
X is N or CR^{a};
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 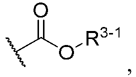 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 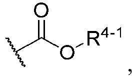 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R^{a} is H or C₁₋₆ alkyl;
or, R^{a} and R² together form -(NH)ₙ-(CH₂)ₘ-(CH=CH)ₚ-; n, m, and p are independently 0, 1, 2, or 3, and n, m, and p are not simultaneously 0;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula VI-A is shown in the following Formula VI: 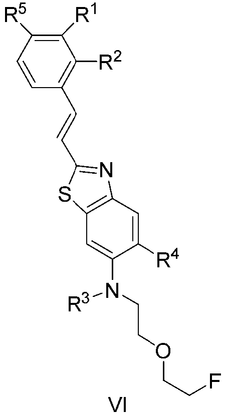
in Formula VI,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 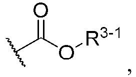 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₂₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 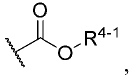 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S.

6. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula I-A or Formula I, R¹ is Br, -OR¹⁻¹, or -NR¹⁻²R¹⁻³; preferably -OR¹⁻¹ or - NR¹⁻²R¹⁻³; more preferably -OH, -OCH₃, -NH₂, or -NHCH₃;
Condition (2): in Formula I-A or Formula I, R² is H, -OH, -OC₁₋₆ alkyl, or -NR²⁻⁰R²⁻¹;
Condition (3): in Formula I-A or Formula I, R³ is H, -OH, or C₁₋₆ alkyl;
Condition (4): in Formula I-A or Formula I, R⁴ is C₁₋₆ alkyl;
Condition (5): in Formula I-A or Formula I, R¹⁻¹ is H or C₁₋₆ alkyl;
Condition (6): in Formula I-A or Formula I, R¹⁻² and R¹⁻³ are independently H or C₁₋₆ alkyl;
Condition (7): in Formula I-A or Formula I, R²⁻¹ is H;
Condition (8): in Formula I-A or Formula I, R²⁻⁰ is H or C₁₋₆ alkyl; and
Condition (9): in Formula I-A or Formula I, R⁵ is -OH, -NH₂, or -NHCH₃; preferably -OH.

7. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula II-A or Formula II, R¹ is Br, F, Cl, I, -OR¹⁻¹, or -NR¹⁻²R¹⁻³;
Condition (2): in Formula II-A or Formula II, R² is -OH, -OC₁₋₆ alkyl, or -NR²⁻⁰R²⁻¹; preferably - OH, -OCH₃, -NH₂, or -NHCH₃;
Condition (3): in Formula II-A or Formula II, R³ is H, -OH, or C₁₋₆ alkyl;
Condition (4): in Formula II-A or Formula II, R⁴ is C₁₋₆ alkyl;
Condition (5): in Formula II-A or Formula II, R¹⁻¹ is H or C₁₋₆ alkyl;
Condition (6): in Formula II-A or Formula II, R¹⁻² and R¹⁻³ are independently H or C₁₋₆ alkyl;
Condition (7): in Formula II-A or Formula II, R²⁻¹ is H;
Condition (8): in Formula II-A or Formula II, R²⁻⁰ is H or C₁₋₆ alkyl; and
Condition (9): in Formula II-A or Formula II, R⁵ is -OH, -NH₂, or -NHCH₃; preferably -OH.

8. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula V-A or Formula V, R¹ is Br, F, Cl, I, -OR¹⁻¹, or -NR¹⁻²R¹⁻³;
Condition (2): in Formula V-A or Formula V, R² is H, -OH, -OC₁₋₆ alkyl, or -NR²⁻⁰R²⁻¹;
Condition (3): in Formula V-A or Formula V, R³ is H, -OH, or C₂₋₆ alkyl; preferably H, -OH, or isopropyl; or preferably -OH, ethyl, or isopropyl; most preferably ethyl;
Condition (4): in Formula V-A or Formula V, R⁴ is C₁₋₆ alkyl;
Condition (5): in Formula V-A or Formula V, R¹⁻¹ is H or C₁₋₆ alkyl;
Condition (6): in Formula V-A or Formula V, R¹⁻² and R¹⁻³ are independently H or C₁₋₆ alkyl;
Condition (7): in Formula V-A or Formula V, R²⁻¹ is H;
Condition (8): in Formula V-A or Formula V, R²⁻⁰ is H or C₁₋₆ alkyl; and
Condition (9): in Formula V-A or Formula V, R⁵ is -OH, -NH₂, or -NHCH₃; preferably -OH.

9. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula III-A or Formula III, R¹ is Br, F, Cl, I, -OR¹⁻¹, or -NR¹⁻²R¹⁻³;
Condition (2): in Formula III-A or Formula III, R² is H, -OH, -OC₁₋₆ alkyl, or -NR²⁻⁰R²⁻¹; or, R^{a} and R² together form -(NH)ₙ-(CH₂)ₘ-(CH=CH)ₚ-; n, m, and p are independently 0, 1, 2, or 3, and n, m, and p are not simultaneously 0; preferably, R² is H, or R^{a} and R² together form -NH- or -CH=CH-;
Condition (3): in Formula III-A or Formula III, R³ is H, -OH, or C₁₋₆ alkyl;
Condition (4): in Formula III-A or Formula III, R⁴ is C₁₋₆ alkyl;
Condition (5): in Formula III-A or Formula III, R¹⁻¹ is H or C₁₋₆ alkyl;
Condition (6): in Formula III-A or Formula III, R¹⁻² and R¹⁻³ are independently H or C₁₋₆ alkyl;
Condition (7): in Formula III-A or Formula III, R²⁻¹ is H;
Condition (8): in Formula III-A or Formula III, R²⁻⁰ is H or C₁₋₆ alkyl; and
Condition (9): in Formula III-A or Formula III, R⁵ is -OH, -NH₂, or -NHCH₃; preferably -OH.

10. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula IV-A or Formula IV, R¹ is Br, F, Cl, I, -OR¹⁻¹, or -NR¹⁻²R¹⁻³;
Condition (2): in Formula IV-A or Formula IV, R² is H, -OH, -OC₁₋₆ alkyl, or -NR²⁻⁰R²⁻¹; or, R^{a} and R² together form -(NH)ₙ-(CH₂)ₘ-(CH=CH)ₚ-; n, m, and p are independently 0, 1, 2, or 3, and n, m, and p are not simultaneously 0; preferably, R² is H, or R^{a} and R² together form -NH- or -CH=CH-;
Condition (3): in Formula IV-A or Formula IV, R³ is H, -OH, or C₁₋₆ alkyl;
Condition (4): in Formula IV-A or Formula IV, R⁴ is C₁₋₆ alkyl;
Condition (5): in Formula IV-A or Formula IV, R¹⁻¹ is H or C₁₋₆ alkyl;
Condition (6): in Formula IV-A or Formula IV, R¹⁻² and R¹⁻³ are independently H or C₁₋₆ alkyl;
Condition (7): in Formula IV-A or Formula IV, R²⁻⁰ is C₁₋₆ alkyl; and
Condition (8): in Formula IV-A or Formula IV, R²⁻¹ is H.

11. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula VI-A or Formula VI, R¹ is Br, F, Cl, I, -OR¹⁻¹, or -NR¹⁻²R¹⁻³;
Condition (2): in Formula VI-A or Formula VI, R² is H, -OH, -OC₁₋₆ alkyl, or -NR²⁻⁰R²⁻¹;
Condition (3): in Formula VI-A or Formula VI, R³ is H, -OH, or C₁₋₆ alkyl;
Condition (4): in Formula VI-A or Formula VI, R⁴ is C₂₋₆ alkyl; preferably ethyl or isopropyl;
Condition (5): in Formula VI-A or Formula VI, R¹⁻¹ is H or C₁₋₆ alkyl;
Condition (6): in Formula VI-A or Formula VI, R¹⁻² and R¹⁻³ are independently H or C₁₋₆ alkyl;
Condition (7): in Formula VI-A or Formula VI, R²⁻¹ is H;
Condition (8): in Formula VI-A or Formula VI, R²⁻⁰ is H or C₁₋₆ alkyl; and
Condition (8): in Formula VI-A or Formula VI, R⁵ is -OH, -NH₂, or -NHCH₃; preferably -OH.

12. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula VII, R¹ is Br, F, Cl, I, -OR¹⁻¹, or -NR¹⁻²R¹⁻³;
Condition (2): in Formula VII, R² is H, -OH, -OC₁₋₆ alkyl, or -NR²⁻⁰R²⁻¹;
Condition (3): in Formula VII, R³ is H, -OH, or C₁₋₆ alkyl;
Condition (4): in Formula VII, R⁴ is C₂₋₆ alkyl;
Condition (5): in Formula VII, R¹⁻¹ is H or C₁₋₆ alkyl;
Condition (6): in Formula VII, R¹⁻² and R¹⁻³ are independently H or C₁₋₆ alkyl;
Condition (7): in Formula VII, R²⁻¹ is H;
Condition (8): in Formula VII, R²⁻⁰ is H or C₁₋₆ alkyl;
Condition (9): in Formula VII, R⁵ is -OH, -NH₂, or -NHCH₃; preferably -OH;
Condition (10): in Formula I-A, II-A, III-A, IV-A, V-A, VI-A, or VII, R⁶ is H or -OC₁₋₆ alkyl; preferably H or methoxy.

13. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula I-A or Formula I, R¹ is Br, -OH, -OCH₃, -NH₂, or -NHCH₃;
R² is H, -OH, -OCH₃, -NH₂, or -NHCH₃;
R³ is H, -OH, or C₁₋₆ alkyl;
R⁴ is C₁₋₆ alkyl;
R⁵ is -OH, -NH₂, or -NHCH₃;
Condition (2): in Formula II-A or Formula II, R¹ is Br, F, Cl, I, -OH, -OCH₃, -NH₂, or -NHCH₃;
R² is -OH, -OCH₃, -NH₂, or -NHCH₃;
R³ is H, -OH, or C₁₋₆ alkyl;
R⁴ is C₁₋₆ alkyl;
R⁵ is -OH, -NH₂, or -NHCH₃;
Condition (3): in Formula III-A or Formula III, X is N or CR^{a};
R' is Br, F, Cl, I, -OH, -OCH₃, -NH₂, or -NHCH₃;
R² is H; or, R^{a} and R² together form -NH- or -CH=CH-;
R³ is H, -OH, or C₁₋₆ alkyl;
R⁴ is C₁₋₆ alkyl;
R⁵ is -OH, -NH₂, or -NHCH₃;
Condition (4): in Formula IV-A or Formula IV, X is N or CR^{a};
R¹ is Br, F, Cl, I, -OH, -OCH₃, -NH₂, or -NHCH₃;
R² is H; or, R^{a} and R² together form -NH- or -(CH=CH)-;
R³ is H, -OH, or C₁₋₆ alkyl;
R⁴ is C₁₋₆ alkyl;
Condition (5): in Formula V-A or Formula V, R¹ is Br, F, Cl, I, -OH, -OCH₃, -NH₂, or -NHCH₃;
R² is H, -OH, -OCH₃, -NH₂, or -NHCH₃;
R³ is H, -OH, or isopropyl; or R³ is -OH, ethyl, or isopropyl;
R⁴ is C₁₋₆ alkyl;
R⁵ is -OH, -NH₂, or -NHCH₃; and
Condition (6): in Formula VI-A and Formula VI, R¹ is Br, F, Cl, I, -OH, -OCH₃, -NH₂, or -NHCH₃;
R² is H, -OH, -OCH₃, -NH₂, or -NHCH₃;
R³ is H, -OH, or C₁₋₆ alkyl;
R⁴ is C₂₋₆ alkyl;
R⁵ is -OH, -NH₂, or -NHCH₃;
preferably, the sulfur-containing fused ring compound of Formula I-A, II-A, III-A, IV-A, V-A, VIA, or VII, the pharmaceutically acceptable salt thereof, the tautomer thereof, or the radioisotope-labeled derivative thereof satisfies one or more of the following conditions:
Condition (1): in Formula I-A or Formula I, R¹ is Br, -OH, -OCH₃, -NH₂, or -NHCH₃;
R² is H;
R³ is C₁₋₆ alkyl;
R⁴ is C₁₋₆ alkyl;
R⁵ is -OH, -NH₂, or -NHCH₃;
Condition (2): in Formula II-A or Formula II, R¹ is Br;
R² is -OH, -OCH₃, -NH₂, or -NHCH₃;
R³ is C₁₋₆ alkyl;
R⁴ is C₁₋₆ alkyl;
R⁵ is -OH;
Condition (3): in Formula III-A or Formula III, X is N or CR^{a};
R¹ is Br;
R² is H; or, R^{a} and R² together form -NH- or -CH=CH-;
R³ is C₁₋₆ alkyl;
R⁴ is C₁₋₆ alkyl;
R⁵ is -OH;
Condition (4): in Formula IV-A or Formula IV, X is N or CR^{a};
R¹ is Br;
R² is H; or, R^{a} and R² together form -NH- or -(CH=CH)-;
R³ is C₁₋₆ alkyl;
R⁴ is C₁₋₆ alkyl;
Condition (5): in Formula V-A or Formula V, R¹ is Br;
R² is H;
R³ is H, -OH, or isopropyl; or R³ is -OH, ethyl, or isopropyl;
R⁴ is C₁₋₆ alkyl;
R⁵ is -OH; and
Condition (6): in Formula VI-A or Formula VI, R¹ is Br;
R² is H;
R³ is H, -OH, or C₁₋₆ alkyl;
R⁴ is ethyl or isopropyl;
R⁵ is -OH.

14. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula VII, R¹ is Br, F, Cl, I, -OH, -OCH₃, -NH₂, or -NHCH₃;
R² is H, -OH, -OCH₃, -NH₂, or -NHCH₃;
R³ is H, -OH, or C₁₋₆ alkyl;
R⁴ is C₁₋₆ alkyl;
R⁵ is -OH, -NH₂, or -NHCH₃;
R⁶ is methoxy;
Condition (2): R¹ is Br, -OH, -OCH₃, -NH₂, or -NHCH₃;
R² is H;
R³ is C₁₋₆ alkyl;
R⁴ is C₁₋₆ alkyl;
R⁵ is -OH, -NH₂, or -NHCH₃;
R⁶ is methoxy.

15. The sulfur-containing fused ring compound of Formula A, the pharmaceutically acceptable salt thereof, the tautomer thereof, or the radioisotope-labeled derivative thereof according to claim 1, or the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula A, the pharmaceutically acceptable salt thereof, the tautomer thereof, or the radioisotope-labeled derivative thereof, or the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula A, I-A, II-A, III-A, IV-A, V-A, VI-A, VII, I, II, III, IV, V, or VI, the C₁₋₆ alkyl is independently methyl, ethyl, n-propyl, isopropyl, *n*-butyl, isobutyl, or *tert*-butyl; for example, methyl, ethyl, or isopropyl; and for another example, methyl;
Condition (2): in Formula A, I-A, II-A, III-A, IV-A, V-A, VI-A, VII, I, II, III, IV, V, or VI, the C₂₋₆ alkenyl is independently vinyl, propenyl, n-propenyl, isopropenyl, *n-*butenyl, isobutenyl, *sec*-butenyl, or *tert*-butenyl;
Condition (3): in Formula A, I-A, II-A, III-A, IV-A, V-A, VI-A, VII, I, II, III, IV, V, or VI, the C₂₋₆ alkynyl is independently ethynyl, *n*-propynyl, isopropynyl, *n*-butynyl, isobutynyl, *sec*-butynyl, or *tert-*butynyl;
Condition (4): in Formula A, I-A, II-A, III-A, IV-A, V-A, VI-A, VII, I, II, III, IV, V, or VI, the C₃₋₆ cycloalkyl is independently cyclopropyl, cyclobutyl, cyclopropyl, cyclopentyl, or cyclohexyl;
Condition (5): in Formula A, I-A, II-A, III-A, IV-A, V-A, VI-A, VII, I, II, III, IV, V, or VI, the -O-C₁₋₆ alkyl is independently methoxy, ethoxy, *n*-propoxy, isopropoxy, *n*-butoxy, isobutoxy, or *tert*-butoxy; for example, methoxy;
Condition (6): in Formula A, I-A, II-A, III-A, IV-A, V-A, VI-A, VII, I, II, III, IV, V, or VI, the halogen is independently F, Cl, Br, or I;
Condition (7): in Formula A, I-A, II-A, III-A, IV-A, V-A, VI-A, VIII, II, III, IV, V, or VI, the C₆₋₁₀ aryl is independently phenyl or naphthyl, for example, phenyl; and
Condition (8): in Formula A, V-A, VI-A, V, or VI, the C₂₋₆ alkyl is independently ethyl, n-propyl, isopropyl, n-butyl, isobutyl, or tert-butyl; for example, ethyl or isopropyl.

16. The sulfur-containing fused ring compound of Formula A, the pharmaceutically acceptable salt thereof, the tautomer thereof, or the radioisotope-labeled derivative thereof according to claim 1, or the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A is any one selected from the group consisting of the following compounds: 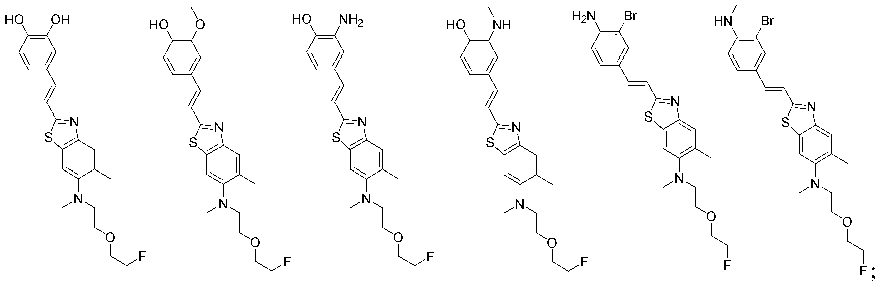
the sulfur-containing fused ring compound of Formula II-A is any one selected from the group consisting of the following compounds: 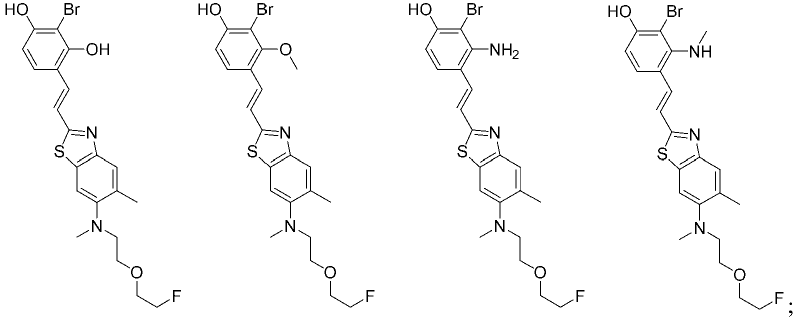
the sulfur-containing fused ring compound of Formula III-A is any one selected from the group consisting of the following compounds: 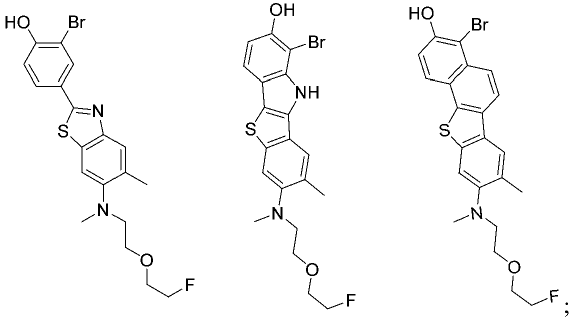
the sulfur-containing fused ring compound of Formula IV-A is: 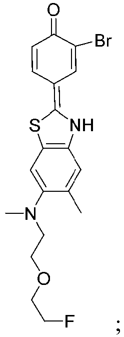
the sulfur-containing fused ring compound of Formula V-A is any one selected from the group consisting of the following compounds: 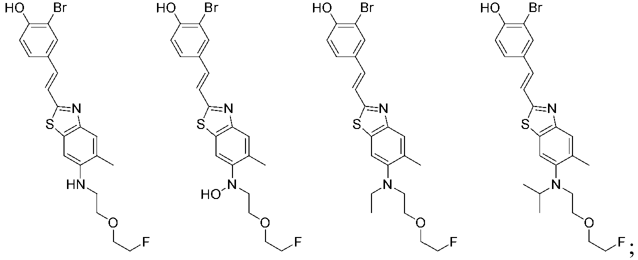
the sulfur-containing fused ring compound of Formula VI-A is any one selected from the group consisting of the following compounds: 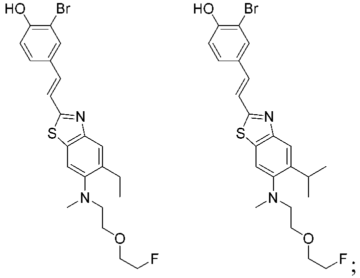
the sulfur-containing fused ring compound of Formula VII is:

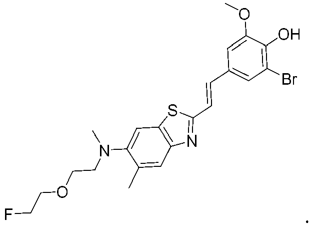

17. The sulfur-containing fused ring compound, the pharmaceutically acceptable salt thereof, the tautomer thereof, or the radioisotope-labeled derivative thereof according to any one of claims 1 to 3, or the sulfur-containing fused ring compound, the pharmaceutically acceptable salt thereof, the tautomer thereof, or the radioisotope-labeled derivative thereof according to any one of claims 4 to 16, wherein the radioisotope-labeled derivative is a compound obtained by replacing the F, C, I, or N atom in the sulfur-containing fused ring compound, the pharmaceutically acceptable salt thereof, or the tautomer thereof with ¹⁸F, ¹¹C, ¹²⁴I, ¹²³I, or ¹³N; preferably ¹⁸F.

18. A pharmaceutical composition, comprising the sulfur-containing fused ring compound of Formula A, the pharmaceutically acceptable salt thereof, the tautomer thereof, or the radioisotope-labeled derivative thereof according to any one of claims 1 to 3, or the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to any one of claims 4 to 16, and a pharmaceutically acceptable excipient.

19. A kit, comprising the sulfur-containing fused ring compound of Formula A, the pharmaceutically acceptable salt thereof, the tautomer thereof, or the radioisotope-labeled derivative thereof according to any one of claims 1 to 3, or the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to any one of claims 4 to 16, and instructions for use.

20. Use of the sulfur-containing fused ring compound of Formula A, the pharmaceutically acceptable salt thereof, the tautomer thereof, or the radioisotope-labeled derivative thereof according to any one of claims 1 to 3, or the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to any one of claims 4 to 16 in the manufacture of a PET-CT tracer; the PET-CT tracer can be used for imaging α-synuclein aggregates in Lewy bodies and Lewy neurites; for example, the PET-CT tracer is used for imaging Parkinson's disease, dementia with Lewy bodies, or multiple system atrophy; for example, the PET-CT tracer can be used for diagnostic exclusion of progressive supranuclear palsy or corticobasal degeneration.

## Amended claims

### Amended claims under Art. 19.1 PCT

1. A sulfur-containing fused ring compound of Formula A, a pharmaceutically acceptable salt thereof, a tautomer thereof, or a radioisotope-labeled derivative thereof;
wherein the sulfur-containing fused ring compound of Formula A is as follows: 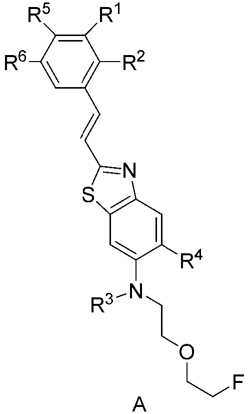
in Formula A,
R¹ is F, Cl, Br, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 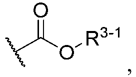 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 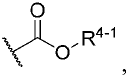 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
when R⁵ is -OH, then R¹ is F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the compound satisfies one or more of the following conditions:
Condition (1): R¹ is F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
Condition (2): R² is -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
Condition (3): R³ is H, -OH, C₂₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 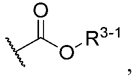 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹.

2. The sulfur-containing fused ring compound of Formula A, the pharmaceutically acceptable salt thereof, the tautomer thereof, or the radioisotope-labeled derivative thereof according to claim 1, wherein the sulfur-containing fused ring compound of Formula A is as shown in Formula A-1:

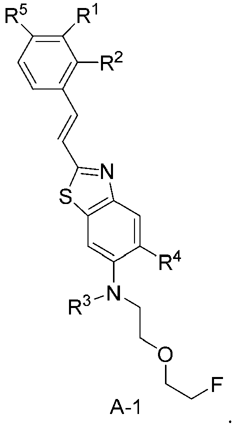

3. The sulfur-containing fused ring compound of Formula A, the pharmaceutically acceptable salt thereof, the tautomer thereof, or the radioisotope-labeled derivative thereof according to claim 1, wherein the sulfur-containing fused ring compound of Formula A, the pharmaceutically acceptable salt thereof, the tautomer thereof, or the radioisotope-labeled derivative thereof satisfies one or more of the following conditions:
Condition (1): in Formula A, R¹ is Br, F, Cl, I, -OR¹⁻¹, or -NR¹⁻²R¹⁻³; preferably -OR¹⁻¹ or -NR¹⁻²R¹⁻³; more preferably -OH, -OCH₃, -NH₂, or -NHCH₃;
Condition (2): in Formula A, R² is H, -OH, -OC₁₋₆ alkyl, or -NR²⁻⁰R²⁻¹; preferably -OH, -OC₁₋₆ alkyl, or -NR²⁻⁰R²⁻¹; more preferably -OH, -OCH₃, -NH₂, or -NHCH₃;
Condition (3): in Formula A, R³ is H, -OH, or C₁₋₆ alkyl; preferably H, -OH, or C₂₋₆ alkyl; more preferably H, -OH, or isopropyl, or, more preferably -OH, ethyl, or isopropyl;
Condition (4): in Formula A, R⁴ is C₁₋₆ alkyl;
Condition (5): in Formula A, R¹⁻¹ is H or C₁₋₆ alkyl;
Condition (6): in Formula A, R¹⁻² and R¹⁻³ are independently H or C₁₋₆ alkyl;
Condition (7): in Formula A, R²⁻¹ is H;
Condition (8): in Formula A, R²⁻⁰ is H or C₁₋₆ alkyl; and
Condition (9): in Formula A, R⁵ is -OH, -NH₂, or -NHCH₃; preferably, R⁵ is -OH.

4. A sulfur-containing fused ring compound of Formula I-A, II-A, III-A, IV-A, V-A, VI-A, or VII, a pharmaceutically acceptable salt thereof, a tautomer thereof, or a radioisotope-labeled derivative thereof, wherein
the sulfur-containing fused ring compound of Formula I-A is as follows: 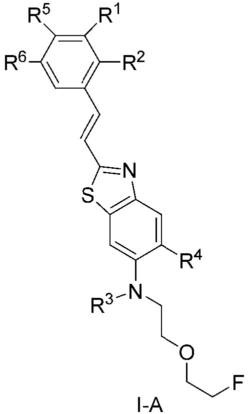
in Formula I-A,
R¹ is F, Cl, Br, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 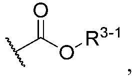 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 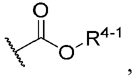 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
when R⁵ is -OH, then R¹ is F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶; the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula II-A is as follows: 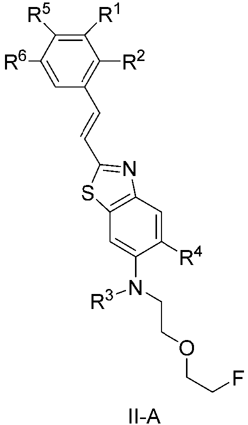
in Formula II-A,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 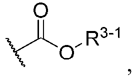 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 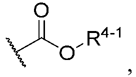 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula V-A is as follows: 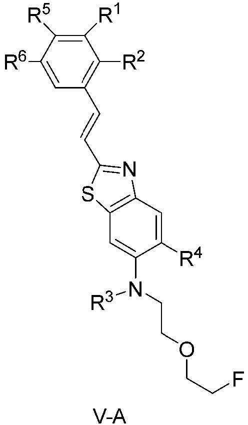
in Formula V-A,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₂₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 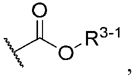 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 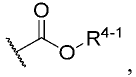 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula III-A is as follows: 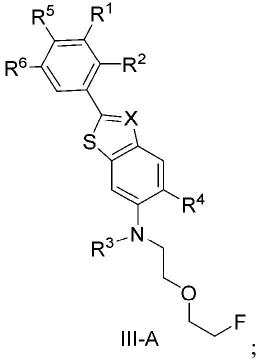
in Formula III-A,
X is N or CR^{a};
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 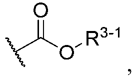 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 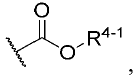 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R^{a} is H or C₁₋₆ alkyl;
or, R^{a} and R² together form -(NH)ₙ-(CH₂)ₘ-(CH=CH)ₚ-; n, m, and p are independently 0, 1, 2, or 3, and n, m, and p are not simultaneously 0;
R⁵ is -OH or -NR⁵⁻¹R⁵⁻²;
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula IV-A is as follows: 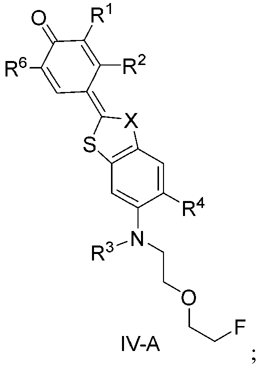
in Formula IV-A,
X is N or CR^{a};
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 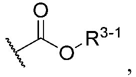 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 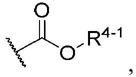 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R^{a} is H or C₁₋₆ alkyl;
or, R^{a} and R² together form -(NH)ₙ-(CH₂)ₘ-(CH=CH)ₚ-; n, m, and p are independently 0, 1, 2, or 3, and n, m, and p are not simultaneously 0;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula VI-A is as follows: 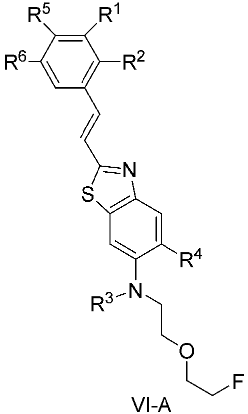
in Formula VI-A,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 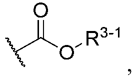 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₂₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 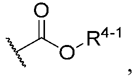 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
R⁶ is H, -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula VII is as follows: 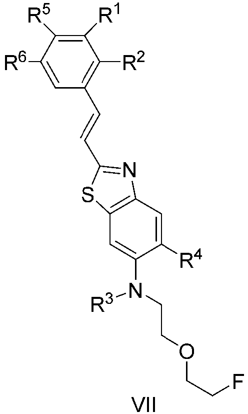
R¹ is F, Cl, Br, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 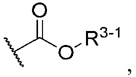 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 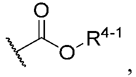 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
R⁶ is -OC₁₋₆ alkyl, or -OC₁₋₆ alkyl substituted by 1, 2, or 3 R⁶⁻¹;
R⁶⁻¹ is independently halogen, -OH, -COOH, -CN, -NO₂, or -NH₂;
the 3- to 6-membered heterocycloalkyl has 1, 2, or 3 heteroatoms selected from the group consisting of N, O, and S.

5. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4, wherein
the sulfur-containing fused ring compound of Formula I-A is shown in the following Formula I: 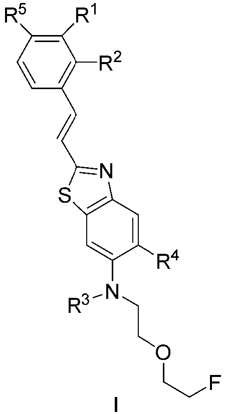
in Formula I,
R¹ is F, Cl, Br, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 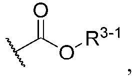 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 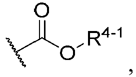 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻³, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
when R⁵ is -OH, then R¹ is F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶; the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula II-A is shown in the following Formula II: 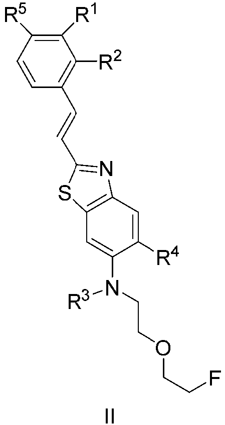
in Formula II,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 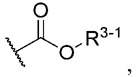 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻³, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 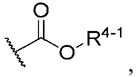 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻³, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula V-A is shown in the following Formula V: 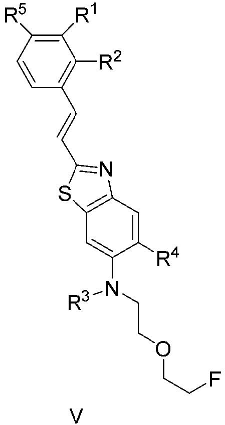
in Formula V,
R¹ is Br, F, Cl, I, -OR¹⁻¹, -NR¹⁻²R¹⁻³, C₁₋₆ alkyl substituted by **1, 2,** or 3 R¹⁻⁴, C₂₋₆ alkenyl substituted **by 1, 2,** or 3 R¹⁻⁵, or C₃₋₆ cycloalkyl substituted by **1, 2,** or 3 R¹⁻⁶;
R¹⁻¹ is H, C₁₋₆ alkyl, or C₁₋₆ alkyl substituted by **1, 2,** or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by **1,** 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₂₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 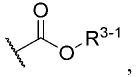 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 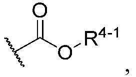 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula III-A is shown in the following Formula III: 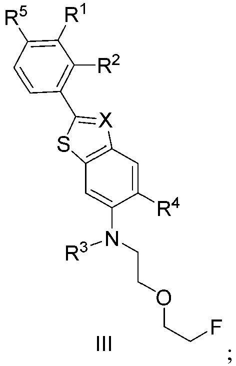
in Formula III,
X is N or CR^{a};
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 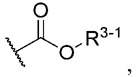 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 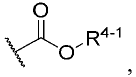 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻³, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R^{a} is H or C₁₋₆ alkyl;
or, R^{a} and R² together form -(NH)ₙ-(CH₂)ₘ-(CH=CH)ₚ-; n, m, and p are independently 0, 1, 2, or 3, and n, m, and p are not simultaneously 0;
R⁵ is -OH or -NR⁵⁻¹R⁵⁻²;
R⁵⁻¹ and R⁵⁻² are independently H or C₁₋₆ alkyl;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula IV-A is shown in the following Formula IV: 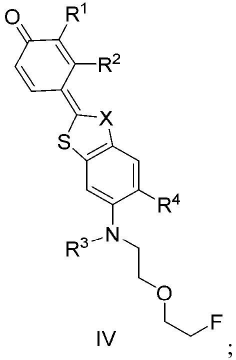
in Formula IV,
X is N or CR^{a};
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 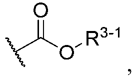 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 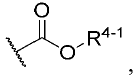 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻⁸, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R^{a} is H or C₁₋₆ alkyl;
or, R^{a} and R² together form -(NH)ₙ-(CH₂)ₘ-(CH=CH)ₚ-; n, m, and p are independently 0, 1, 2, or 3, and n, m, and p are not simultaneously 0;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S;
the sulfur-containing fused ring compound of Formula VI-A is shown in the following Formula VI: 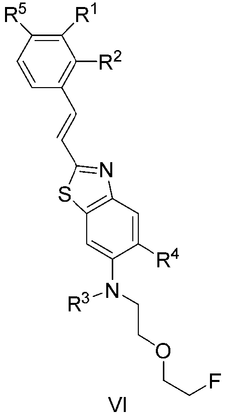
in Formula VI,
R¹ is Br, F, Cl, I, -OR¹⁻¹, -NR¹⁻²R¹⁻³, C₁₋₆ alkyl substituted by 1, 2, or 3 R¹⁻⁴, C₂₋₆ alkenyl substituted by 1, 2, or 3 R¹⁻⁵, or C₃₋₆ cycloalkyl substituted by 1, 2, or 3 R¹⁻⁶;
R¹⁻¹ is H, C₁₋₆ alkyl, or C₁₋₆ alkyl substituted by 1, 2, or 3 R¹⁻¹⁻¹;
R¹⁻² and R¹⁻³ are independently H, C₁₋₆ alkyl, or C₁₋₆ alkyl substituted by 1, 2, or 3 R¹⁻²⁻¹;
R¹⁻⁴, R¹⁻⁵, and R¹⁻⁶ are independently -OH or -NR¹⁻⁴⁻¹R¹⁻⁴⁻²;
R¹⁻¹⁻¹ is independently halogen, -OH, -COOH, -CN, -NO₂, or -NH₂;
R¹⁻²⁻¹ is independently halogen, -OH, -COOH, -CN, -NO₂, or -NH₂;
R¹⁻⁴⁻¹ and R¹⁻⁴⁻² are independently H or C₁₋₆ alkyl;
R² is H, -OH, -OC₁₋₆ alkyl, -NR²⁻⁰R²⁻¹, C₁₋₆ alkyl substituted by 1, 2, or 3 R²⁻², C₂₋₆ alkenyl substituted by 1, 2, or 3 R²⁻³, C₃₋₆ cycloalkyl substituted by 1, 2, or 3 R²⁻⁴, or -O-C₁₋₆ alkyl substituted by 1, 2, or 3 R²⁻⁵;
R²⁻⁰ and R²⁻¹ are independently H, C₁₋₆ alkyl, or C₁₋₆ alkyl substituted by 1, 2, or 3 R²⁻¹⁻¹;
R²⁻², R²⁻³, R²⁻⁴, and R²⁻⁵ are independently halogen, -OH, or -NR²⁻²⁻⁰R²⁻²⁻¹;
R²⁻¹⁻¹ is independently halogen, -OH, -COOH, -CN, -NO₂, or -NH₂;
R²⁻²⁻⁰ and R²⁻²⁻¹ are independently H or C₁₋₆ alkyl;
R³ is H, -OH, C₁₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 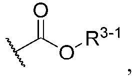 C₁₋₆ alkyl substituted by 1, 2, or 3 R³⁻², C₂₋₆ alkenyl substituted by 1, 2, or 3 R³⁻³, C₂₋₆ alkynyl substituted by 1, 2, or 3 R³⁻⁴, C₃₋₆ cycloalkyl substituted by 1, 2, or 3 R³⁻⁵, 3- to 6-membered heterocycloalkyl substituted by 1, 2, or 3 R³⁻⁶, -O-C₁₋₆ alkyl substituted by 1, 2, or 3 R³⁻⁷, C₆₋₁₀ aryl substituted by 1, 2, or 3 R³⁻⁸, or 5- to 10-membered heteroaryl substituted by 1, 2, or 3 R³⁻⁹;
R³⁻¹ is C₁₋₆ alkyl or C₁₋₆ alkyl substituted by 1, 2, or 3 R³⁻¹⁻¹;
R³⁻², R³⁻³, R³⁻⁴, R³⁻⁵, R³⁻⁶, R³⁻⁷, R³⁻⁸, and R³⁻⁹ are independently halogen, -OH, -COOH, -CN, -NO₂, or -NH₂;
R³⁻¹⁻¹ is independently halogen, -OH, -COOH, -CN, -NO₂, or -NH₂;
R⁴ is H, C₂₋₆ alkyl, C₂₋₆ alkenyl, C₂₋₆ alkynyl, C₃₋₆ cycloalkyl, 3- to 6-membered heterocycloalkyl, -O-C₁₋₆ alkyl, C₆₋₁₀ aryl, 5- to 10-membered heteroaryl, 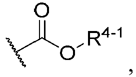 C₁₋₆ alkyl substituted by 1, 2, or 3 R⁴⁻², C₂₋₆ alkenyl substituted by 1, 2, or 3 R⁴⁻³, C₂₋₆ alkynyl substituted by 1, 2, or 3 R⁴⁻⁴, C₃₋₆ cycloalkyl substituted by 1, 2, or 3 R⁴⁻⁵, 3- to 6-membered heterocycloalkyl substituted by 1, 2, or 3 R⁴⁻⁶, -O-C₁₋₆ alkyl substituted by 1, 2, or 3 R⁴⁻⁷, C₆₋₁₀ aryl substituted by 1, 2, or 3 R⁴⁻⁸, or 5- to 10-membered heteroaryl substituted by 1, 2, or 3 R⁴⁻⁹;
R⁴⁻¹ is C₁₋₆ alkyl or C₁₋₆ alkyl substituted by 1, 2, or 3 R⁴⁻¹⁻¹;
R⁴⁻², R⁴⁻³, R⁴⁻⁴, R⁴⁻⁵, R⁴⁻⁶, R⁴⁻⁷, R⁴⁻³, and R⁴⁻⁹ are independently halogen, -OH, -COOH, -CN, -NO₂, or -NH₂;
R⁴⁻¹⁻¹ is independently halogen, -OH, -COOH, -CN, -NO₂, or -NH₂;
R⁵ is -OH or -NR⁵⁻¹R⁵⁻²;
R⁵⁻¹ and R⁵⁻² are independently H or C₁₋₆ alkyl;
the 5- to 10-membered heteroaryl has 1, 2, or 3 heteroatoms selected from the group consisting of N, O, and S;
the 3- to 6-membered heterocycloalkyl has 1, 2, or 3 heteroatoms selected from the group consisting of N, O, and S.

6. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula I-A or Formula I, R¹ is Br, -OR¹⁻¹, or -NR¹⁻²R¹⁻³; preferably -OR¹⁻¹ or - NR¹⁻²R¹⁻³; more preferably -OH, -OCH₃, -NH₂, or -NHCH₃;
Condition (2): in Formula I-A or Formula I, R² is H, -OH, -OC₁₋₆ alkyl, or -NR²⁻⁰R²⁻¹;
Condition (3): in Formula I-A or Formula I, R³ is H, -OH, or C₁₋₆ alkyl;
Condition (4): in Formula I-A or Formula I, R⁴ is C₁₋₆ alkyl;
Condition (5): in Formula I-A or Formula I, R¹⁻¹ is H or C₁₋₆ alkyl;
Condition (6): in Formula I-A or Formula I, R¹⁻² and R¹⁻³ are independently H or C₁₋₆ alkyl;
Condition (7): in Formula I-A or Formula I, R²⁻¹ is H;
Condition (8): in Formula I-A or Formula I, R²⁻⁰ is H or C₁₋₆ alkyl; and
Condition (9): in Formula I-A or Formula I, R⁵ is -OH, -NH₂, or -NHCH₃; preferably -OH.

7. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula II-A or Formula II, R¹ is Br, F, Cl, I, -OR¹⁻¹, or -NR¹⁻²R¹⁻³;
Condition (2): in Formula II-A or Formula II, R² is -OH, -OC₁₋₆ alkyl, or -NR²⁻⁰R²⁻¹; preferably - OH, -OCH₃, -NH₂, or -NHCH₃;
Condition (3): in Formula II-A or Formula II, R³ is H, -OH, or C₁₋₆ alkyl;
Condition (4): in Formula II-A or Formula II, R⁴ is C₁₋₆ alkyl;
Condition (5): in Formula II-A or Formula II, R¹⁻¹ is H or C₁₋₆ alkyl;
Condition (6): in Formula II-A or Formula II, R¹⁻² and R¹⁻³ are independently H or C₁₋₆ alkyl;
Condition (7): in Formula II-A or Formula II, R²⁻¹ is H;
Condition (8): in Formula II-A or Formula II, R²⁻⁰ is H or C₁₋₆ alkyl; and
Condition (9): in Formula II-A or Formula II, R⁵ is -OH, -NH₂, or -NHCH₃; preferably -OH.

8. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula V-A or Formula V, R¹ is Br, F, Cl, I, -OR¹⁻¹, or -NR¹⁻²R¹⁻³;
Condition (2): in Formula V-A or Formula V, R² is H, -OH, -OC₁₋₆ alkyl, or -NR²⁻⁰R²⁻¹;
Condition (3): in Formula V-A or Formula V, R³ is H, -OH, or C₂₋₆ alkyl; preferably H, -OH, or isopropyl; or preferably -OH, ethyl, or isopropyl; most preferably ethyl;
Condition (4): in Formula V-A or Formula V, R⁴ is C₁₋₆ alkyl;
Condition (5): in Formula V-A or Formula V, R¹⁻¹ is H or C₁₋₆ alkyl;
Condition (6): in Formula V-A or Formula V, R¹⁻² and R¹⁻³ are independently H or C₁₋₆ alkyl;
Condition (7): in Formula V-A or Formula V, R²⁻¹ is H;
Condition (8): in Formula V-A or Formula V, R²⁻⁰ is H or C₁₋₆ alkyl; and
Condition (9): in Formula V-A or Formula V, R⁵ is -OH, -NH₂, or -NHCH₃; preferably -OH.

9. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula III-A or Formula III, R¹ is Br, F, Cl, I, -OR¹⁻¹, or -NR¹⁻²R¹⁻³;
Condition (2): in Formula III-A or Formula III, R² is H, -OH, -OC₁₋₆ alkyl, or -NR²⁻⁰R²⁻¹; or, R^{a} and R² together form -(NH)ₙ-(CH₂)ₘ-(CH=CH)ₚ-; n, m, and p are independently 0, 1, 2, or 3, and n, m, and p are not simultaneously 0; preferably, R² is H, or R^{a} and R² together form -NH- or -CH=CH-;
Condition (3): in Formula III-A or Formula III, R³ is H, -OH, or C₁₋₆ alkyl;
Condition (4): in Formula III-A or Formula III, R⁴ is C₁₋₆ alkyl;
Condition (5): in Formula III-A or Formula III, R¹⁻¹ is H or C₁₋₆ alkyl;
Condition (6): in Formula III-A or Formula III, R¹⁻² and R¹⁻³ are independently H or C₁₋₆ alkyl;
Condition (7): in Formula III-A or Formula III, R²⁻¹ is H;
Condition (8): in Formula III-A or Formula III, R²⁻⁰ is H or C₁₋₆ alkyl; and
Condition (9): in Formula III-A or Formula III, R⁵ is -OH, -NH₂, or -NHCH₃; preferably -OH.

10. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula IV-A or Formula IV, R¹ is Br, F, Cl, I, -OR¹⁻¹, or -NR¹⁻²R¹⁻³;
Condition (2): in Formula IV-A or Formula IV, R² is H, -OH, -OC₁₋₆ alkyl, or -NR²⁻⁰R²⁻¹; or, R^{a} and R² together form -(NH)ₙ-(CH₂)ₘ-(CH=CH)ₚ-; n, m, and p are independently 0, 1, 2, or 3, and n, m, and p are not simultaneously 0; preferably, R² is H, or R^{a} and R² together form -NH- or -CH=CH-;
Condition (3): in Formula IV-A or Formula IV, R³ is H, -OH, or C₁₋₆ alkyl;
Condition (4): in Formula IV-A or Formula IV, R⁴ is C₁₋₆ alkyl;
Condition (5): in Formula IV-A or Formula IV, R¹⁻¹ is H or C₁₋₆ alkyl;
Condition (6): in Formula IV-A or Formula IV, R¹⁻² and R¹⁻³ are independently H or C₁₋₆ alkyl;
Condition (7): in Formula IV-A or Formula IV, R²⁻⁰ is C₁₋₆ alkyl; and
Condition (8): in Formula IV-A or Formula IV, R²⁻¹ is H.

11. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula VI-A or Formula VI, R¹ is Br, F, Cl, I, -OR¹⁻¹, or -NR¹⁻²R¹⁻³;
Condition (2): in Formula VI-A or Formula VI, R² is H, -OH, -OC₁₋₆ alkyl, or -NR²⁻⁰R²⁻¹;
Condition (3): in Formula VI-A or Formula VI, R³ is H, -OH, or C₁₋₆ alkyl;
Condition (4): in Formula VI-A or Formula VI, R⁴ is C₂₋₆ alkyl; preferably ethyl or isopropyl;
Condition (5): in Formula VI-A or Formula VI, R¹⁻¹ is H or C₁₋₆ alkyl;
Condition (6): in Formula VI-A or Formula VI, R¹⁻² and R¹⁻³ are independently H or C₁₋₆ alkyl;
Condition (7): in Formula VI-A or Formula VI, R²⁻¹ is H;
Condition (8): in Formula VI-A or Formula VI, R²⁻⁰ is H or C₁₋₆ alkyl; and
Condition (8): in Formula VI-A or Formula VI, R⁵ is -OH, -NH₂, or -NHCH₃; preferably -OH.

12. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula VII, R¹ is Br, F, Cl, I, -OR¹⁻¹, or -NR¹⁻²R¹⁻³;
Condition (2): in Formula VII, R² is H, -OH, -OC₁₋₆ alkyl, or -NR²⁻⁰R²⁻¹;
Condition (3): in Formula VII, R³ is H, -OH, or C₁₋₆ alkyl;
Condition (4): in Formula VII, R⁴ is C₂₋₆ alkyl;
Condition (5): in Formula VII, R¹⁻¹ is H or C₁₋₆ alkyl;
Condition (6): in Formula VII, R¹⁻² and R¹⁻³ are independently H or C₁₋₆ alkyl;
Condition (7): in Formula VII, R²⁻¹ is H;
Condition (8): in Formula VII, R²⁻⁰ is H or C₁₋₆ alkyl;
Condition (9): in Formula VII, R⁵ is -OH, -NH₂, or -NHCH₃; preferably -OH;
Condition (10): in Formula I-A, II-A, III-A, IV-A, V-A, or VI-A, R⁶ is H;
Condition (11): in Formula VII, R⁶ is -OC₁₋₆ alkyl, preferably methoxy.

13. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula I-A or Formula I, R¹ is Br, -OH, -OCH₃, -NH₂, or -NHCH₃;
R² is H, -OH, -OCH₃, -NH₂, or -NHCH₃;
R³ is H, -OH, or C₁₋₆ alkyl;
R⁴ is C₁₋₆ alkyl;
R⁵ is -OH, -NH₂, or -NHCH₃;
Condition (2): in Formula II-A or Formula II, R¹ is Br, F, Cl, I, -OH, -OCH₃, -NH₂, or -NHCH₃;
R² is -OH, -OCH₃, -NH₂, or -NHCH₃;
R³ is H, -OH, or C₁₋₆ alkyl;
R⁴ is C₁₋₆ alkyl;
R⁵ is -OH, -NH₂, or -NHCH₃;
Condition (3): in Formula III-A or Formula III, X is N or CR^{a};
R' is Br, F, Cl, I, -OH, -OCH₃, -NH₂, or -NHCH₃;
R² is H; or, R^{a} and R² together form -NH- or -CH=CH-;
R³ is H, -OH, or C₁₋₆ alkyl;
R⁴ is C₁₋₆ alkyl;
R⁵ is -OH, -NH₂, or -NHCH₃;
Condition (4): in Formula IV-A or Formula IV, X is N or CR^{a};
R' is Br, F, Cl, I, -OH, -OCH₃, -NH₂, or -NHCH₃;
R² is H; or, R^{a} and R² together form -NH- or -(CH=CH)-;
R³ is H, -OH, or C₁₋₆ alkyl;
R⁴ is C₁₋₆ alkyl;
Condition (5): in Formula V-A or Formula V, R¹ is Br, F, Cl, I, -OH, -OCH₃, -NH₂, or -NHCH₃;
R² is H, -OH, -OCH₃, -NH₂, or -NHCH₃;
R³ is H, -OH, or isopropyl; or R³ is -OH, ethyl, or isopropyl;
R⁴ is C₁₋₆ alkyl;
R⁵ is -OH, -NH₂, or -NHCH₃; and
Condition (6): in Formula VI-A and Formula VI, R¹ is Br, F, Cl, I, -OH, -OCH₃, -NH₂, or -NHCH₃;
R² is H, -OH, -OCH₃, -NH₂, or -NHCH₃;
R³ is H, -OH, or C₁₋₆ alkyl;
R⁴ is C₂₋₆ alkyl;
R⁵ is -OH, -NH₂, or -NHCH₃;
preferably, the sulfur-containing fused ring compound of Formula I-A, II-A, III-A, IV-A, V-A, VIA, or VII, the pharmaceutically acceptable salt thereof, the tautomer thereof, or the radioisotope-labeled derivative thereof satisfies one or more of the following conditions:
Condition (1): in Formula I-A or Formula I, R¹ is Br, -OH, -OCH₃, -NH₂, or -NHCH₃;
R² is H;
R³ is C₁₋₆ alkyl;
R⁴ is C₁₋₆ alkyl;
R⁵ is -OH, -NH₂, or -NHCH₃;
Condition (2): in Formula II-A or Formula II, R¹ is Br;
R² is -OH, -OCH₃, -NH₂, or -NHCH₃;
R³ is C₁₋₆ alkyl;
R⁴ is C₁₋₆ alkyl;
R⁵ is -OH;
Condition (3): in Formula III-A or Formula III, X is N or CR^{a};
R¹ is Br;
R² is H; or, R^{a} and R² together form -NH- or -CH=CH-;
R³ is C₁₋₆ alkyl;
R⁴ is C₁₋₆ alkyl;
R⁵ is -OH;
Condition (4): in Formula IV-A or Formula IV, X is N or CR^{a};
R¹ is Br;
R² is H; or, R^{a} and R² together form -NH- or -(CH=CH)-;
R³ is C₁₋₆ alkyl;
R⁴ is C₁₋₆ alkyl;
Condition (5): in Formula V-A or Formula V, R¹ is Br;
R² is H;
R³ is H, -OH, or isopropyl; or R³ is -OH, ethyl, or isopropyl;
R⁴ is C₁₋₆ alkyl;
R⁵ is -OH; and
Condition (6): in Formula VI-A or Formula VI, R¹ is Br;
R² is H;
R³ is H, -OH, or C₁₋₆ alkyl;
R⁴ is ethyl or isopropyl;
R⁵ is -OH.

14. The sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula VII, R¹ is Br, F, Cl, I, -OH, -OCH₃, -NH₂, or -NHCH₃;
R² is H, -OH, -OCH₃, -NH₂, or -NHCH₃;
R³ is H, -OH, or C₁₋₆ alkyl;
R⁴ is C₁₋₆ alkyl;
R⁵ is -OH, -NH₂, or -NHCH₃;
R⁶ is methoxy;
Condition (2): R¹ is Br, -OH, -OCH₃, -NH₂, or -NHCH₃;
R² is H;
R³ is C₁₋₆ alkyl;
R⁴ is C₁₋₆ alkyl;
R⁵ is -OH, -NH₂, or -NHCH₃;
R⁶ is methoxy.

15. The sulfur-containing fused ring compound of Formula A, the pharmaceutically acceptable salt thereof, the tautomer thereof, or the radioisotope-labeled derivative thereof according to claim 1, or the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula A, the pharmaceutically acceptable salt thereof, the tautomer thereof, or the radioisotope-labeled derivative thereof, or the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof satisfies one or more of the following conditions:
Condition (1): in Formula A, I-A, II-A, III-A, IV-A, V-A, VI-A, VII, I, II, III, IV, V, or VI, the C₁₋₆ alkyl is independently methyl, ethyl, *n-*propyl, isopropyl, *n*-butyl, isobutyl, or *tert*-butyl; for example, methyl, ethyl, or isopropyl; and for another example, methyl;
Condition (2): in Formula A, I-A, II-A, III-A, IV-A, V-A, VI-A, VII, I, II, III, IV, V, or VI, the C₂₋₆ alkenyl is independently vinyl, propenyl, n-propenyl, isopropenyl, n-butenyl, isobutenyl, *sec*-butenyl, or *tert*-butenyl;
Condition (3): in Formula A, I-A, II-A, III-A, IV-A, V-A, VI-A, VII, I, II, III, IV, V, or VI, the C₂₋₆ alkynyl is independently ethynyl, n-propynyl, isopropynyl, *n*-butynyl, isobutynyl, *sec*-butynyl, or *tert-*butynyl;
Condition (4): in Formula A, I-A, II-A, III-A, IV-A, V-A, VI-A, VII, I, II, III, IV, V, or VI, the C₃₋₆ cycloalkyl is independently cyclopropyl, cyclobutyl, cyclopropyl, cyclopentyl, or cyclohexyl;
Condition (5): in Formula A, I-A, II-A, III-A, IV-A, V-A, VI-A, VII, I, II, III, IV, V, or VI, the -O-C₁₋₆ alkyl is independently methoxy, ethoxy, *n*-propoxy, isopropoxy, n-butoxy, isobutoxy, or *tert*-butoxy; for example, methoxy;
Condition (6): in Formula A, I-A, II-A, III-A, IV-A, V-A, VI-A, VII, I, II, III, IV, V, or VI, the halogen is independently F, Cl, Br, or I;
Condition (7): in Formula A, I-A, II-A, III-A, IV-A, V-A, VI-A, VIII, II, III, IV, V, or VI, the C₆₋₁₀ aryl is independently phenyl or naphthyl, for example, phenyl; and
Condition (8): in Formula A, V-A, VI-A, V, or VI, the C₂₋₆ alkyl is independently ethyl, n-propyl, isopropyl, n-butyl, isobutyl, or tert-butyl; for example, ethyl or isopropyl.

16. The sulfur-containing fused ring compound of Formula A, the pharmaceutically acceptable salt thereof, the tautomer thereof, or the radioisotope-labeled derivative thereof according to claim 1, or the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to claim 4 or 5, wherein the sulfur-containing fused ring compound of Formula I-A is any one selected from the group consisting of the following compounds: 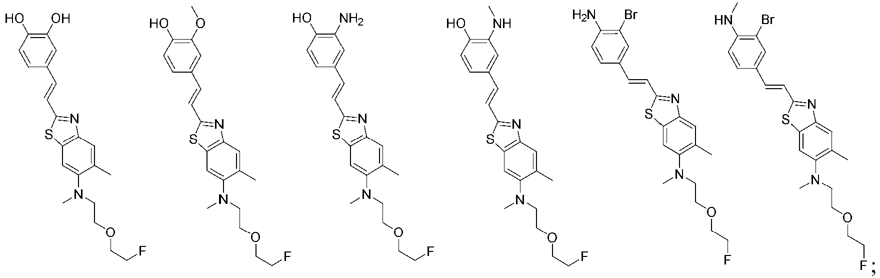
the sulfur-containing fused ring compound of Formula II-A is any one selected from the group consisting of the following compounds: 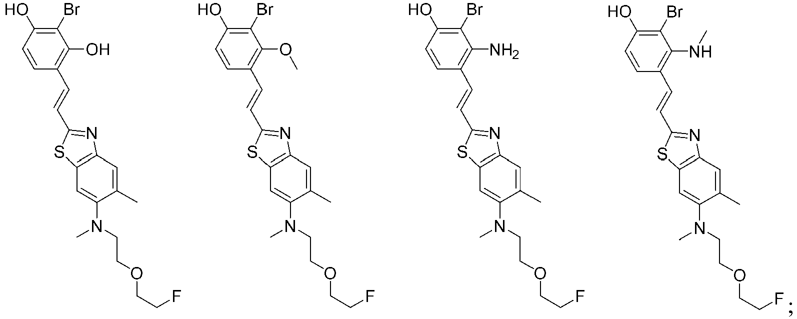
the sulfur-containing fused ring compound of Formula III-A is any one selected from the group consisting of the following compounds: 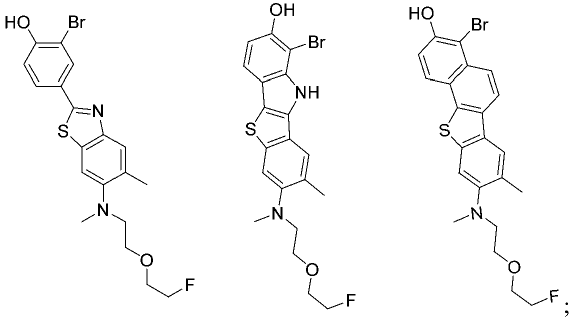
the sulfur-containing fused ring compound of Formula IV-A is: 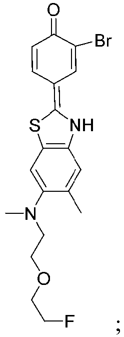
the sulfur-containing fused ring compound of Formula V-A is any one selected from the group consisting of the following compounds: 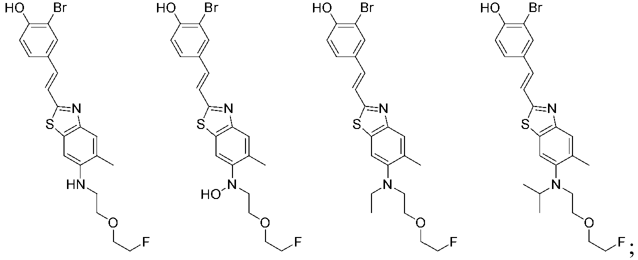
the sulfur-containing fused ring compound of Formula VI-A is any one selected from the group consisting of the following compounds: 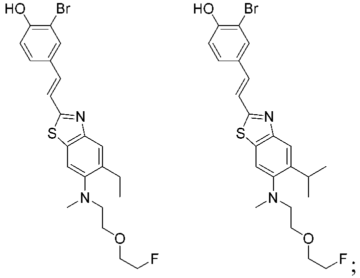
the sulfur-containing fused ring compound of Formula VII is:

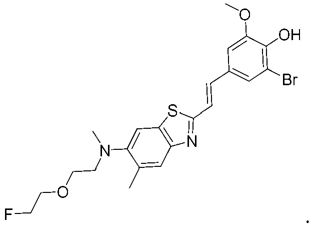

17. The sulfur-containing fused ring compound, the pharmaceutically acceptable salt thereof, the tautomer thereof, or the radioisotope-labeled derivative thereof according to any one of claims 1 to 3, or the sulfur-containing fused ring compound, the pharmaceutically acceptable salt thereof, the tautomer thereof, or the radioisotope-labeled derivative thereof according to any one of claims 4 to 16, wherein the radioisotope-labeled derivative is a compound obtained by replacing the F, C, I, or N atom in the sulfur-containing fused ring compound, the pharmaceutically acceptable salt thereof, or the tautomer thereof with ¹⁸F, ¹¹C, ¹²⁴I, ¹²³I, or ¹³N; preferably ¹⁸F.

18. A pharmaceutical composition, comprising the sulfur-containing fused ring compound of Formula A, the pharmaceutically acceptable salt thereof, the tautomer thereof, or the radioisotope-labeled derivative thereof according to any one of claims 1 to 3, or the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to any one of claims 4 to 16, and a pharmaceutically acceptable excipient.

19. A kit, comprising the sulfur-containing fused ring compound of Formula A, the pharmaceutically acceptable salt thereof, the tautomer thereof, or the radioisotope-labeled derivative thereof according to any one of claims 1 to 3, or the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to any one of claims 4 to 16, and instructions for use.

20. Use of the sulfur-containing fused ring compound of Formula A, the pharmaceutically acceptable salt thereof, the tautomer thereof, or the radioisotope-labeled derivative thereof according to any one of claims 1 to 3, or the sulfur-containing fused ring compound of Formula I-A, II-A, III-A, IV-A, V-A, VI-A, or VII, the pharmaceutically acceptable salt thereof, the tautomer thereof, or the radioisotope-labeled derivative thereof according to any one of claims 4 to 16 in the manufacture of a PET-CT tracer; the PET-CT tracer can be used for imaging α-synuclein aggregates in Lewy bodies and Lewy neurites; for example, the PET-CT tracer is used for imaging Parkinson's disease, dementia with Lewy bodies, or multiple system atrophy; for example, the PET-CT tracer can be used for diagnostic exclusion of progressive supranuclear palsy or corticobasal degeneration.
